# EUROPEAN PATENT APPLICATION

(11) **EP 4 238 969 A1**
(43) Date of publication of application: **06.09.2023**
(21) Application number: 21886619.2
(22) Date of filing: 13.10.2021
(51) Int. Cl.: C07D 405/14, C07D 409/14, H01L 51/00, H01L 51/50

(54) **HETEROCYCLIC COMPOUND, ORGANIC LIGHT-EMITTING DEVICE COMPRISING SAME, COMPOSITION FOR ORGANIC LAYER OF ORGANIC LIGHT-EMITTING DEVICE, AND METHOD FOR MANUFACTURING ORGANIC LIGHT-EMITTING DEVICE**

(30) Priority: 30.10.2020 KR 20200143049
(71) Applicant: LT Materials Co., Ltd., Yongin-si, Gyeonggi-do 17118 (KR)
(72) Inventor: SEO, Young Beom, Yongin-si, Gyeonggi-do 17118 (KR); NO, Young Seok, Yongin-si, Gyeonggi-do 17118 (KR); PARK, Geon Yu, Yongin-si, Gyeonggi-do 17118 (KR); KIM, Dong Jun, Yongin-si, Gyeonggi-do 17118 (KR)
(74) Representative: Goddar, Heinz J.
(86) International application number: PCT/KR2021/014110
(87) International publication number: WO 2022/092625

(57) **Abstract**

The present specification relates to a heterocyclic compound represented by Formula 1, an organic light emitting device comprising the same, a composition for organic layer of the organic light emitting device, and a method of manufacturing the organic light emitting device.

## Description

### [Technical Field]

The present application claims the benefit of priority based on Korean Patent Application No. 10-2020-0143049 filed on October 30, 2020, the entire contents of which are incorporated herein by reference.

The present invention relates to a heterocyclic compound, an organic light emitting device comprising the same, a composition for organic layer of the organic light emitting device, and a method for manufacturing the organic light emitting device.

### [Background Art]

An organic light emitting device is a kind of self-emitting display device, which has the advantage of having a wide viewing angle, excellent contrast, and also fast response speed.

The organic light emitting device has a structure in which an organic thin film is disposed between two electrodes. When a voltage is applied to the organic light emitting device having such structure, electrons and holes injected from the two electrodes combine in the organic thin film to form a pair, and then emit light while disappearing. The organic thin film may be composed of a single layer or multiple layers, as required.

The material of the organic thin film may have a light emitting function, if necessary. For example, as a material for the organic thin film, a compound capable of forming a light emitting layer by itself alone may be used, or a compound capable of serving as a host or dopant of a host-dopant-based light emitting layer may also be used. In addition, as a material for the organic thin film, a compound capable of performing the roles of hole injection, hole transport, electron blocking, hole blocking, electron transport, electron injection, and the like may be used.

In order to improve the performance, lifetime or efficiency of an organic light emitting device, there is a continuous demand for the development of materials for the organic thin film.

### [Prior Art Document]

### [Patent Document]

(Patent Document 1) US Patent No. 4,356,429

### [Disclosure]

### [Technical Problem]

It is an object of the present invention to provide a heterocyclic compound, an organic light emitting device comprising the same, a manufacturing method thereof, and a composition for organic layer.

### [Technical Solution]

The present invention provides a heterocyclic compound represented by Formula 1 below: 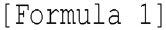 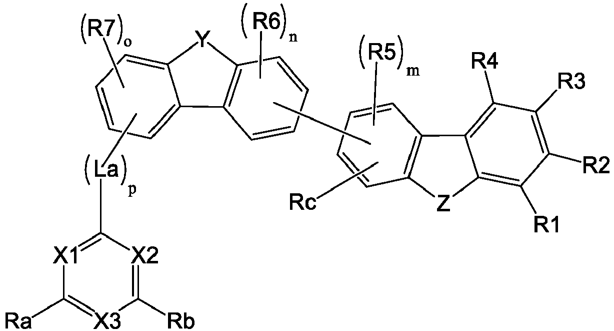 wherein,
X1 to X3 are each independently N or CR10, at least two of X1 to X3 are N,
R10 is hydrogen, deuterium, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group,
Ra and Rb are the same as or different from each other, and are each independently a substituted or unsubstituted C6 to C60 aryl group, or a substituted or unsubstituted C2 to C60 heteroaryl group,
Rc is a substituted or unsubstituted C6 to C60 aryl group, or a substituted or unsubstituted C2 to C60 heteroaryl group,
La is a single bond, a substituted or unsubstituted C6 to C60 arylene group, or a substituted or unsubstituted C2 to C60 heteroarylene group,
Y and Z are the same as or different from each other and are each independently O, S or CR11R12,
R11 and R12 are hydrogen, deuterium, a cyano group, a substituted or unsubstituted C1 to C60 alkyl group, or a substituted or unsubstituted C6 to C60 aryl group,
R1 to R7 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, or form a substituted or unsubstituted C6 to C60 aromatic hydrocarbon ring or a substituted or unsubstituted C2 to C60 heterocyclic ring by combining two or more groups adjacent to each other, R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group,
m is an integer from 0 to 2, n is an integer from 0 to 3, o is an integer from 0 to 3, and p is an integer from 0 to 5.

In addition, the present invention provides an organic light emitting device comprising a first electrode; a second electrode provided to face the first electrode; and one or more organic layers provided between the first electrode and the second electrode, wherein at least one of the one or more organic layers comprises a heterocyclic compound represented by Formula 1 above.

In addition, the present invention provides an organic light emitting device wherein the one or more organic layers further comprises a heterocyclic compound represented by Formula 2 below: 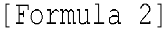 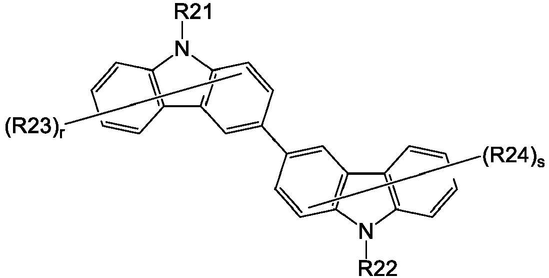 wherein,
R21 and R22 are the same as or different from each other and are each independently a substituted or unsubstituted C6 to C60 aryl group or a substituted or unsubstituted C2 to C60 heteroaryl group,
R23 and R24 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR201R202R203, - P(=O)R201R202; and an amine group substituted or unsubstituted by a substituted or unsubstituted C1 to C60 alkyl group, a substituted or unsubstituted C6 to C60 aryl group or a substituted or unsubstituted C2 to C60 heteroaryl group, or form a substituted or unsubstituted C6 to C60 aromatic hydrocarbon ring or a substituted or unsubstituted C2 to C60 heterocyclic ring by combining two or more groups adjacent to each other,
R201, R202, and R203 are the same as or different from each other and are each independently hydrogen, deuterium, a cyano group, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group,
r and s are integers from 0 to 7.

In addition, the present invention provides a composition for organic layer of an organic light emitting device comprising the heterocyclic compound represented by Formula 1 above and the heterocyclic compound represented by Formula 2 above.

In addition, the present invention provides a method for manufacturing an organic light emitting device comprising the steps of preparing a substrate; forming a first electrode on the substrate; forming one or more organic layers on the first electrode; and forming a second electrode on the one or more organic layers, wherein the step of forming the one or more organic layers comprises a step of forming the one or more organic layers using the composition for organic layer of the organic light emitting device.

### [Advantageous Effects]

The compounds described herein can be used as a material for the organic layer of an organic light emitting device. The compound may serve as a material for a hole injection layer, a material for a hole transport layer, a material for a light emitting layer, a material for an electron transport layer, a material for an electron injection layer, and the like in an organic light emitting device. In particular, the compound can be used as a material for a light emitting layer of an organic light emitting device.

Specifically, the compound may be used alone as a light emitting material, and may be used as a host material or a dopant material of the light emitting layer. When the compound represented by Formula 1 is used for the organic layer, it is possible to lower the operating voltage of the device, improve the light efficiency, and improve the lifetime characteristics of the device by thermal stability of the compound.

In addition, as the heterocyclic compound represented by Formula 1 has a heterocyclic substituent containing at least one N on one benzene ring of the heterocyclic ring (dibenzofuran, dibenzothiophene or carbazole) including Y, and a heterocyclic substituent substituted by Rc on the other benzene ring, an organic light emitting device comprising the same has excellent characteristics in lifetime and efficiency.

In particular, the heterocyclic compound represented by Formula 1 may be used together with the heterocyclic compound represented by Formula 2 as a material of a light emitting layer of an organic light emitting device. In this case, it is possible to lower the operating voltage of the device, improve the light efficiency, and improve the lifetime characteristics of the device by thermal stability of the compound.

### [Description of Drawing]

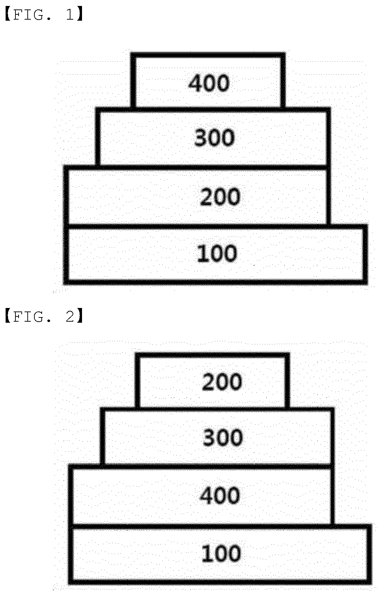

FIGs. 1 to 3 are views schematically showing a stacked structure of an organic light emitting device according to an embodiment of the present invention, respectively.

### [Best Mode]

Hereinafter, the present application will be described in detail.

In the present specification, the term "substituted" means that a hydrogen atom bonded to a carbon atom of a compound is replaced with another substituent, the position to be substituted is not limited as long as it is a position where a hydrogen atom may be substituted, that is, it is a position that can be substituted by a substituent, and when substituted by two or more substituents, two or more substituents may be the same or different from each other.

In the present specification, the term "substituted or unsubstituted" means that it is substituted or unsubstituted by one or more substituents selected from the group consisting of C1 to C60 straight or branched chain alkyl; C2 to C60 straight or branched alkenyl; C2 to C60 straight or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; - P(=O)RR'; C1 to C20 alkylamine; C6 to C60 monocyclic or polycyclic arylamine; and C2 to C60 monocyclic or polycyclic heteroarylamine, or means that it is substituted or unsubstituted with a substituent formed by connecting two or more substituents selected from the above-exemplified substituents.

In the present specification, the halogen may be fluorine, chlorine, bromine or iodine.

In the present specification, the alkyl group comprises a straight or branched chain having 1 to 60 carbon atoms, and may be further substituted by other substituents. The number of carbon atoms in the alkyl group may be 1 to 60, specifically 1 to 40, more specifically, 1 to 20. Specific examples of the alkyl group may be, but is not limited to, a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethylpropyl group, an isohexyl group, a 2-methylpentyl group, a 4-methylhexyl group, a 5-methylhexyl group and the like.

In the present specification, the alkenyl group comprises a straight or branched chain having 2 to 60 carbon atoms, and may be further substituted by other substituents. The carbon number of the alkenyl group may be 2 to 60, specifically 2 to 40, more specifically 2 to 20. Specific examples of the alkenyl group may be, but is not limited to, a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like.

In the present specification, the alkynyl group comprises a straight or branched chain having 2 to 60 carbon atoms, and may be further substituted by other substituents. The carbon number of the alkynyl group may be 2 to 60, specifically 2 to 40, more specifically 2 to 20.

In the present specification, the alkoxy group may be a straight-chain, a branched-chain or a cyclic chain. Although the number of carbon atoms in the alkoxy group is not particularly limited, it is preferable that the number of carbon atoms is 1-20. Specific examples of the alkoxy group may be, but is not limited to, methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like.

In the present specification, the cycloalkyl group comprises a monocyclic or polycyclic ring having 3 to 60 carbon atoms, and may be further substituted by other substituents. In this case, the polycyclic ring refers to a group formed by directly connecting or condensing a cycloalkyl group with another cyclic group. In this case, the other cyclic group may be a cycloalkyl group, but may be a different type of cyclic group, for example, a heterocycloalkyl group, an aryl group, a heteroaryl group, or the like. The number of carbon atoms in the cycloalkyl group may be 3 to 60, specifically 3 to 40, more specifically 5 to 20. Specific examples of the cycloalkyl group may be, but is not limited to, a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like.

In the present specification, the heterocycloalkyl group comprises a monocyclic or polycyclic ring containing O, S, Se, N or Si as a hetero atom and having 2 to 60 carbon atoms, and may be further substituted by other substituents. In this case, the polycyclic ring refers to a group formed by directly connecting or condensing a heterocycloalkyl group with another cyclic group. In this case, the other cyclic group may be a heterocycloalkyl group, but may be a different type of cyclic group, for example, a cycloalkyl group, an aryl group, a heteroaryl group, or the like. The number of carbon atoms in the heterocycloalkyl group may be 2 to 60, specifically 2 to 40, more specifically 3 to 20.

In the present specification, the aryl group comprises a monocyclic or polycyclic ring having 6 to 60 carbon atoms, and may be further substituted by other substituents. In this case, the polycyclic ring means a group formed by directly connecting or condensing an aryl group with another cyclic group. In this case, the other cyclic group may be an aryl group, but may be a different type of cyclic group, for example, a cycloalkyl group, a heterocycloalkyl group, a heteroaryl group, or the like. The aryl group comprises a spiro group. The number of carbon atoms in the aryl group may be 6 to 60, specifically 6 to 40, more specifically 6 to 25. Specific examples of the aryl group may be, but is not limited to, a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenylenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, condensed ring groups thereof and the like.

In the present specification, the phosphine oxide group is represented by -P (=O) R₁₀₁R₁₀₂, wherein R₁₀₁ and R₁₀₂ are the same as or different from each other, and may each independently be a substituent consisting of at least one of hydrogen; deuterium; a halogen group; an alkyl group; an alkenyl group; an alkoxy group; a cycloalkyl group; an aryl group; and a heterocyclic group. Specifically, it may be substituted with an aryl group, wherein the aryl group may be as exemplified above. For example, the phosphine oxide group is, but is not limited to, a diphenyl phosphine oxide group, dinaphthyl phosphine oxide, or the like.

In the present specification, the silyl group contains Si and is a substituent formed by directly connecting the Si atoms as a radical, and is represented by -SiR₁₀₄R₁₀₅R₁₀₆, wherein R₁₀₄ to R₁₀₆ are the same as or different from each other, and may each independently be a substituent consisting of at least one of hydrogen; deuterium; a halogen group; an alkyl group; an alkenyl group; an alkoxy group; a cycloalkyl group; an aryl group; and a heterocyclic group. Specific examples of the silyl group may be, but is not limited to, a trimethylsilyl group, a triethylsilyl group, a t-butyldimethylsilyl group, vinyldimethylsilyl group, a propyldimethylsilyl group, a triphenylsilyl group, a diphenylsilyl group, a phenylsilyl group and the like.

In the present specification, the fluorenyl group may be substituted, and adjacent substituents may be combined with each other to form a ring.

If the fluorenyl group is substituted, the fluorenyl group may be, but is not limited to,

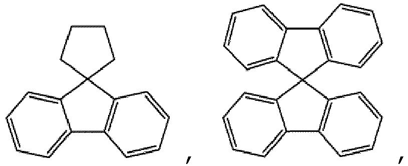

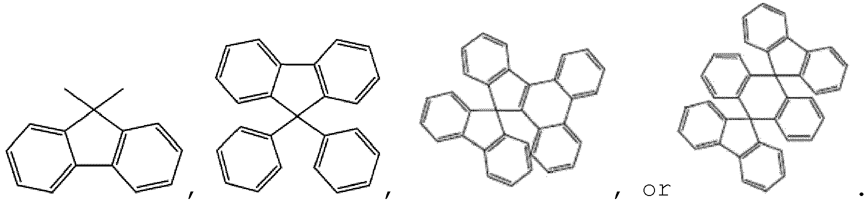

In the present specification, the heteroaryl group comprises a monocyclic or polycyclic ring containing S, O, Se, N or Si as a hetero atom and having 2 to 60 carbon atoms, and may be further substituted by other substituents. In this case, the polycyclic ring refers to a group formed by directly connecting or condensing a heteroaryl group with another cyclic group. In this case, the other cyclic group may be a heteroaryl group, but may be a different type of cyclic group, for example, a cycloalkyl group, a heterocycloalkyl group, an aryl group, or the like. The number of carbon atoms in the heteroaryl group may be 2 to 60, specifically 2 to 40, more specifically 3 to 25. Specific examples of the heteroaryl group may be, but is not limited to, a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, tetrazinyl group, a quinolyl group, an isoquinolyl group, a quinazolinyl group, an isoquinazolinyl group, a quinazolylyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilol group, spirobi(dibenzosilol), a dihydrophenazinyl group, a phenoxazinyl group, a phenanthridyl group, an imidazopyridinyl group, a thienyl group, an indolo[2,3-a] carbazolyl group, an indolo[2,3-b] carbazolyl group, an indolinyl group, a 10,11-dihydro-dibenzo[b,f]azepine group, a 9,10-dihydroacridinyl group, a phenanthrazinyl group, a phenothiazinyl group, phthalazinyl group, a naphthylidinyl group, a phenanthrolinyl group, a benzo[c] [1,2,5]thiadiazolyl group, 5,10-dihydrodibenzo[b,e] [1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms of the amine group is not particularly limited, but is preferably 1 to 30. Specific examples of the amine group may be, but is not limited to, a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like.

In the present specification, the arylene group means that the aryl group has two bonding positions, that is, a divalent group. These are the same as those described for the aryl group described above, except that each of them is a divalent group. In addition, the heteroaryl group means that the heteroaryl group has two bonding positions, that is, a divalent group. These are the same as those described for the heteroaryl group described above, except that each of them is a divalent group.

In the present specification, an "adjacent" group may mean a substituent substituted at the atom directly linked to the atom substituted by that substituent, a substituent that is sterically closest to that substituent, or another substituent substituted at the atom substituted by that substituent. For example, two substituents substituted at an ortho position in a benzene ring and two substituents substituted at the same carbon in an aliphatic ring may be interpreted as "adjacent" groups to each other.

In the present invention, "when a substituent is not indicated in the structure of the formula or the compound" means that a hydrogen atom is bonded to a carbon atom. However, since deuterium (²H) and tritium (³H) are isotopes of hydrogen, some hydrogen atoms may be deuterium or tritium.

In one embodiment of the present invention, "when a substituent is not indicated in the structure of the formula or the compound" may mean that all positions that may be substituted by the substituent are hydrogen, deuterium, or tritium. That is, in the case of deuterium and tritium, some hydrogen atoms as isotopes of hydrogen may be isotopes of deuterium and tritium. In this case, the content of deuterium or tritium may be 0% to 100%.

In one embodiment of the present invention, in the case of "when a substituent is not indicated in the structure of the formula or the compound," if deuterium and tritium are not explicitly excluded, except that "the content of deuterium is 0%", "the content of tritium is 0%", "the content of hydrogen is 100%", "all substituents are hydrogen" etc., thus hydrogen, deuterium, and tritium may be used in admixture in the compound.

In one embodiment of the present invention, deuterium is one of the isotopes of hydrogen, and is an element having as an atomic nucleus a deuteron consisting of one proton and one neutron, and can be expressed as hydrogen-2, and the element symbol can also be written as D or ²H. Similarly, the element symbol for tritium can also be written as T or ³H.

In one embodiment of the present invention, an isotope means an atom with the same atomic number (Z) but a different mass number (A), and can also be interpreted as an element that has the same number of protons but the different number of neutrons.

In one embodiment of the present invention, the meaning of content T% of a specific substituent may be defined as T2/T1×100 = T% wherein T1 is defined as the total number of substituents that the basic compound can have, and T2 is defined as the number of specific substituents substituted among them.

That is, in one example, the 20% content of deuterium in the phenyl group represented by 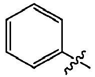 may mean that the total number of substituents that the phenyl group can have is 5 (T1 in the formula), and the number of deuterium among them is 1 (T2 in the formula). That is, in the phenyl group, that the content of deuterium is 20% may be represented by Structural Formulas below:

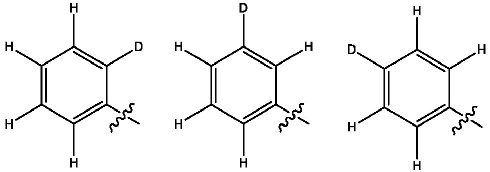

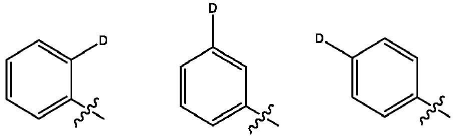

Also, in one embodiment of the present invention, in the case of "a phenyl group having a deuterium content of 0%", it means a phenyl group that does not contain a deuterium atom, i.e., has 5 hydrogen atoms.

In the present invention, C6 to C60 aromatic hydrocarbon ring means a compound containing an aromatic ring consisting of C6 to C60 carbons and hydrogen, and for example, may be, but is not limited to, benzene, biphenyl, triphenyl, triphenylene, naphthalene, anthracene, phenalene, phenanthrene, fluorene, pyrene, chrysene, perylene, azulene and the like, and comprises all of the aromatic hydrocarbon ring compounds known in the art as satisfying the carbon number described above.

The present invention provides a heterocyclic compound represented by the following Formula 1: 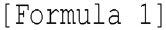 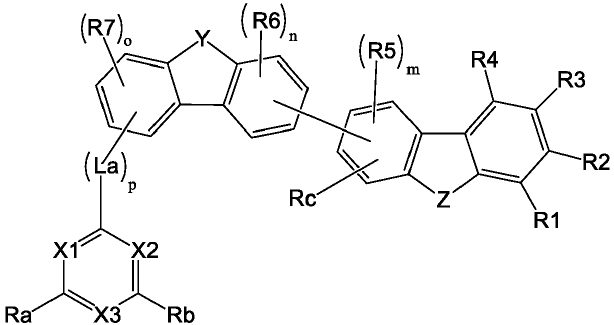 wherein,
X1 to X3 are each independently N or CR10, and at least two of X1 to X3 are N,
R10 is hydrogen, deuterium, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group,
Ra and Rb are the same as or different from each other, and are each independently a substituted or unsubstituted C6 to C60 aryl group, or a substituted or unsubstituted C2 to C60 heteroaryl group,
Rc is a substituted or unsubstituted C6 to C60 aryl group, or a substituted or unsubstituted C2 to C60 heteroaryl group,
La is a single bond, a substituted or unsubstituted C6 to C60 arylene group, or a substituted or unsubstituted C2 to C60 heteroarylene group,
Y and Z are the same as or different from each other and are each independently O, S or CR11R12,
R11 and R12 are hydrogen, deuterium, a cyano group, a substituted or unsubstituted C1 to C60 alkyl group, or a substituted or unsubstituted C6 to C60 aryl group,
R1 to R7 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, or form a substituted or unsubstituted C6 to C60 aromatic hydrocarbon ring or a substituted or unsubstituted C2 to C60 heterocyclic ring by combining two or more groups adjacent to each other, and R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group,
m is an integer from 0 to 2, n is an integer from 0 to 3, o is an integer from 0 to 3, p is an integer from 0 to 5.

In one embodiment of the present invention, X1 and X2, X1 and X3, or X2 and X3 may be N.

In another embodiment of the present invention, all of X1 to X3 may be N.

In one embodiment of the present invention, Ra and Rb are the same as or different from each other, and are each independently a substituted or unsubstituted C6 to C12 aryl group, or a substituted or unsubstituted C2 to C12 heteroaryl group.

In another embodiment of the present invention, Ra and Rb are the same as or different from each other, and may each independently be a substituted or unsubstituted phenyl group, or a substituted or unsubstituted C12 heteroaryl group.

In another embodiment of the present invention, Ra and Rb are the same as or different from each other, and may each independently be a substituted or unsubstituted phenyl group, or substituted or unsubstituted dibenzofuran or dibenzothiophene.

In another embodiment of the present invention, Ra and Rb are the same as or different from each other, and may each independently be a phenyl group substituted with a phenyl group, an unsubstituted phenyl group, or unsubstituted dibenzofuran or dibenzothiophene.

In another embodiment of the present invention, the 'substitution' of Ra and Rb may each independently consist of one or more substituents selected from the group consisting of C1 to C10 alkyl; C2 to C10 alkenyl; C2 to C10 alkynyl; C3 to C15 cycloalkyl; C2 to C20 heterocycloalkyl; C6 to C30 aryl; C2 to C30 heteroaryl; C1 to C10 alkylamine; C6 to C30 arylamine; and C2 to C30 heteroarylamine group.

In another embodiment of the present invention, the 'substitution' of Ra and Rb may each independently consist of one or more substituents selected from the group consisting of C1 to C10 alkyl; C6 to C30 aryl; C2 to C30 heteroaryl group.

In another embodiment of the present invention, the 'substitution' of Ra and Rb may each independently consist of one or more substituents selected from the group consisting of C1 to C5 alkyl; C6 to C20 aryl; and C2 to C20 heteroaryl group.

In another embodiment of the present invention, the 'substitution' of Ra and Rb may each independently consist of one or more substituents selected from the group consisting of methyl, ethyl, straight or branched propyl, straight or branched butyl, straight or branched pentyl, phenyl, naphthalenyl, pyridinyl, anthracenyl, carbazole, dibenzothiophene, dibenzofuran, and phenanthrenyl groups.

In another embodiment of the present invention, the 'substitution' of Ra and Rb may each independently consist of methyl, ethyl, straight or branched chain propyl, straight or branched chain butyl, and straight or branched chain pentyl group.

In one embodiment of the present invention, Rc may be a substituted or unsubstituted C6 to C12 aryl group, or a substituted or unsubstituted C2 to C12 heteroaryl group.

In another embodiment of the present invention, Rc may be a substituted or unsubstituted C6 to C12 aryl group, or a substituted or unsubstituted C2 to C12 heteroaryl group.

In another embodiment of the present invention, Rc may be a substituted or unsubstituted C6 to C12 aryl group.

In another embodiment of the present invention, Rc may be a substituted or unsubstituted phenyl group.

In another embodiment of the present invention, Rc may be a phenyl group substituted with a phenyl group or an unsubstituted phenyl group.

In another embodiment of the present invention, the 'substitution' of Rc may each independently consist of one or more substituents selected from the group consisting of C1 to C10 alkyl; C2 to C10 alkenyl; C2 to C10 alkynyl; C3 to C15 cycloalkyl; C2 to C20 heterocycloalkyl; C6 to C30 aryl; C2 to C30 heteroaryl; C1 to C10 alkylamine; C6 to C30 arylamine; and C2 to C30 heteroarylamine groups.

In another embodiment of the present invention, the 'substitution' of Rc may each independently consist of one or more substituents selected from the group consisting of C1 to C10 alkyl; C6 to C30 aryl; C2 to C30 heteroaryl groups.

In another embodiment of the present invention, the 'substitution' of Rc may each independently consist of one or more substituents selected from the group consisting of C1 to C5 alkyl; C6 to C20 aryl; and C2 to C20 heteroaryl groups.

In another embodiment of the present invention, the 'substitution' of Rc may each independently consist of one or more substituents selected from the group consisting of methyl, ethyl, straight or branched chain propyl, straight or branched chain butyl, straight or branched chain pentyl, phenyl, naphthalenyl, pyridinyl, anthracenyl, carbazole, dibenzothiophene, dibenzofuran, and phenanthrenyl groups.

In another embodiment of the present invention, the 'substitution' of Rc may be independently composed of methyl, ethyl, straight or branched chain propyl, straight or branched chain butyl, and straight or branched chain pentyl groups.

In one embodiment of the present invention, La may be a single bond, a substituted or unsubstituted C6 to C12 arylenyl group, or a substituted or unsubstituted C2 to C12 heteroarylenyl group.

In another embodiment of the present invention, La may be a single bond, a substituted or unsubstituted phenylenyl group, or a substituted or unsubstituted C12 heteroarylenyl group.

In another embodiment of the present invention, La may be a single bond.

In one embodiment of the present invention, Y and Z may be the same as or different from each other and may each independently be O or S.

In another embodiment of the present invention, Y and Z may be O.

In another embodiment of the present invention, Y and Z may be S.

In another embodiment of the present invention, Y may be O, and Z may be S.

In another embodiment of the present invention, Y may be S, and Z may be O.

In one embodiment of the present invention, R1 to R7 are the same as or different from each other and are each independently selected from the group consisting of hydrogen; deuterium; halogen; a cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C1 to C10 alkoxy group; a substituted or unsubstituted C3 to C20 cycloalkyl group; a substituted or unsubstituted C2 to C20 heterocycloalkyl group; a substituted or unsubstituted C6 to C30 aryl group; a substituted or unsubstituted C2 to C30 heteroaryl group; -P(=O)R101R102; -SiR101R102R103; and -NR101R102, or form a substituted or unsubstituted C6 to C30 aromatic hydrocarbon ring or a substituted or unsubstituted C2 to C30 hetero ring by combining two or more groups adjacent to each other, R101, R102, and R103 are the same as or different from each other and may each independently be a substituted or unsubstituted C1 to C10 alkyl group; a substituted or unsubstituted C6 to C30 aryl group; or a substituted or unsubstituted C2 to C30 heteroaryl group.

In another embodiment of the present invention, R1 to R7 are the same as or different from each other and may each independently be hydrogen; deuterium; halogen; a cyano group; a substituted or unsubstituted C1 to C10 alkyl group; a substituted or unsubstituted C2 to C10 alkenyl group; a substituted or unsubstituted C2 to C10 alkynyl group; a substituted or unsubstituted C6 to C30 aryl group; or a substituted or unsubstituted C2 to C30 heteroaryl group.

In another embodiment of the present invention, R1 to R7 are the same as or different from each other and may each independently be hydrogen; deuterium; a substituted or unsubstituted C1 to C5 alkyl group; a substituted or unsubstituted C2 to C5 alkenyl group; a substituted or unsubstituted C2 to C5 alkynyl group; a substituted or unsubstituted C6 to C20 aryl group; or a substituted or unsubstituted C2 to C20 heteroaryl group.

In another embodiment of the present invention, R1 to R7 are the same as or different from each other and may each independently be hydrogen; deuterium; a substituted or unsubstituted C1 to C5 alkyl group; a substituted or unsubstituted C6 to C20 aryl group; or a substituted or unsubstituted C2 to C20 heteroaryl group.

In another embodiment of the present invention, R1 to R7 are the same as or different from each other and may each independently be hydrogen, deuterium, a substituted or unsubstituted C1 to C5 alkyl group, substituted or unsubstituted phenyl, naphthalenyl, pyridinyl, anthracenyl, carbazole, dibenzothiophene, dibenzofuran, or phenanthrenyl group.

In another embodiment of the present invention, R1 to R7 are the same as or different from each other and may each independently be hydrogen, deuterium, and a substituted or unsubstituted C1 to C5 alkyl group. In this case, the C1 to C5 alkyl group may be methyl, ethyl, straight or branched chain propyl, straight or branched chain butyl, or a straight or branched chain pentyl group.

In another embodiment of the present invention, R1 to R7 are the same as or different from each other and may each independently be hydrogen, deuterium.

In another embodiment of the present invention, the 'substitution' of R1 to R7 may consist each independently of one or more substituents selected from the group consisting of C1 to C10 alkyl; C2 to C10 alkenyl; C2 to C10 alkynyl; C3 to C15 cycloalkyl; C2 to C20 heterocycloalkyl; C6 to C30 aryl; C2 to C30 heteroaryl; C1 to C10 alkylamine; C6 to C30 arylamine; and C2 to C30 heteroarylamine groups.

In another embodiment of the present invention, the 'substitution' of R1 to R7 may consist each independently of one or more substituents selected from the group consisting of C1 to C10 alkyl; C6 to C30 aryl; C2 to C30 heteroaryl groups.

In another embodiment of the present invention, the 'substitution' of R1 to R7 may consist each independently of one or more substituents selected from the group consisting of C1 to C5 alkyl; C6 to C20 aryl; and C2 to C20 heteroaryl groups.

In another embodiment of the present invention, the 'substitution' of R1 to R7 may consist each independently of one or more substituents selected from the group consisting of methyl, ethyl, straight or branched chain propyl, straight or branched chain butyl, straight or branched chain pentyl, phenyl, naphthalenyl, pyridinyl, anthracenyl, carbazole, dibenzothiophene, dibenzofuran, and phenanthrenyl groups.

In another embodiment of the present invention, the 'substitution' of R1 to R7 may consist each independently of methyl, ethyl, straight or branched chain propyl, straight or branched chain butyl, and straight or branched chain pentyl groups.

In one embodiment of the present invention, Formula 1 above may be represented by any one of Formulas 1-1 to 1-3 below: 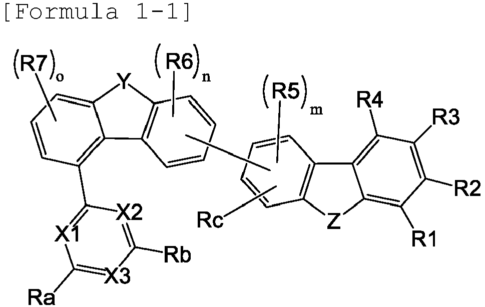 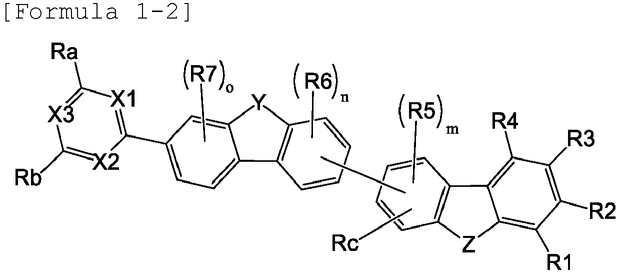 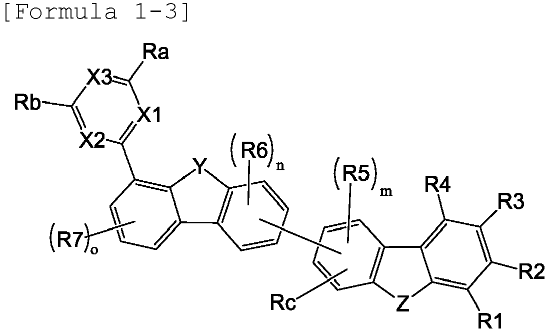 wherein the definitions of X1 to X3, Ra, Rb, Rc, Y, Z, R1 to R7, m, n and o are the same as those in Formula 1 above.

In one embodiment of the present invention, Formula 1-1 may be represented by Formula 1-4 or 1-5 below: 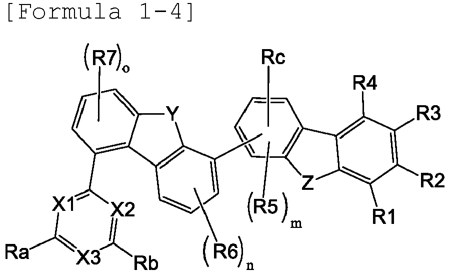 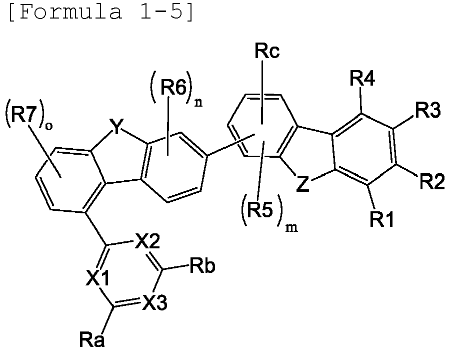 wherein the definitions of X1 to X3, Ra, Rb, Rc, Y, Z, R1 to R7, m, n and o are the same as those in Formula 1 above.

In one embodiment of the present invention, Formula 1 may be represented by any one of Formulas 1-6 to 1-11: 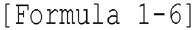 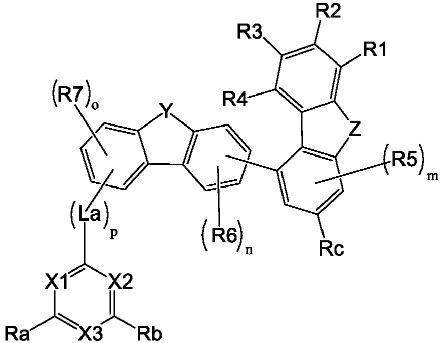 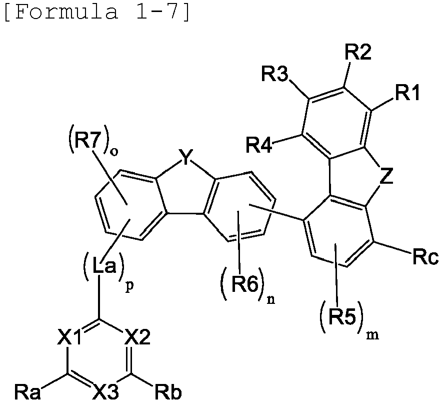 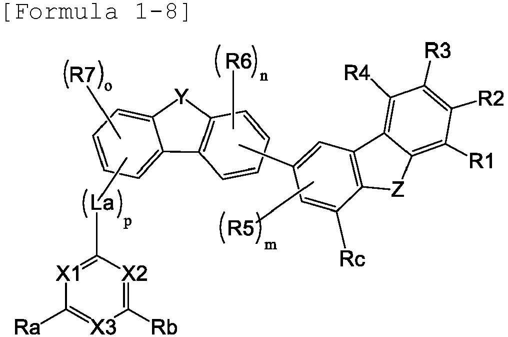 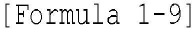 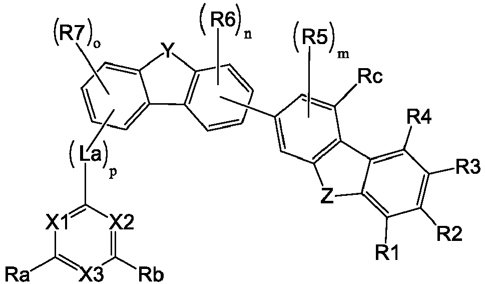 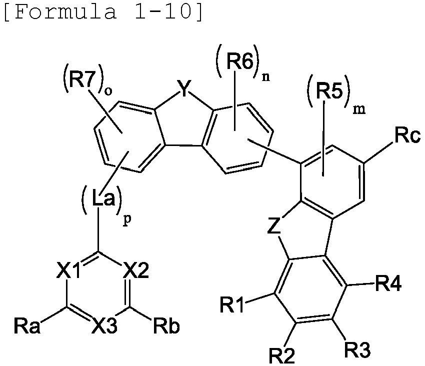 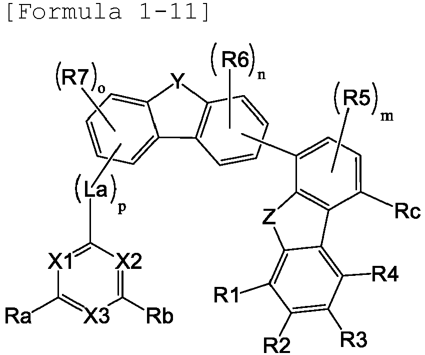 wherein the definitions of X1 to X3, Ra, Rb, Rc, La, Y, Z, R1 to R7, m, n, o and p are the same as those in Formula 1 above.

In one embodiment of the present invention, the compound represented by Formula 1 may comprises at least one deuterium as a substituent.

In another embodiment of the present invention, the content of deuterium may be 0% or more, 1% or more, 10% or more, 20% or more, 30% or more, 40% or more, or 50% or more, and 100% or less, 90% or less, 80% or less, 70% or less, or 60% or less, based on the total number of hydrogen atoms and deuterium atoms in Formula 1.

In another embodiment of the present invention, the content of deuterium may be 20% to 90%, based on the total number of hydrogen atoms and deuterium atoms in Formula 1.

In another embodiment of the present invention, the content of deuterium may be 30% to 80%, based on the total number of hydrogen atoms and deuterium atoms in Formula 1.

In another embodiment of the present invention, the content of deuterium may be 50% to 60%, based on the total number of hydrogen atoms and deuterium atoms in Formula 1.

In one embodiment of the present invention, the heterocyclic compound represented by Formula 1 may be at least one selected from the compounds below:

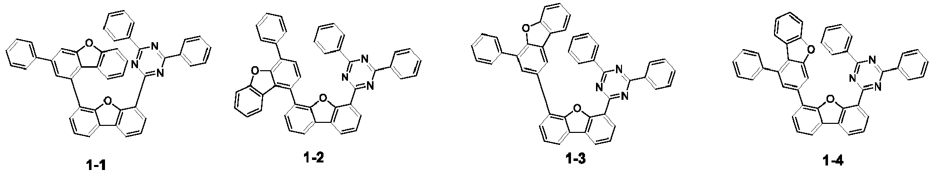

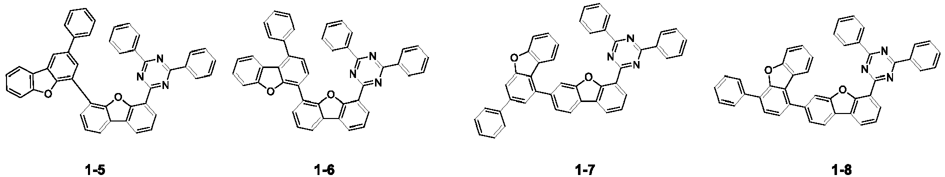

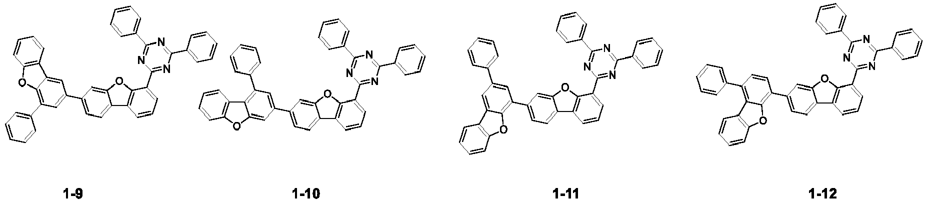

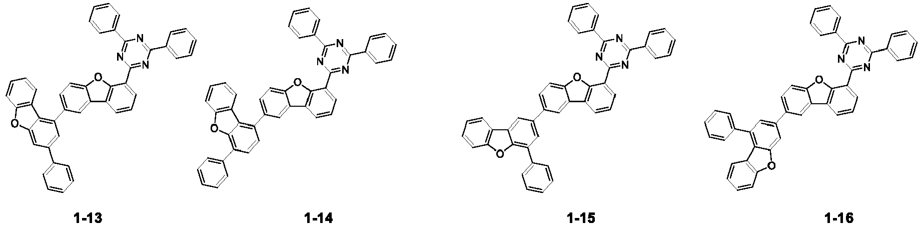

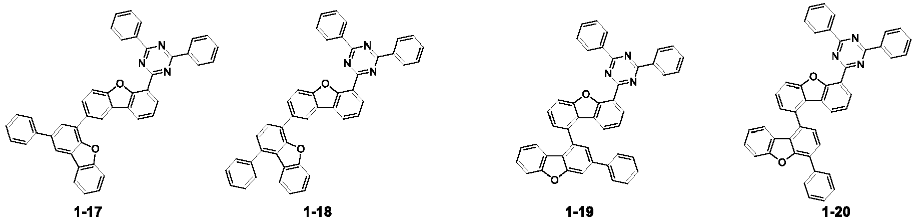

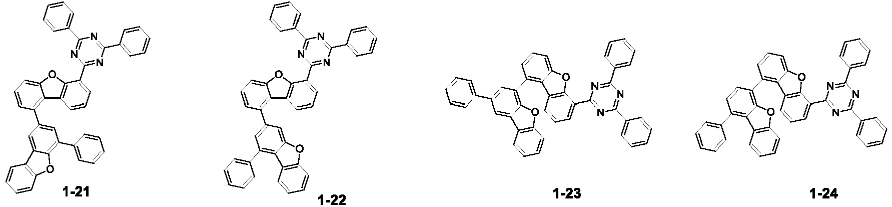

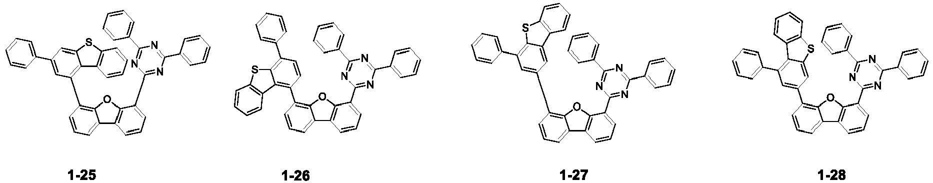

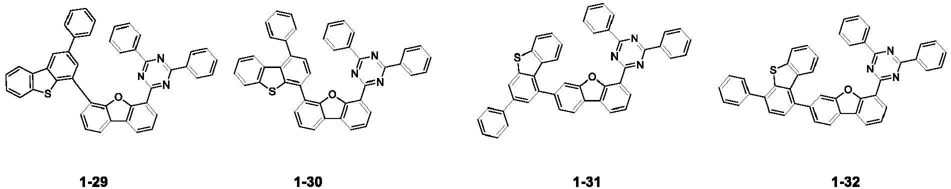

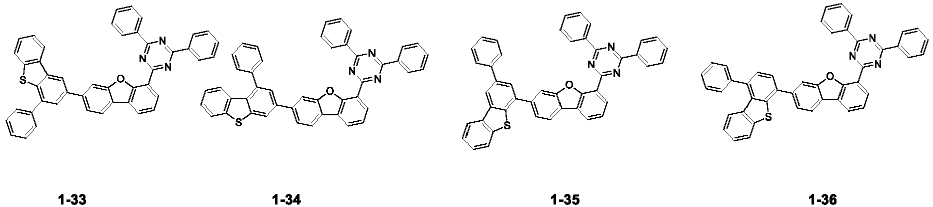

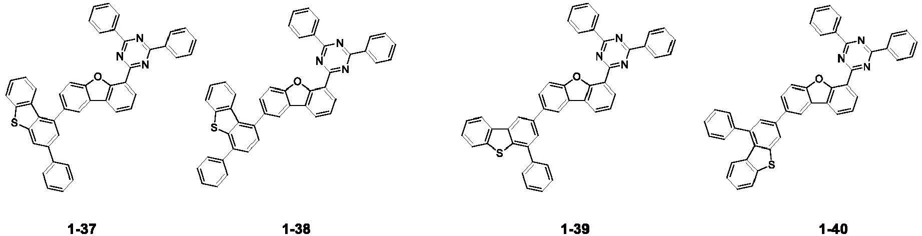

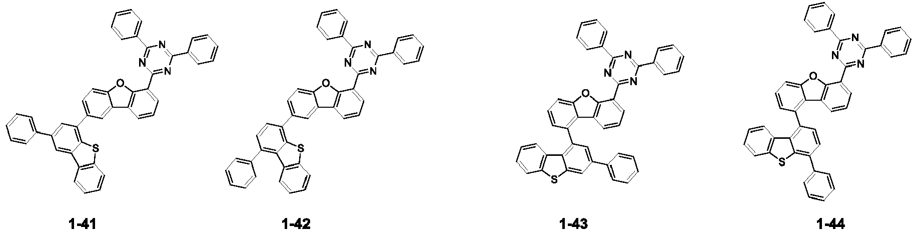

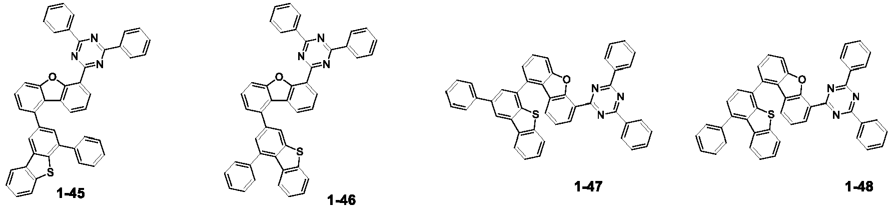

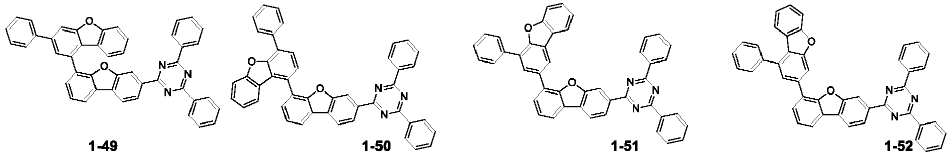

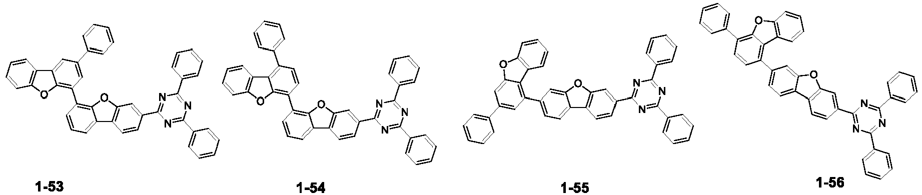

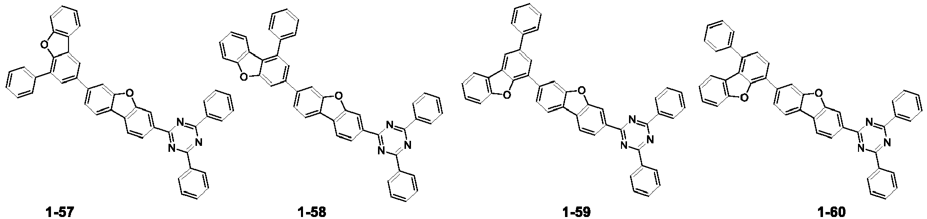

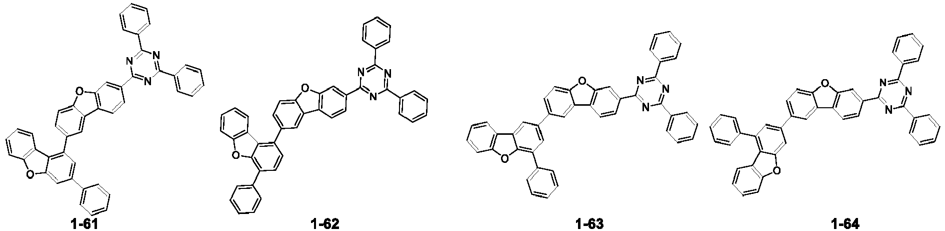

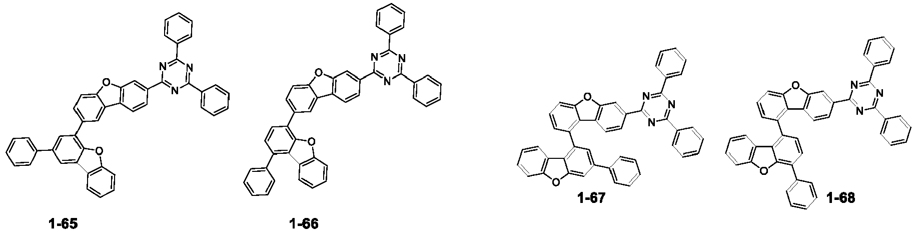

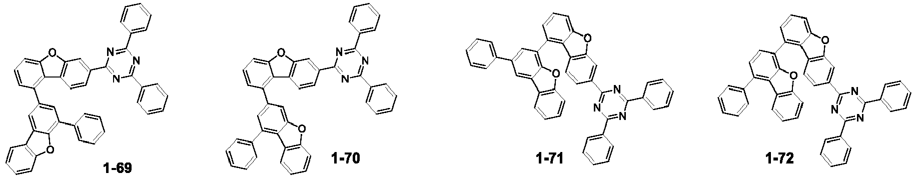

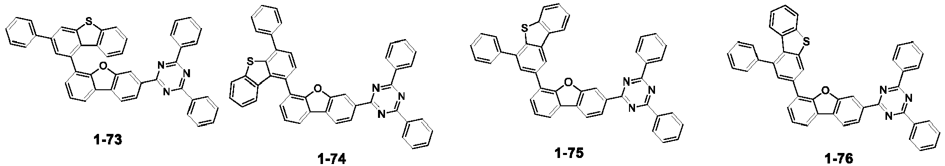

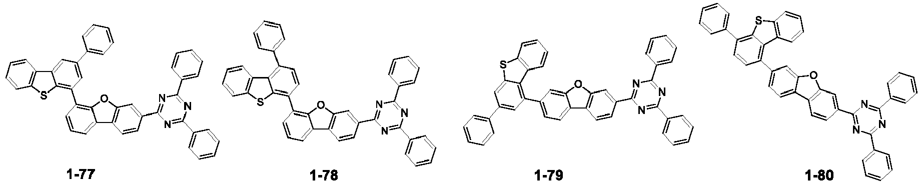

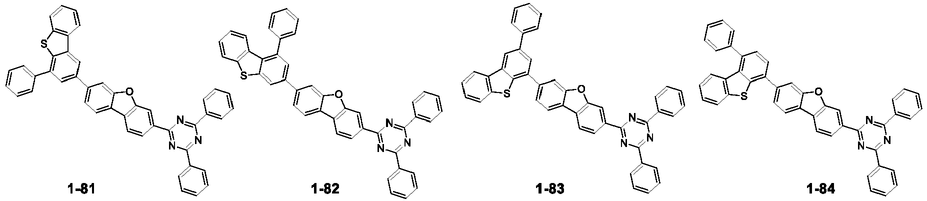

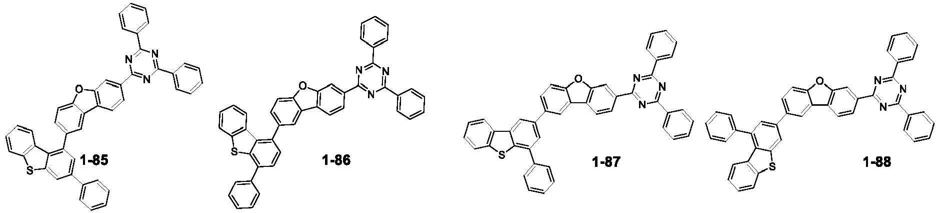

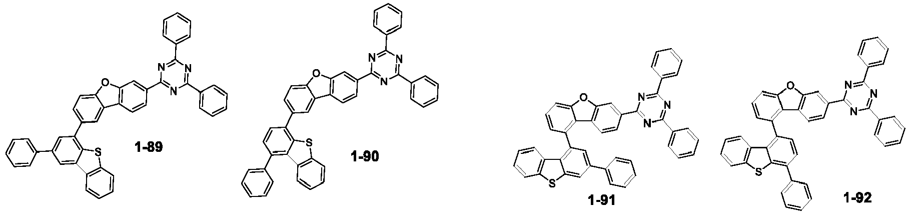

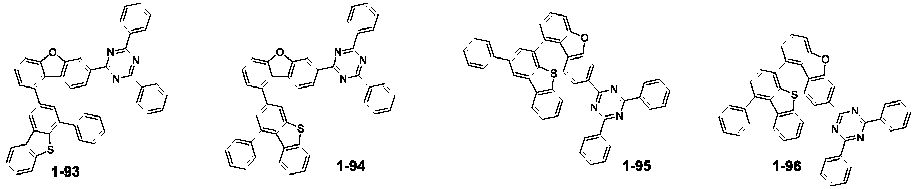

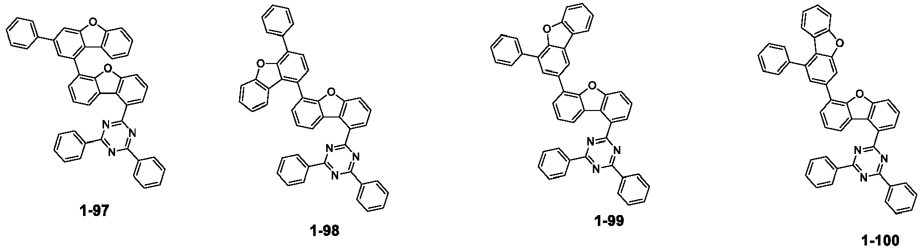

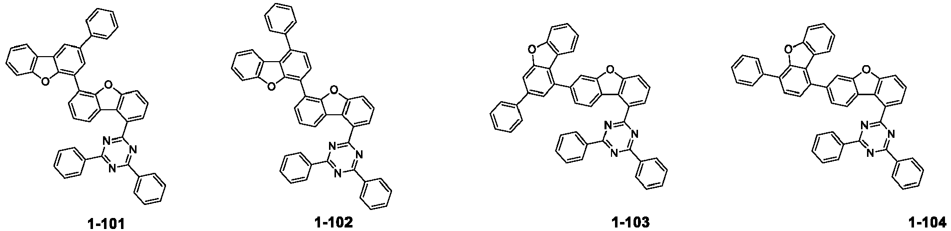

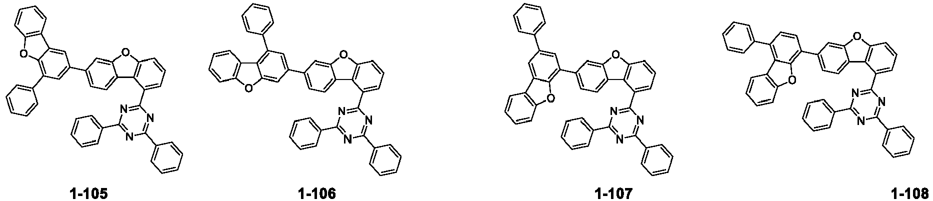

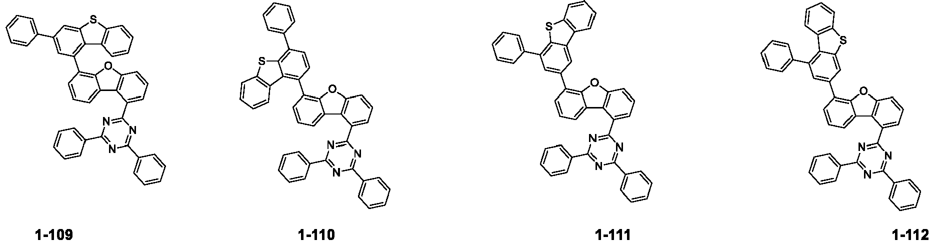

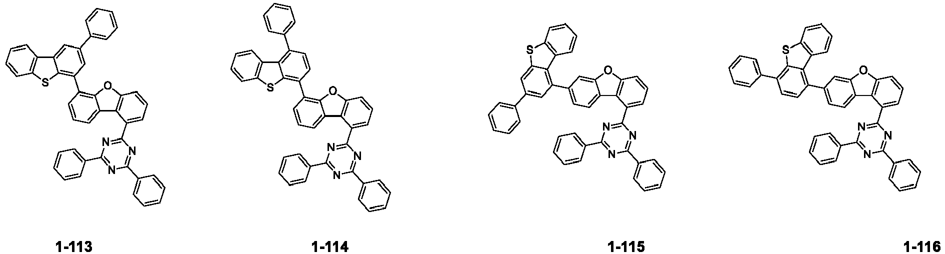

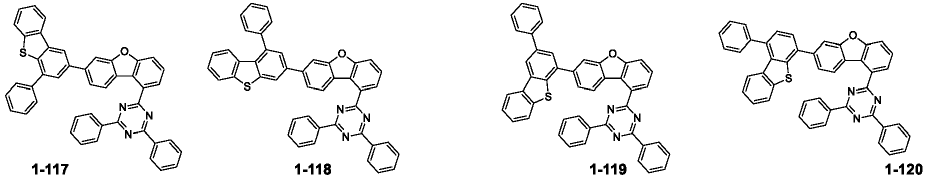

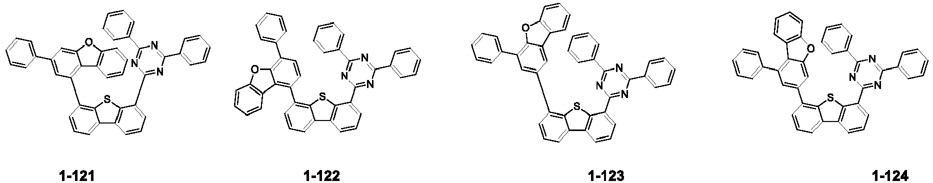

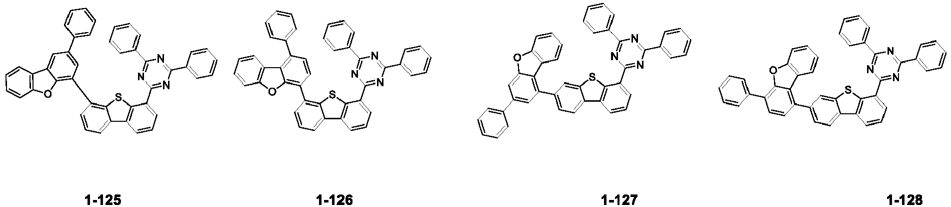

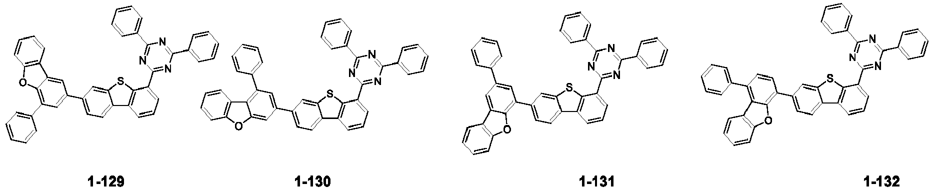

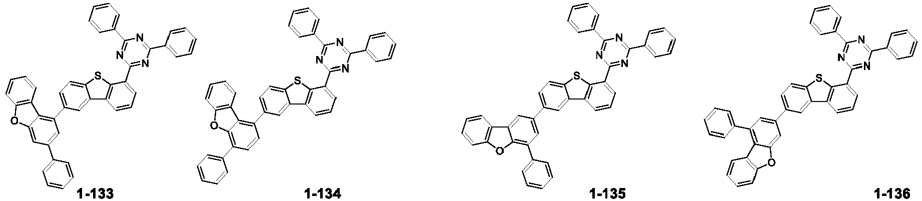

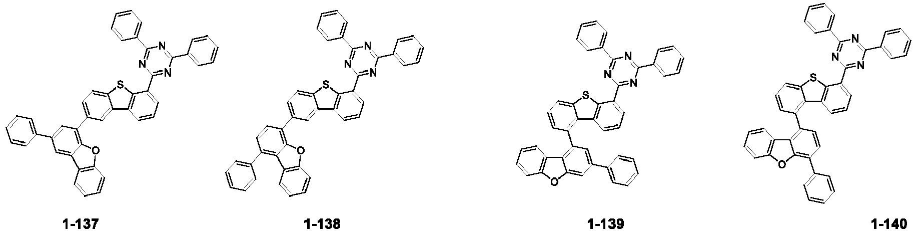

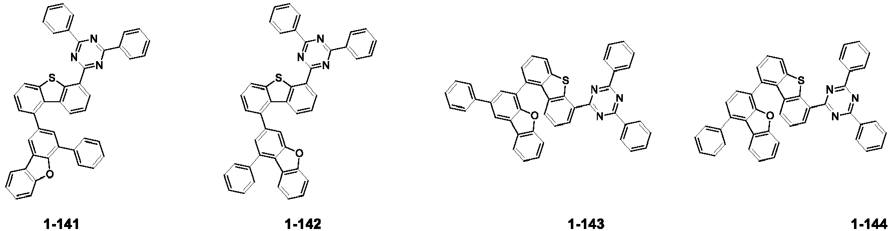

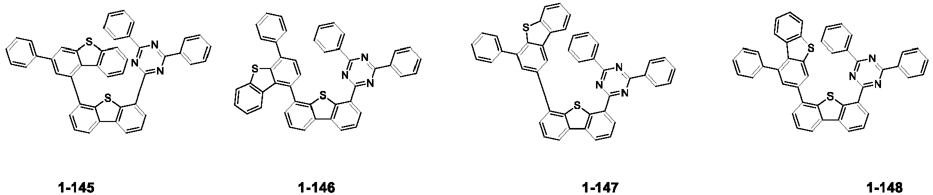

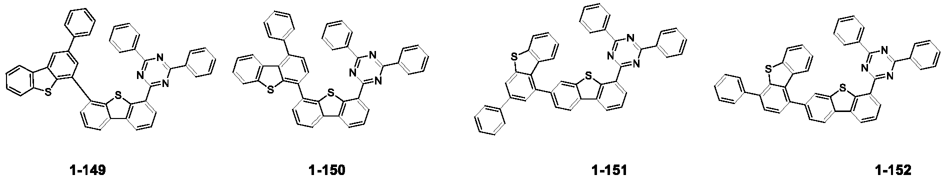

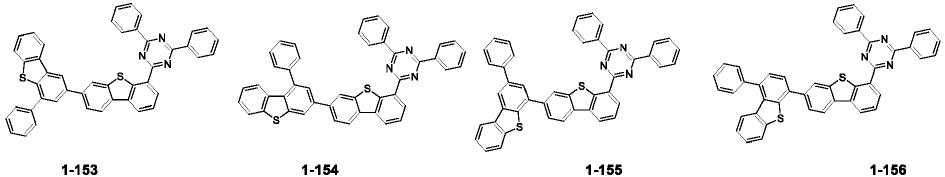

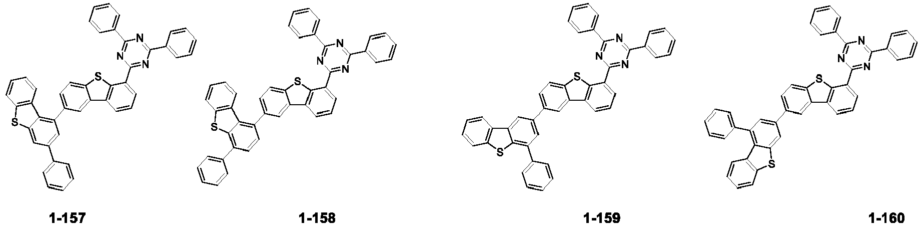

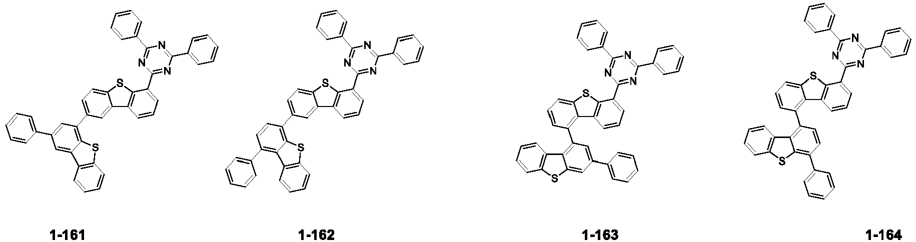

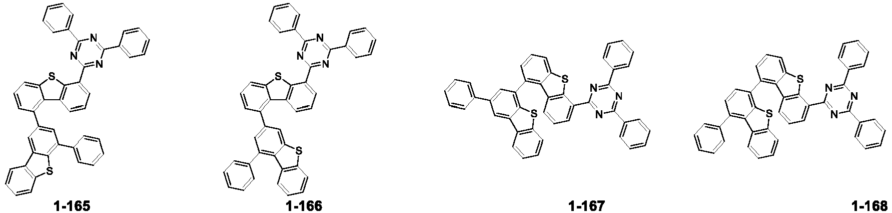

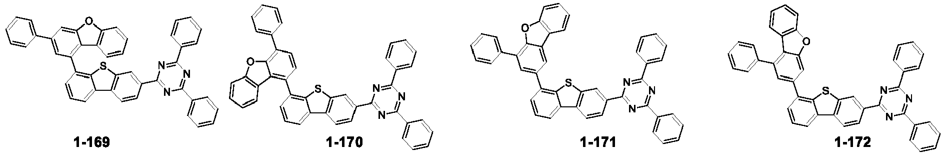

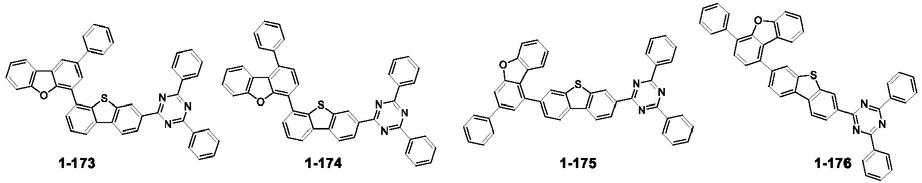

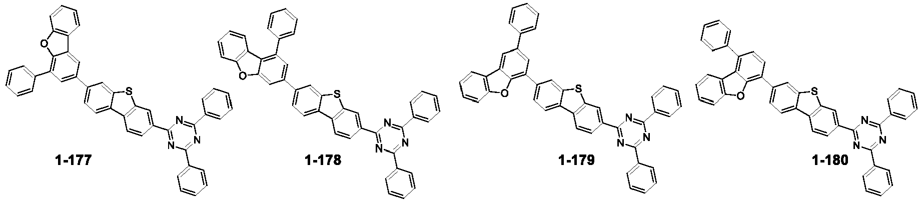

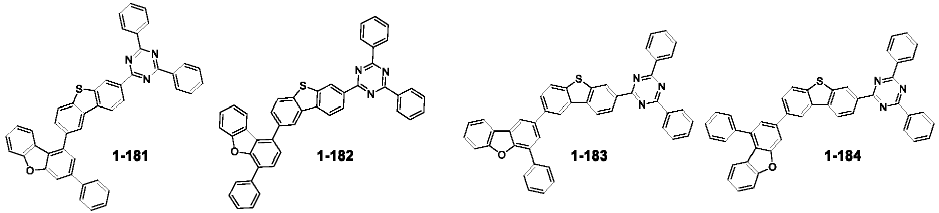

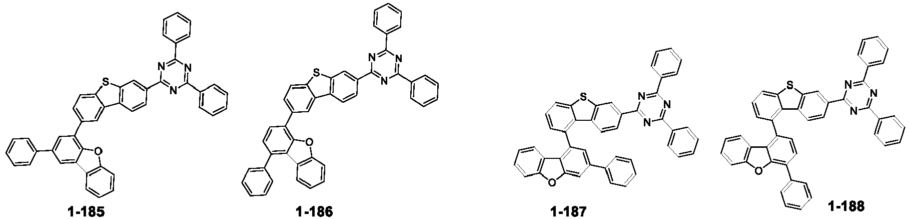

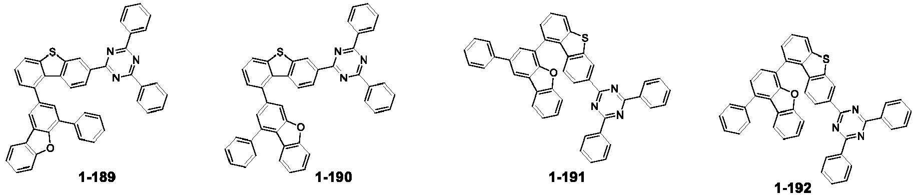

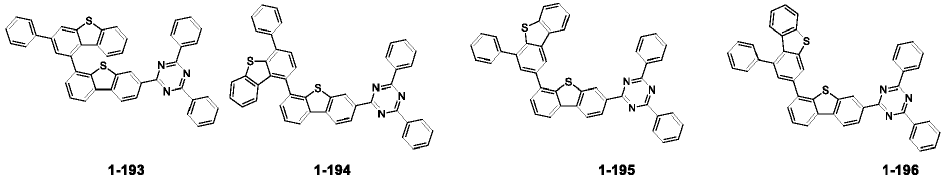

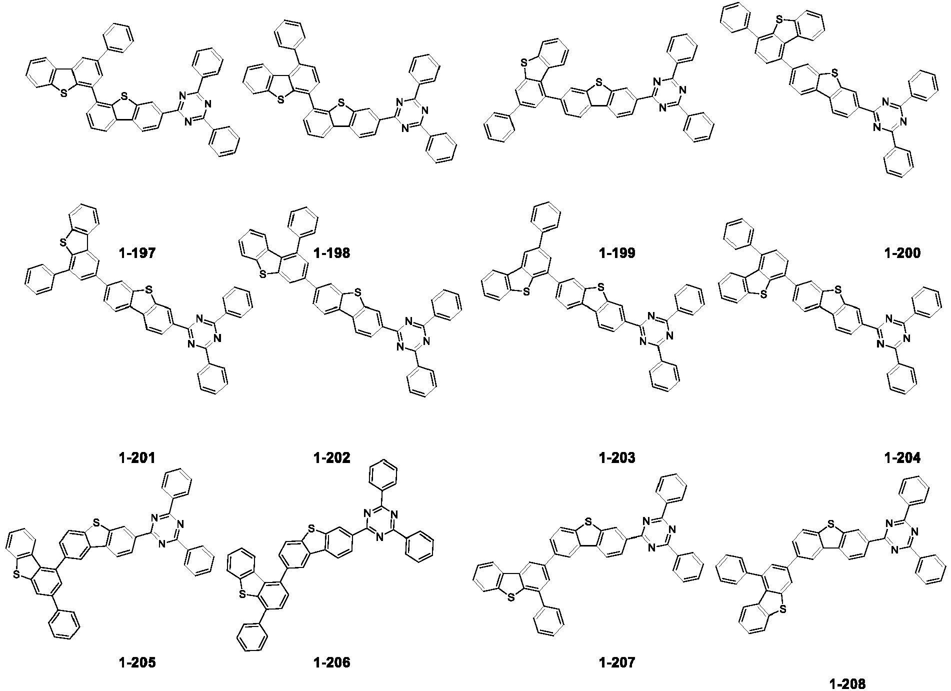

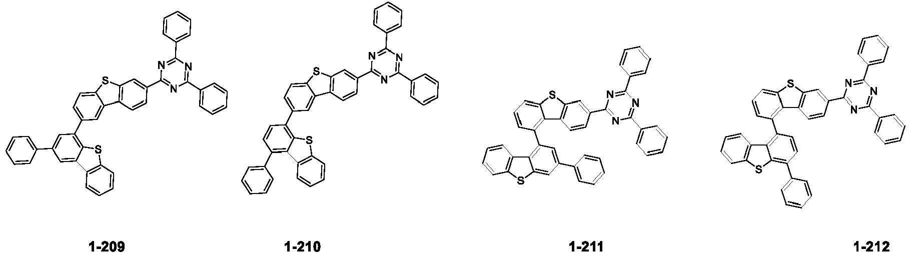

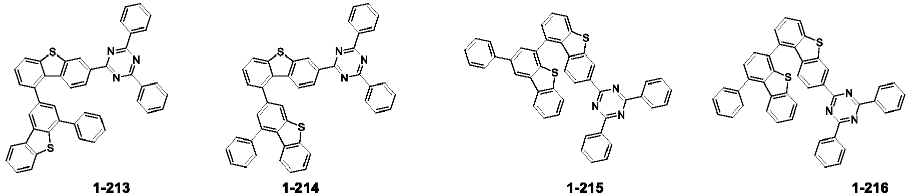

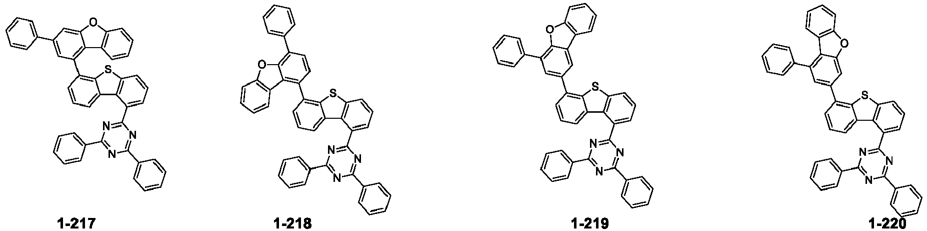

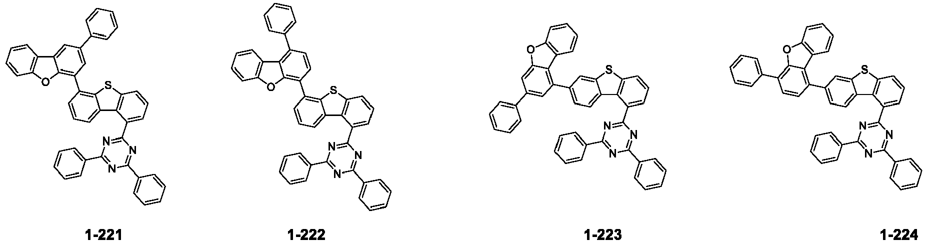

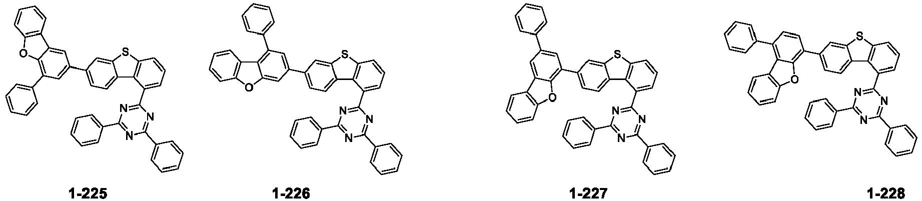

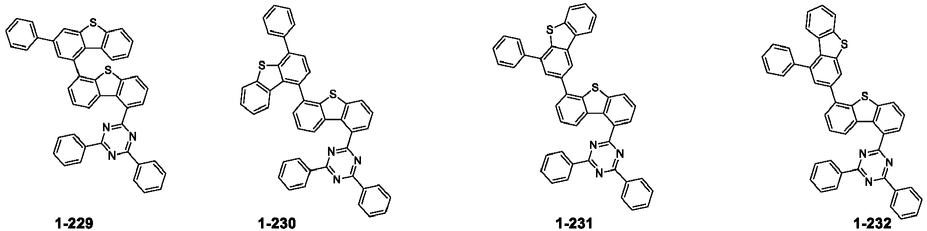

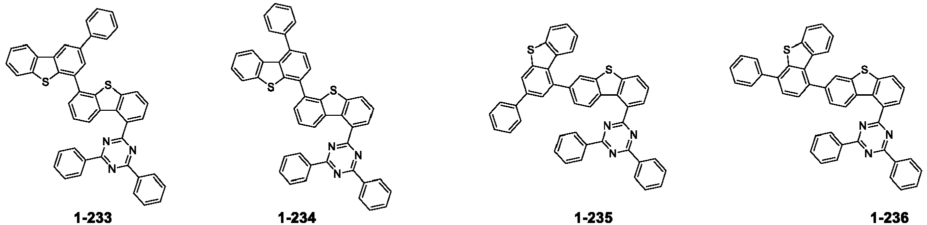

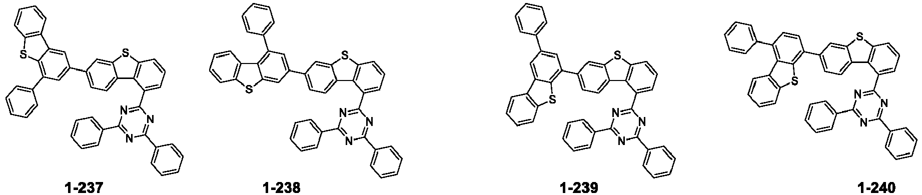

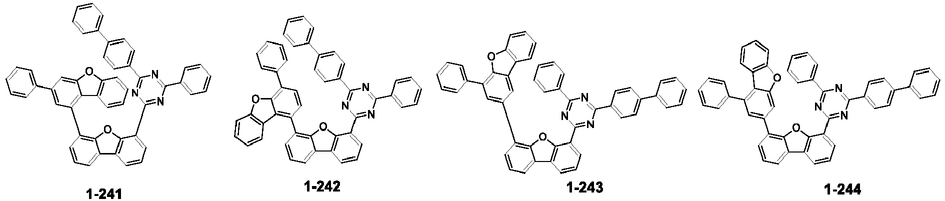

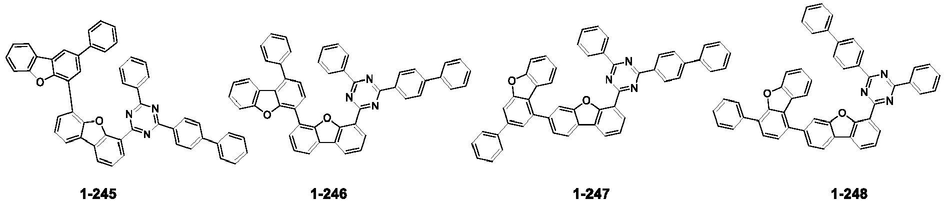

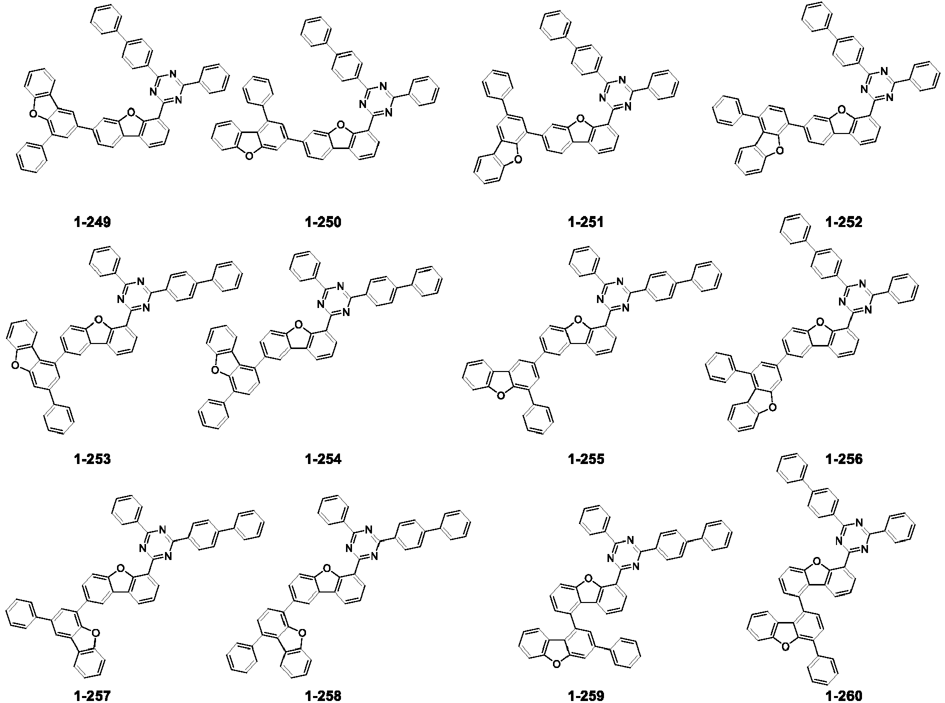

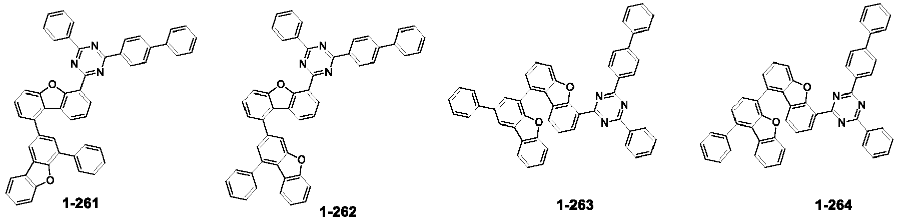

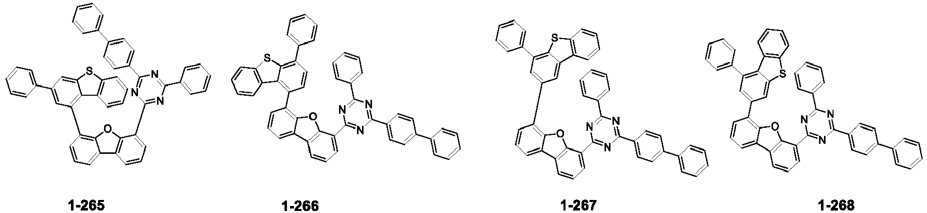

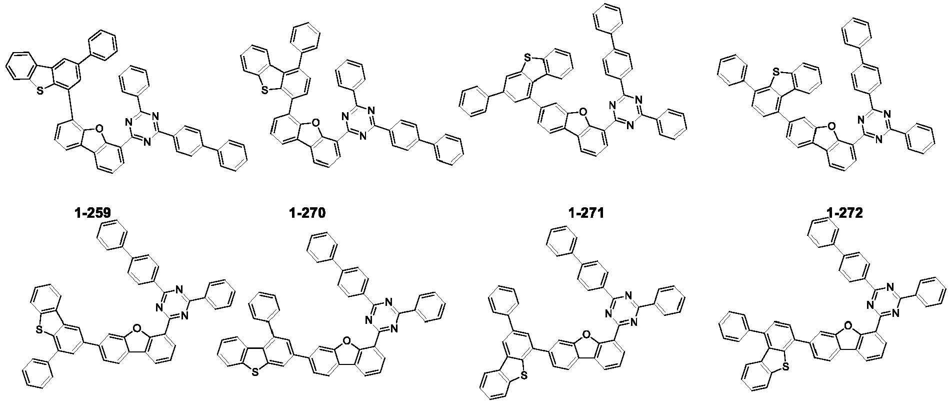

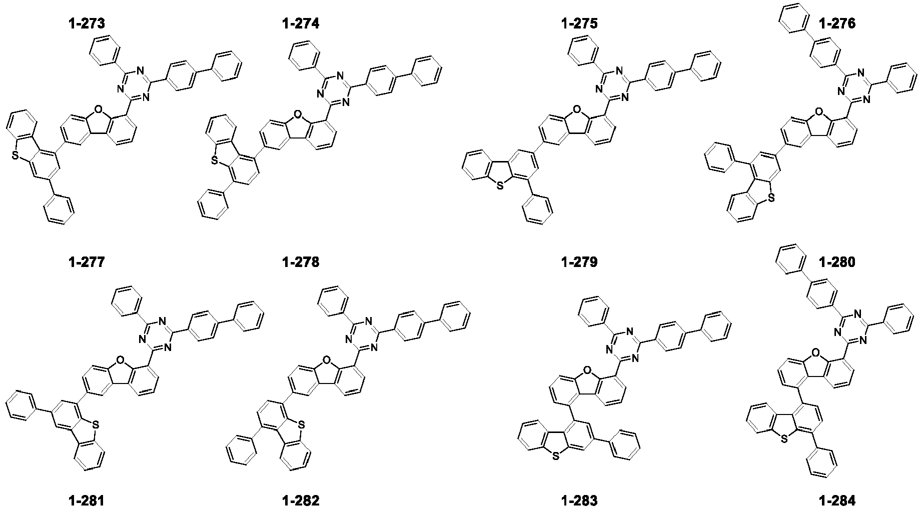

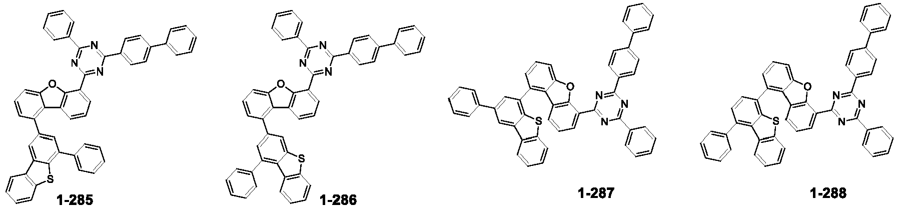

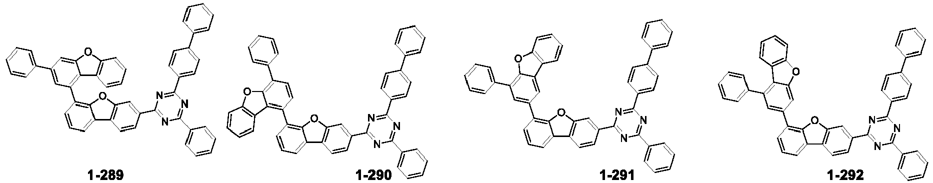

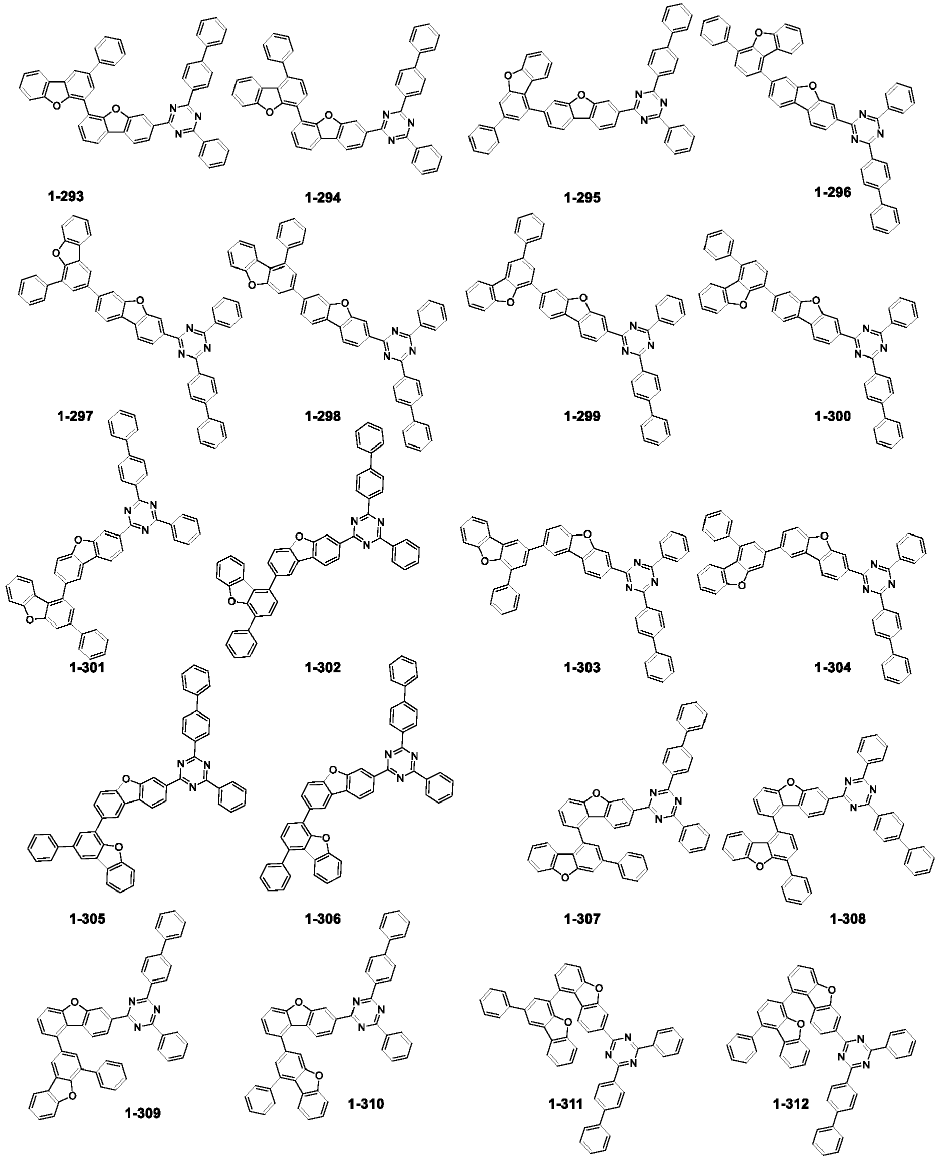

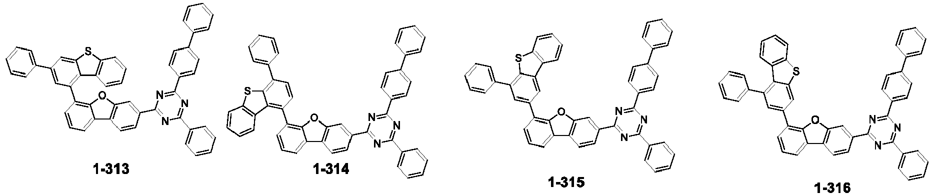

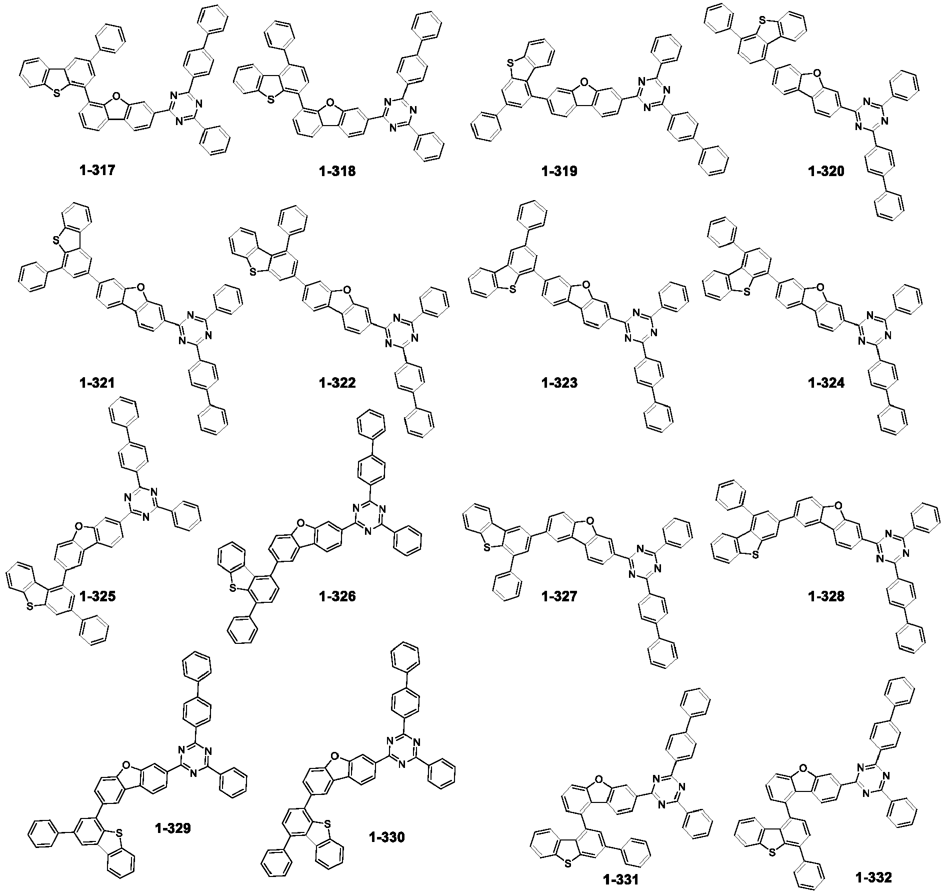

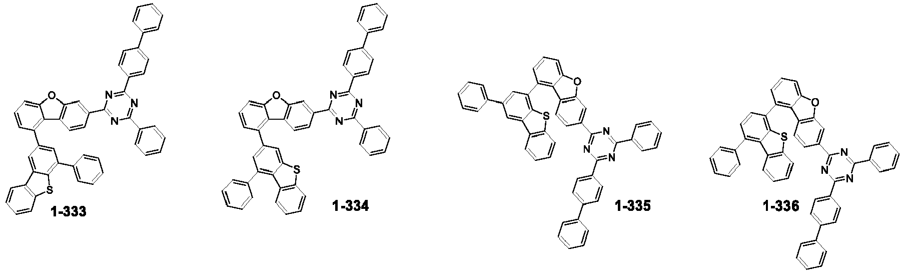

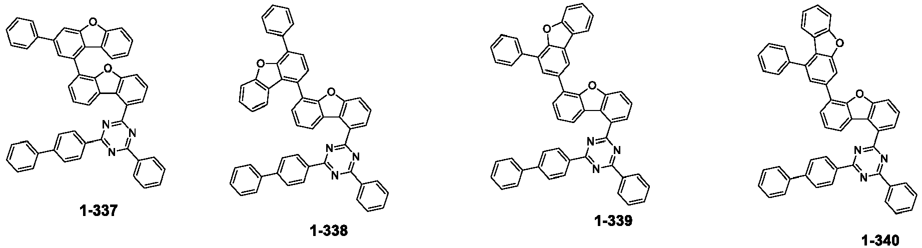

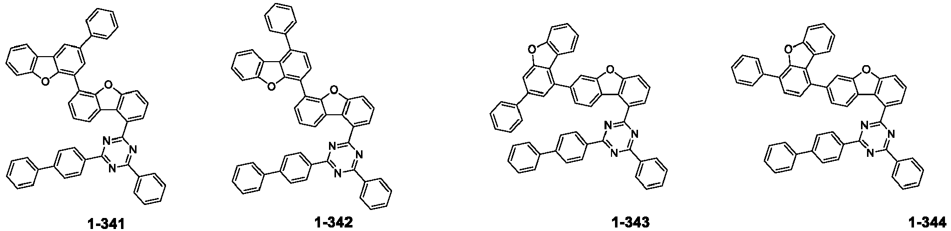

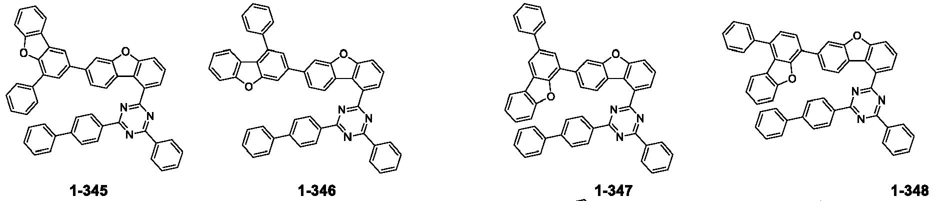

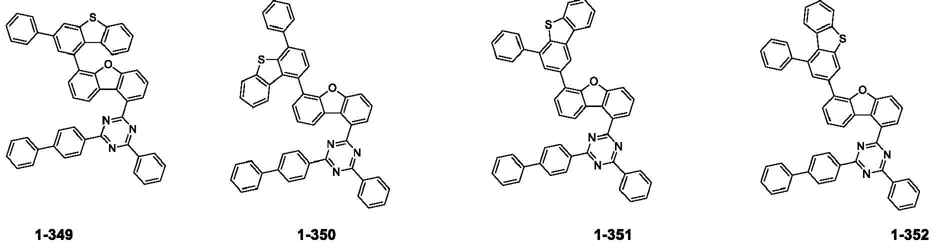

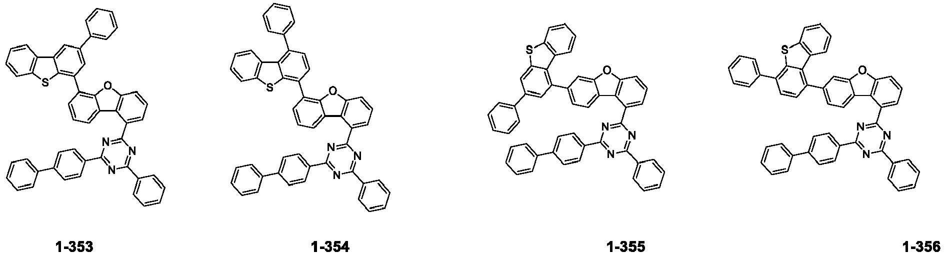

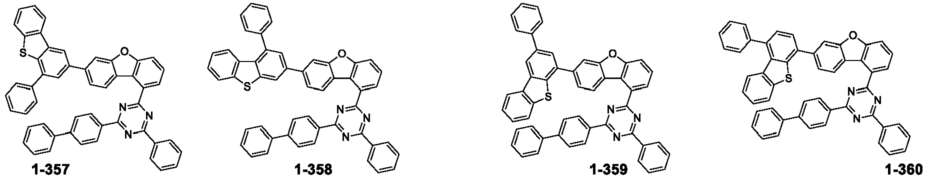

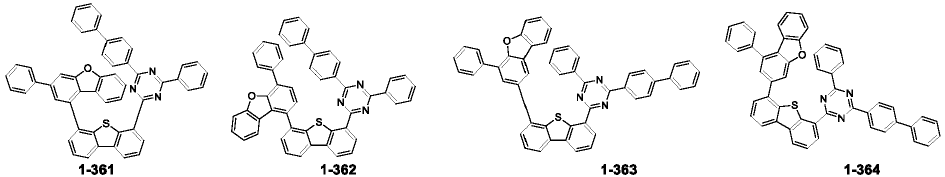

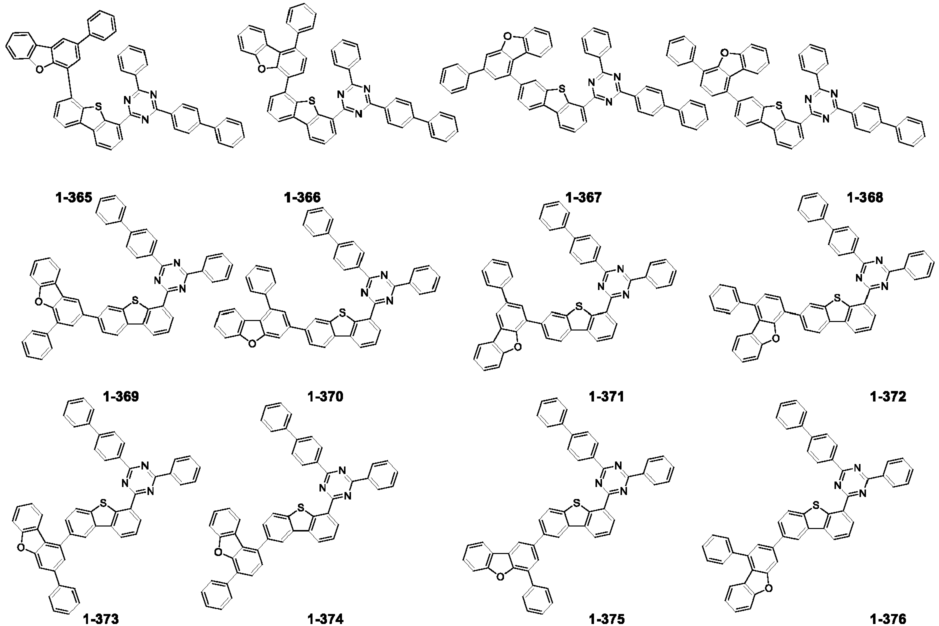

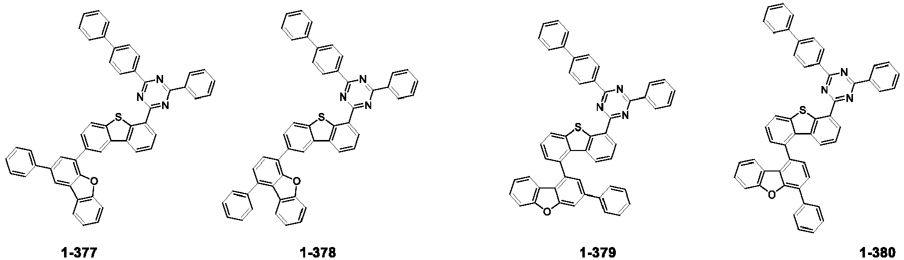

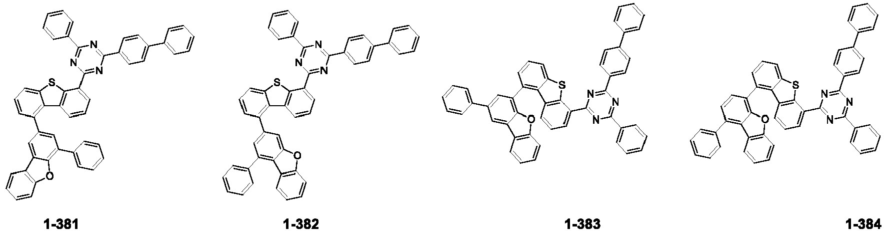

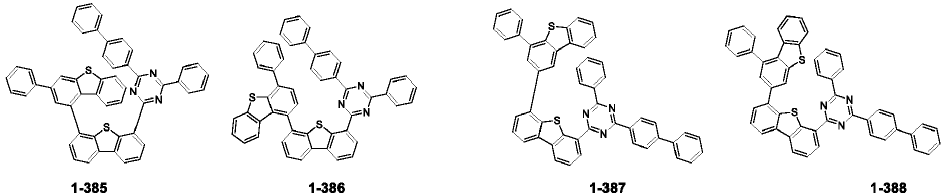

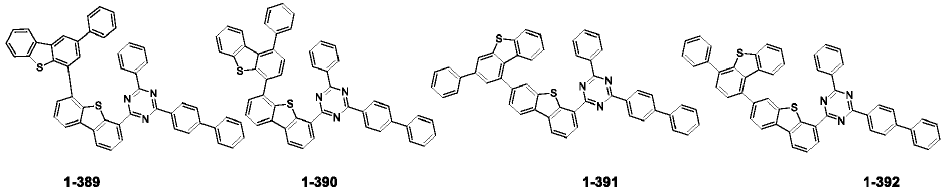

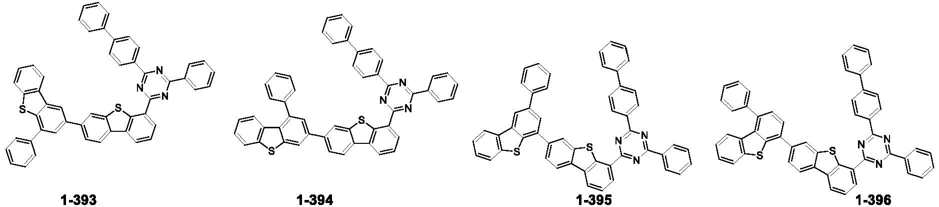

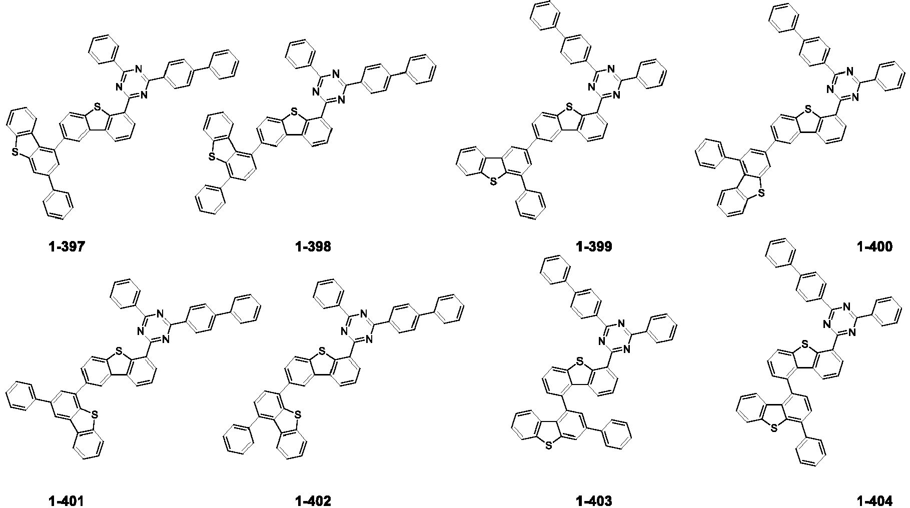

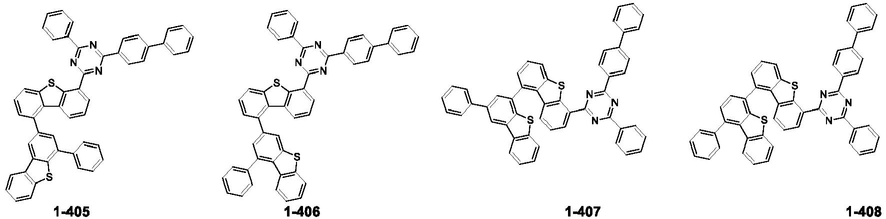

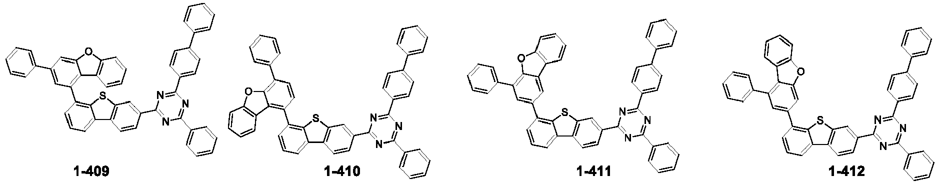

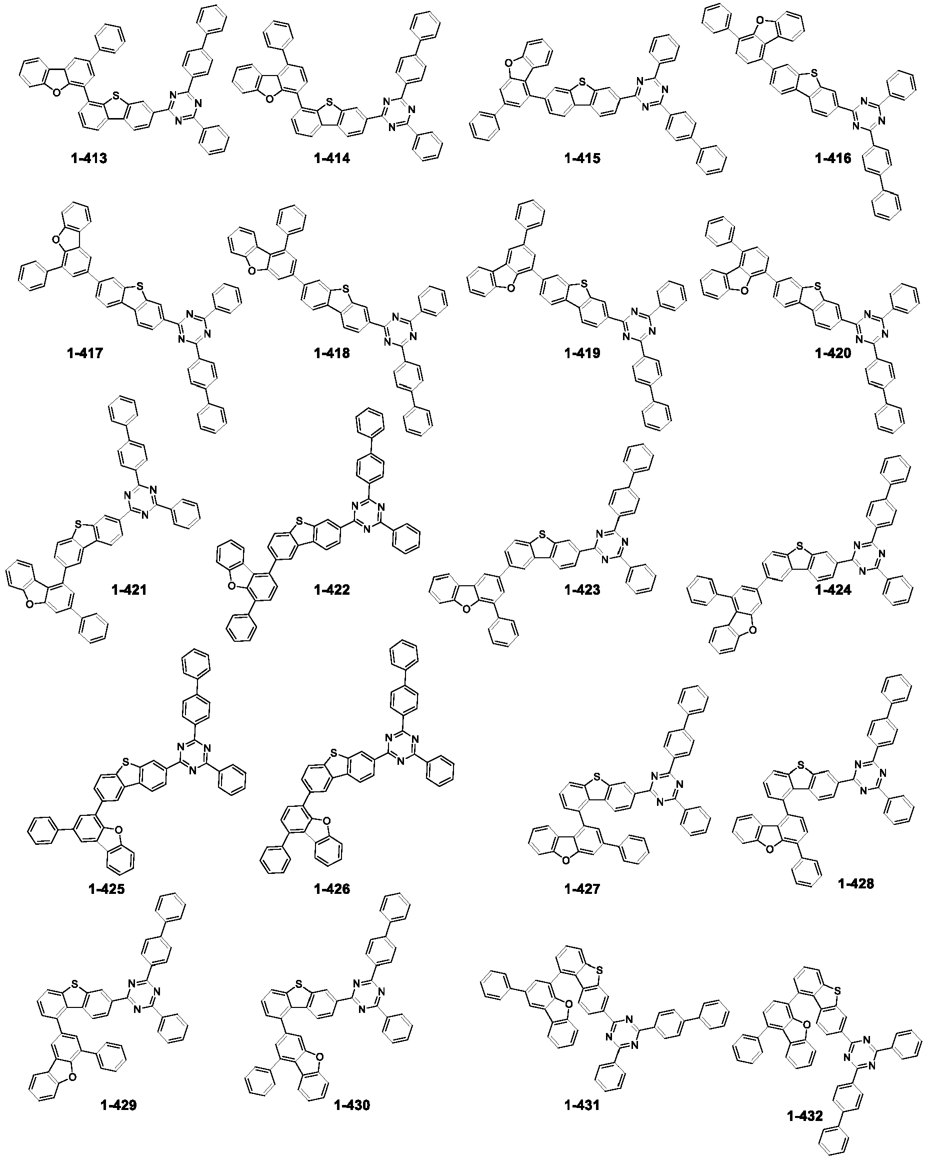

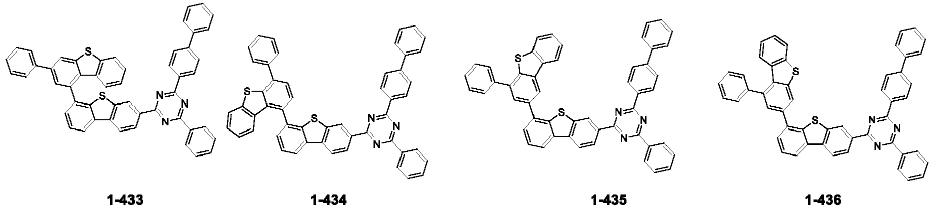

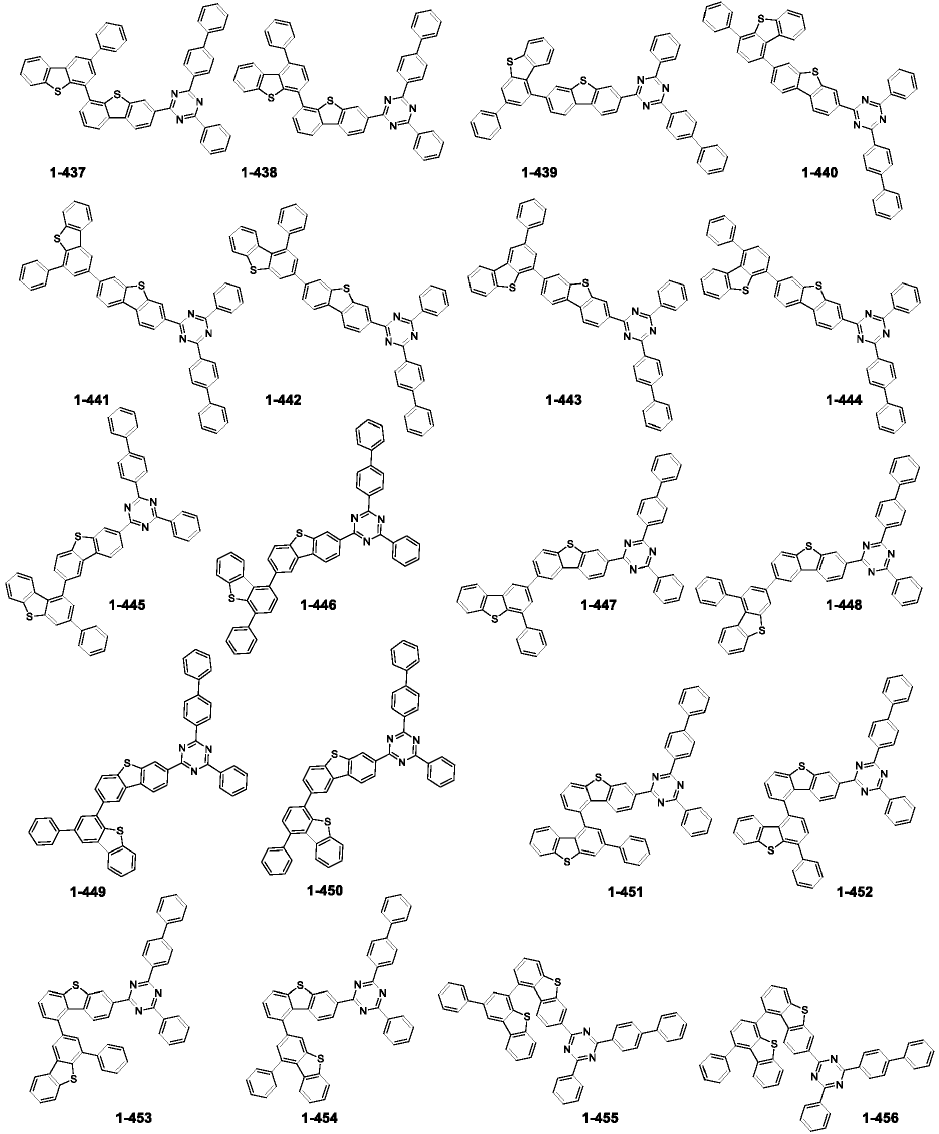

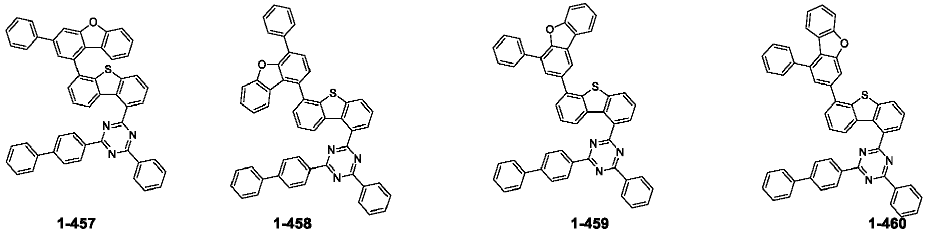

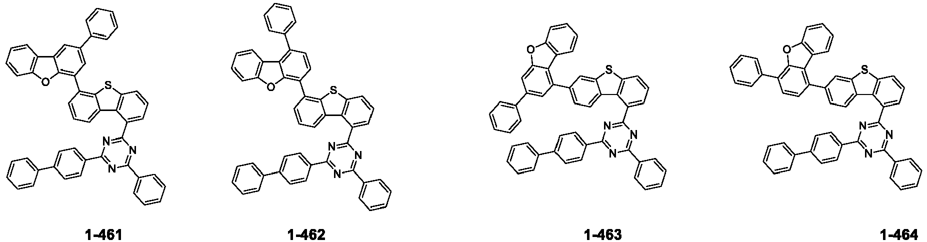

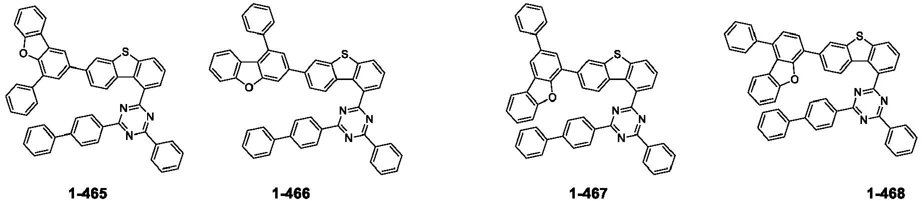

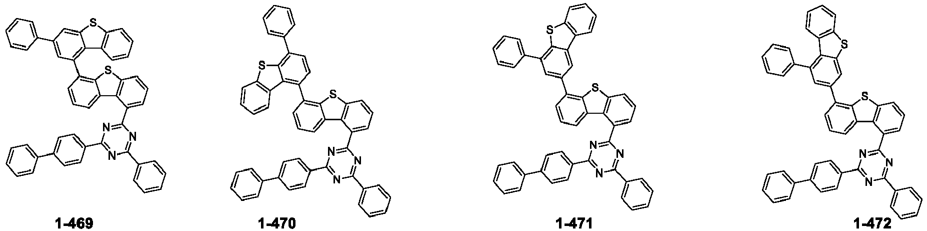

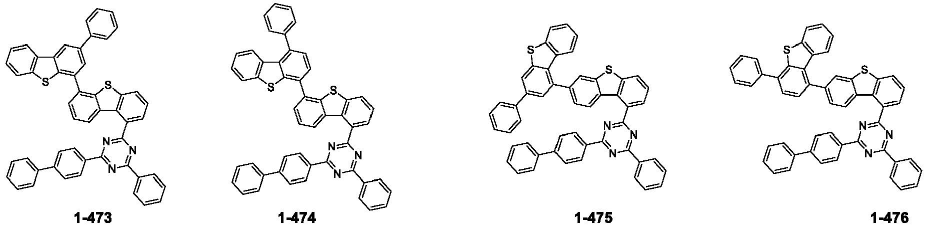

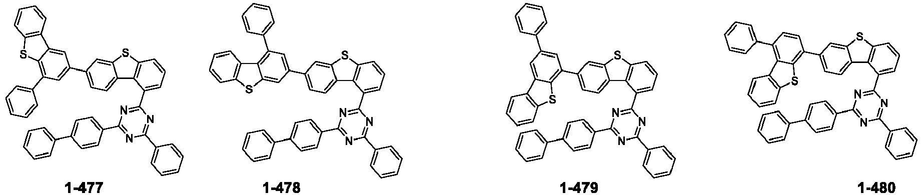

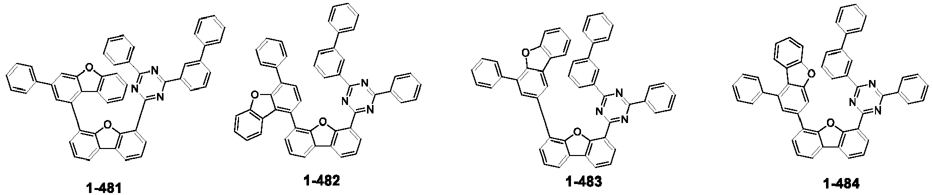

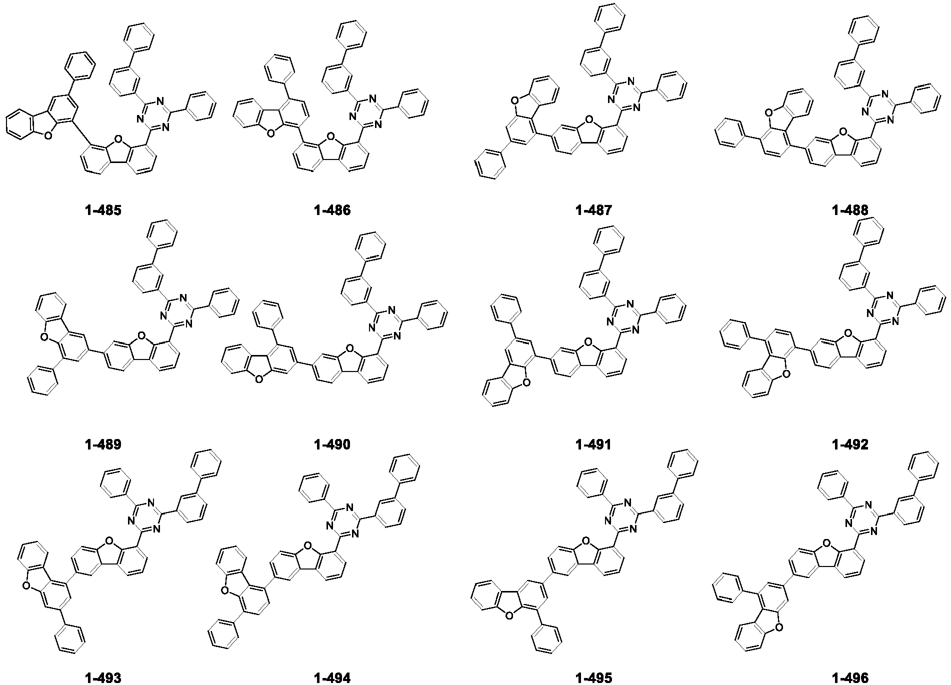

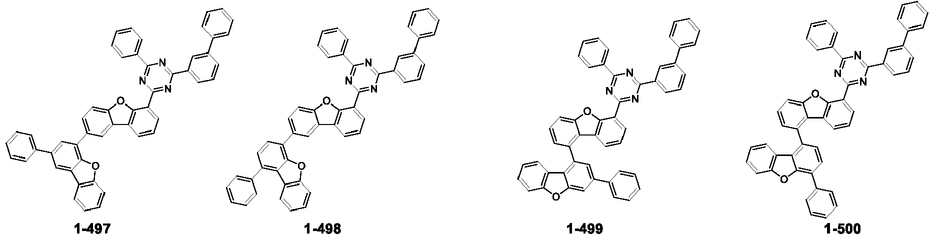

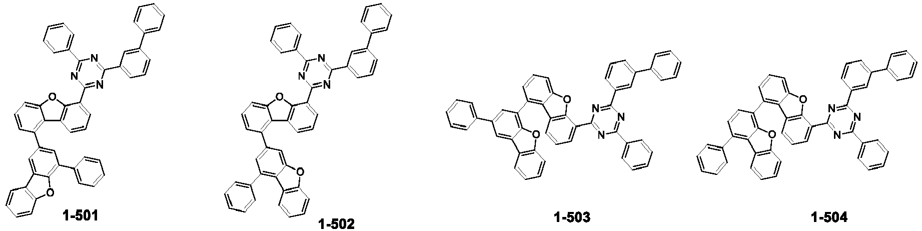

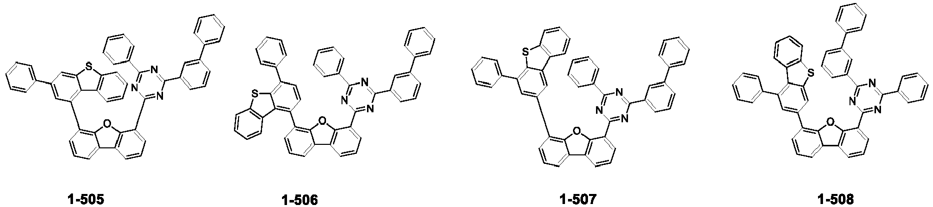

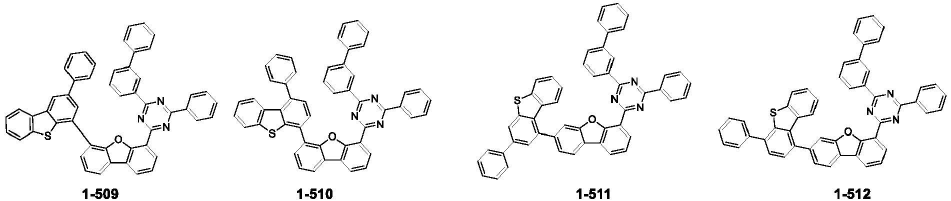

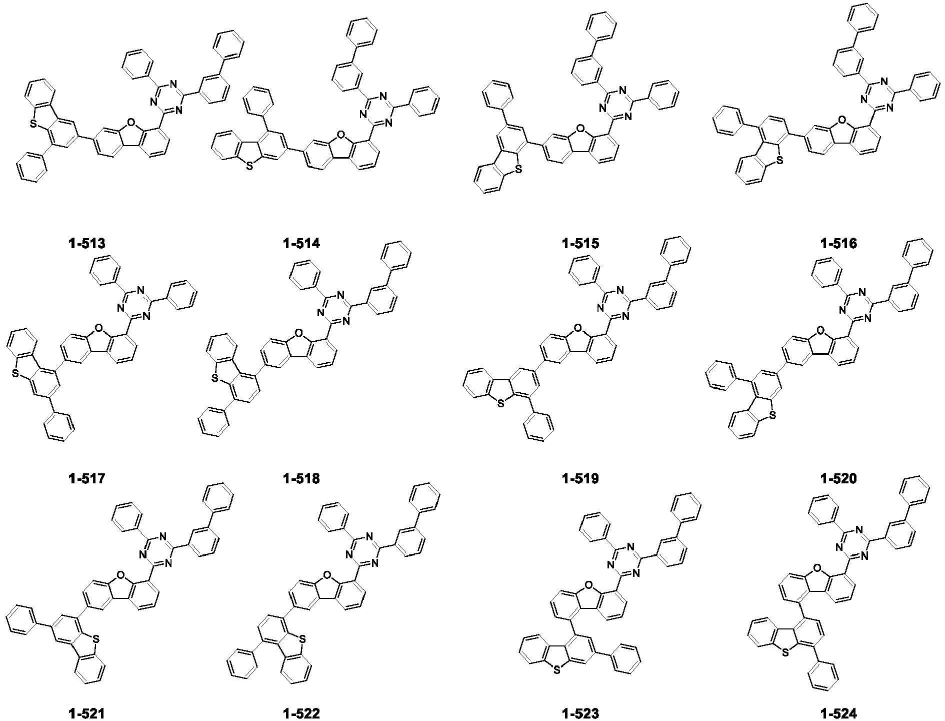

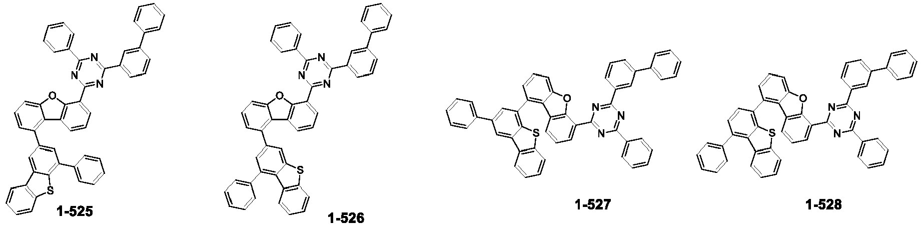

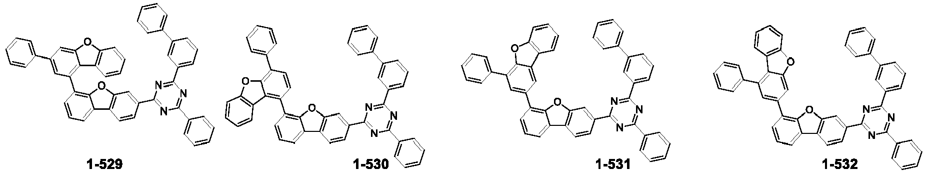

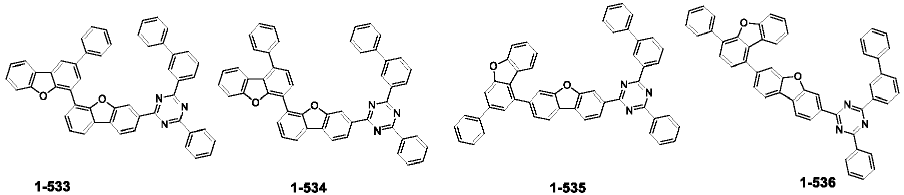

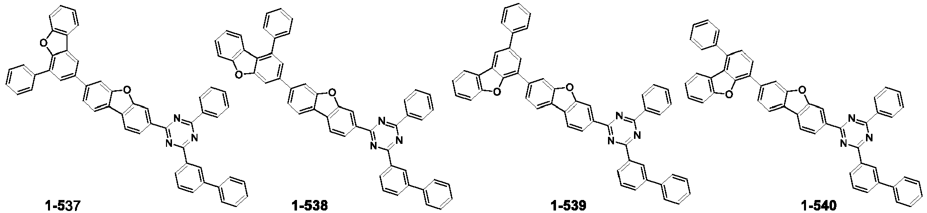

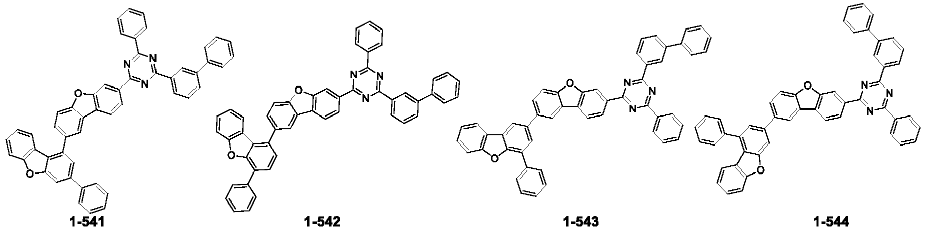

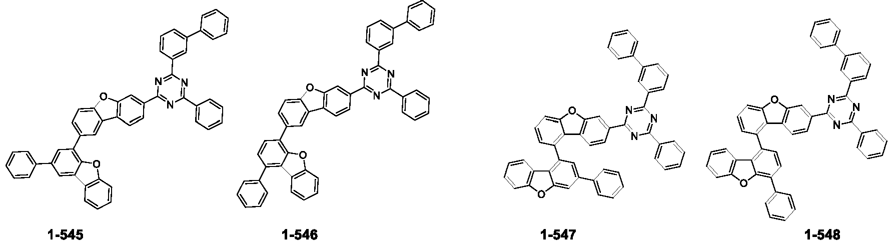

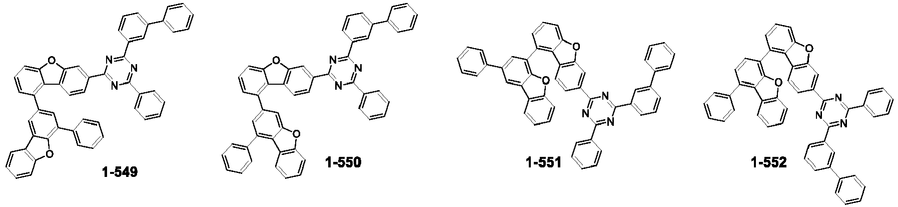

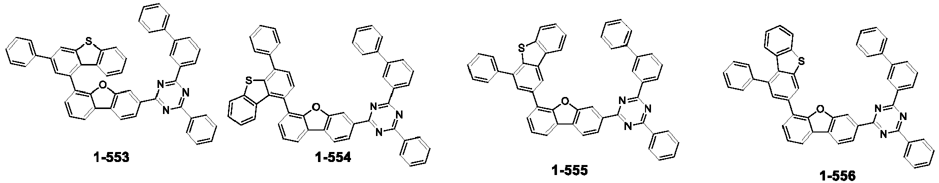

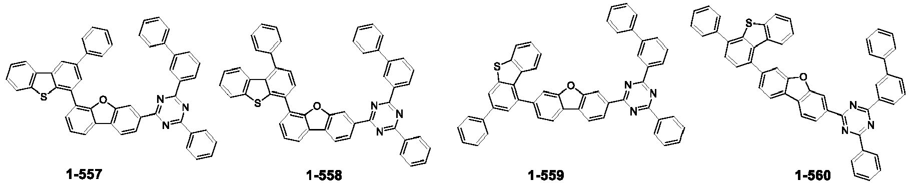

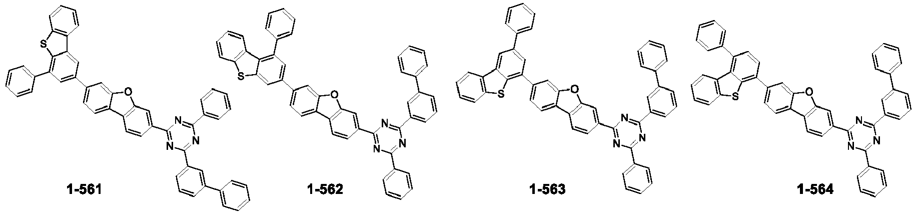

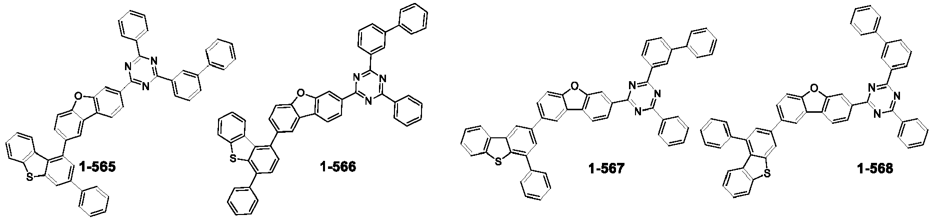

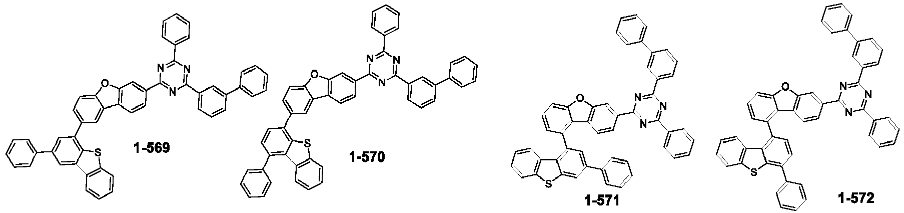

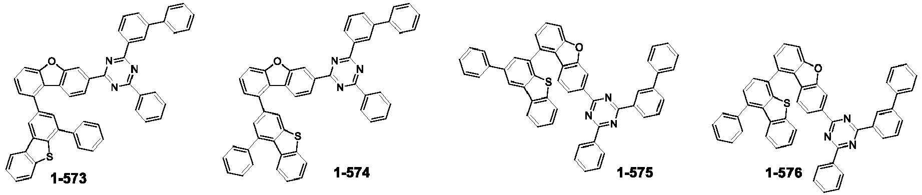

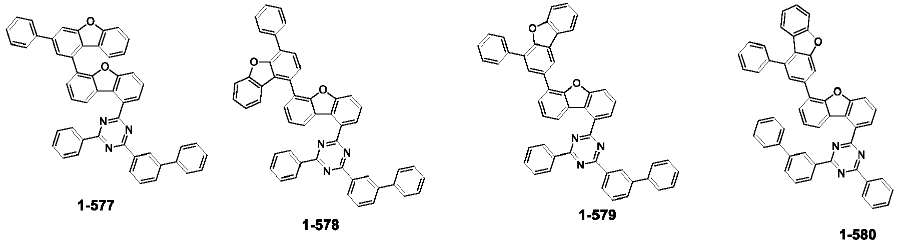

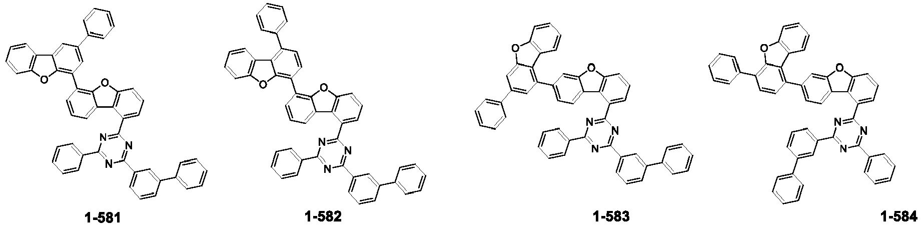

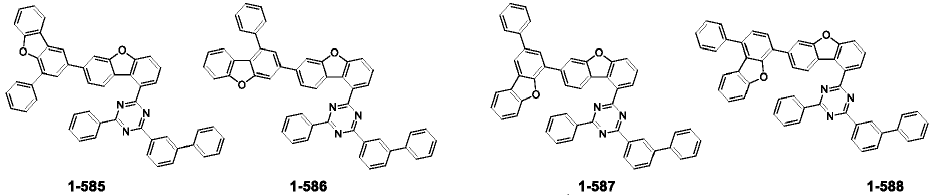

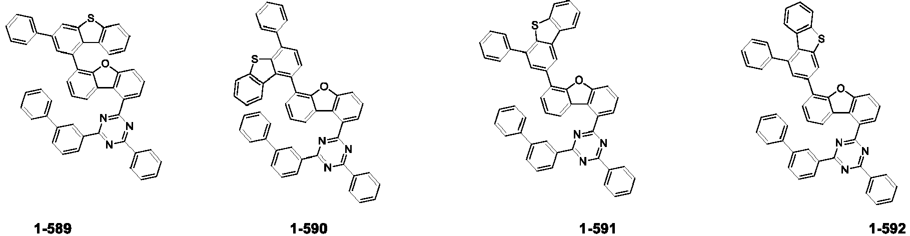

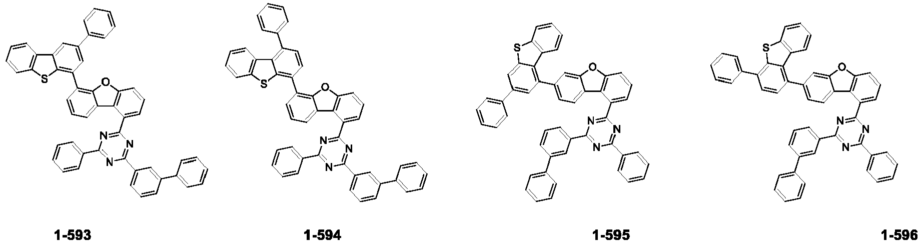

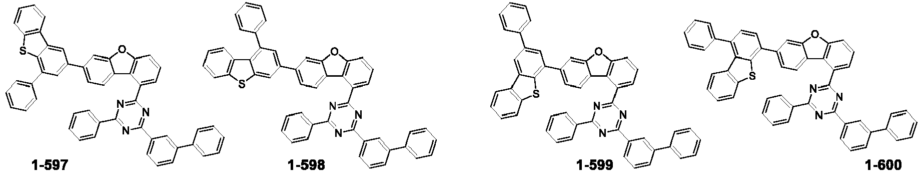

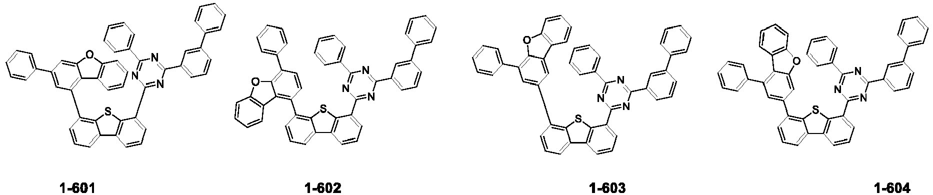

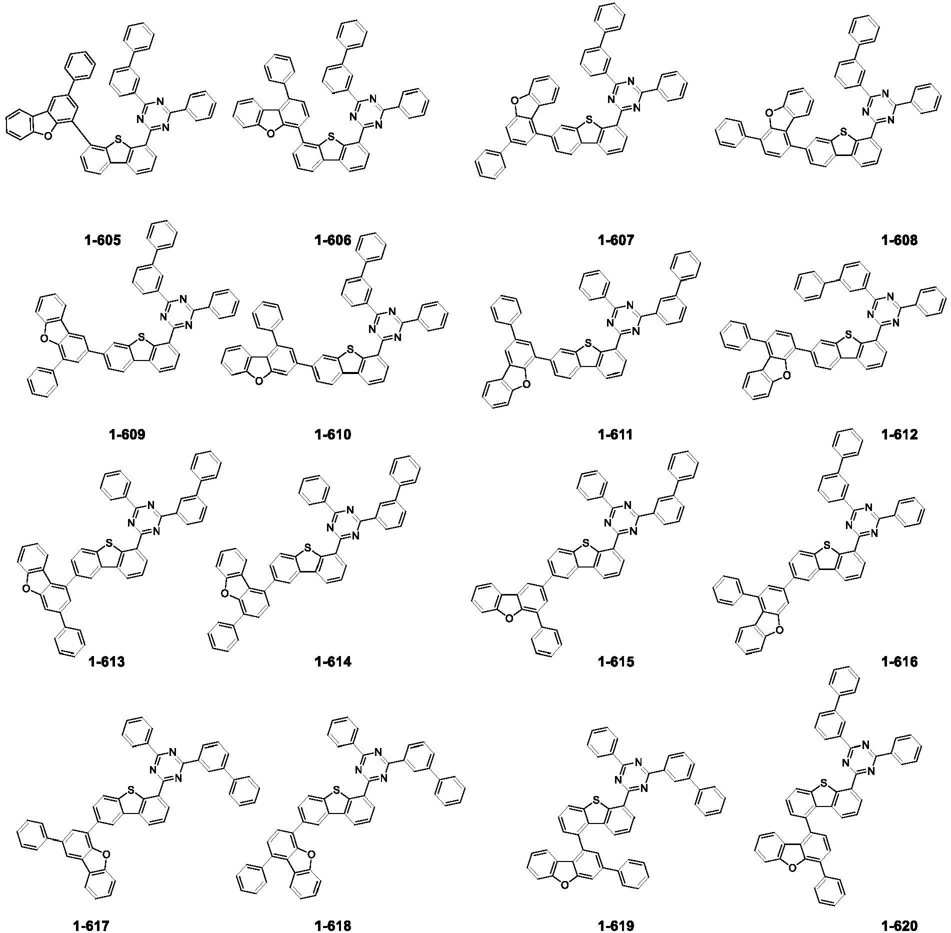

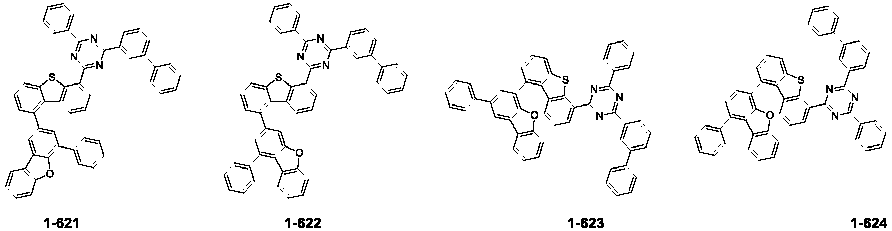

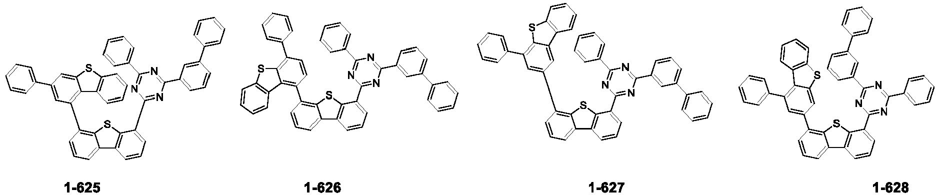

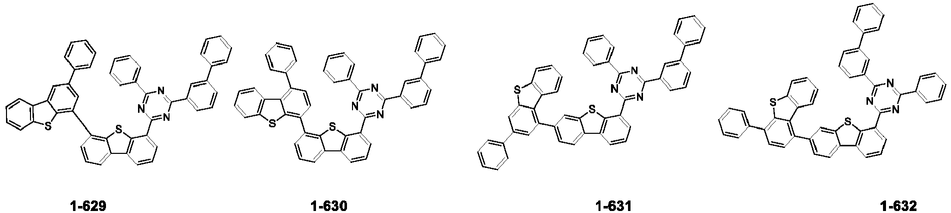

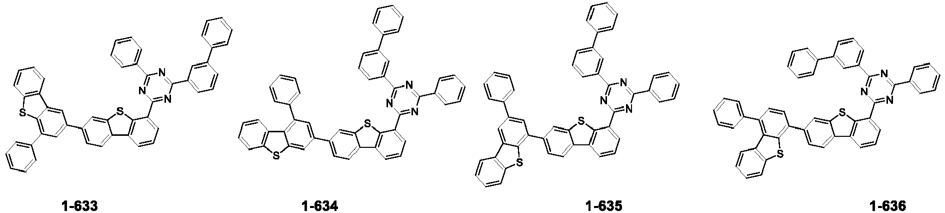

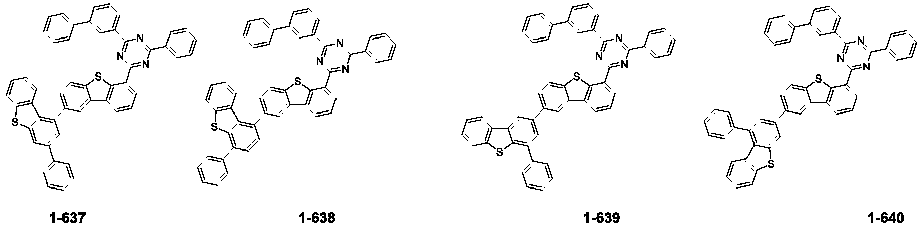

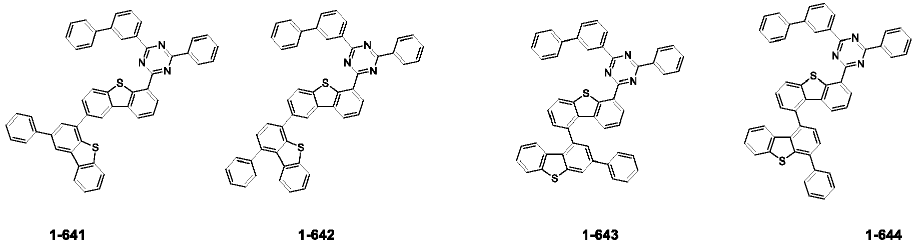

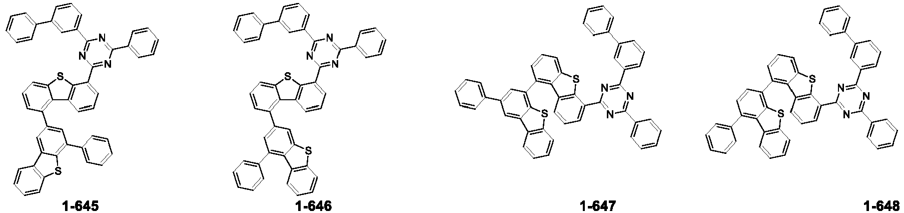

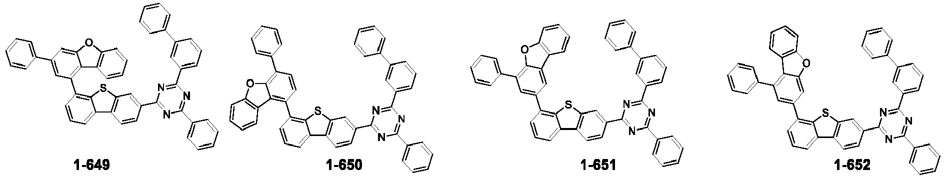

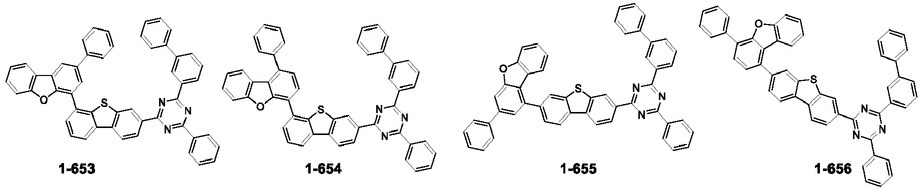

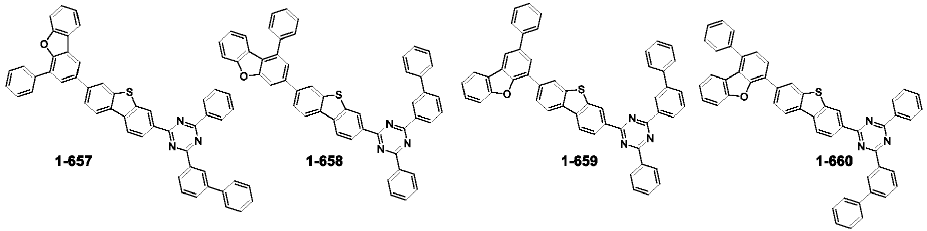

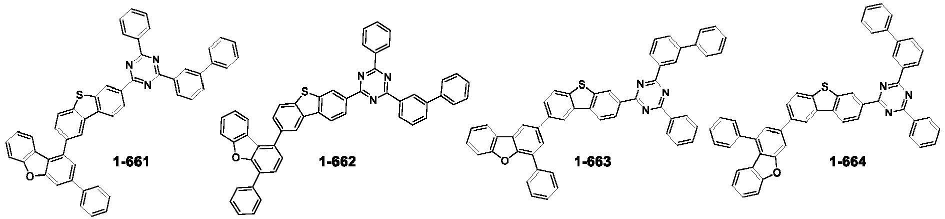

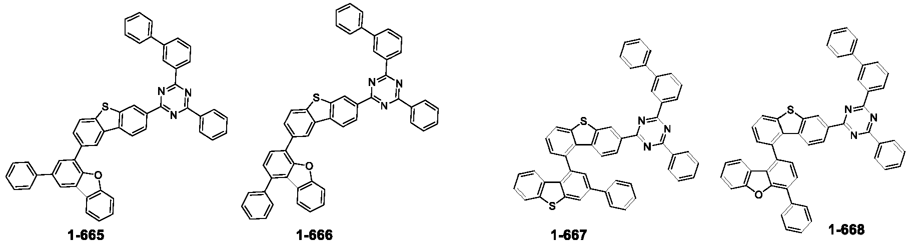

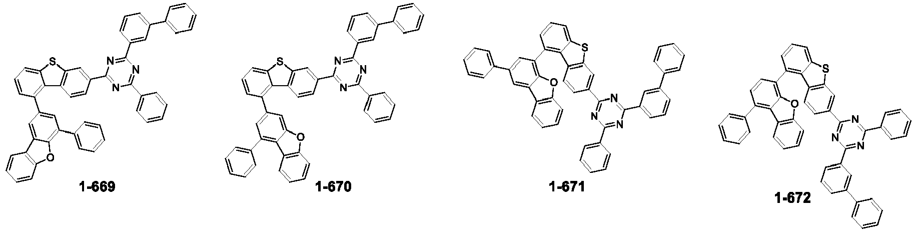

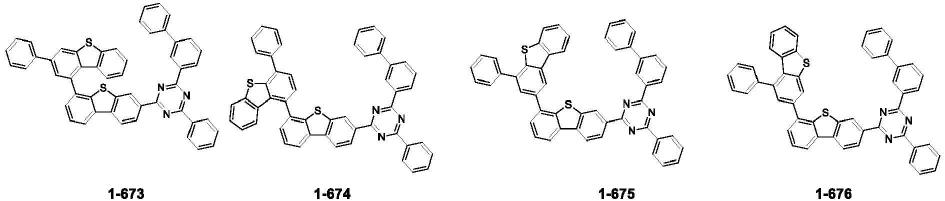

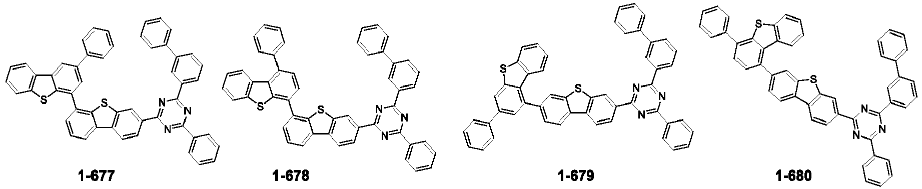

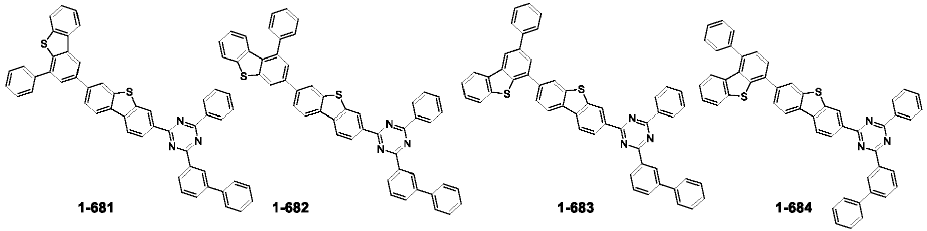

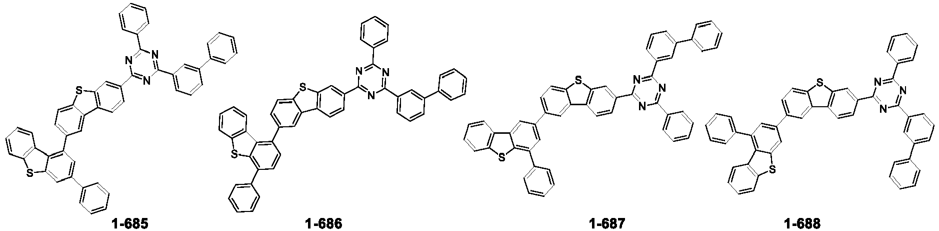

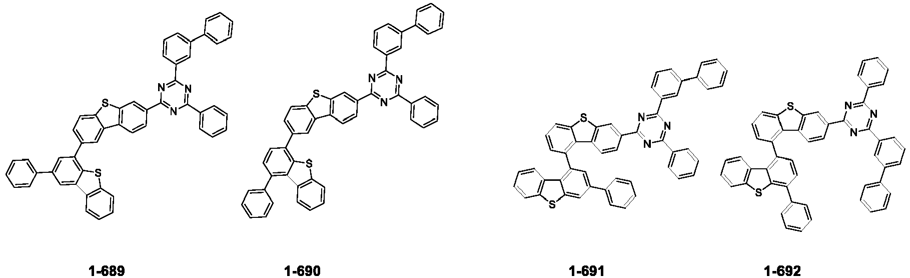

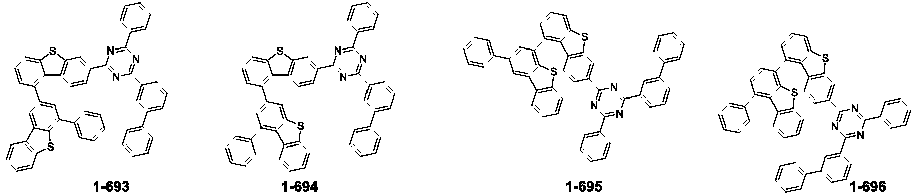

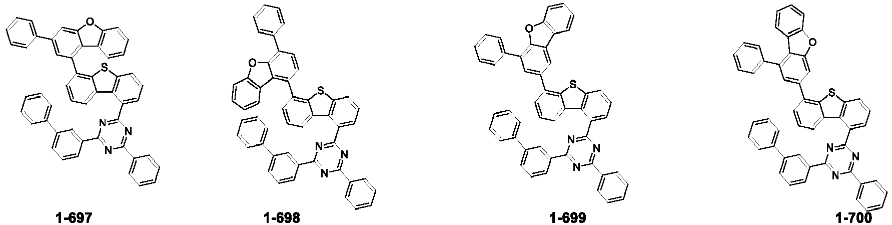

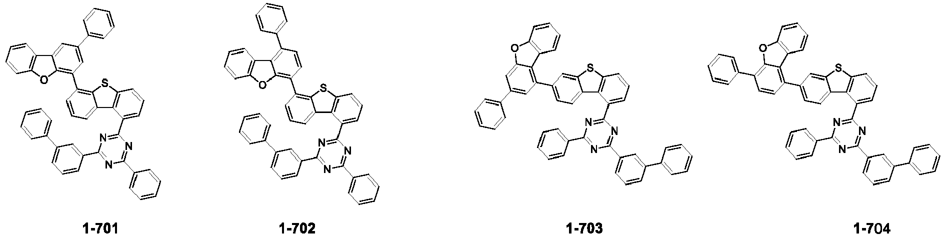

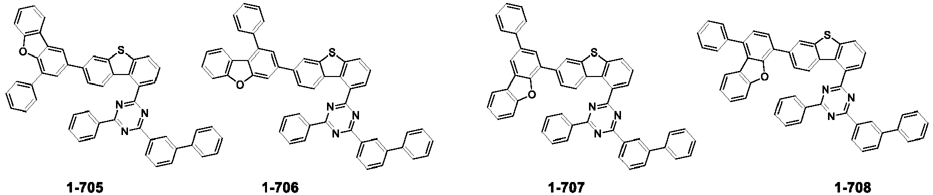

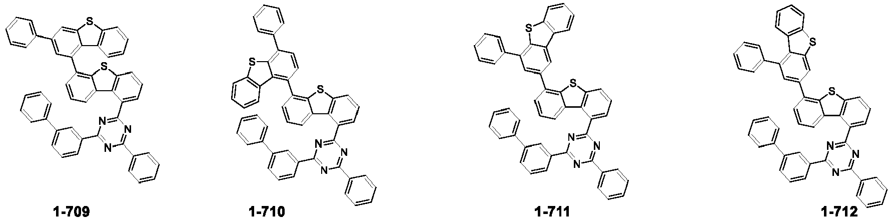

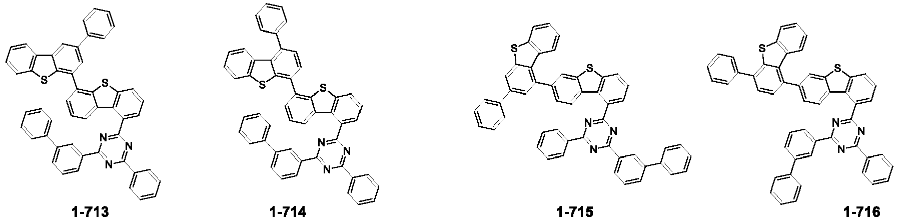

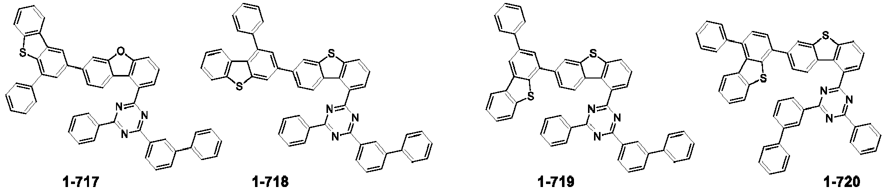

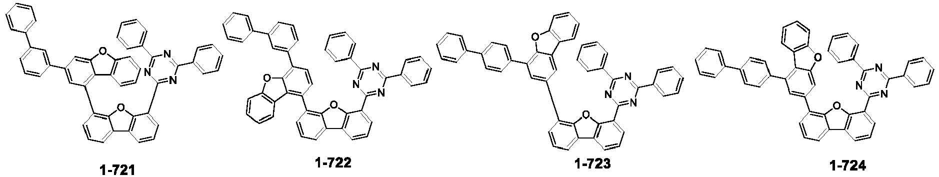

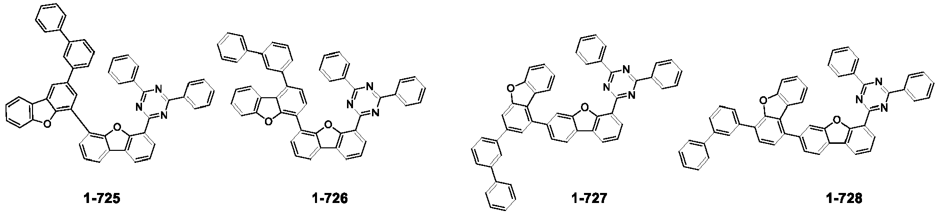

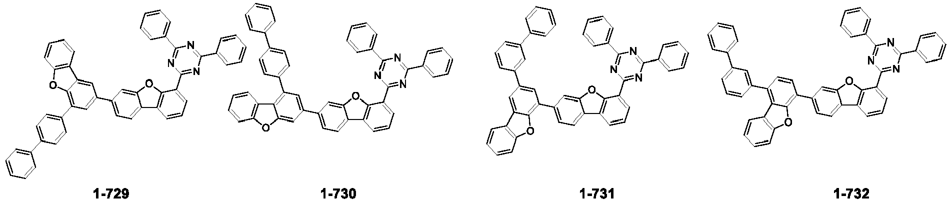

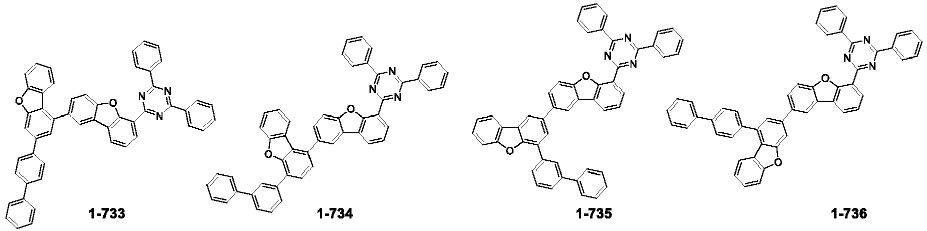

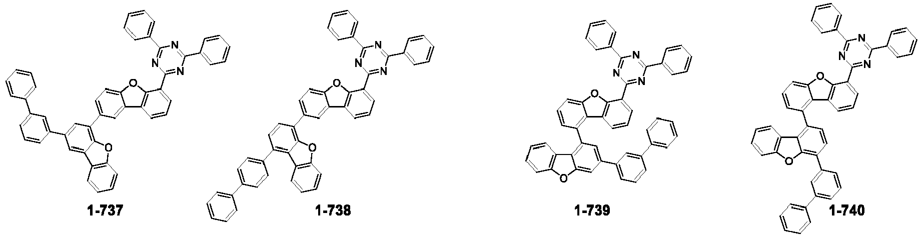

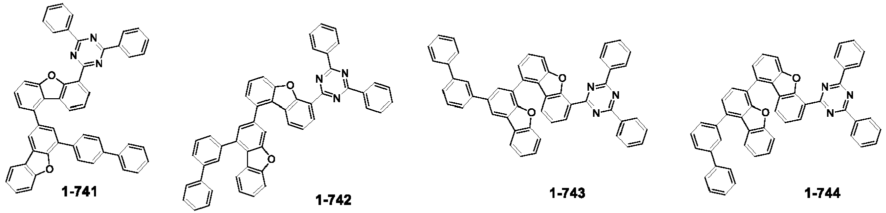

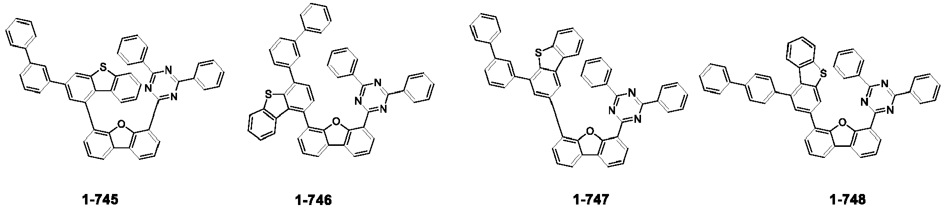

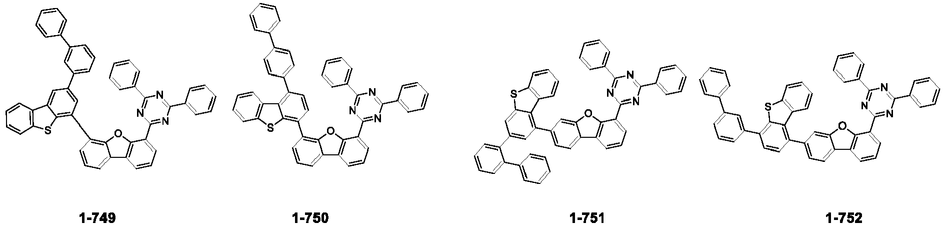

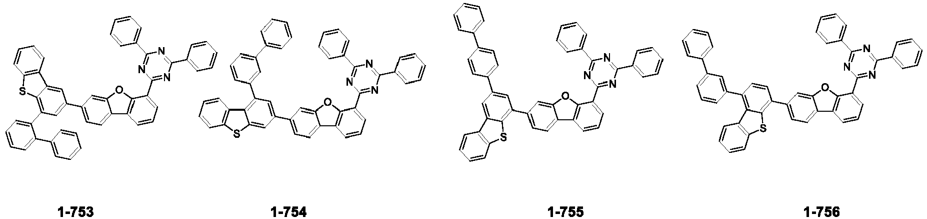

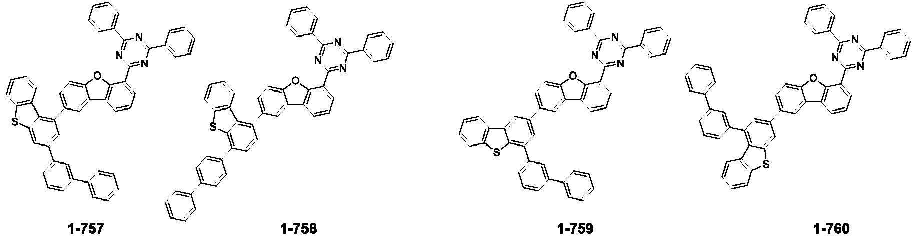

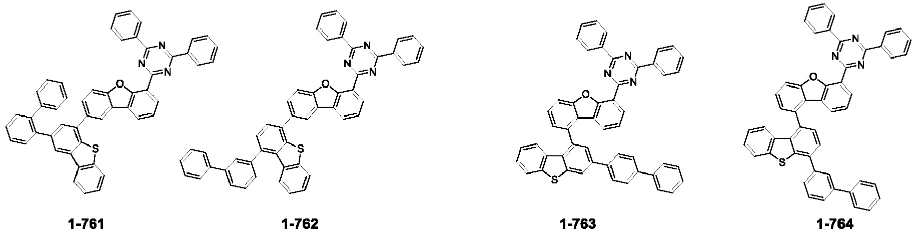

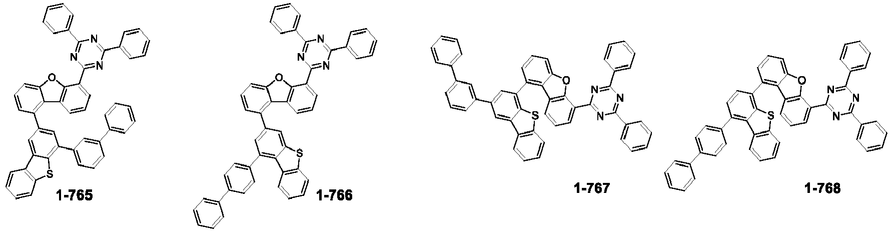

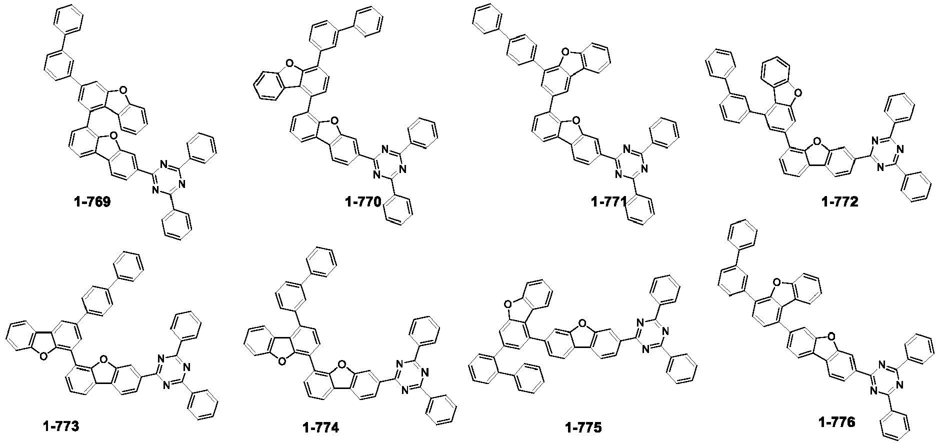

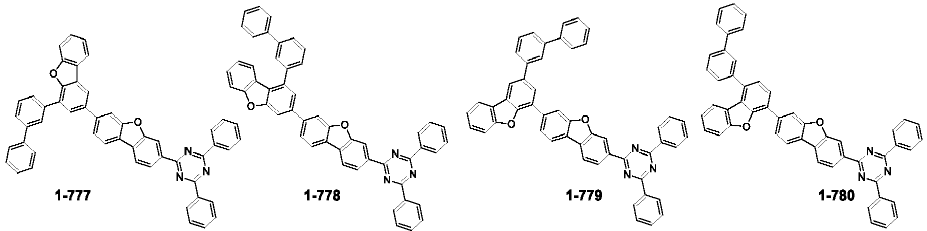

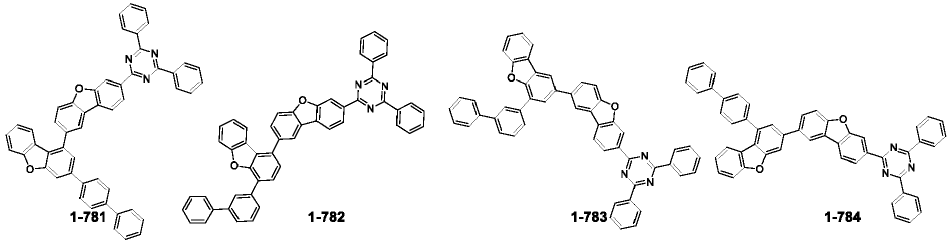

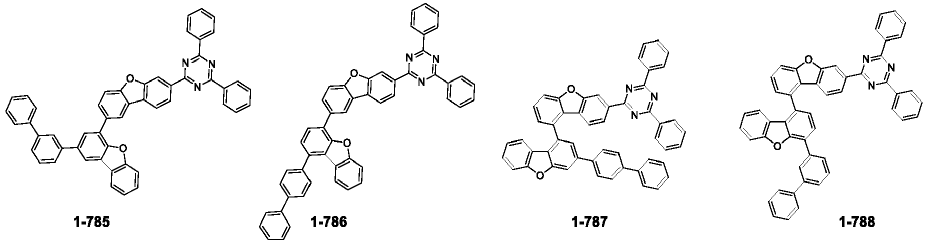

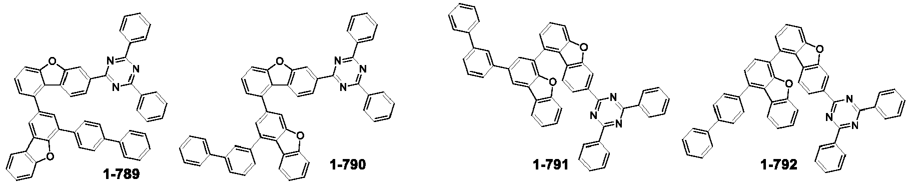

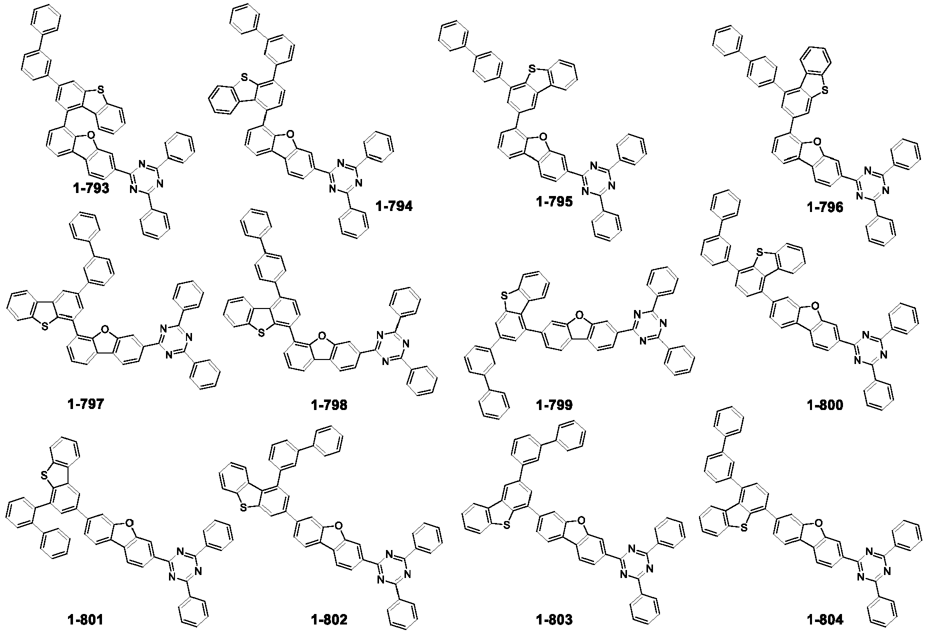

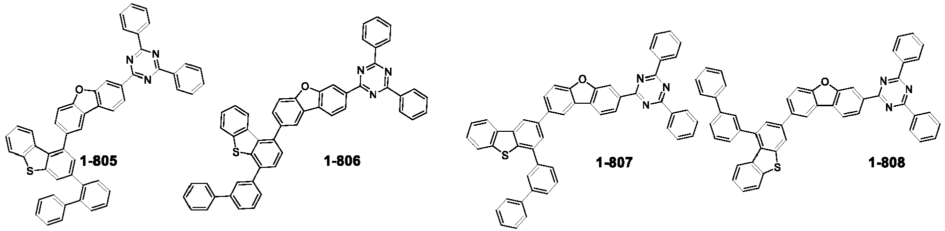

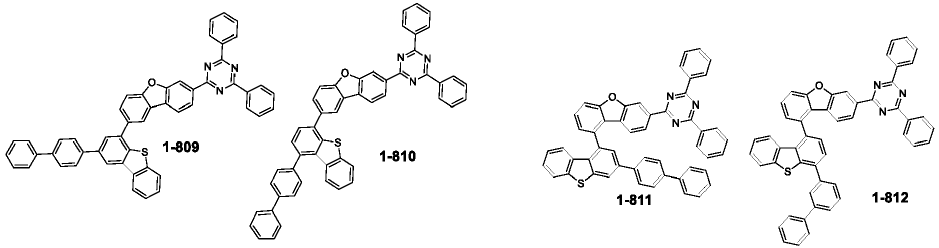

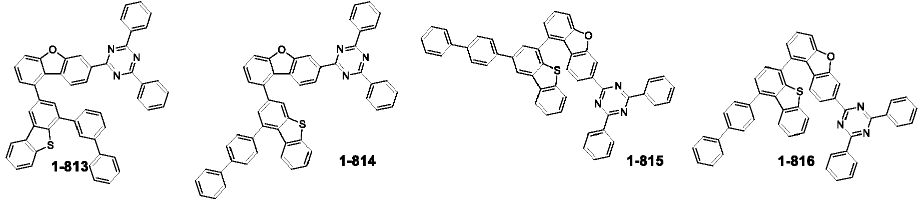

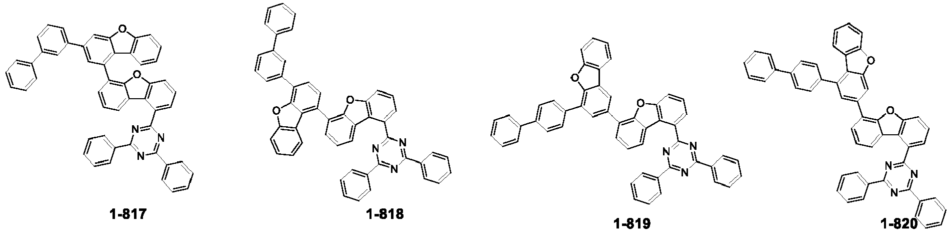

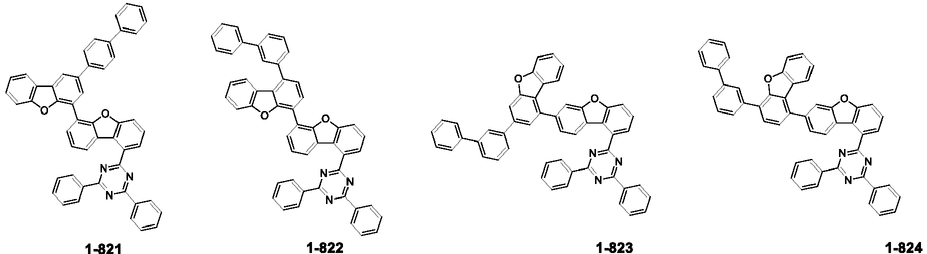

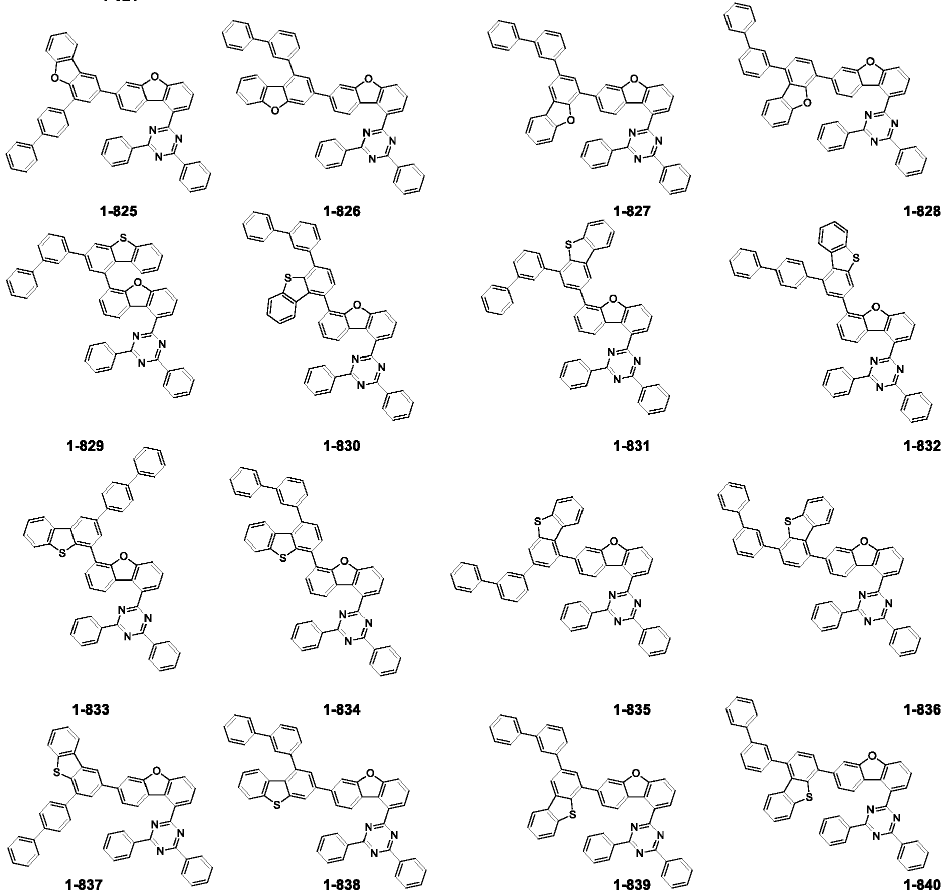

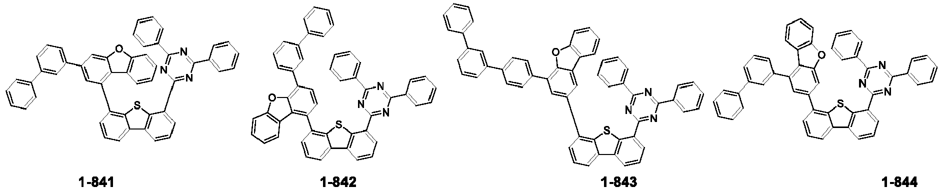

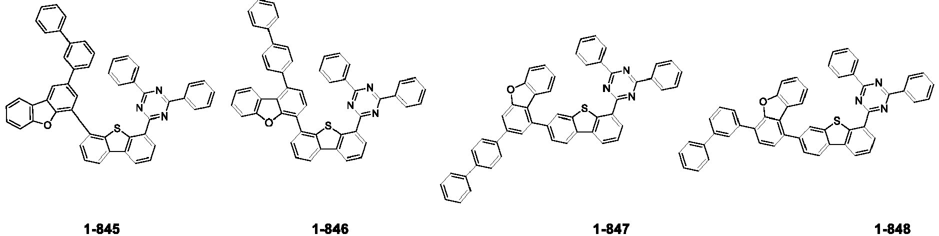

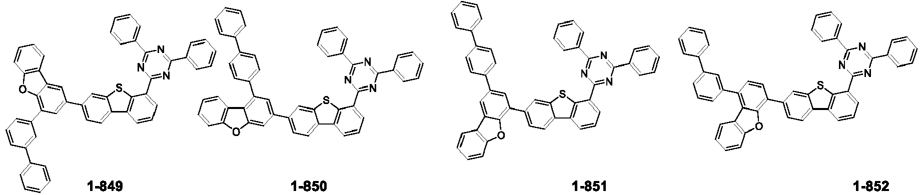

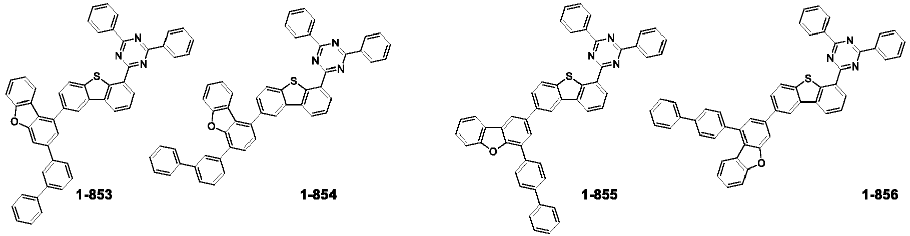

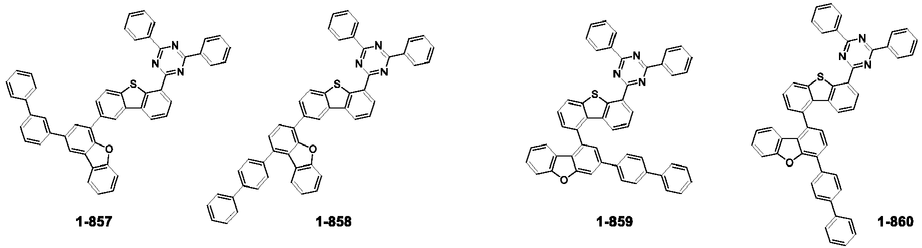

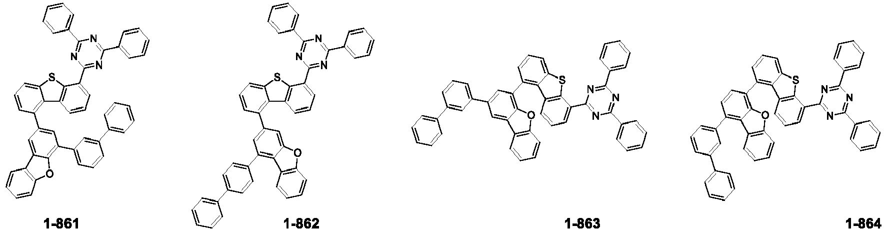

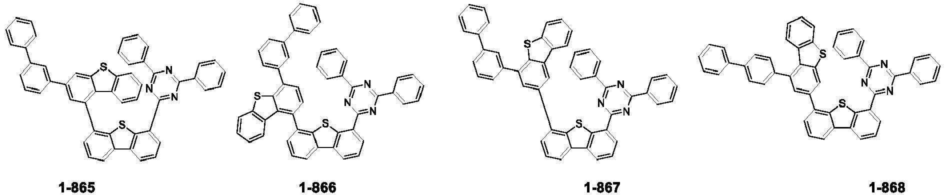

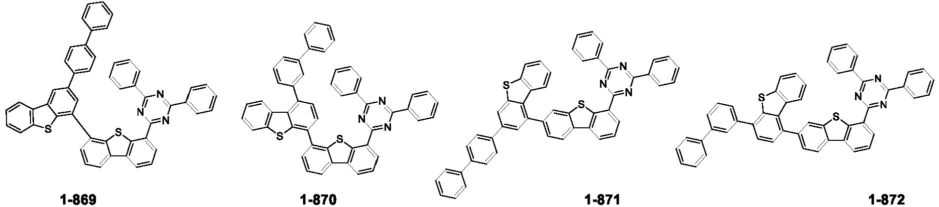

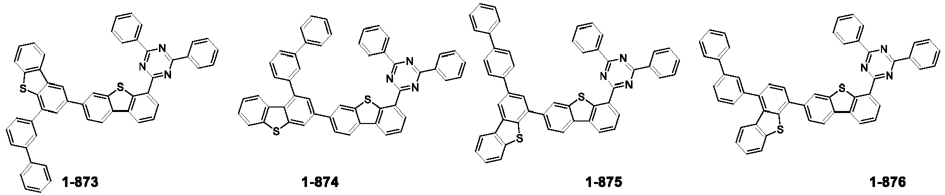

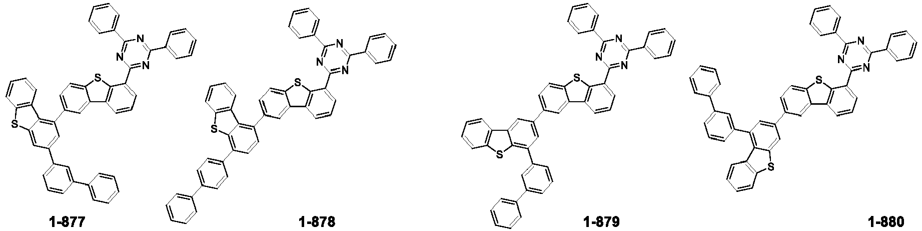

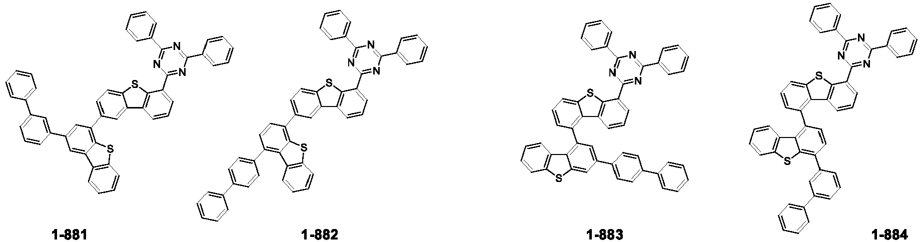

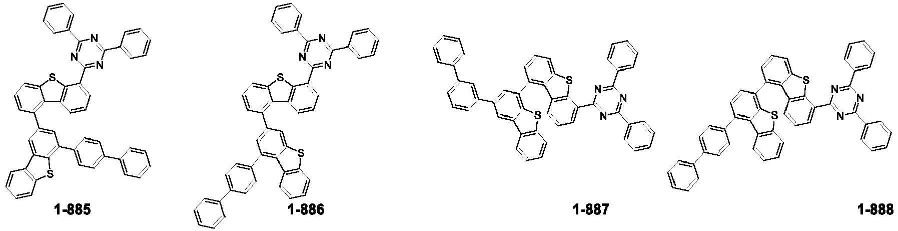

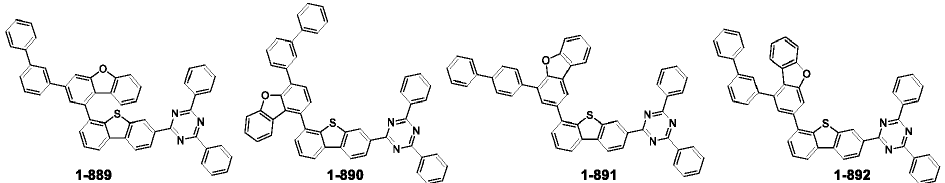

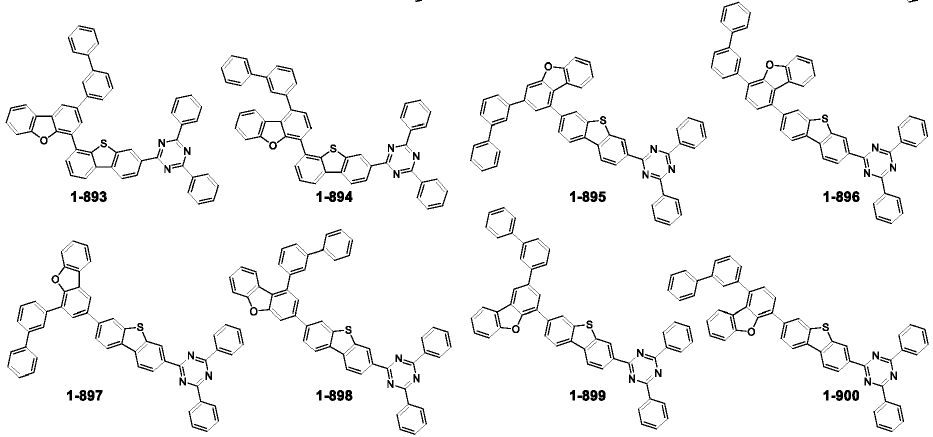

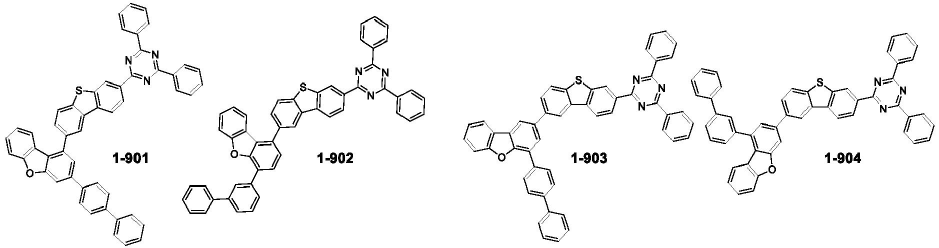

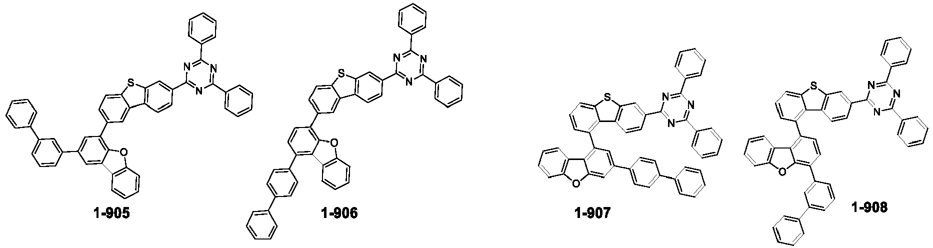

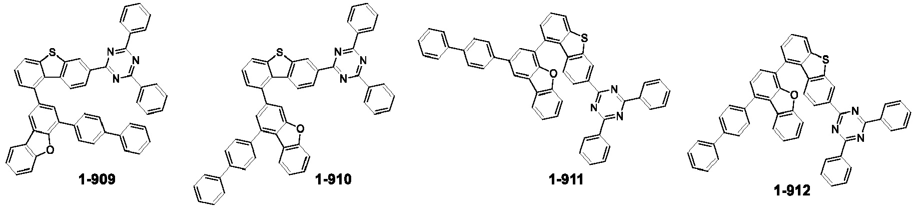

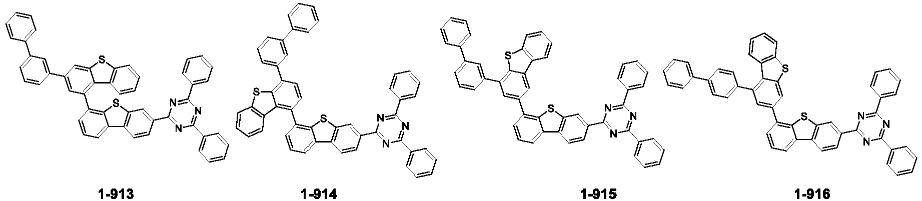

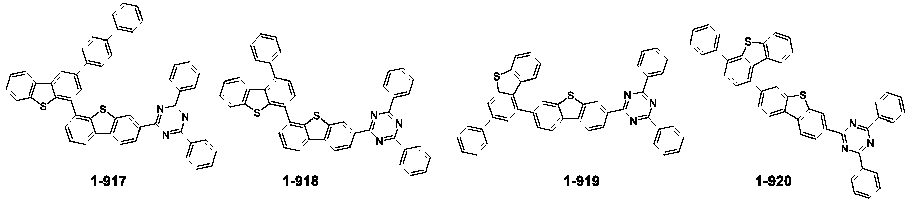

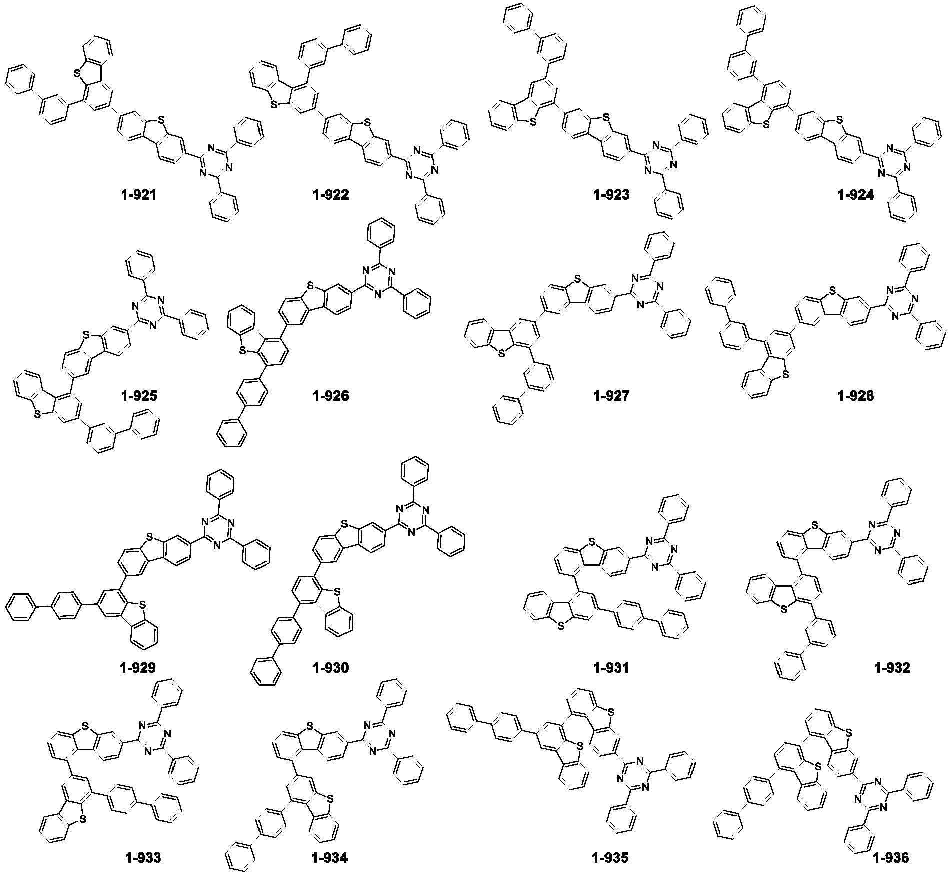

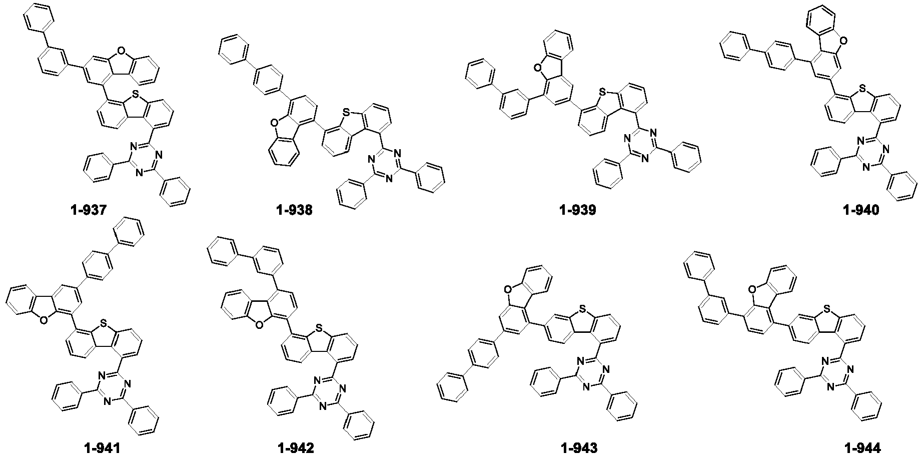

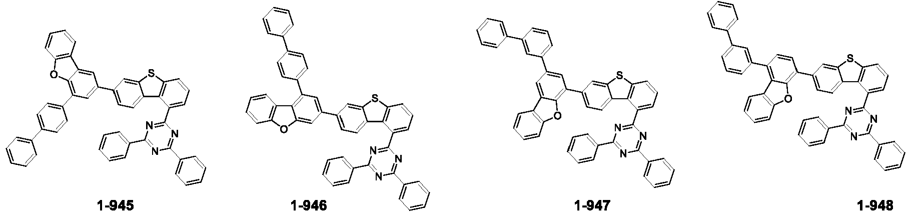

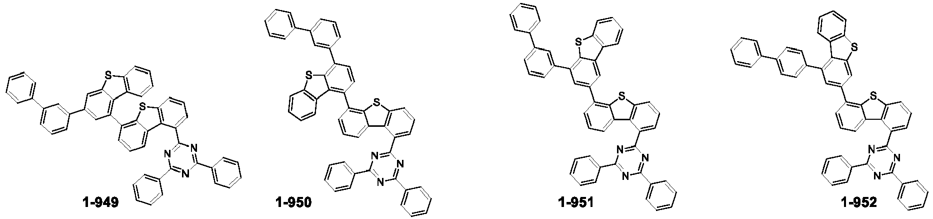

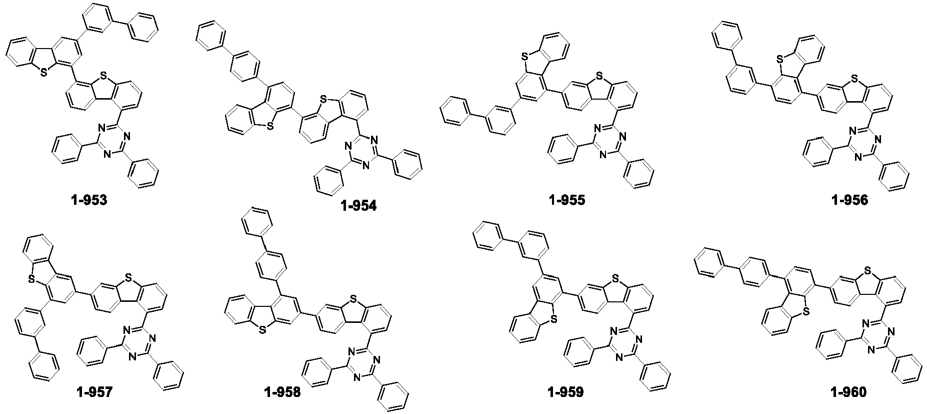

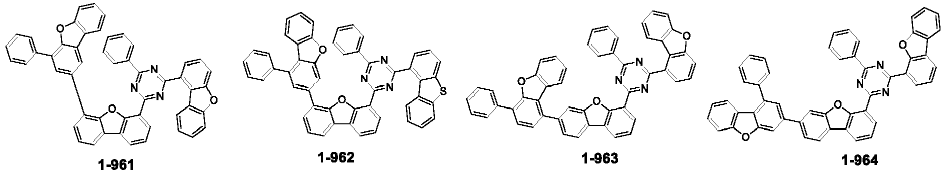

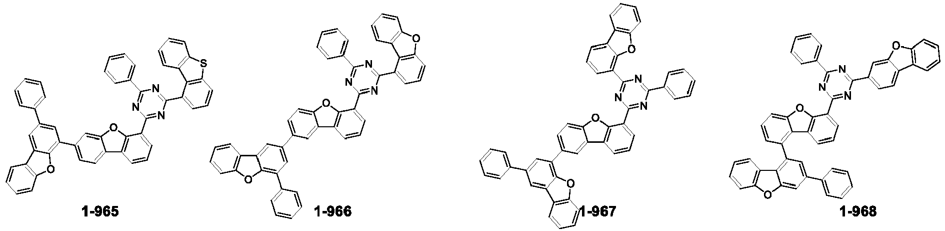

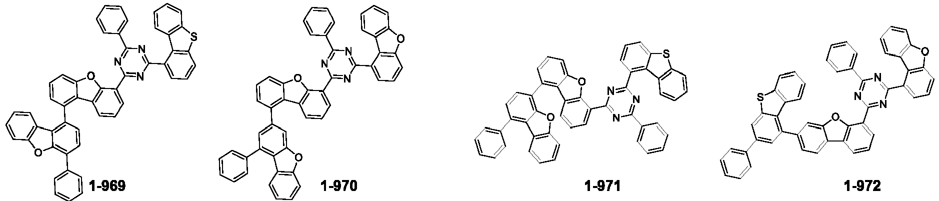

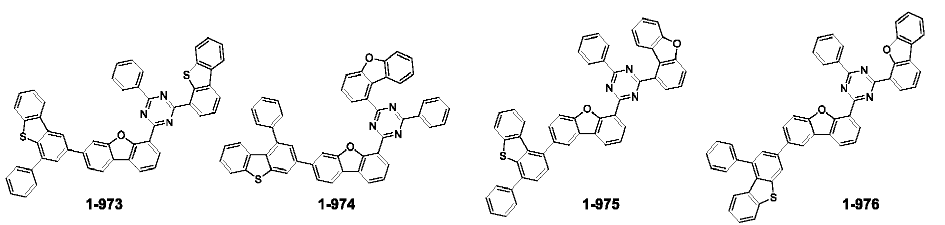

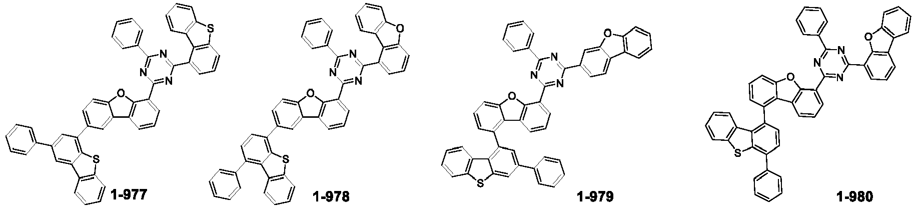

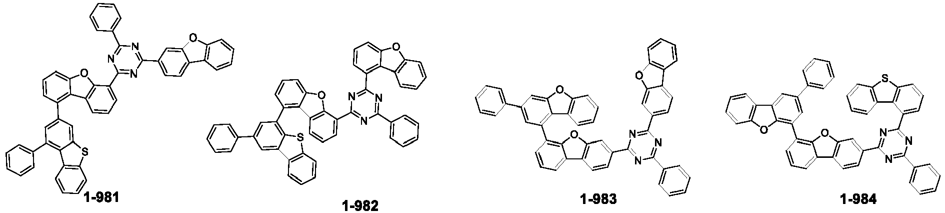

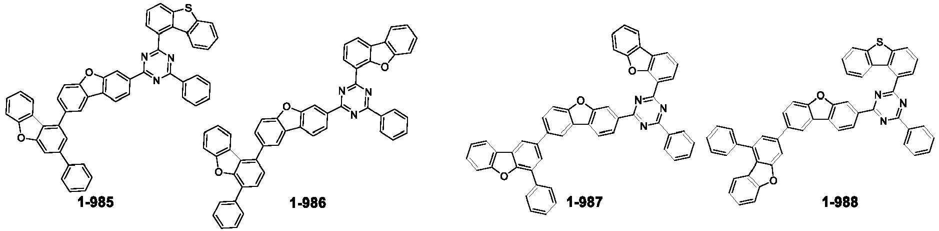

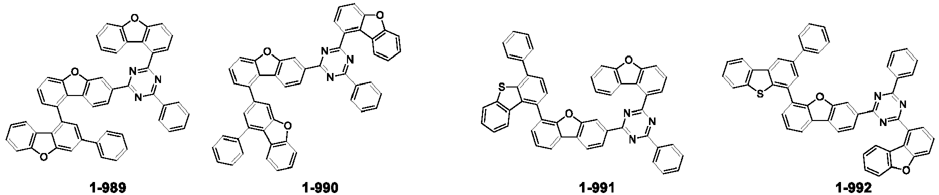

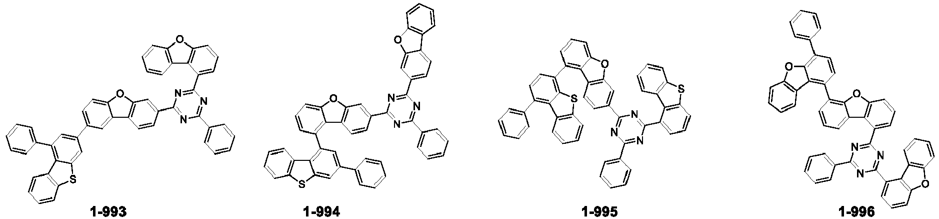

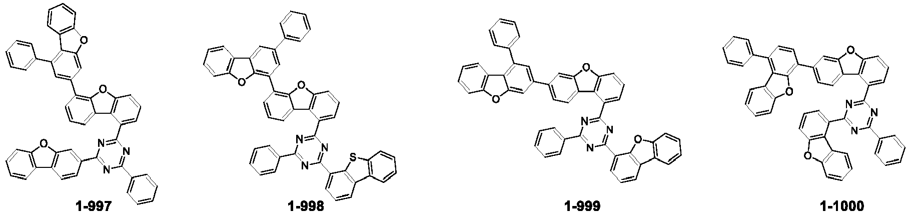

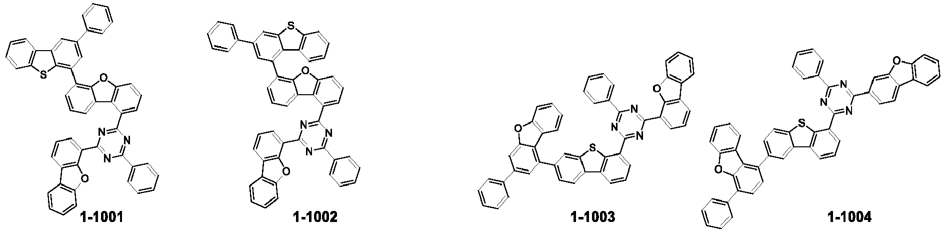

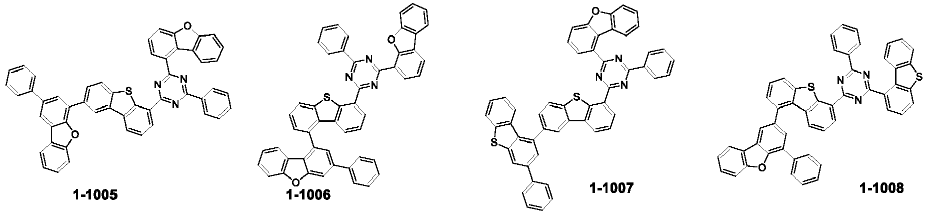

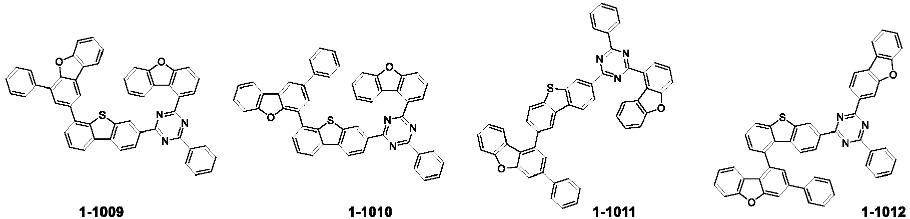

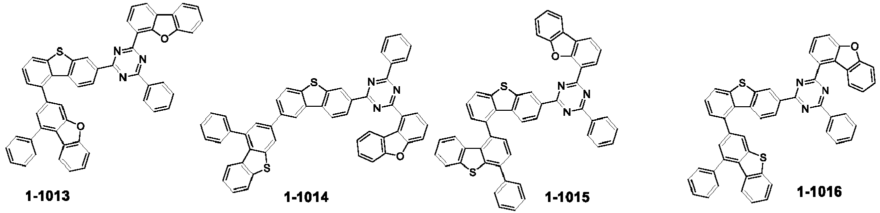

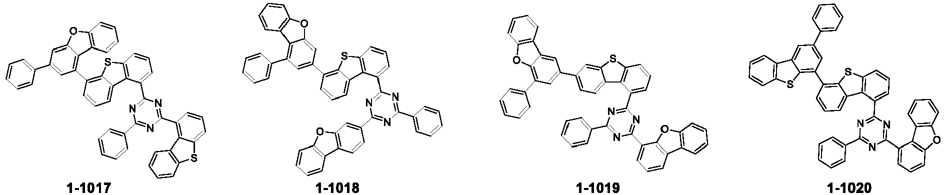

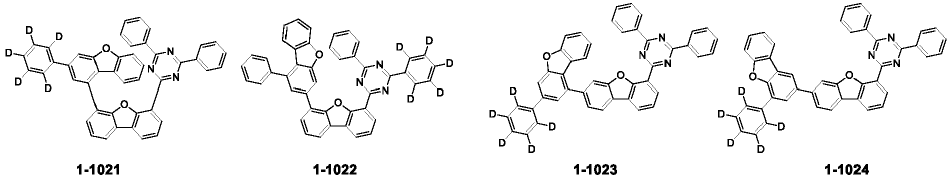

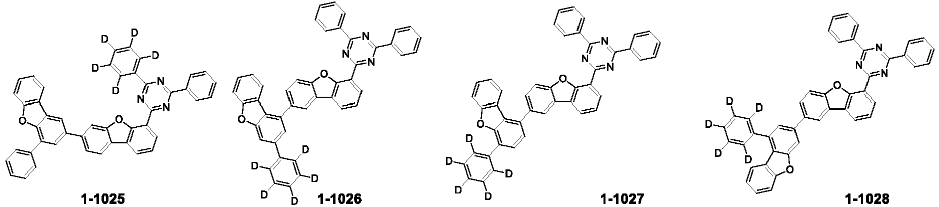

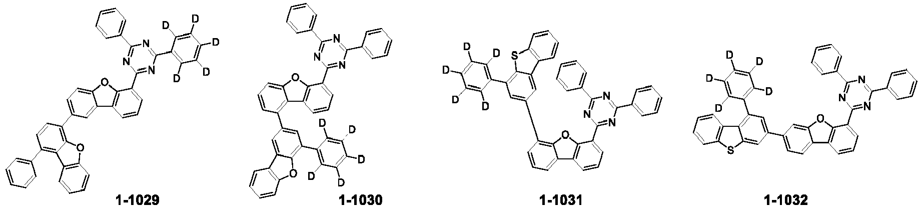

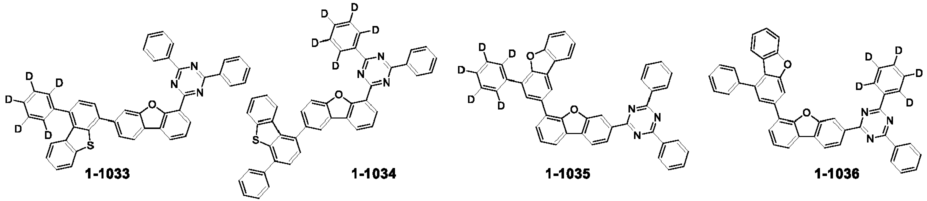

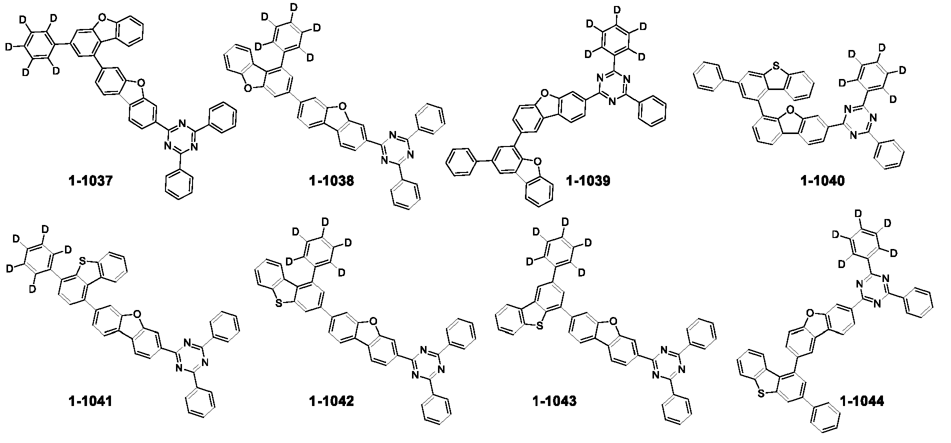

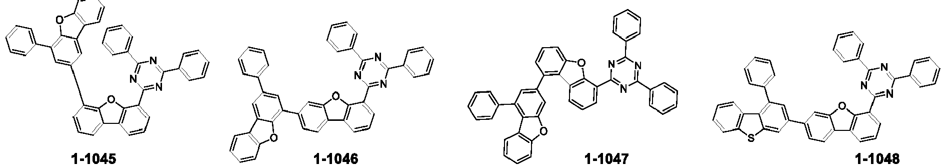

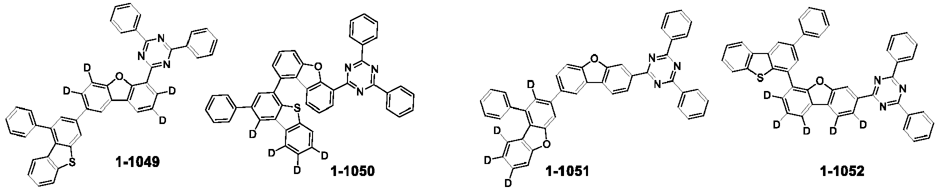

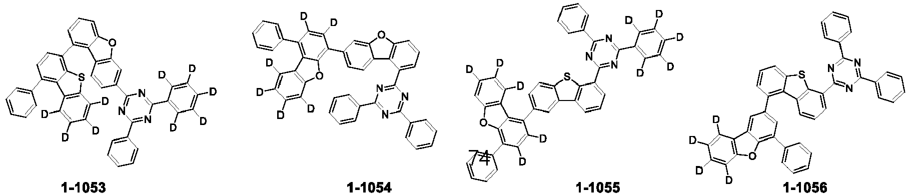

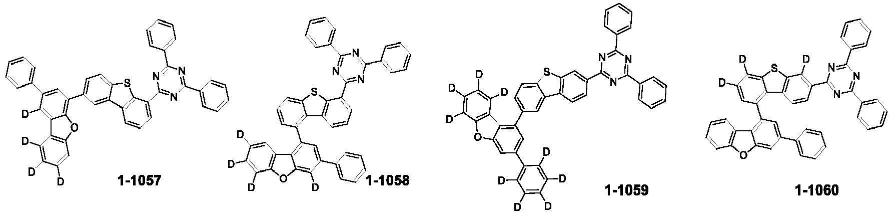

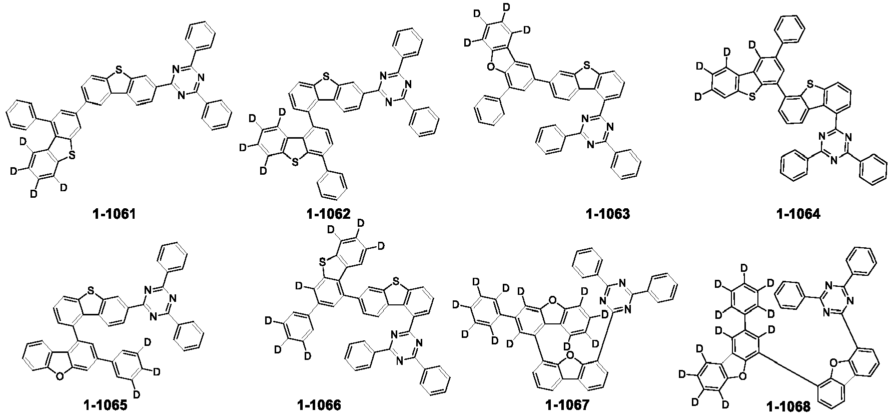

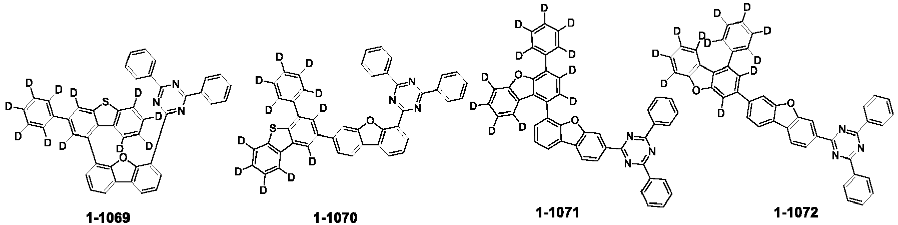

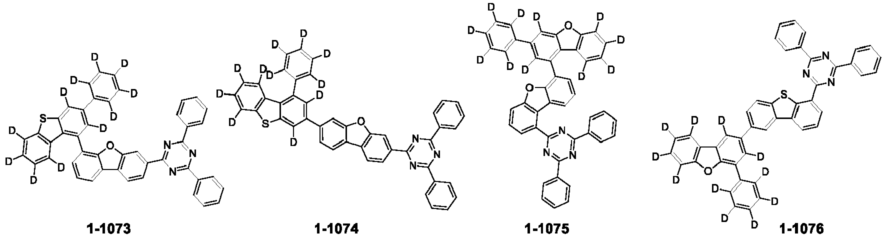

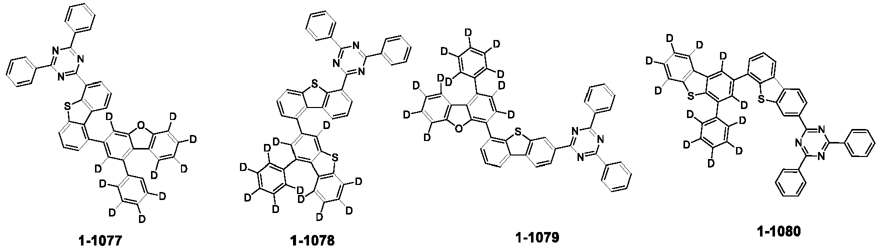

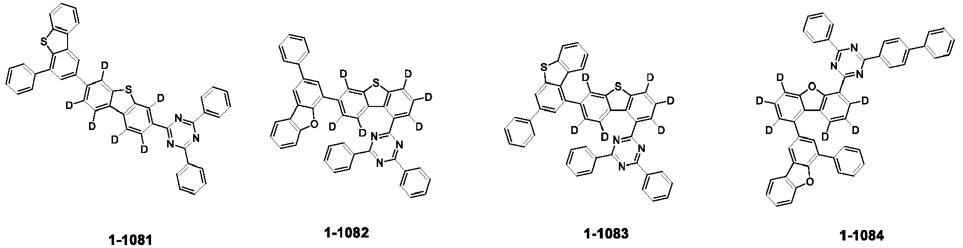

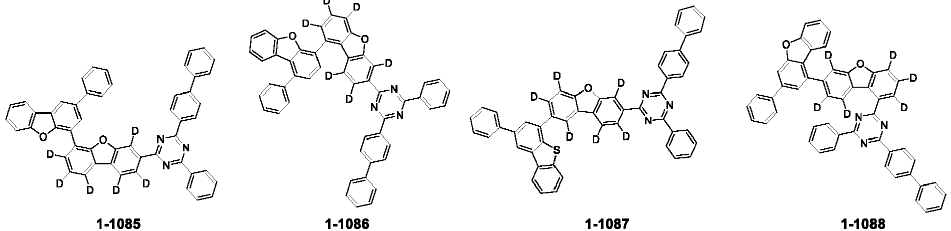

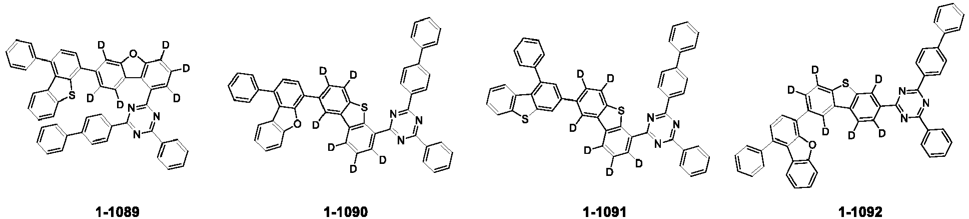

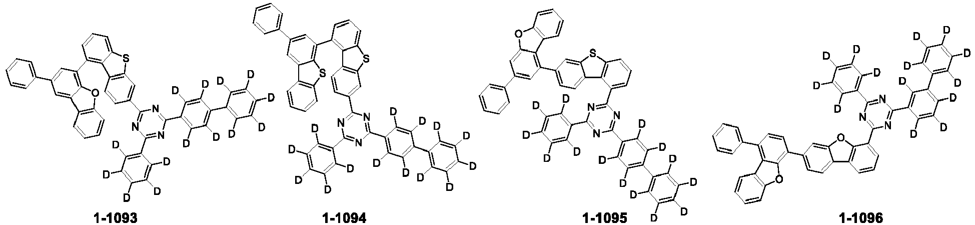

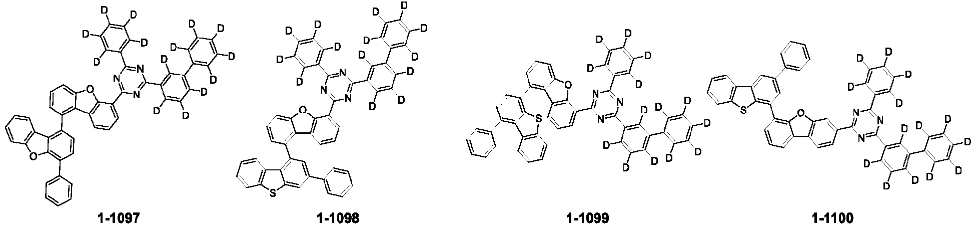

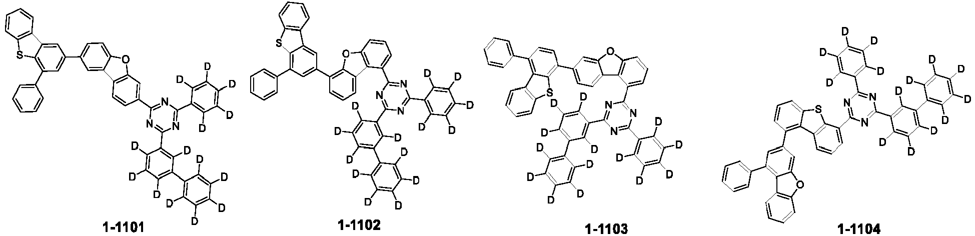

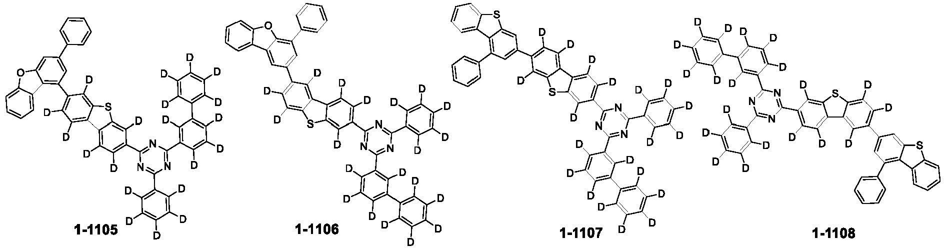

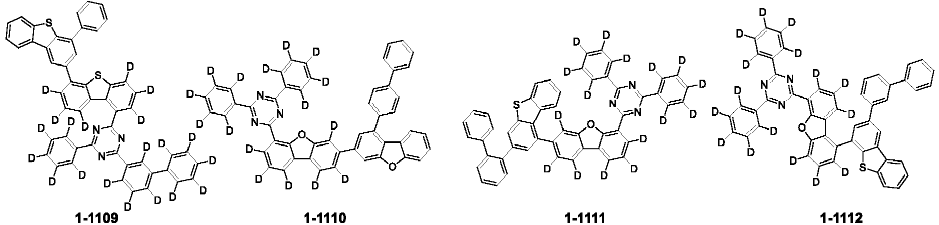

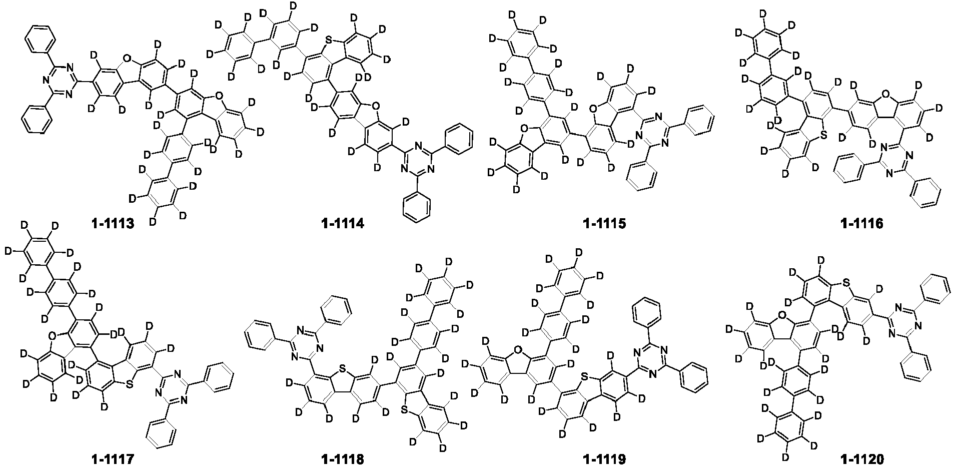

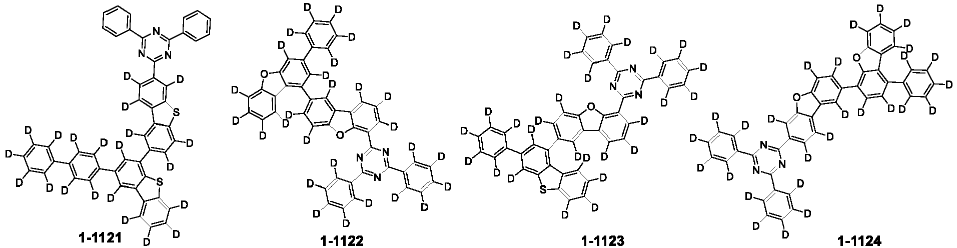

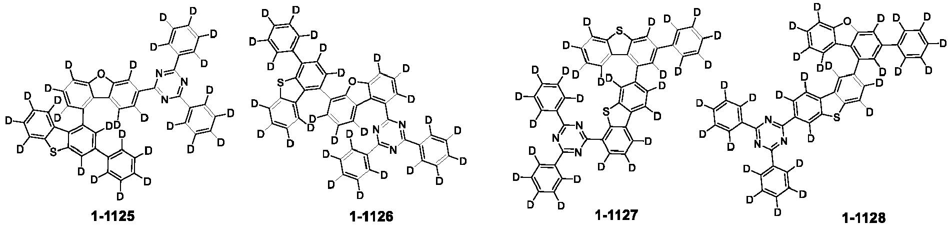

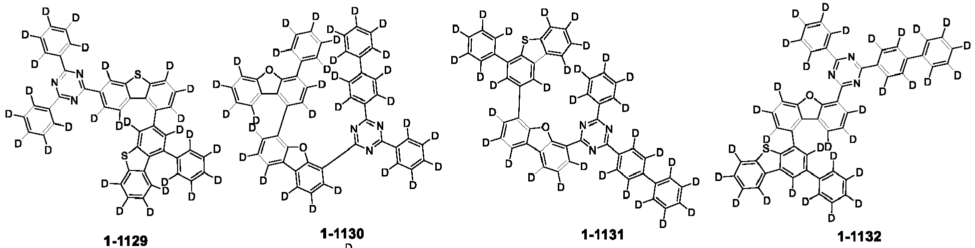

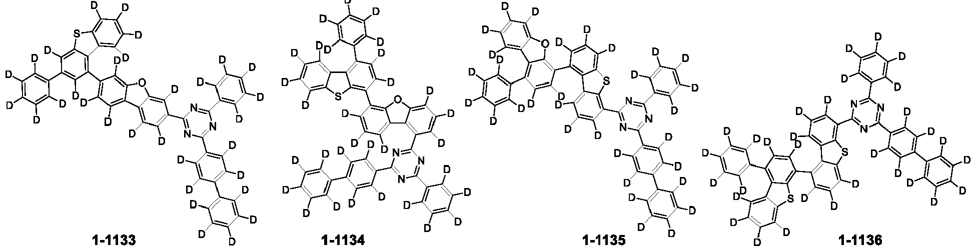

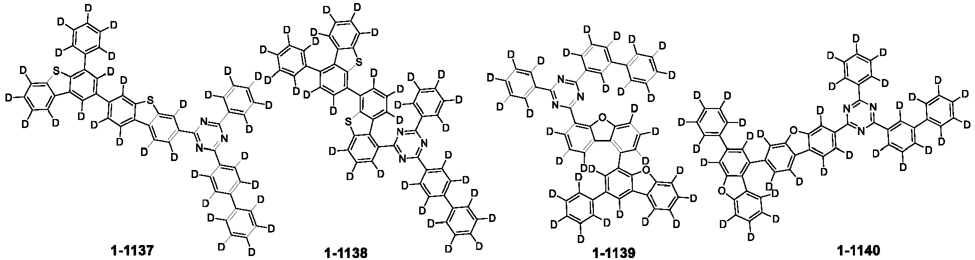

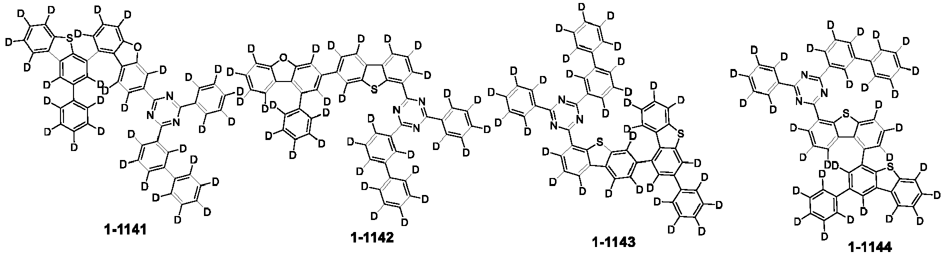

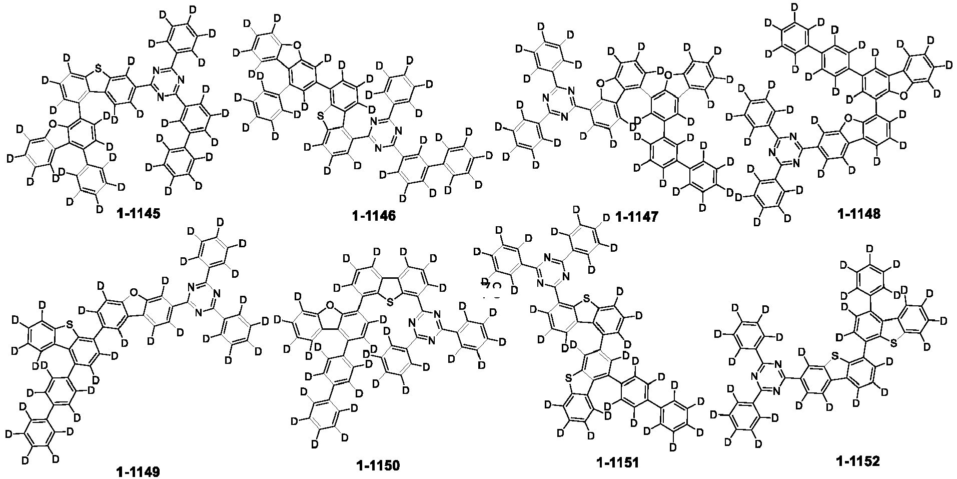

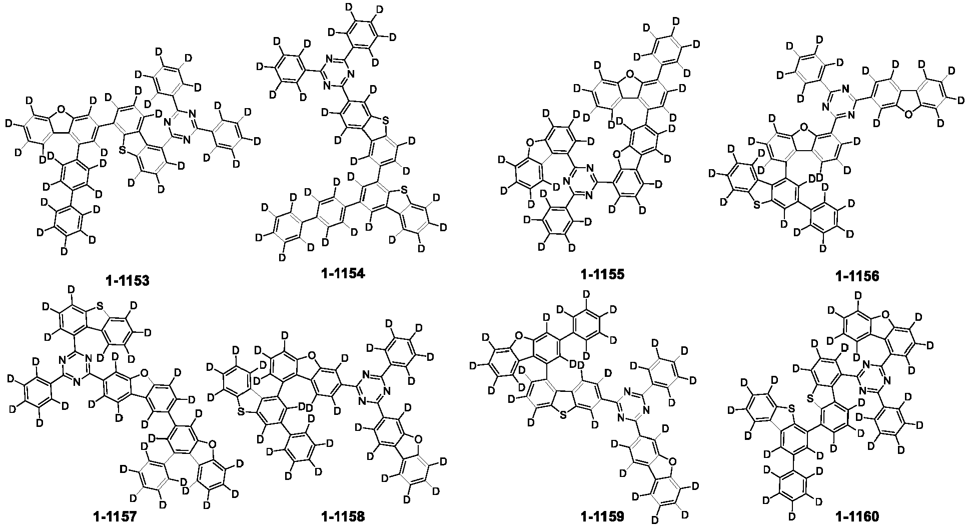

In addition, by introducing various substituents into the structure of Formula 1, compounds having intrinsic properties of the introduced substituents can be synthesized. For example, by introducing into the core structure a substituent mainly used in a material for a hole injection layer, a material for a hole transport layer, a material for a light emitting layer, a material for an electron transport layer, and a material for a charge generating layer used in manufacturing an organic light emitting device, substances that satisfy the requirements of each organic layer can be synthesized.

Also, by introducing various substituents into the structure of Formula 1, it is possible to finely control the energy bandgap, and on the other hand, to improve the properties at the interface between organic substances, and to diversify the use of the substances.

Also, in one embodiment of the present invention, the present invention provides an organic light emitting device comprising a first electrode; a second electrode provided to face the first electrode; and one or more organic layers provided between the first electrode and the second electrode, wherein at least one of the one or more organic layers comprises a heterocyclic compound represented by Formula 1 above.

In one embodiment of the present invention, the first electrode may be a positive electrode, and the second electrode may be a negative electrode.

In another embodiment, the first electrode may be a negative electrode, and the second electrode may be a positive electrode.

In one embodiment of the present invention, the organic light emitting device may be a blue organic light emitting device, and the heterocyclic compound represented by Formula 1 may be used as a material of the blue organic light emitting device.

In one embodiment of the present invention, the organic light emitting device may be a green organic light emitting device, and the heterocyclic compound represented by Formula 1 may be used as a material of the green organic light emitting device.

In one embodiment of the present invention, the organic light emitting device may be a red organic light emitting device, and the heterocyclic compound represented by Formula 1 may be used as a material of the red organic light emitting device.

In one embodiment of the present invention, the organic light emitting device may be a blue organic light emitting device, and the heterocyclic compound represented by Formula 1 may be used as a material for a light emitting layer of the blue organic light emitting device.

In one embodiment of the present invention, the organic light emitting device may be a green organic light emitting device, and the heterocyclic compound represented by Formula 1 may be used as a material for a light emitting layer of the green organic light emitting device.

In one embodiment of the present invention, the organic light emitting device may be a red organic light emitting device, and the heterocyclic compound represented by Formula 1 may be used as a material for a light emitting layer of the red organic light emitting device.

Specific details of the heterocyclic compound represented by Formula 1 are the same as described above.

The organic light emitting device of the present invention may be manufactured by a conventional method and material for manufacturing an organic light emitting device, except that one or more organic layer are formed using the aforementioned heterocyclic compound.

The heterocyclic compound may be formed as an organic layer by a solution coating method as well as a vacuum deposition method when manufacturing an organic light emitting device. In this case, the solution coating method means, but is not limited to, spin coating, dip coating, inkjet printing, screen printing, spraying, roll coating, and the like.

The organic layer of the organic light emitting device of the present invention may have a single-layer structure, and may also have a multi-layer structure formed by stacking two or more organic layers. For example, the organic light emitting device of the present invention may have a structure including a hole injection layer, a hole transport layer, a light emitting layer, an electron transport layer, an electron injection layer, etc. as an organic layer. However, the structure of the organic light emitting device is not limited thereto and may include a smaller number of organic layers.

In the organic light emitting device according to an embodiment of the present invention, an organic light emitting device is provided, wherein the organic layer including the heterocyclic compound represented by Formula 1 further includes a heterocyclic compound represented by Formula 2 below: 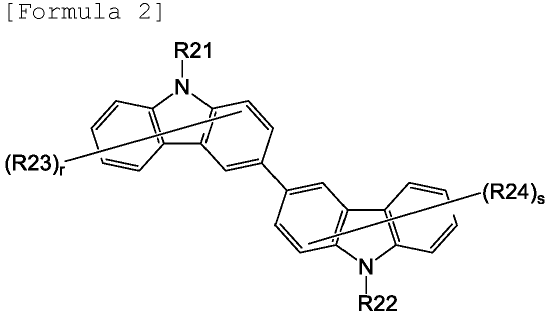 wherein,
R21 and R22 are the same as or different from each other and are each independently a substituted or unsubstituted C6 to C60 aryl group or a substituted or unsubstituted C2 to C60 heteroaryl group,
R23 and R24 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR201R202R203, - P(=O)R201R202; and an amine group substituted or unsubstituted by a substituted or unsubstituted C1 to C60 alkyl group, a substituted or unsubstituted C6 to C60 aryl group or a substituted or unsubstituted C2 to C60 heteroaryl group, or form a substituted or unsubstituted C6 to C60 aromatic hydrocarbon ring or a substituted or unsubstituted C2 to C60 heterocyclic ring by combining two or more groups adjacent to each other,
R201, R202, and R203 are the same as or different from each other and are each independently hydrogen, deuterium, a cyano group, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group,
r and s are integers from 0 to 7.

When the compound represented by Formula 1 and the compound represented by Formula 2 are included at the same time, better efficiency and lifetime effect are exhibited. From this, it can be expected that the exciplex phenomenon occurs when both compounds are included at the same time.

The exciplex phenomenon is a phenomenon in which energy having the size of the HOMO energy level of the donor (p-host) and the LUMO energy level of the acceptor (n-host) is released through electron exchange between two molecules. If the exciplex phenomenon occurs between two molecules, Reverse Intersystem Crossing (RISC) occurs, and thus the internal quantum efficiency of fluorescence can be increased up to 100%. If a donor (p-host) with good hole transport ability and an acceptor (n-host) with good electron transport ability are used as hosts for the light emitting layer, since holes are injected into the p-host and electrons are injected into the n-host, the operating voltage can be lowered, thereby helping to improve lifetime. That is, if the compound represented by Formula 2 is used as the donor and the compound represented by Formula 1 is used as the acceptor, excellent device characteristics are exhibited.

In the organic light emitting device according to an embodiment of the present invention, r and s may be 0.

In the organic light emitting device according to an embodiment of the present invention, R23 and R24 may be hydrogen.

In the organic light emitting device according to an embodiment of the present invention, R21 and R22 are the same as or different from each other and may each independently be a substituted or unsubstituted C6 to C60 aryl group; or a substituted or unsubstituted C2 to C60 heteroaryl group.

In an organic light emitting device according to another embodiment, R21 and R22 are the same as or different from each other and may each independently be a substituted or unsubstituted C6 to C40 aryl group; or a substituted or unsubstituted C6 to C40 heteroaryl group.

In an organic light emitting device according to another embodiment, R21 and R22 are the same as or different from each other and may each independently be a C6 to C40 aryl group substituted or unsubstituted with one or more substituents selected from the group consisting of a C1 to C40 alkyl group, a C6 to C40 aryl group, a cyano group, and -SiR201R202R203; or a C2 to C40 heteroaryl group substituted or unsubstituted with one or more substituents selected from the group consisting of a C6 to C40 aryl group and a C2 to C40 heteroaryl group.

In an organic light emitting device according to another embodiment, R21 and R22 are the same as or different from each other and may each independently be a C6 to C40 aryl group substituted or unsubstituted with one or more substituents selected from the group consisting of a C1 to C40 alkyl group, a C6 to C40 aryl group, a cyano group, and -SiR201R202R203.

In an organic light emitting device according to another embodiment, R21 and R22 are the same as or different from each other and may each independently be a phenyl group substituted or unsubstituted with a phenylgroup, a cyano group or -SiR201R202R203; a biphenyl group substituted or unsubstituted with a phenyl group; a naphthyl group; a fluorenyl group unsubstituted or substituted with a methyl group or a phenyl group; a spirobifluorenyl group; or a triphenylenyl group.

In the organic light emitting device according to an embodiment of the present invention, 201, R202, and R203 may be a phenyl group.

In the organic light emitting device according to an embodiment of the present invention, Formula 2 may be a heterocyclic compound represented by Formula 2-1 below: 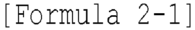 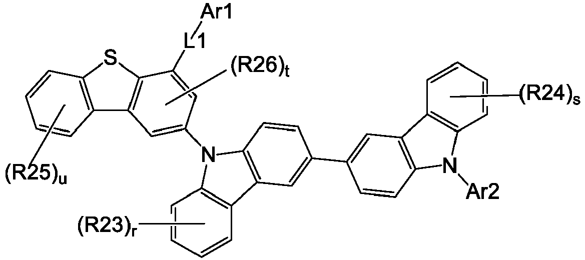 wherein,
R25 and R26 are the same as or different from each other and are each independently selected from the group consisting of hydrogen, deuterium, a substituted or unsubstituted C1 to C60 alkyl group and a substituted or unsubstituted C2 to C60 heteroaryl group, t is an integer from 0 to 2, u is an integer from 0 to 4,
L1 is a single bond, a substituted or unsubstituted C1 to C60 arylenyl group, or a substituted or unsubstituted C2 to C60 heteroarylene group,
Ar1 and Ar2 are the same as or different from each other and are each independently a substituted or unsubstituted C1 to C60 alkyl group or a substituted or unsubstituted C2 to C60 heteroaryl group, and
the definitions of R23, R24, r and s are the same as those in Formula 2 above.

In one embodiment of the present invention, the compound represented by Formula 2 may include at least one deuterium as a substituent.

In another embodiment of the present invention, the content of deuterium may be 0% or more, 1% or more, 10% or more, 20% or more, 30% or more, 40% or more, or 50% or more, and 100% or less, 90% or less, 80% or less, 70% or less, or 60% or less, based on the total number of hydrogen atoms and deuterium atoms in Formula 2.

In another embodiment of the present invention, the content of deuterium may be 20% to 90%, based on the total number of hydrogen atoms and deuterium atoms in Formula 2.

In another embodiment of the present invention, the content of deuterium may be 30% to 80%, based on the total number of hydrogen atoms and deuterium atoms in Formula 2.

In another embodiment of the present invention, the content of deuterium may be 50% to 60%, based on the total number of hydrogen atoms and deuterium atoms in Formula 2.

In one embodiment of the present invention, the heterocyclic compound represented by Formula 2 may be at least one selected from compounds below:

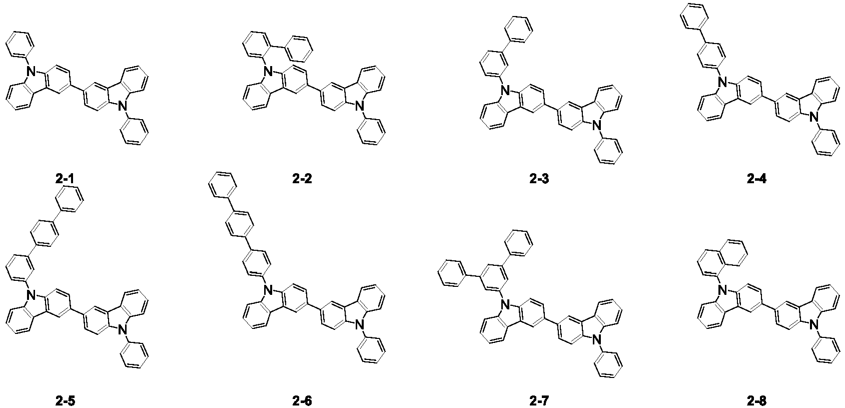

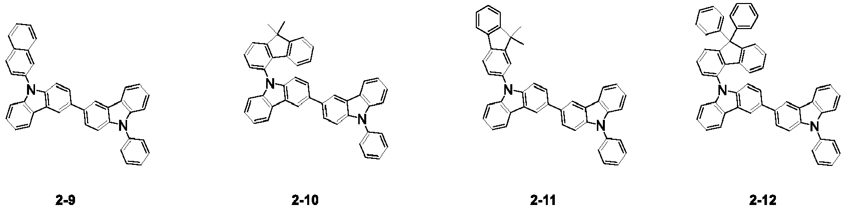

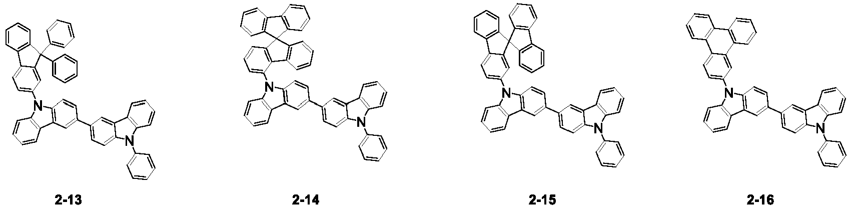

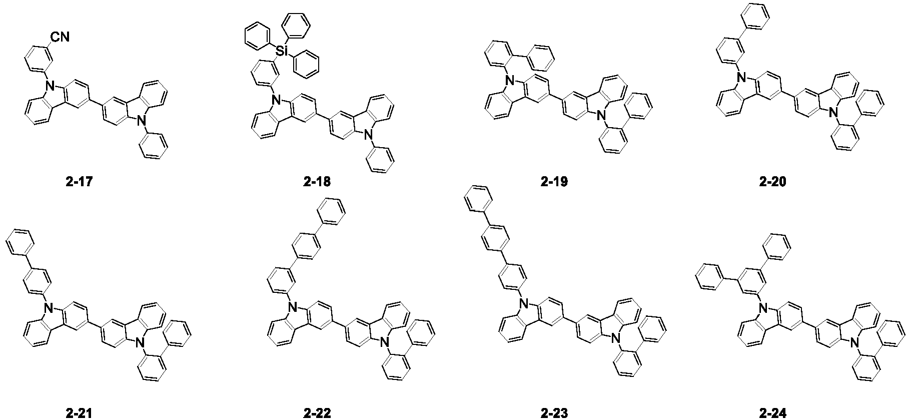

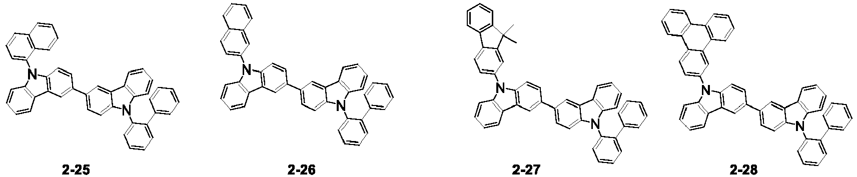

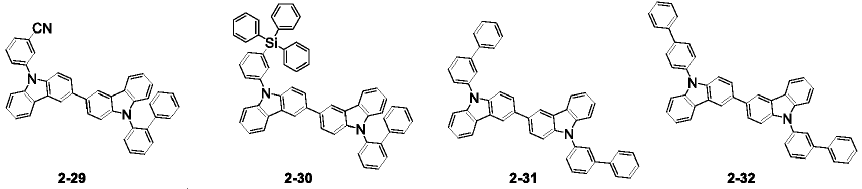

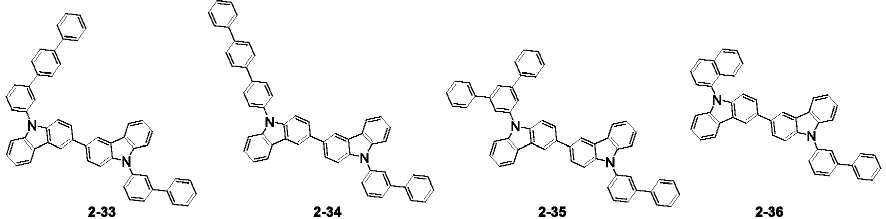

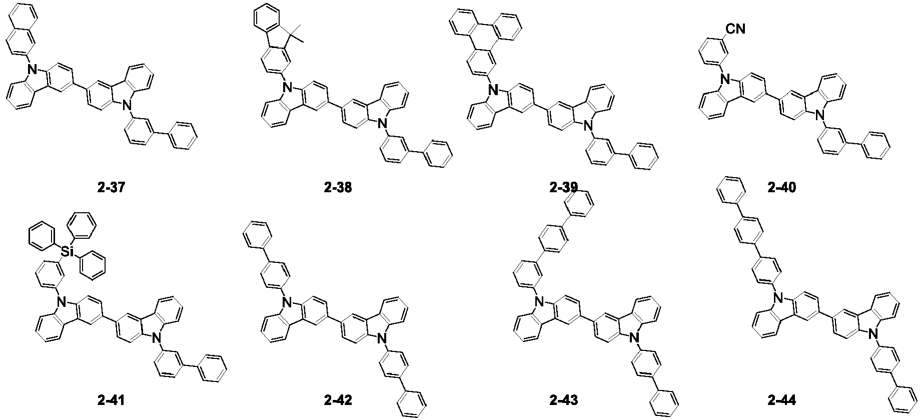

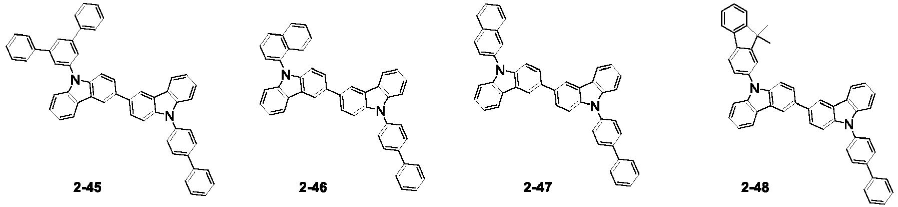

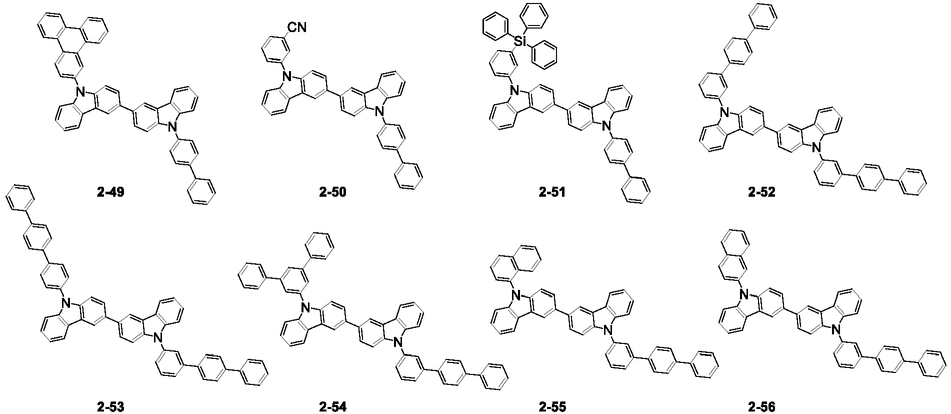

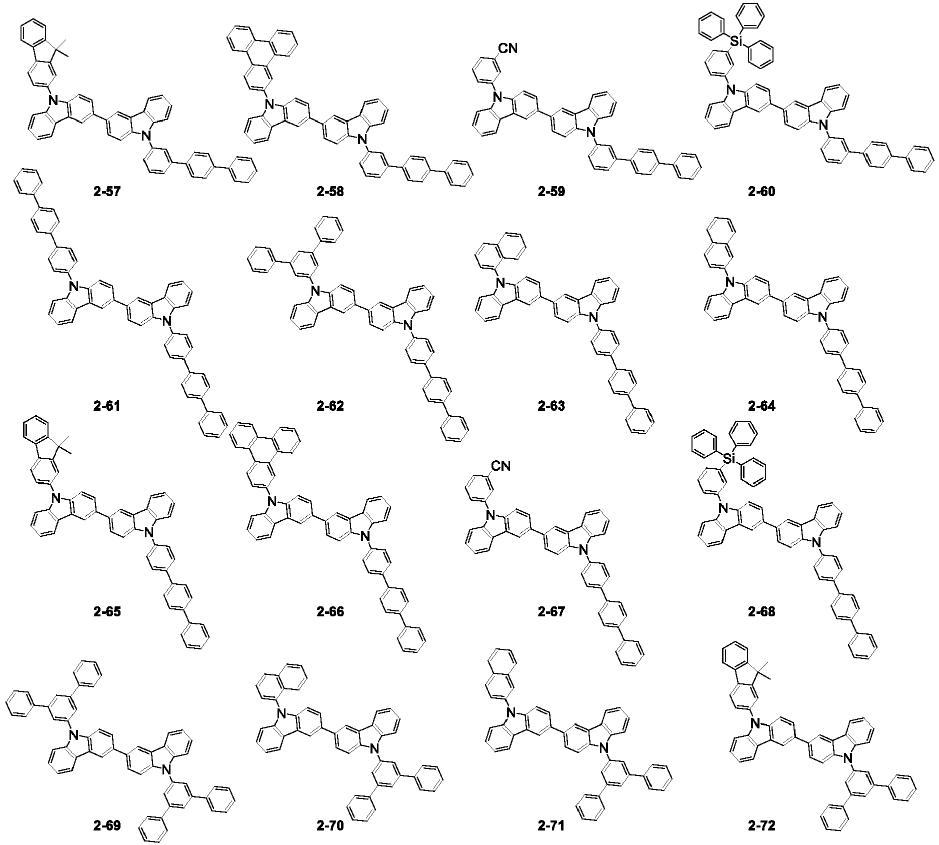

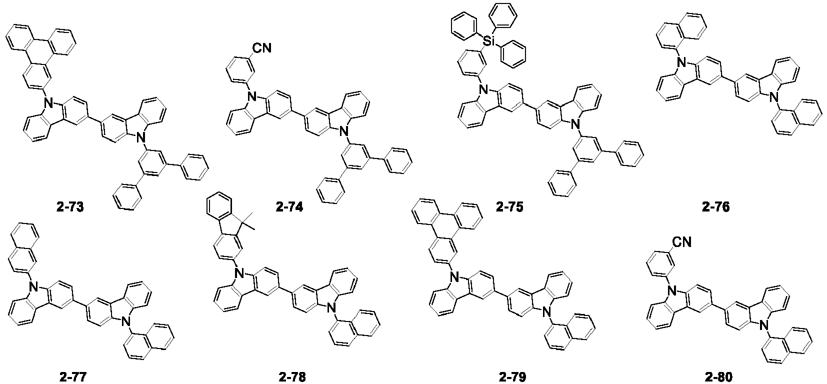

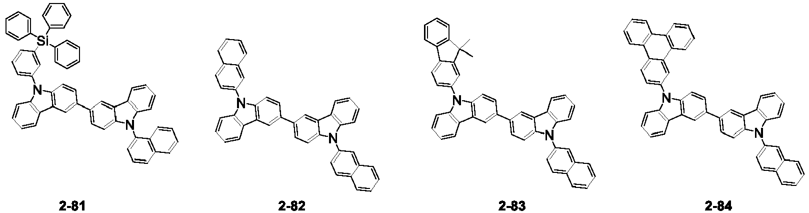

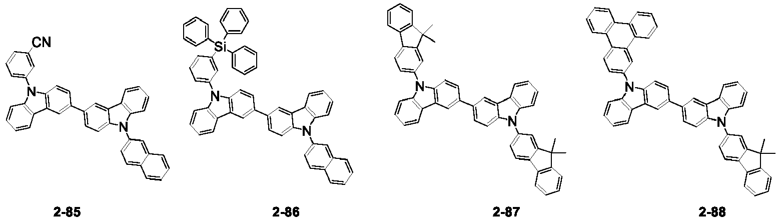

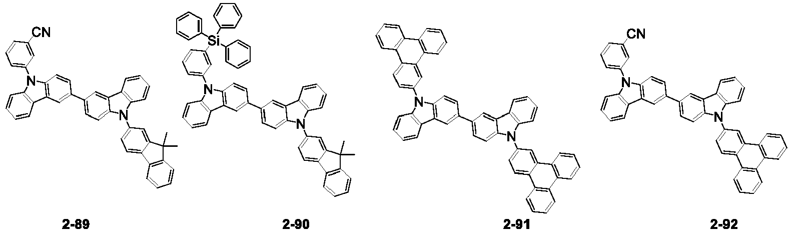

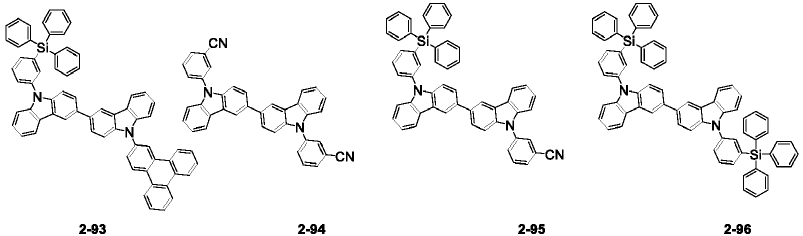

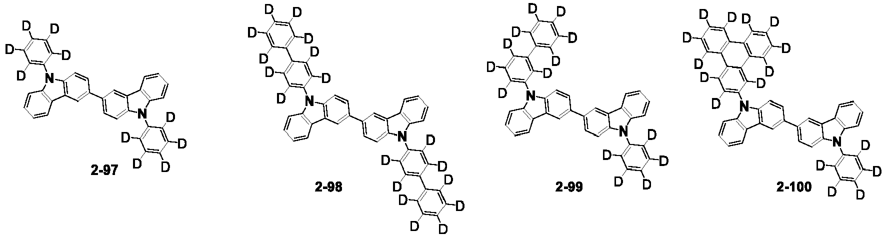

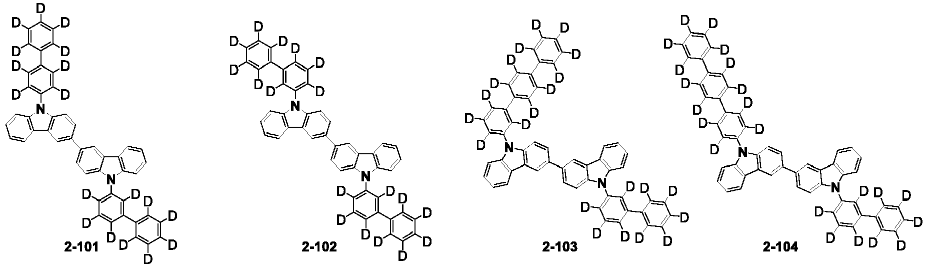

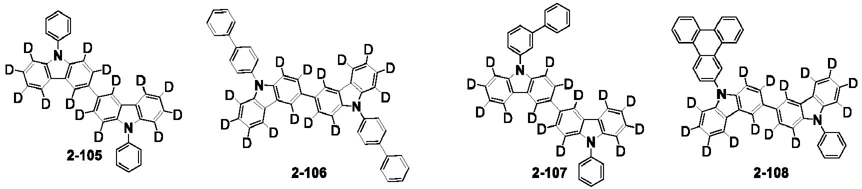

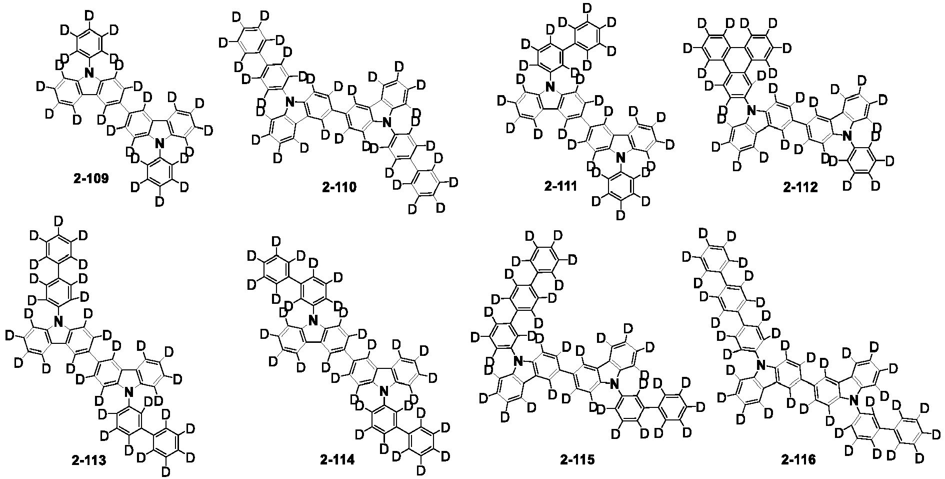

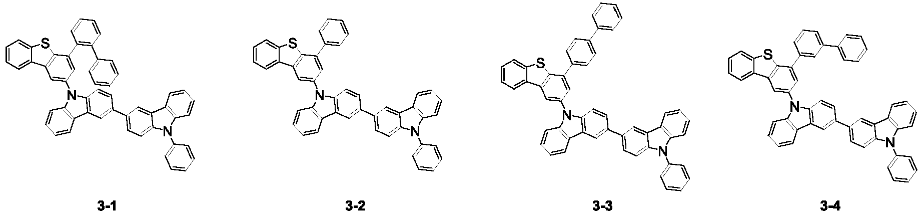

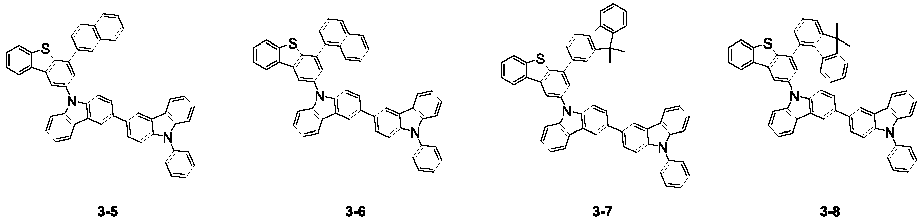

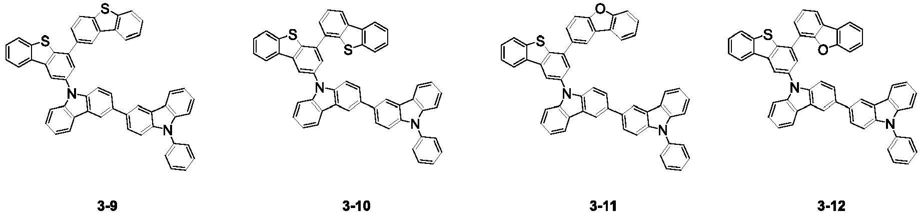

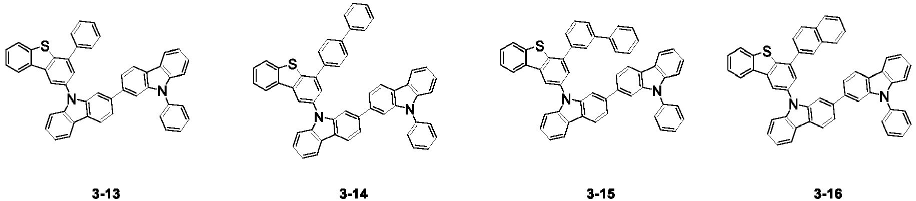

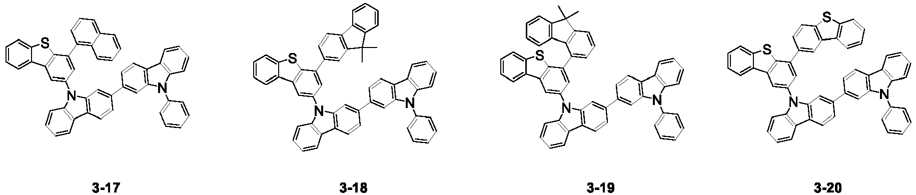

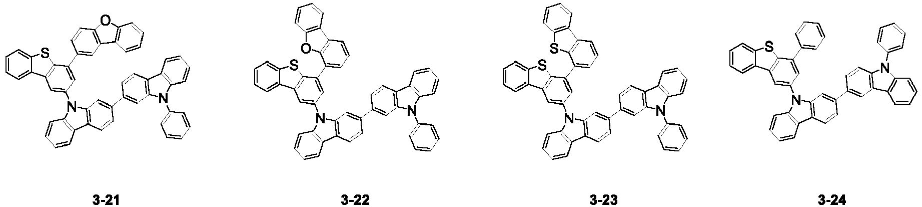

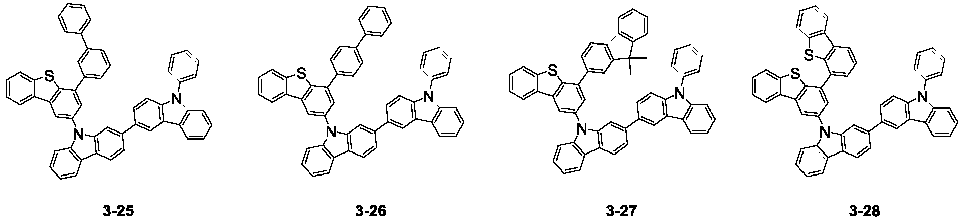

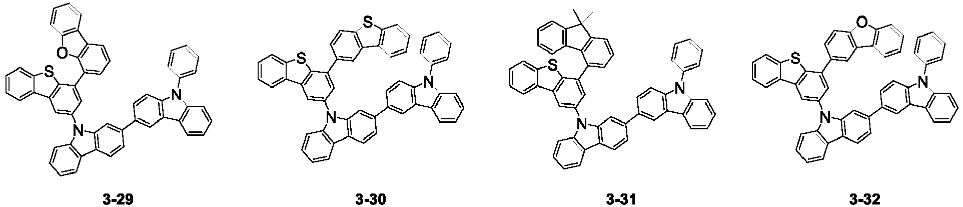

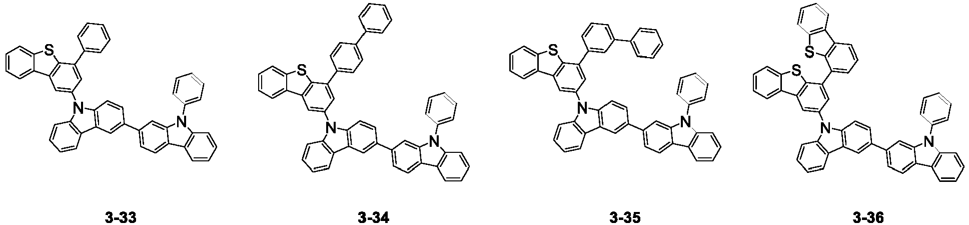

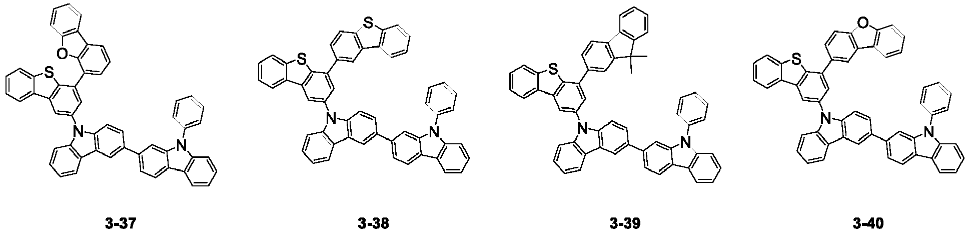

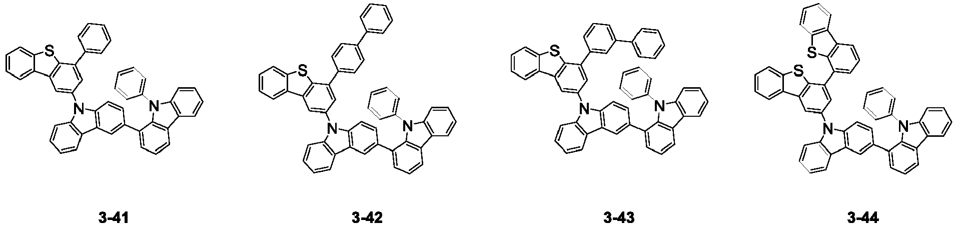

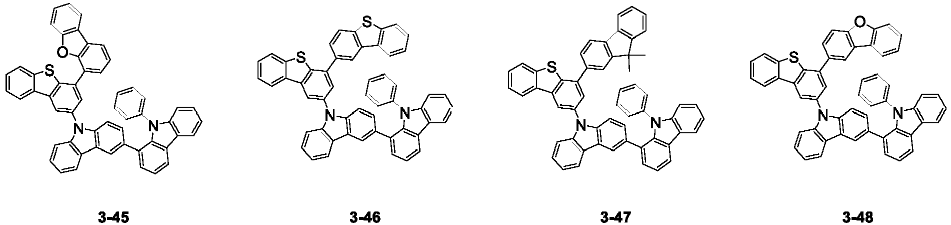

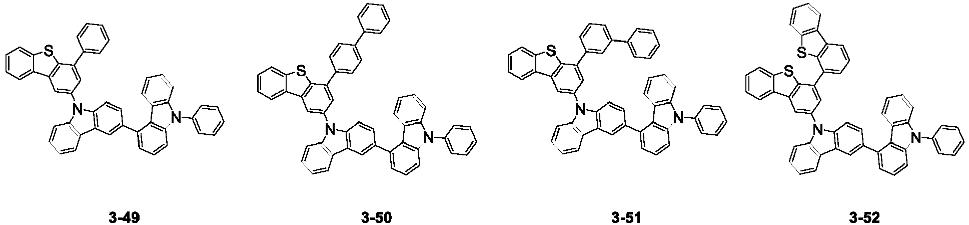

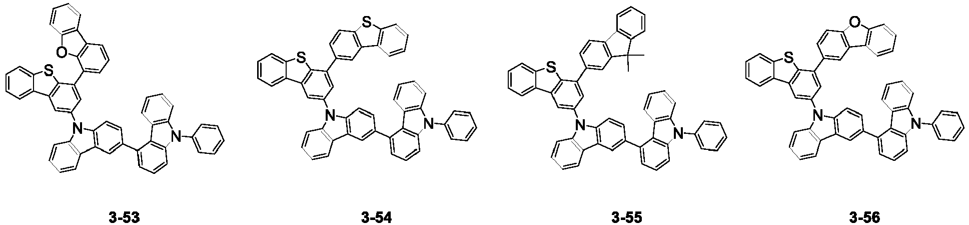

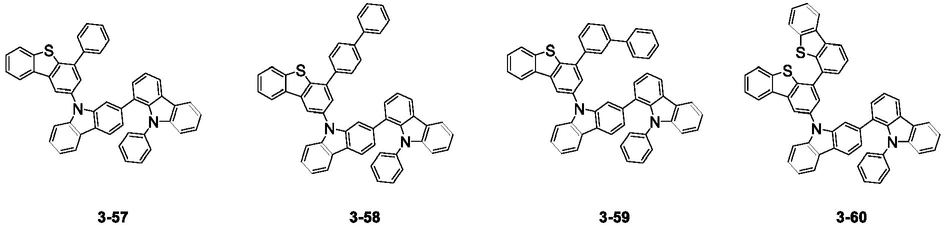

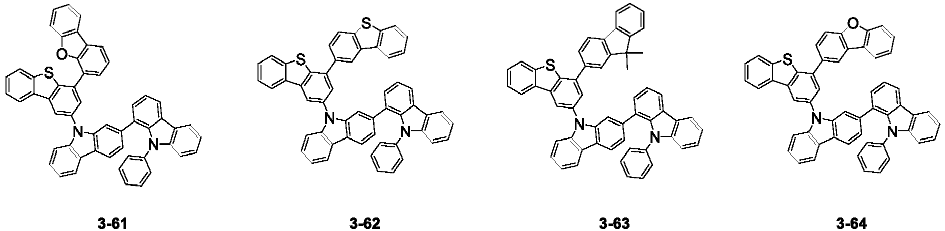

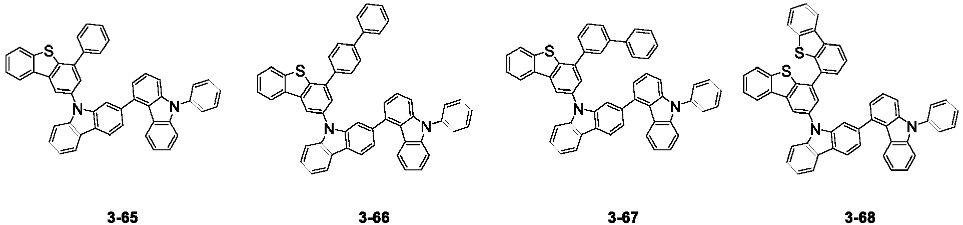

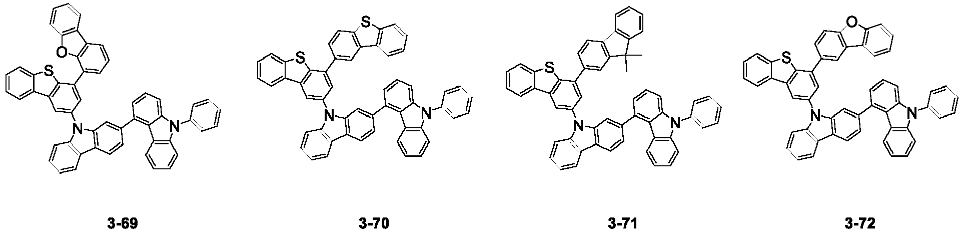

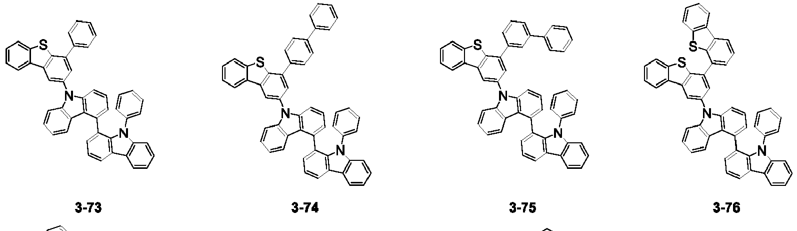

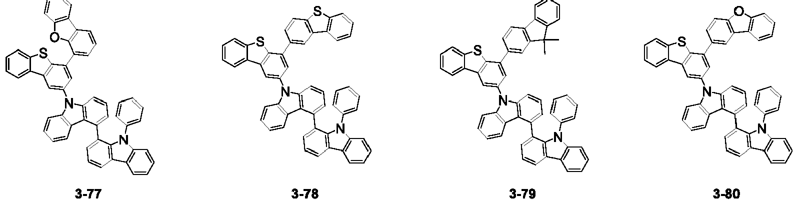

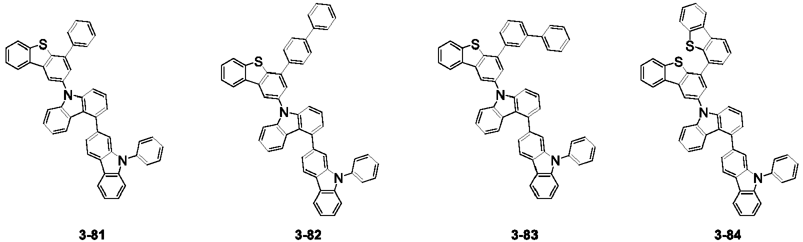

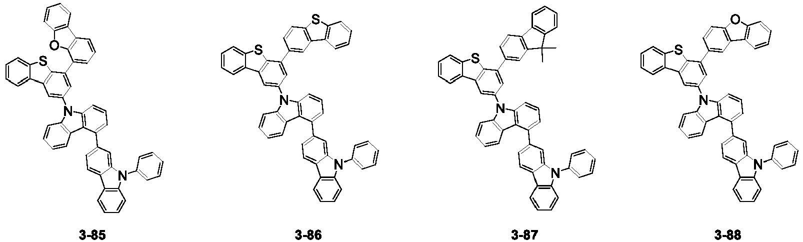

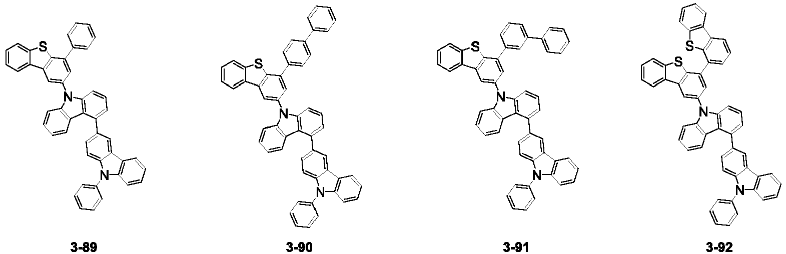

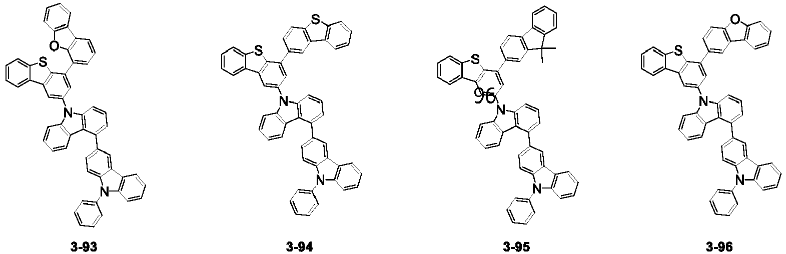

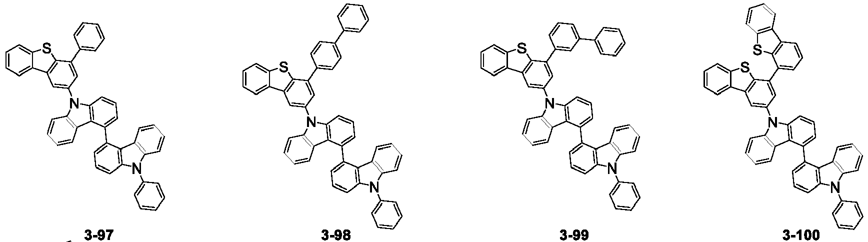

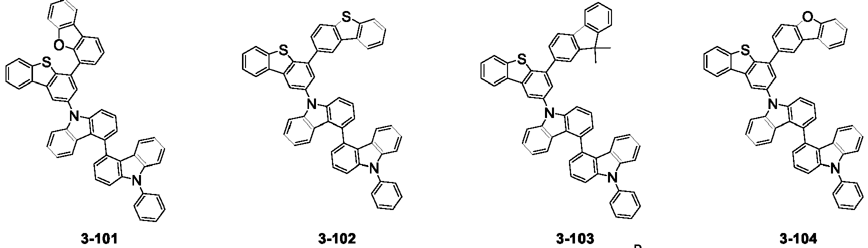

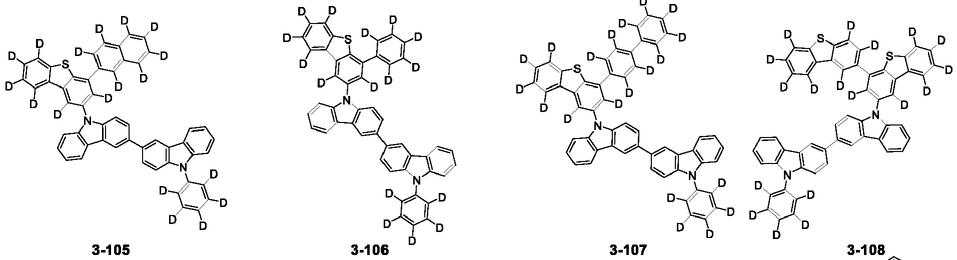

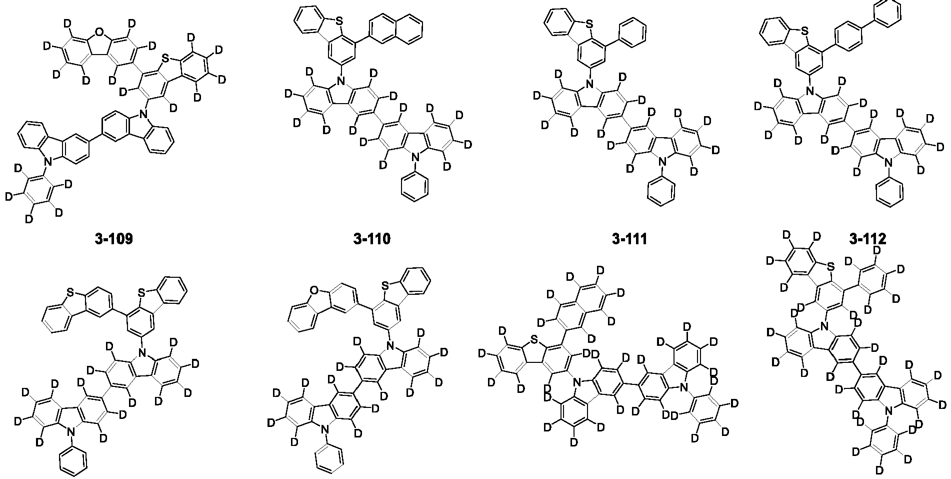

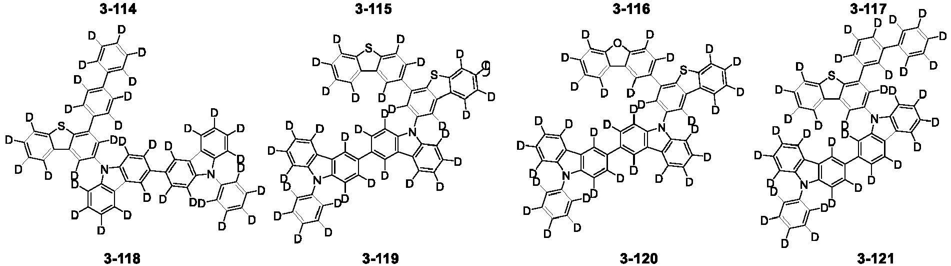

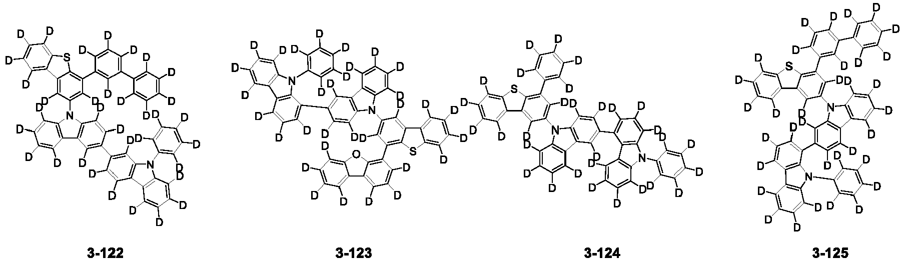

In addition, an embodiment of the present invention provides a composition for organic layer of an organic light emitting device comprising the heterocyclic compound represented by Formula 1 above and the heterocyclic compound represented by Formula 2 above.

Specific details of the heterocyclic compound represented by Formula 1 and the heterocyclic compound represented by Formula 2 are the same as described above.

In one embodiment of the present invention, a weight ratio of the heterocyclic compound represented by Formula 1 and the heterocyclic compound represented by Formula 2 in the composition for organic layer of the organic light emitting device may be 1:10 to 10:1, 1:8 to 8:1, 1:5 to 5:1, 1:2 to 2:1, but is not limited thereto.

The composition for organic layer of the organic light emitting device may be used when forming an organic material of the organic light emitting device, and may be in particular used when forming a host of the light emitting layer.

In one embodiment of the present invention, the organic layer includes the heterocyclic compound represented by Formula 1 and the heterocyclic compound represented by Formula 2, and may be used together with a phosphorescent dopant.

The phosphorescent dopant material may be those known in the art. For example, phosphorescent dopant materials represented by LL'MX', LL'L"M, LMX'X", L₂MX' and L₃M may be used, but the scope of the present invention is not limited to these examples.

M may be iridium, platinum, osmium, or the like.

L is an anionic bidentate ligand coordinated to M by sp² carbon and a hetero atom, and X may function to trap electrons or holes. Non-limiting examples of L comprise 2-(1-naphthyl)benzoxazole, (2-phenylbenzoxazole), (2-phenylbenzothiazole), (2-phenylbenzothiazole), (7,8-benzoquinoline), phenylpyridine, benzothiophene group pyridine, 3-methoxy-2-phenylpyridine, thiophene group pyridine, tolyl pyridine and the like. Non-limiting examples of X' and X" comprise acetylacetonate (acac), hexafluoroacetylacetonate, salicylidene, picolinate, 8-hydroxyquinolinate, and the like.

Specific examples of the phosphorescent dopant are shown below, but are not limited thereto:

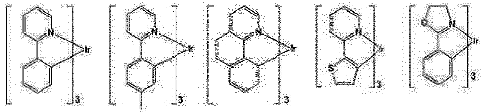

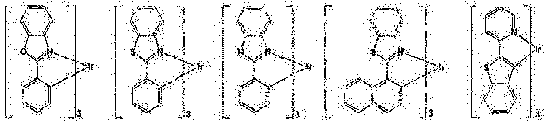

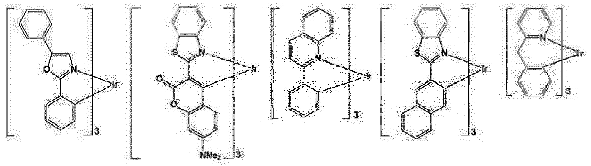

In one embodiment of the present invention, the organic layer comprises the heterocyclic compound represented by Formula 1 and the heterocyclic compound represented by Formula 2, and may be used together with an iridium-based dopant.

In one embodiment of the present invention, Ir(ppy)₃, which is a green phosphorescent dopant, may be used as the iridium-based dopant.

In one embodiment of the present invention, the content of the dopant may be 1% to 15%, preferably 3% to 10%, more preferably 5% to 10%, based on the total of the light emitting layer.

In the organic light emitting device according to an embodiment of the present invention, the organic layer may include an electron injection layer or an electron transport layer, and the electron injection layer or the electron transport layer may include the heterocyclic compound.

In the organic light emitting device according to another embodiment, the organic layer may comprise an electron blocking layer or a hole blocking layer, and the electron blocking layer or the hole blocking layer may comprise the heterocyclic compound.

In the organic light emitting device according to another embodiment, the organic layer may comprise an electron transport layer, a light emitting layer, or a hole blocking layer, and the electron transport layer, the light emitting layer, or the hole blocking layer may comprise the heterocyclic compound.

The organic light emitting device according to an embodiment of the present invention may further comprise one layer or more layers selected from the group consisting of a light emitting layer, a hole injection layer, a hole transport layer, an electron injection layer, an electron transport layer, an electron blocking layer and a hole blocking layer.

FIGs. 1 to 3 illustrate the stacking order of the electrode and the organic layer of the organic light emitting device according to an embodiment of the present invention. However, it is not intended that the scope of the present application be limited by these drawings, and the structure of the organic light emitting device known in the art may also be applied to the present application.

According to FIG. 1, an organic light emitting device formed by sequentially stacking a positive electrode 200, an organic layer 300, and a negative electrode 400 on a substrate 100 is shown. However, it is not limited to such a structure, and an organic light emitting device formed by sequentially stacking a negative electrode, an organic layer, and a positive electrode on a substrate may be implemented, as shown in FIG. 2.

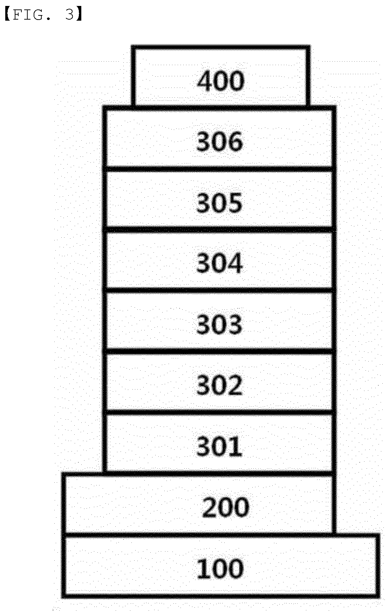

FIG. 3 illustrates a case in which the organic layer is composed of multiple layers. The organic light emitting device according to FIG. 3 comprises a hole injection layer 301, a hole transport layer 302, a light emitting layer 303, a hole blocking layer 304, an electron transport layer 305, and an electron injection layer 306. However, the scope of the present application is not limited by the stacked structure as described above, and if necessary, the remaining layers except for the light emitting layer may be omitted, and other necessary functional layers may be further added.

In one embodiment of the present invention, the present invention provides a method of manufacturing an organic light emitting device comprising the steps of preparing a substrate; forming a first electrode on the substrate; forming one or more organic layers on the first electrode; and forming a second electrode on the one or more organic layers, wherein the step of forming the one or more organic layers comprises a step of forming the one or more organic layers using the composition for organic layer according to an embodiment of the present invention.

In one embodiment of the present invention, the step of forming the organic layer may comprise pre-mixing the heterocyclic compound represented by Formula 1 and the heterocyclic compound represented by Formula 2, and depositing it using a thermal vacuum deposition method.

The pre-mixing refers to first mixing the heterocyclic compound represented by Formula 1 and the heterocyclic compound represented by Formula 2 and putting them in one source to mix them, before deposition on the organic layer.

The pre-mixed material may be referred to as a composition for organic layer according to an exemplary embodiment of the present application.

The organic layer comprising the heterocyclic compound represented by Formula 1 may further comprise another material, if necessary.

The organic layer simultaneously comprising the heterocyclic compounds represented by Formula 1 and Formula 2 may further comprise another material, if necessary.

In the organic light emitting device according to an embodiment of the present invention, the materials other than the heterocyclic compounds represented by Formula 1 or Formula 2 are exemplified below, but these are for illustrative purposes only and are not intended to limit the scope of the present application, and may be replaced by materials known in the art.

As the positive electrode material, materials having a relatively large work function can be used, and a transparent conductive oxide, metal, a conductive polymer or the like may be used. Specific examples of the positive electrode material may be, but is not limited to, metals such as vanadium, chromium, copper, zinc, and gold or alloys thereof; metal oxides such as zinc oxide, indium oxide, indium tin oxide (ITO), and indium zinc oxide (IZO); a combination of metal and oxide such as ZnO : Al or SnO₂ : Sb; conductive polymers such as poly(3-methylthiophene), poly[3,4-(ethylene-1,2-dioxy)thiophene](PEDOT), polypyrrole and polyaniline; and the like.

As the negative electrode material, materials having a relatively low work function may be used, and metal, metal oxide, a conductive polymer or the like may be used. Specific examples of the negative electrode material may be, but is not limited to, metals such as magnesium, calcium, sodium, potassium, titanium, indium, yttrium, lithium, gadolinium, aluminum, silver, tin and lead or alloys thereof; multilayer-structured materials such as LiF/Al or LiO₂/Al; and the like.

As the hole injection layer material, a known material for the hole injection layer may be used, and for example, phthalocyanine compounds such as copper phthalocyanine disclosed in U.S. Patent No. 4,356,429, or starburst-type amine derivatives described in [Advanced Material, 6, p.677 (1994)], such as tris(4-carbazoyl-9-ylphenyl)amine(TCTA), 4,4',4"-tri[phenyl(m-tolyl)amino]triphenylamine(m-MTDATA), 1,3,5-tris[4-(3-methylphenylphenylamino)phenyl]benzene(m-MTDAPB), polyaniline/dodecylbenzenesulfonic acid which is a soluble conductive polymer, or poly(3,4-ethylenedioxythiophene)/poly(4-styrenesulfonate), polyaniline/camphor sulfonic acid, polyaniline/poly(4-styrene-sulfonate), or the like may be used.

As a material for the hole transport layer, a pyrazoline derivative, an arylamine-based derivative, a stilbene derivative, a triphenyldiamine derivative, or the like may be used, and a low-molecular or high-molecular material may be used.

As a material for the electron transport layer, metal complexes of oxadiazole derivatives, anthraquinodimethane and derivatives thereof, benzoquinone and derivatives thereof, naphthoquinone and its derivatives, anthraquinone and its derivatives, tetracyanoanthraquinodimethane and its derivatives, fluorenone derivatives, diphenyldicyanoethylene and its derivatives, diphenoquinone derivatives, 8-hydroxyquinoline and its derivatives, and the like may be used, and high-molecular materials as well as low-molecular materials may be also used.

As the electron injection layer material, for example, LiF is typically used in the art, but the present application is not limited thereto.

As a material for the light emitting layer, a red, green or blue light emitting material may be used, and if necessary, two or more light emitting materials may be mixed and used. In this case, 2 or more luminescent materials may be used by deposition from separate sources, or may be pre-mixed and deposited from one source. In addition, as a material for the light emitting layer, a fluorescent material may be used, and a phosphorescent material may also be used. As a material for the light emitting layer, a material that emits light by combining holes and electrons respectively injected from the positive electrode and the negative electrode alone may be used, and materials in which the host material and the dopant material together participate in light emission may be also used.

If the hosts of the material for the light emitting layer are mixed and used, hosts of the same type may be mixed and used, or hosts of different types may be mixed and used. For example, any two or more types of n-type host material or p-type host material may be selected and used as a host material for the light emitting layer.

The organic light emitting device according to an embodiment of the present invention may be a top emission type, a bottom emission type, or a double side emission type depending on the material used.

The heterocyclic compound according to an embodiment of the present invention may also act in organic electronic devices including a organic solar cell, a organic photoreceptor, a organic transistor, and the like through the principle similar to that applied to the organic light emitting device.

Hereinafter, preferred examples are presented to help the understanding of the present invention. However, the following examples are provided only to make the present invention easier to understand, and the present invention is not limited thereto.

### <Preparation Example>

### <Preparation Example 1> Synthesis of intermediate C

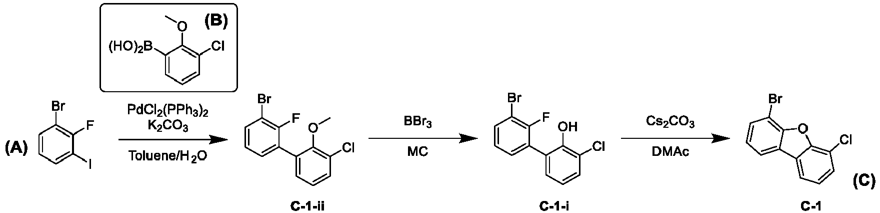

### 1) Preparation of compound C-1-ii

20g (66.47mmol) of 1-bromo-2-fluoro-3-iodobenzene (A), 14.87.g (79.76mmol) of (3-chloro-2-methoxyphenyl)boronic acid (B), 2.33g (3.32mmol) of bis(triphenylphosphine)palladium (II) dichloride ((PPh₃) ₂ (PdCl₂)), 18.37g (132.93mmol) of K₂CO₃ were placed in a 1L one-neck round flask, dissolved in 200ml of toluene and 60ml of water, and reacted under reflux at 111°C for 3 hours.

After completion of the reaction, the mixture was cooled to room temperature, and the resultant was extracted with water and methylchloride (MC), and the organic layer was concentrated. This organic layer was purified through a column using methylchloride (MC) and HX. Next, an excess of methanol was added, the precipitated solid was filtered off, and the filtrate was concentrated and dried to obtain 14.7g (70%) of the desired compound, 3-bromo-3'-chloro-2-fluoro-2'-methoxy-1,1'-biphenyl [C-1-ii] .

### 2) Preparation of compound C-1-i

14.7g(46.53mmol) of 3-bromo-3'-chloro-2-fluoro-2'-methoxy-1,1'-biphenyl [C-1-ii] was placed in a 1L one-neck round flask, dissolved in 150ml of methylchloride (MC), and stirred. After sufficient stirring, 23.31 g (93.06 mmol) of tribromoboron (BBr₃) was added dropwise over 10 minutes while maintaining room temperature, followed by stirring at room temperature for 2 hours.

After the reaction was completed, an excess of water (300 ml) was added, stirred for 1 hour, and then filtered to filter out the solid. The organic layer was separated from the filtrate, concentrated, and then purified through a column using MC and HX. The filtrate through the column was concentrated and dried in vacuo to obtain 11.5g (82%) of the desired compound, 3'-bromo-3-chloro-2'-fluoro-[1,1'-biphenyl]-2-ol [C-1-i].

### 3) Preparation of compound C-1

11.5g (38.15mmol) of 3'-bromo-3-chloro-2'-fluoro-[1,1'-biphenyl]-2-ol [C-1-i] was placed in a 1L one-neck round flask, dissolved in 110ml of dimethylacetamide (DMAc), and then heated to 120°C and stirred. At this time, 24.86 g (76.31 mmol) of cesium carbonate (Cs₂CO₃) was slowly added, heated to 165°C, and then reacted under reflux for 3 hours.

After the reaction was completed, the reaction solution was cooled to room temperature, and then filtered to remove the solid. The filtrate was concentrated and purified through a column using MC and HX. The filtrate was concentrated, stirred in 6T methanol (MeOH) for 3 hours, and then filtered to obtain 9.1g (85%) of the desired compound, 4-bromo-6-chlorodibenzo[b,d]furan [C-1], as a white solid.

The desired compounds C shown in Table 1 were prepared in the same manner as for compound C-1, except that in Preparation Example 1, (A) of Table 1 below instead of 1-bromo-2-fluoro-3-iodobenzene was used, and (B) of Table 1 below instead of (3-chloro-2-methoxyphenyl)boronic acid (B) was used, as an intermediate.

**Table 1:**

| Compo und | A | B | C | Yield |
|---|---|---|---|---|
| C-1 | 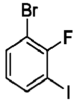 | 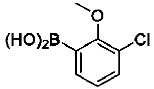 | 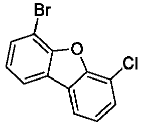 | 49% |
| C-2 | 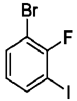 | 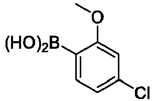 | 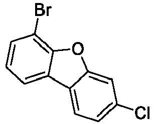 | 52% |
| C-3 | 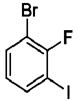 | 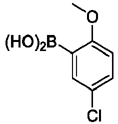 | 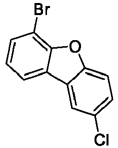 | 55% |
| C-4 | 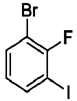 | 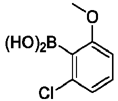 | 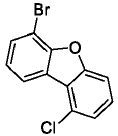 | 45% |
| C-5 | 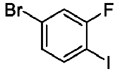 | 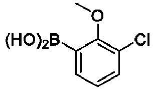 | 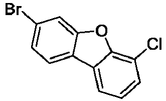 | 52% |
| C-6 | 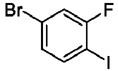 | 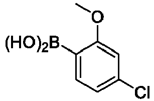 | 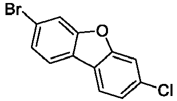 | 55% |
| C-7 | 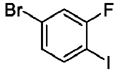 | 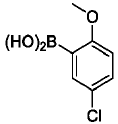 | 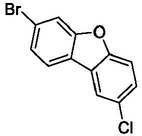 | 52% |
| C-8 | 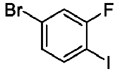 | 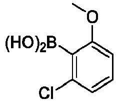 | 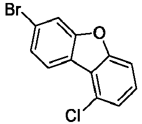 | 49% |
| C-9 | 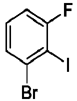 | 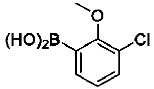 | 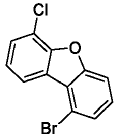 | 46% |
| C-10 | 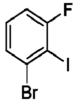 | 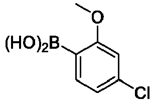 | 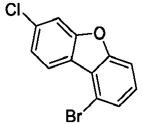 | 48% |
| C-11 | 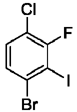 | 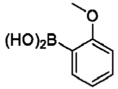 | 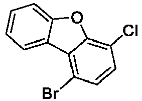 | 42% |
| C-12 | 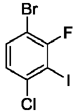 | 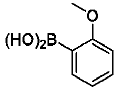 | 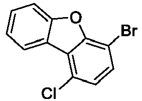 | 39% |
| C-13 | 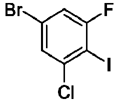 | 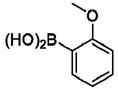 | 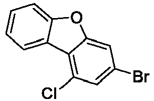 | 45% |
| C-14 | 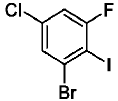 | 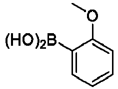 | 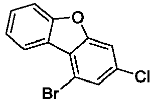 | 44% |
| C-15 | 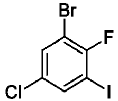 | 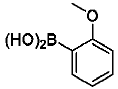 | 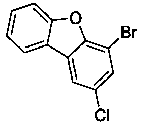 | 48% |
| C-16 | 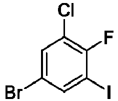 | 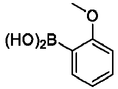 | 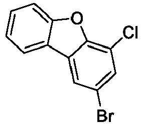 | 45% |

### <Preparation Example 2> Synthesis of intermediate C-17

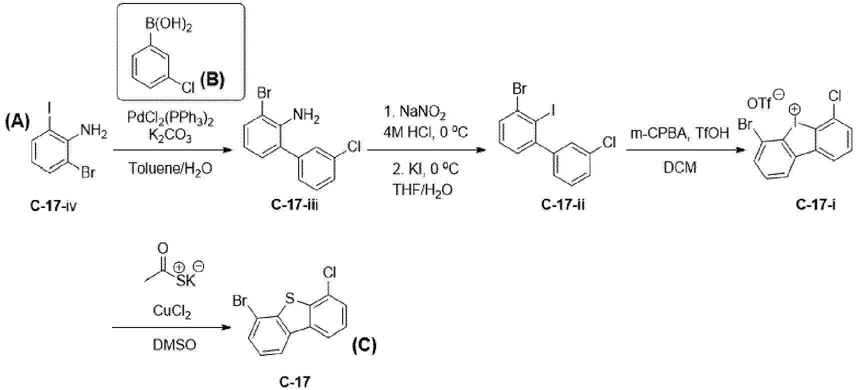

### 1) Preparation of compound C-17-iii

100g (335.56mmol) of 2-bromo-6-iodoaniline (A), 63.0g (402.79mmol) of (4-chlorophenyl)boronic acid (B), 19.39g (16.78mmol) of tetrakis(triphenylhosphine)palladium(0) (Pd(PPh₃)₄), 92.8g (671.32mmol) of K₂CO₃ were placed in a 2L one neck round flask, and then 500ml of 1,4-dioxane and 120ml of H₂O were added, and the mixture was stirred at 101°C under reflux for 6 hours. After completion of the reaction, the reaction solution was cooled to room temperature, and the organic layer was extracted with water and dichloromethane and concentrated. This organic layer was purified through a column using MC and HX to obtain 73g(77%) of the desired compound, 3-bromo-3'-chloro-[1,1'-biphenyl]-2-amine [C-17-iii] .

### 2) Preparation of compound C-17-ii

73g (258.38mmol) of 3-bromo-3'-chloro-[1,1'-biphenyl]-2-amine [C-17-iii], 700 ml of tetrahydrofuran (THF) and 1050 ml of 4M HCl were placed in a 3L one neck round flask, and then cooled to 0°C. Thereafter, 52.2 g (90 ml) of a sodium nitrite (NaNO₂) aqueous solution was added dropwise at 0°C, and then stirred while maintaining the temperature for 1 hour, and then 280 g (1680 ml) of a potassium iodide (KI) aqueous solution was slowly added dropwise.

After completion of the reaction, sodium thiosulfate was added to neutralize the acid. Thereafter, the organic layer was extracted with water and dichloromethane, separated, adsorbed, and purified through a column using MC/HX to obtain 88g (87%) of the desired compound, 3-bromo-3'-chloro-2-iodo-1,1'-biphenyl [C-17-ii] .

### 3)Preparation of compound C-17-i

58.24g (224.8mmol) of 3-bromo-3'-chloro-2-iodo-1,1'-biphenyl [C-17-ii] was placed in a 2L one neck round flask and sufficiently dissolved in 900ml of dichloromethane, and then while maintaining at 0°C, 58.24 g (337.19 mmol) of m-chloroperbenzoic acid (m-CPBA) and 59.6 ml (674.4 mmol) of trifluoromethanesulfonic acid (TfOH) were added and stirred for 5 hours. After completion of the reaction, the resulting solid was filtered and dried, and then dissolved in acetone, and the undissolved solid was removed by filtration again, and only the filtrate was concentrated. The filtrate was stirred in dichloromethane, and the precipitated solid was filtered to obtain 86.4g (71%) of the desired compound, ((trifluoromethyl)sulfonyl)-1-oxidane, 4-bromo-6-chlorodibenzo[b,d]iodol-5-ium salt [C-17-i].

### 4) Preparation of compound C-17

86.4 g (159.6mmol) of ((trifluoromethyl)sulfonyl)-1-oxidane, 4-bromo-6-chlorodibenzo[b,d]iodol-5-ium salt [C-17-i], 91 g (798mmol) of potassium ethanethioate, and 1.4g(8.0mmol) of Cu(OAc)₂ were placed in a 2L one neck round flask, dissolved in 1200 ml of dimethyl sulfoxide (DMSO), and stirred at 110 °C for 24 hours.

After the reaction is completed, the mixture was cooled to room temperature, and then the precipitated solid was removed by filtration, washed thoroughly with dichloromethane, and then the filtered filtrate was concentrated. The concentrated organic material was adsorbed, and then purified through a column using MC/HX to obtain 22g (46%) of the desired compound, 4-bromo-6-chlorodibenzo[b,d]thiophene [C-17].

The following desired compounds C were prepared in the same manner as compound C-17, except that in Preparation Example 2, (A) of Table 2 below instead of 2-bromo-6-iodoaniline was used, and (B) of Table 2 below instead of (2-chlorophenyl)boronic acid was used, as an intermediate.

**Table 2:**

| Compo und | A | B | C | Yield |
|---|---|---|---|---|
| C-17 | 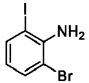 | 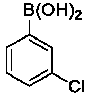 | 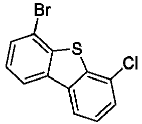 | 21% |
| C-18 | 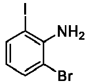 | 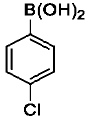 | 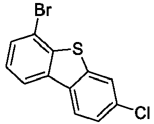 | 21% |
| C-19 | 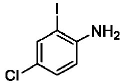 | 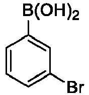 | 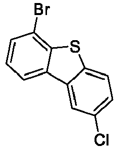 | 22% |
| C-20 | 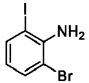 | 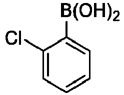 | 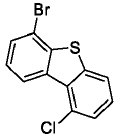 | 24% |
| C-21 | 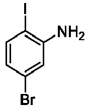 | 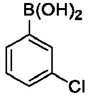 | 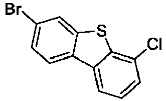 | 20% |
| C-22 | 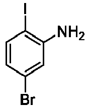 | 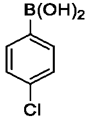 | 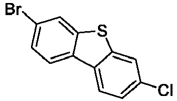 | 22% |
| C-23 | 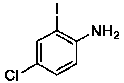 | 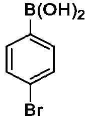 | 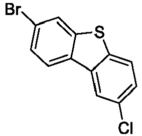 | 21% |
| C-24 | 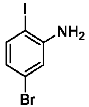 | 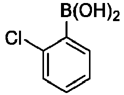 | 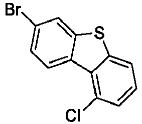 | 17% |
| C-25 | 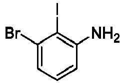 | 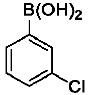 | 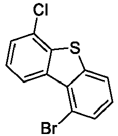 | 23% |
| C-26 | 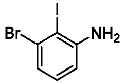 | 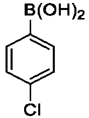 | 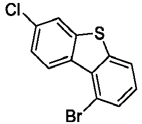 | 19% |
| C-27 | 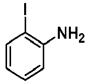 | 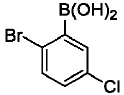 | 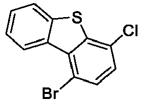 | 19% |
| C-28 | 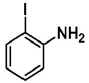 | 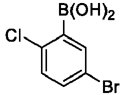 | 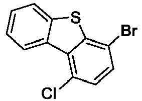 | 20% |
| C-29 | 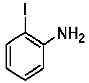 | 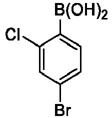 | 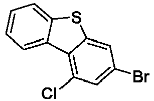 | 15% |
| C-30 | 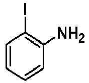 | 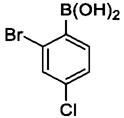 | 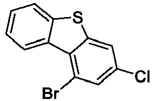 | 14% |
| C-31 | 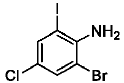 | 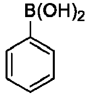 | 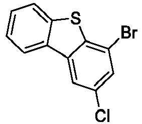 | 16% |
| C-32 | 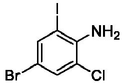 | 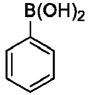 | 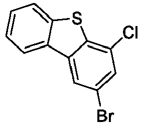 | 15% |

### <Preparation Example 3> Synthesis of compound 1-1

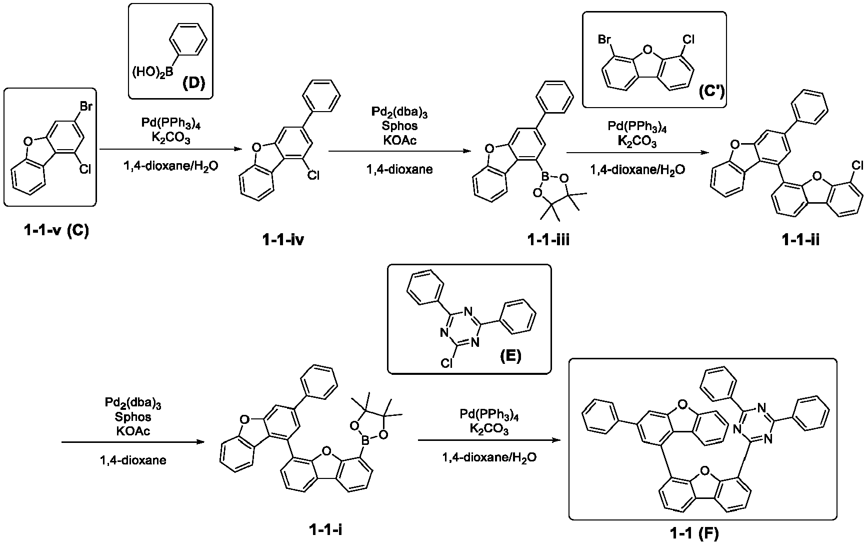

### 1) Preparation of compound 1-1-iv

Into a 1L one-neck round flask, 20g (71.04mmol) of 3-bromo-1-chlorodibenzo[b,d]furan (C), 10.61mmol of phenylboronic acid (D), 2.36g (3.36mmol) of tetrakis(triphenylphosphine)palladium(0) (Pd(PPh₃)₄), and 18.56g(134.26mmol) of K₂CO₃ were placed, and dissolved in 200 ml of 1,4-dioxane and 60 ml of water, and reacted under reflux at 101 °C for 3 hours.

After the reaction was completed, the organic layer was extracted with water and MC after cooling to room temperature.

The extract was concentrated, then dissolved in MC, loaded on a column, and purified by HX (only). The filtrate was concentrated, and then stirred in 100 mL of methanol, and the precipitated solid was filtered to obtain 15.3 g (77%) of 3-chloro-1-phenyl-dibenzofuran ([1-1-iv]) as a white solid.

### 2) Preparation of compound 1-1-iii

15.3g (54.89mmol) of 3-chloro-1-phenyl-dibenzofuran([1-1-iv]), 20.91g (82.34mmol) of 4,4,5,5-tetramethyl-2-(4,4,5,5-tetramethyl-1,3,2-dioxaborolan-2-yl)-1,3,2-dioxaborolane, 5.03g(5.49mmol) of tris(dibenzylideneacetone)dipalladium (Pd₂(dba)₃), 5.63g(13.72mmol) of Sphos(2-dicyclohexylphosphino-2',6'-dimethoxybiphenyl), 10.77g(109.78mmol) of KOAc were placed, and dissolved in 160 mL of 1,4-dioxane, and stirred under reflux at 101 °C for 1 hour.

After the reaction was completed, the organic layer was extracted with water and MC after cooling to room temperature.

The organic layer was concentrated, and then dissolved in a small amount of toluene, filtered through silica gel, and eluted with toluene. The filtered filtrate was concentrated and dried in vacuo to obtain 19.3g (95%) of 4,4,5,5-tetramethyl-2-(1-phenyldibenzofuran-3-yl)-1,3,2-dioxaborolane ([1-1-iii]).

### 3) Preparation of compound 1-1-ii

Into a 500 ml one neck round flask, 19.3g (52.13mmol) of 4,4,5,5-tetramethyl-2-(1-phenyldibenzofuran-3-yl)-1,3,2-dioxaborolane [1-1-iii], 16.14g(57.34mmol) of 1-bromo-7-chlorodibenzofuran (C'), 2.41g(2.09mmol) of tetrakis(triphenylphosphine)palladium(0) (Pd(PPh₃)₄), 14.41g (104.25mmol) of K₂CO₃ were placed, and dissolved in 200 mL of 1,4-dioxane and 60 mL of water, and stirred under reflux at 101 °C for 3 hours.

After the reaction was completed, the mixture was cooled to room temperature, and water and methanol were added and stirred. The precipitated solid was filtered and washed with an excessive amount of water and methanol.

The solid was dried in vacuo to obtain 18 g (78%) of 3-(7-chlorodibenzofuran-1-yl)-1-phenyl-1-dibenzofuran ([1-1-ii]) as a white solid.

### 4) Preparation of compound 1-1-i

Into a 1L one-neck round flask, 18g (40.46mmol) of 3-(7-chlorodibenzofuran-1-yl)-1-phenyl-1-dibenzofuran([1-1-ii]), 15.41g(60.69mmol) of 4,4,5,5-tetramethyl-2-(4,4,5,5-tetramethyl-1,3,2-dioxaborolan-2-yl)-1,3,2-dioxaborolane, 3.7g (4.05mmol) of tris(dibenzylideneacetone)dipalladium (Pd₂(dba)₃), 4.15g (10.11mmol) of Sphos (2-Dicyclohexylphosphino-2',6'-dimethoxybiphenyl), and 7.94g (80.92mmol) of KOAc were placed, and dissolved in 180 mL of 1,4-dioxane and stirred under reflux at 101 °C for 1.5 hours.

After the reaction was completed, the organic layer was extracted with water and MC from the reactant cooled to room temperature. The extracted organic layer was concentrated, dissolved in a small amount of toluene, filtered through silica gel, and eluted with toluene. The filtered filtrate was concentrated, stirred in 100 ml of ethyl acetate, and the precipitated solid was filtered to obtain 18.9g (85%) of 4,4,5,5-tetramethyl-2-[9-(1-phenyldibenzofuran-3-yl)dibenzofuran-3-yl]-1,3,2-dioxaborolane ([1-1-i]).

### 5) Preparation of compound 1-1

Into a 500ml one-neck round flask, 8.8g(16.41mmol) of 4,4,5,5-tetramethyl-2-[9-(1-phenyldibenzofuran-3-yl)dibenzofuran-3-yl]-1,3,2-dioxaborolane ([1-1-i]), 4.83g (18.05mmol) of 2-chloro-4,6-diphenyl-1,3,5-triazine (E), 0.66g (0.57mmol) of tetrakis(triphenylphosphine)palladium(0) (Pd(PPh₃)₄), and 4.08g (29.53mmol) of K₂CO₃ were placed, and dissolved in 90 mL of 1,4-dioxane and 30 mL of water, and stirred under reflux at 101 °C for 12 hours.

After the reaction was completed, the mixture was cooled to room temperature, and the precipitated solid was filtered, and washed with an excessive amount of water and acetone. The sufficiently dried solid was dissolved in dichlorobenzene (DCB), filtered through silica gel, and eluted with toluene. The filtered filtrate was concentrated, stirred in 100 mL of ethyl actate (EA), and white crystals obtained were filtered to obtain 8.9g (84%) of the desired compound, 2,4-diphenyl-6-[9-(1-phenyldibenzofuran-3-yl)dibenzofuran-3-yl]-1,3,5-triazine [1-1].

The following desired compounds F were prepared in the same manner as for compound C-17, except that in Preparation Example 3, (C) of Table 3 below instead of 3-bromo-1-chlorodibenzo[b,d]furan was used, (D) of Table 3 below instead of phenylboronic acid was used, (C') in Table 3 below instead of 1-bromo-7-chloro-dibenzofuran was used, and (E) of Table 3 below instead of 2-chloro-4,6-diphenyl-1,3,5-triazine was used, as an intermediate.

**Table 3:**

| Com pou nd | C | D | C' | E | F | Yie ld |
|---|---|---|---|---|---|---|
| 1-1 | 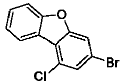 | 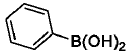 | 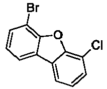 | 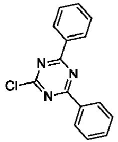 | 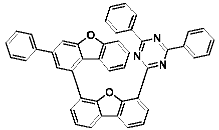 | 41% |
| 1-7 | 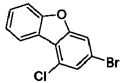 | 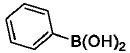 | 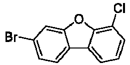 | 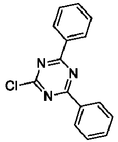 | 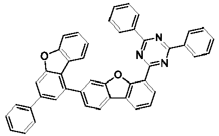 | 43% |
| 1-14 | 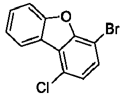 | 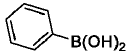 | 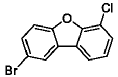 | 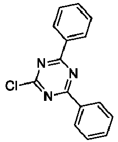 | 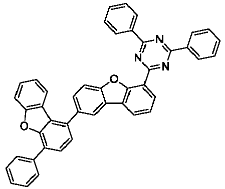 | 40% |
| 1-16 | 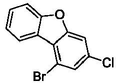 | 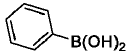 | 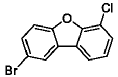 | 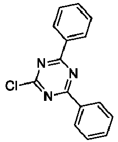 | 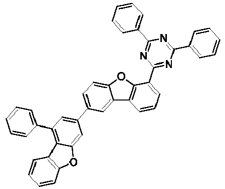 | 44% |
| 1-19 | 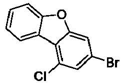 | 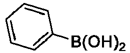 | 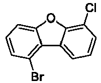 | 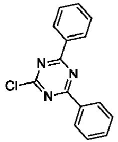 | 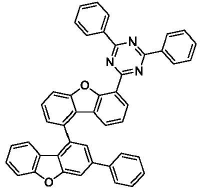 | 43% |
| 1-23 | 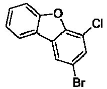 | 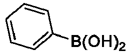 | 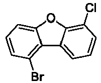 | 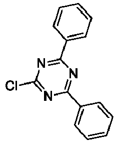 | 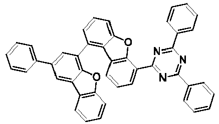 | 42% |
| 1-25 | 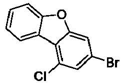 | 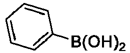 | 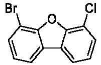 | 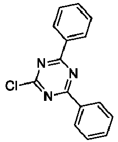 | 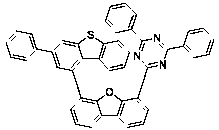 | 38% |
| 1-31 | 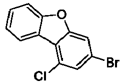 | 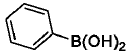 | 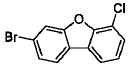 | 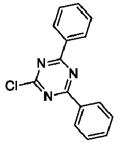 | 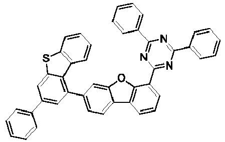 | 40% |
| 1-34 | 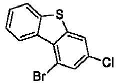 | 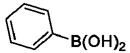 | 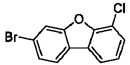 | 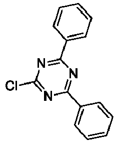 | 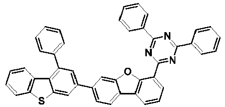 | 43% |
| 1-43 | 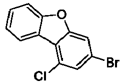 | 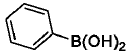 | 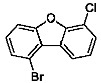 | 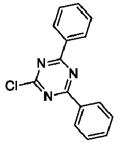 | 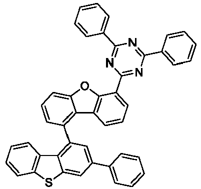 | 45% |
| 1-49 | 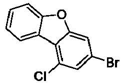 | 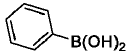 | 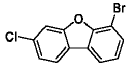 | 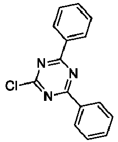 | 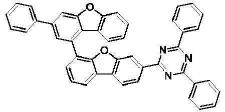 | 43% |
| 1-54 | 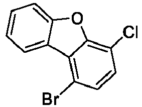 | 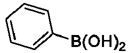 | 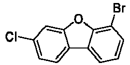 | 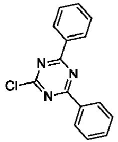 | 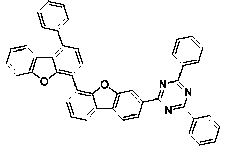 | 40% |
| 1-58 | 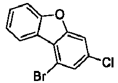 | 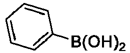 | 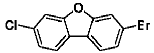 | 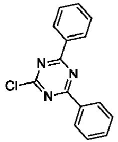 | 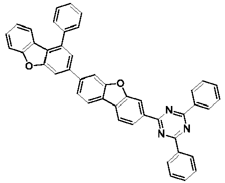 | 41% |
| 1-66 | 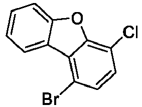 | 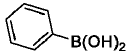 | 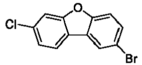 | 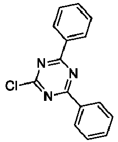 | 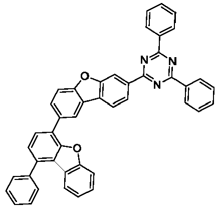 | 44% |
| 1-70 | 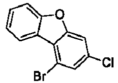 | 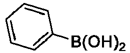 | 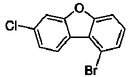 | 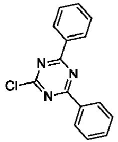 | 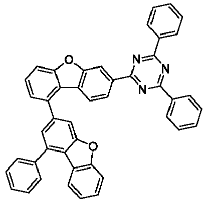 | 41% |
| 1-73 | 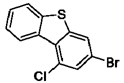 | 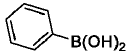 | 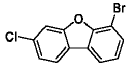 | 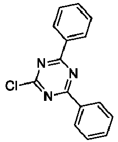 | 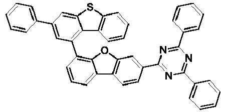 | 42% |
| 1-79 | 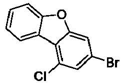 | 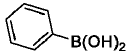 | 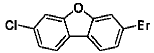 | 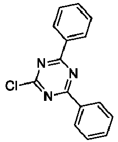 | 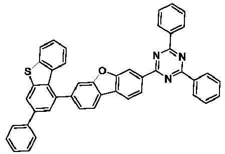 | 44% |
| 1-87 | 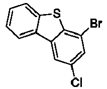 | 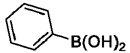 | 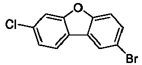 | 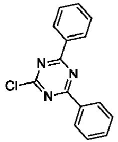 | 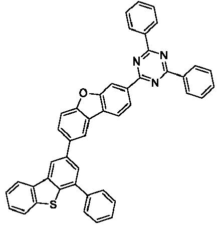 | 42% |
| 1-97 | 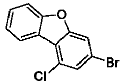 | 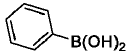 | 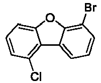 | 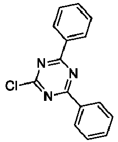 | 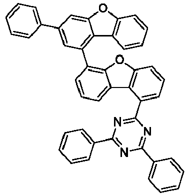 | 44% |
| 1-104 | 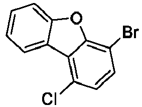 | 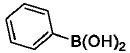 | 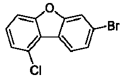 | 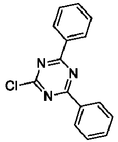 | 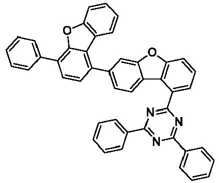 | 39% |
| 1-109 | 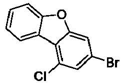 | 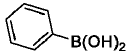 | 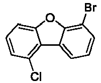 | 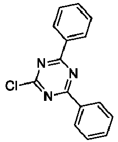 | 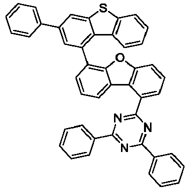 | 38% |
| 1-119 | 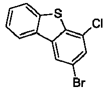 | 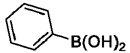 | 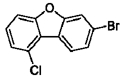 | 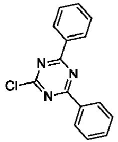 | 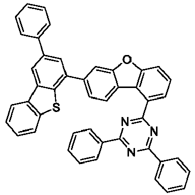 | 39% |
| 1-121 | 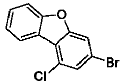 | 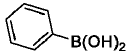 | 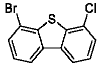 | 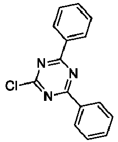 | 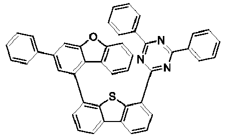 | 38% |
| 1-129 | 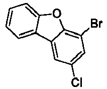 | 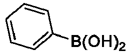 | 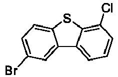 | 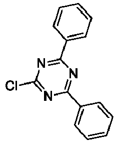 | 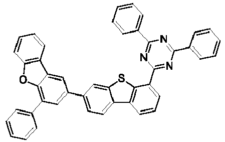 | 44% |
| 1-139 | 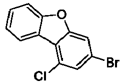 | 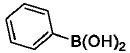 | 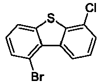 | 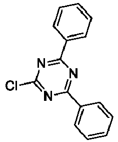 | 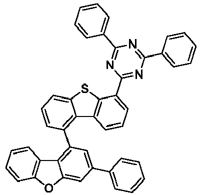 | 42% |
| 1-140 | 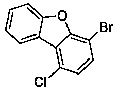 | 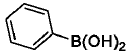 | 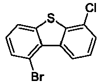 | 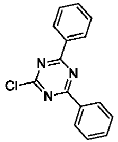 | 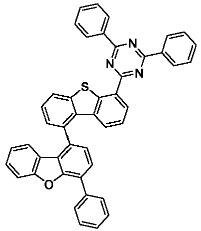 | 43% |
| 1-156 | 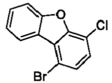 | 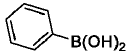 | 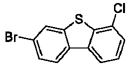 | 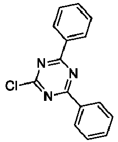 | 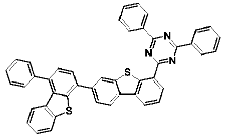 | 42% |
| 1-163 | 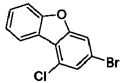 | 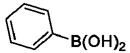 | 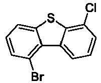 | 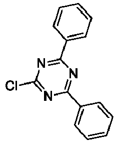 | 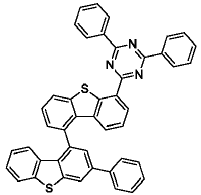 | 41% |
| 1-169 | 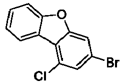 | 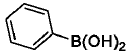 | 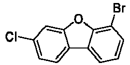 | 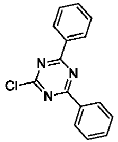 | 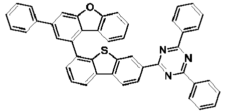 | 40% |
| 1-178 | 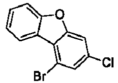 | 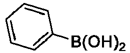 | 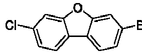 | 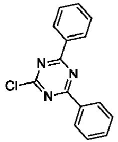 | 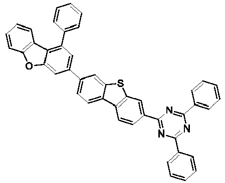 | 43% |
| 1-187 | 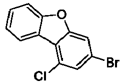 | 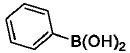 | 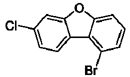 | 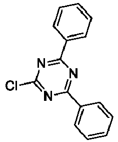 | 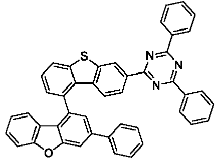 | 45% |
| 1-193 | 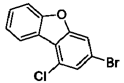 | 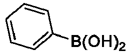 | 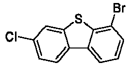 | 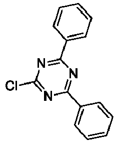 | 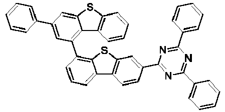 | 42% |
| 1-205 | 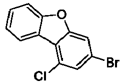 | 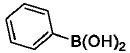 | 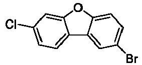 | 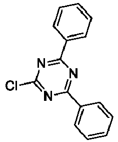 | 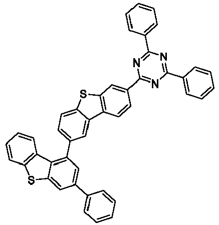 | 43% |
| 1-211 | 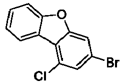 | 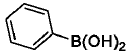 | 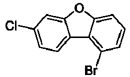 | 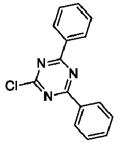 | 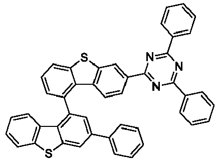 | 42% |
| 1-227 | 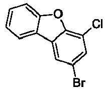 | 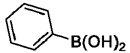 | 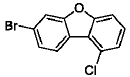 | 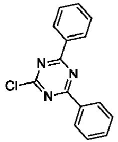 | 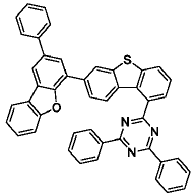 | 41% |
| 1-235 | 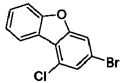 | 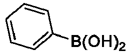 | 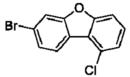 | 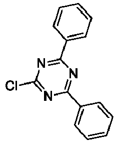 | 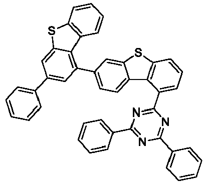 | 43% |
| 1-247 | 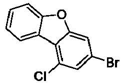 | 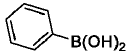 | 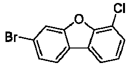 | 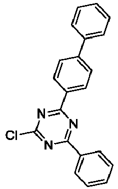 | 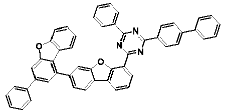 | 45% |
| 1-261 | 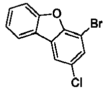 | 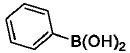 | 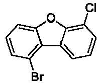 | 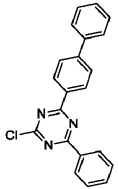 | 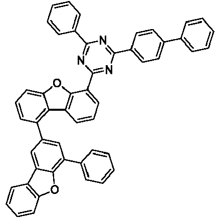 | 41% |
| 1-277 | 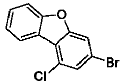 | 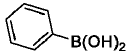 | 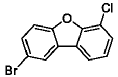 | 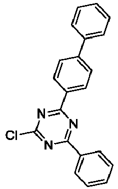 | 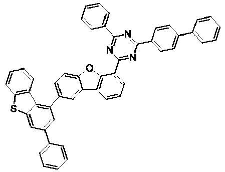 | 40% |
| 1-283 | 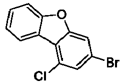 | 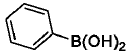 | 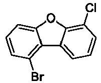 | 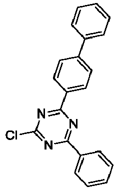 | 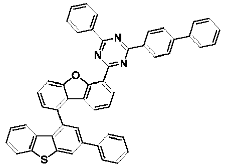 | 42% |
| 1-289 | 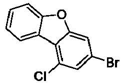 | 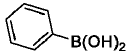 | 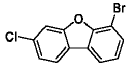 | 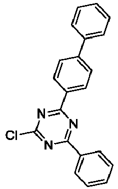 | 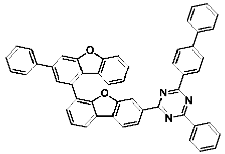 | 43% |
| 1-310 | 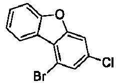 | 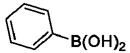 | 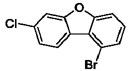 | 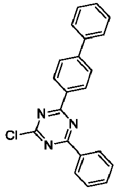 | 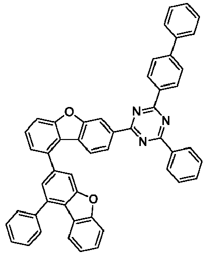 | 43% |
| 1-325 | 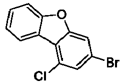 | 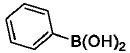 | 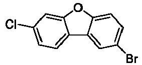 | 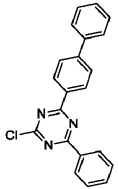 | 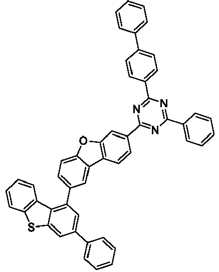 | 44% |
| 1-335 | 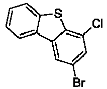 | 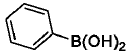 | 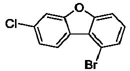 | 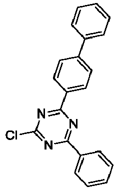 | 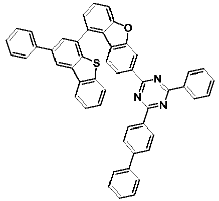 | 46% |
| 1-337 | 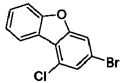 | 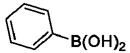 | 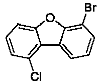 | 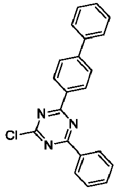 | 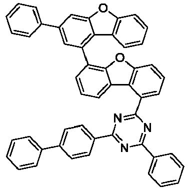 | 41% |
| 1-343 | 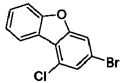 | 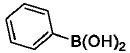 | 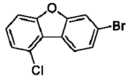 | 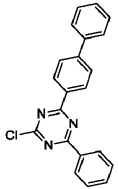 | 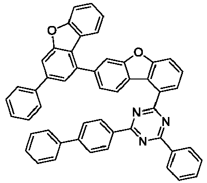 | 40% |
| 1-355 | 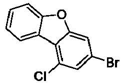 | 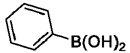 | 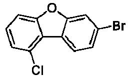 | 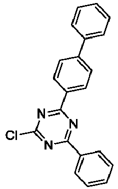 | 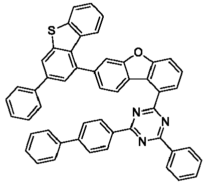 | 42% |
| 1-358 | 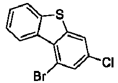 | 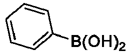 | 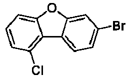 | 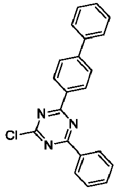 | 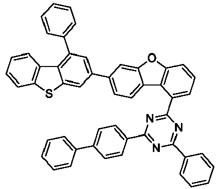 | 41% |
| 1-370 | 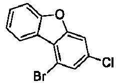 | 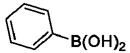 | 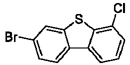 | 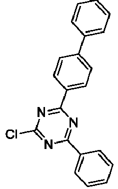 | 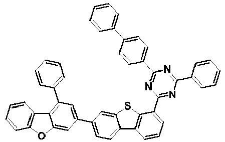 | 42% |
| 1-379 | 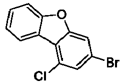 | 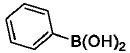 | 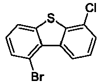 | 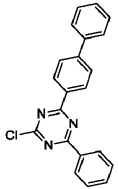 | 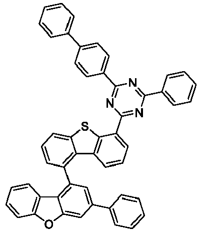 | 43% |
| 1-397 | 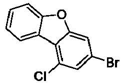 | 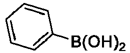 | 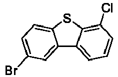 | 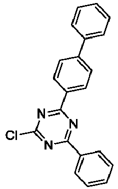 | 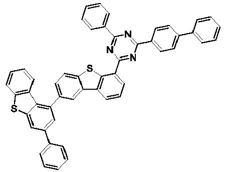 | 42% |
| 1-403 | 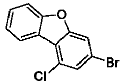 | 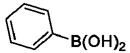 | 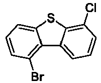 | 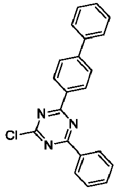 | 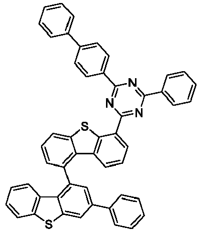 | 41% |
| 1-409 | 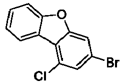 | 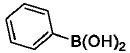 | 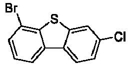 | 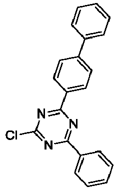 | 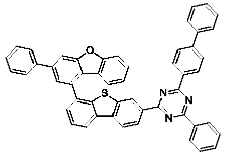 | 44% |
| 1-421 | 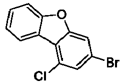 | 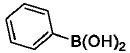 | 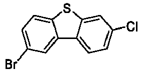 | 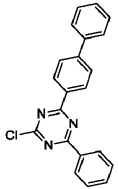 | 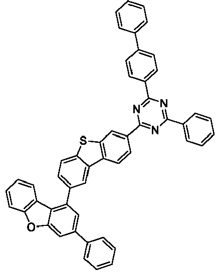 | 45% |
| 1-451 | 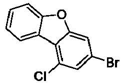 | 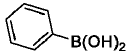 | 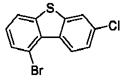 | 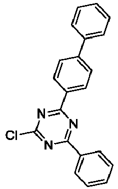 | 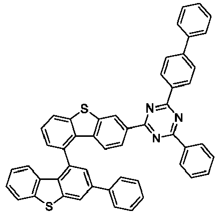 | 42% |
| 1-457 | 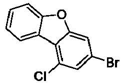 | 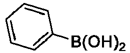 | 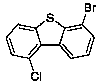 | 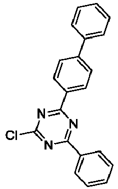 | 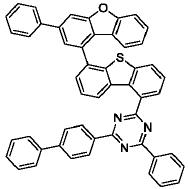 | 44% |
| 1-464 | 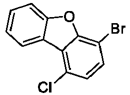 | 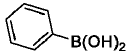 | 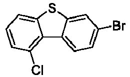 | 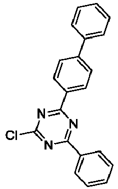 | 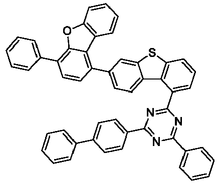 | 41% |
| 1-466 | 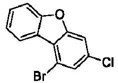 | 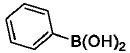 | 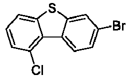 | 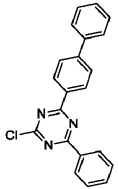 | 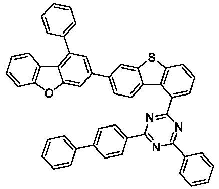 | 43% |
| 1-471 | 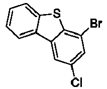 | 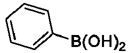 | 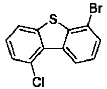 | 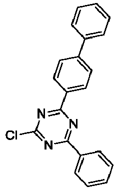 | 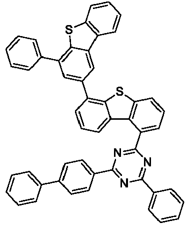 | 42% |
| 1-475 | 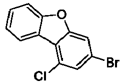 | 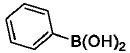 | 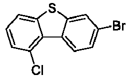 | 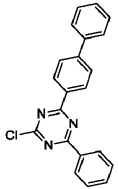 | 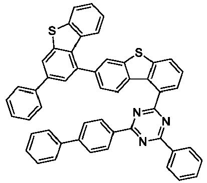 | 45% |
| 1-490 | 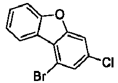 | 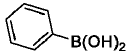 | 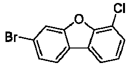 | 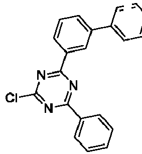 | 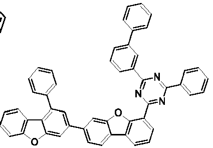 | 46% |
| 1-498 | 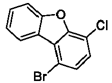 | 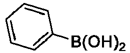 | 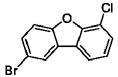 | 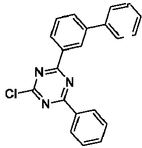 | 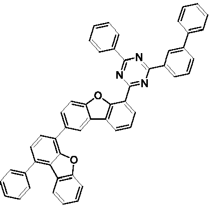 | 41% |
| 1-512 | 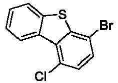 | 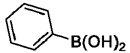 | 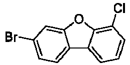 | 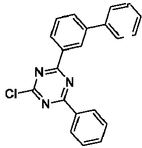 | 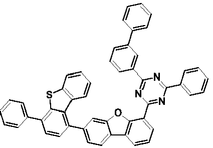 | 42% |
| 1-524 | 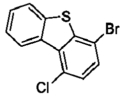 | 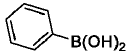 | 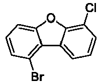 | 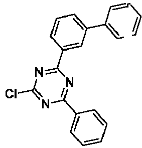 | 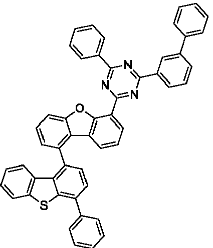 | 43% |
| 1-553 | 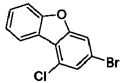 | 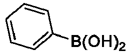 | 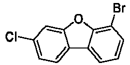 | 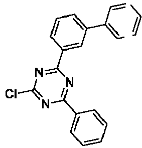 | 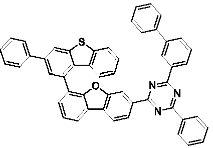 | 44% |
| 1-559 | 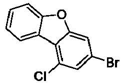 | 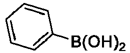 | 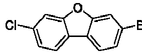 | 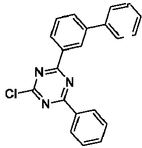 | 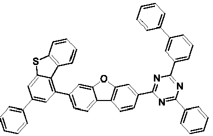 | 43% |
| 1-563 | 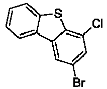 | 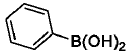 | 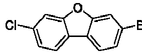 | 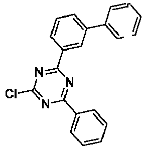 | 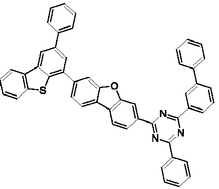 | 45% |
| 1-571 | 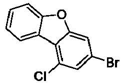 | 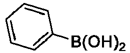 | 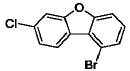 | 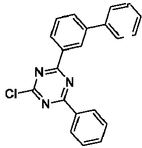 | 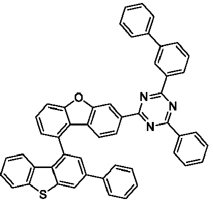 | 41% |
| 1-577 | 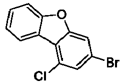 | 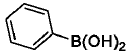 | 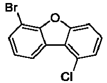 | 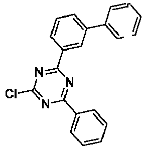 | 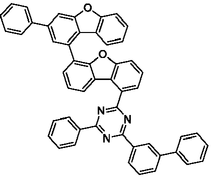 | 42% |
| 1-583 | 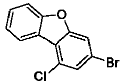 | 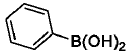 | 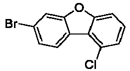 | 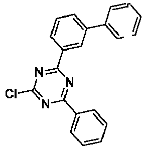 | 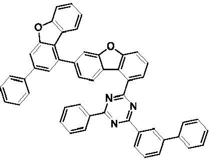 | 40% |
| 1-584 | 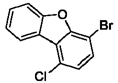 | 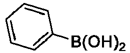 | 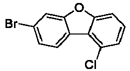 | 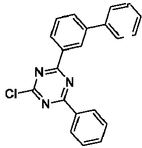 | 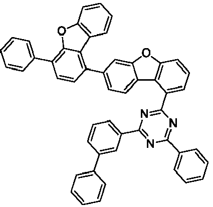 | 41% |
| 1-589 | 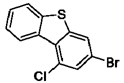 | 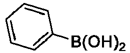 | 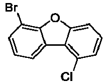 | 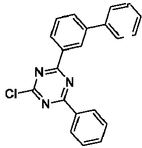 | 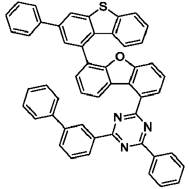 | 40% |
| 1-593 | 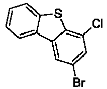 | 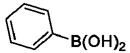 | 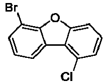 | 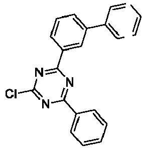 | 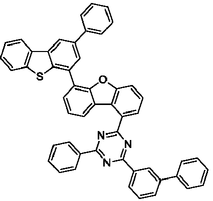 | 42% |
| 1-600 | 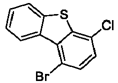 | 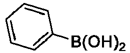 | 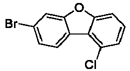 | 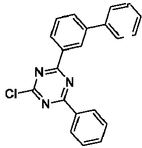 | 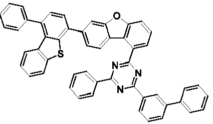 | 45% |
| 1-607 | 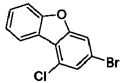 | 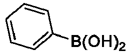 | 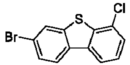 | 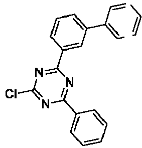 | 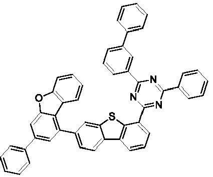 | 44% |
| 1-614 | 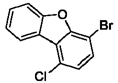 | 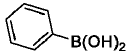 | 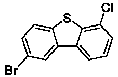 | 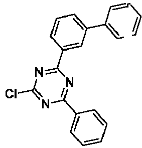 | 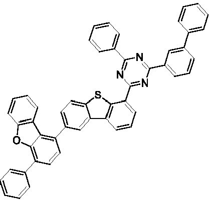 | 41% |
| 1-634 | 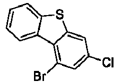 | 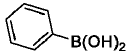 | 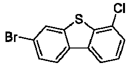 | 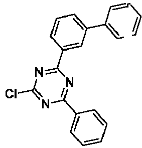 | 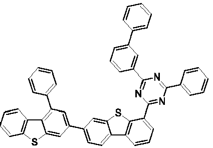 | 38% |
| 1-642 | 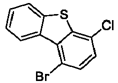 | 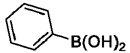 | 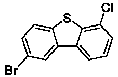 | 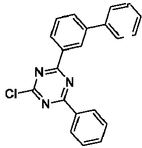 | 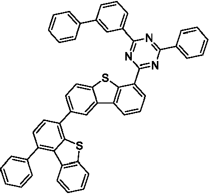 | 39% |
| 1-643 | 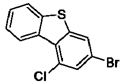 | 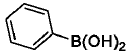 | 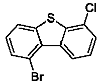 | 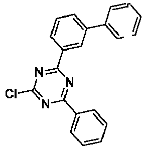 | 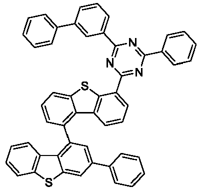 | 45% |
| 1-644 | 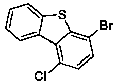 | 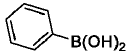 | 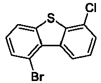 | 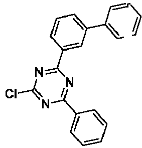 | 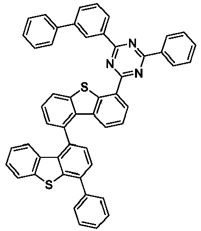 | 42% |
| 1-649 | 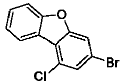 | 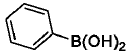 | 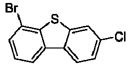 | 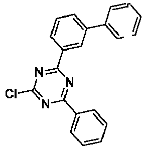 | 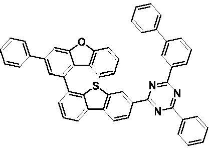 | 41% |
| 1-657 | 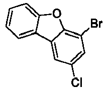 | 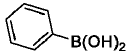 | 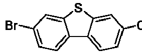 | 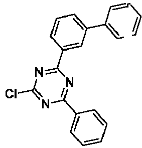 | 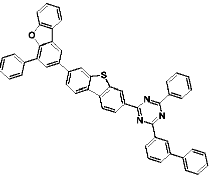 | 41% |
| 1-667 | 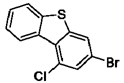 | 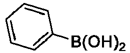 | 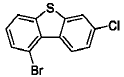 | 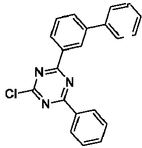 | 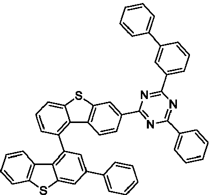 | 42% |
| 1-668 | 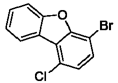 | 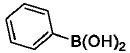 | 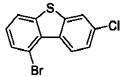 | 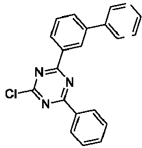 | 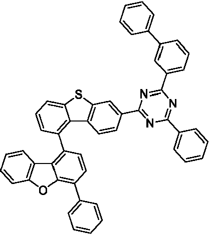 | 41% |
| 1-673 | 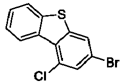 | 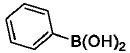 | 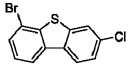 | 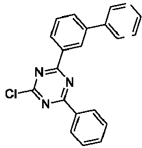 | 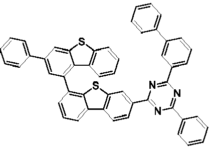 | 40% |
| 1-695 | 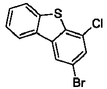 | 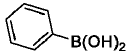 | 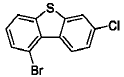 | 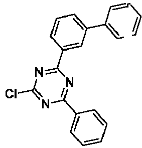 | 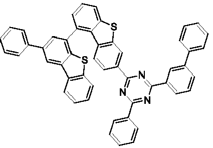 | 43% |
| 1-697 | 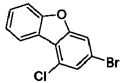 | 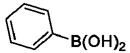 | 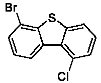 | 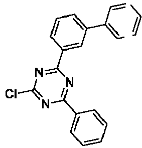 | 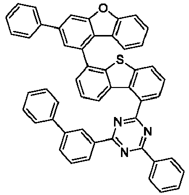 | 39% |
| 1-701 | 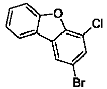 | 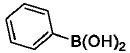 | 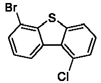 | 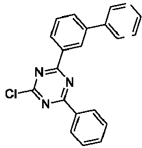 | 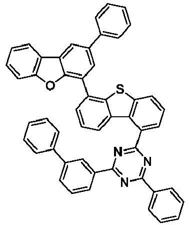 | 42% |
| 1-703 | 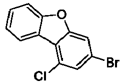 | 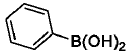 | 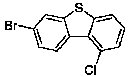 | 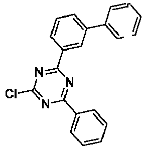 | 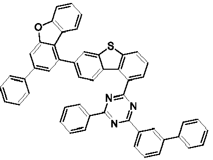 | 44% |
| 1-704 | 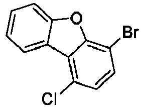 | 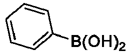 | 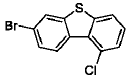 | 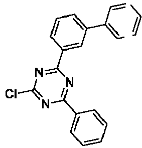 | 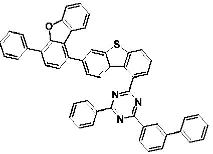 | 44% |
| 1-705 | 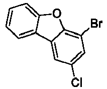 | 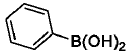 | 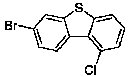 | 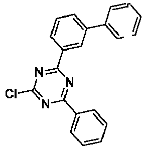 | 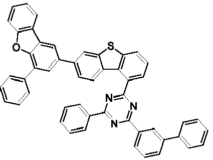 | 45% |
| 1-715 | 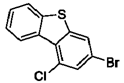 | 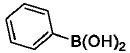 | 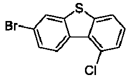 | 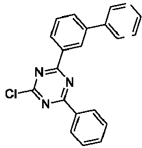 | 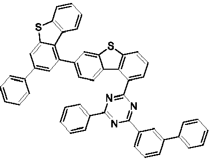 | 41% |
| 1-727 | 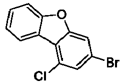 | 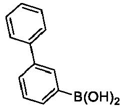 | 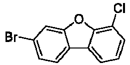 | 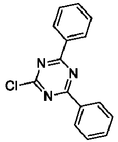 | 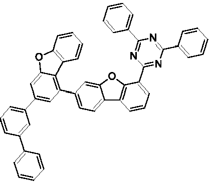 | 42% |
| 1-728 | 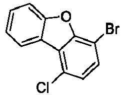 | 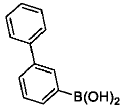 | 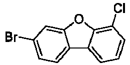 | 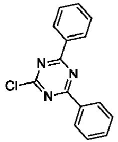 | 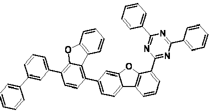 | 40% |
| 1-732 | 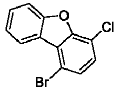 | 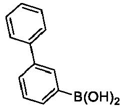 | 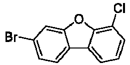 | 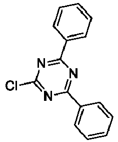 | 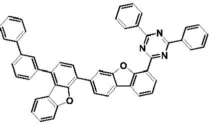 | 43% |
| 1-737 | 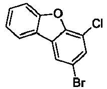 | 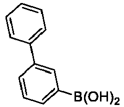 | 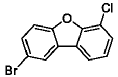 | 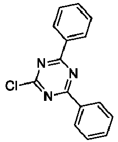 | 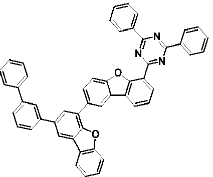 | 44% |
| 1-739 | 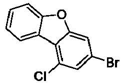 | 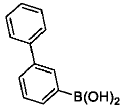 | 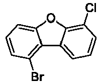 | 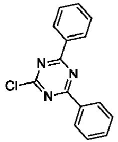 | 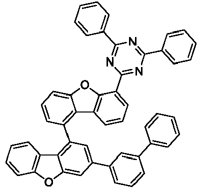 | 41% |
| 1-741 | 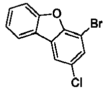 | 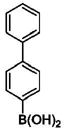 | 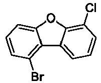 | 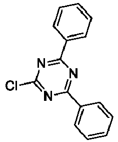 | 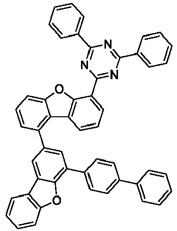 | 42% |
| 1-752 | 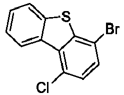 | 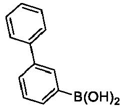 | 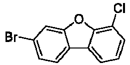 | 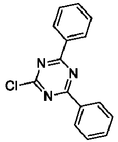 | 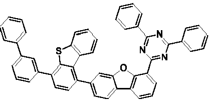 | 43% |
| 1-764 | 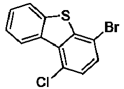 | 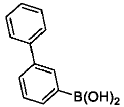 | 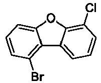 | 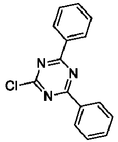 | 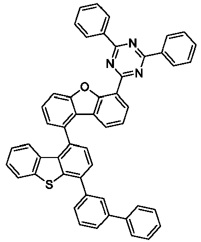 | 46% |
| 1-769 | 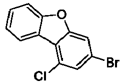 | 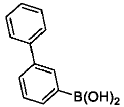 | 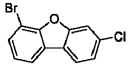 | 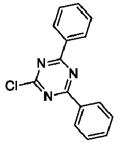 | 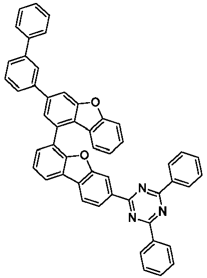 | 42% |
| 1-772 | 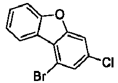 | 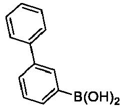 | 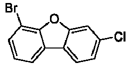 | 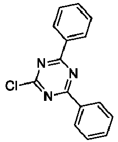 | 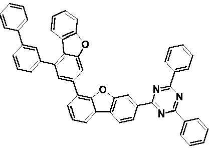 | 43% |
| 1-778 | 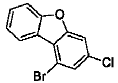 | 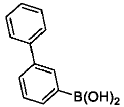 | 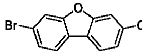 | 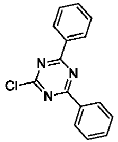 | 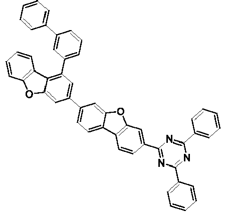 | 41% |
| 1-788 | 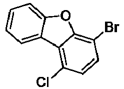 | 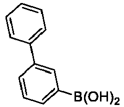 | 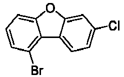 | 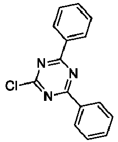 | 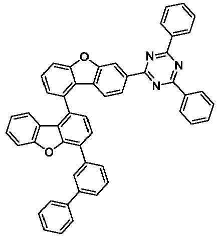 | 40% |
| 1-797 | 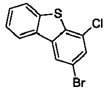 | 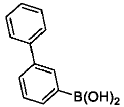 | 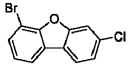 | 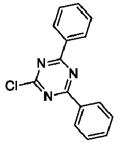 | 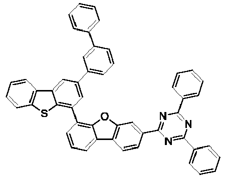 | 41% |
| 1-813 | 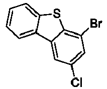 | 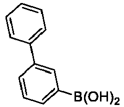 | 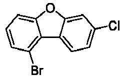 | 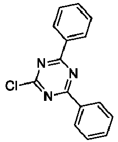 | 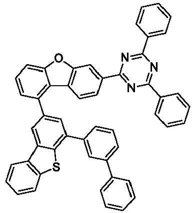 | 41% |
| 1-819 | 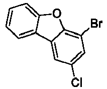 | 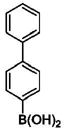 | 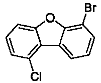 | 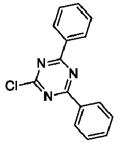 | 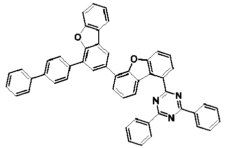 | 42% |
| 1-822 | 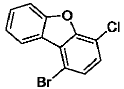 | 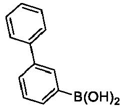 | 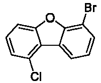 | 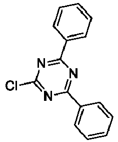 | 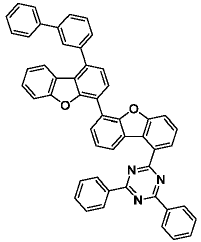 | 40% |
| 1-824 | 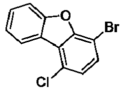 | 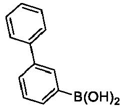 | 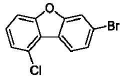 | 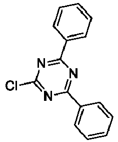 | 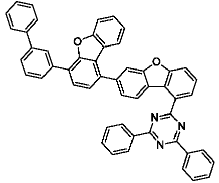 | 42% |
| 1-827 | 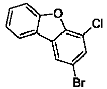 | 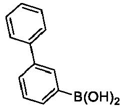 | 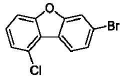 | 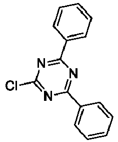 | 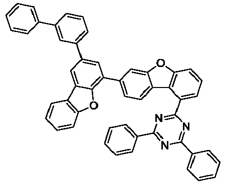 | 43% |
| 1-836 | 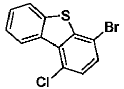 | 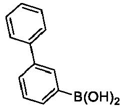 | 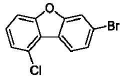 | 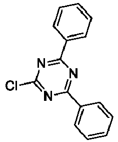 | 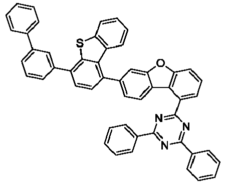 | 44% |
| 1-838 | 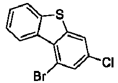 | 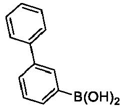 | 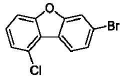 | 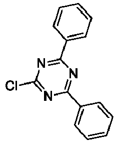 | 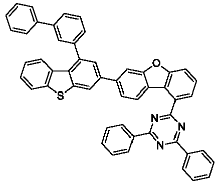 | 41% |
| 1-848 | 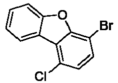 | 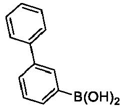 | 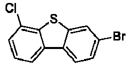 | 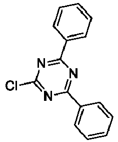 | 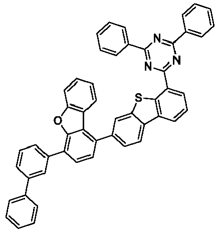 | 41% |
| 1-851 | 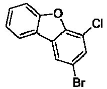 | 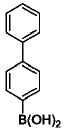 | 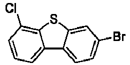 | 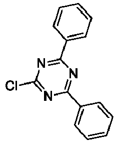 | 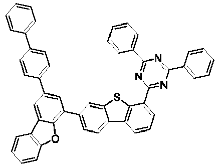 | 42% |
| 1-859 | 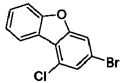 | 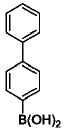 | 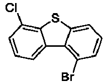 | 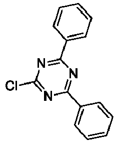 | 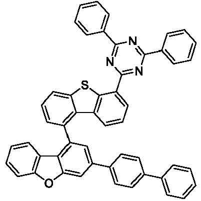 | 45% |
| 1-873 | 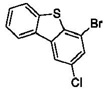 | 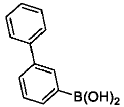 | 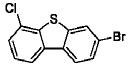 | 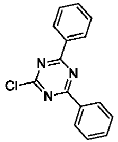 | 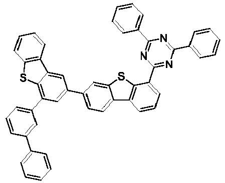 | 41% |
| 1-885 | 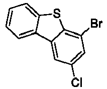 | 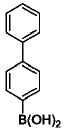 | 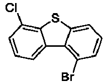 | 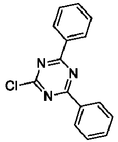 | 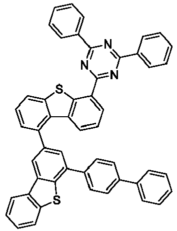 | 39% |
| 1-891 | 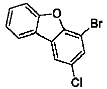 | 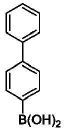 | 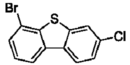 | 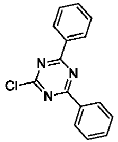 | 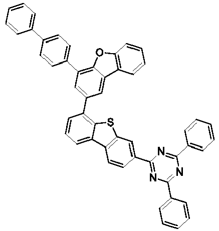 | 38% |
| 1-897 | 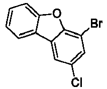 | 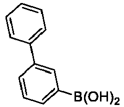 | 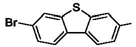 | 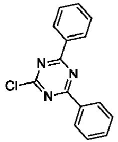 | 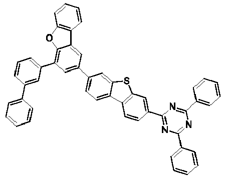 | 42% |
| 1-907 | 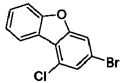 | 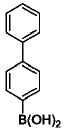 | 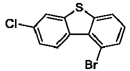 | 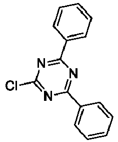 | 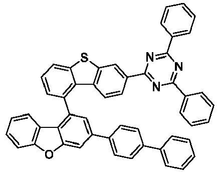 | 46% |
| 1-916 | 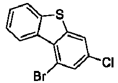 | 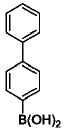 | 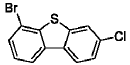 | 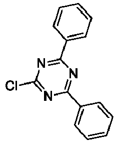 | 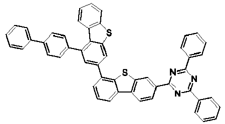 | 42% |
| 1-942 | 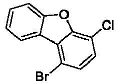 | 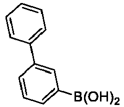 | 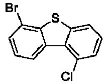 | 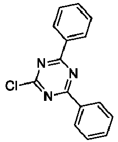 | 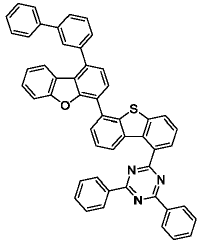 | 41% |
| 1-945 | 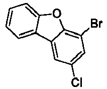 | 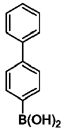 | 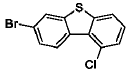 | 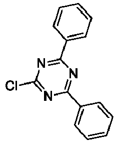 | 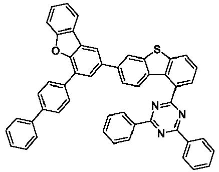 | 41% |
| 1-959 | 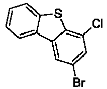 | 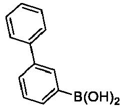 | 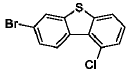 | 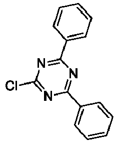 | 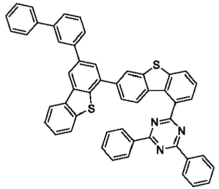 | 40% |
| 1-971 | 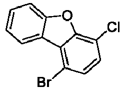 | 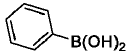 | 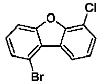 | 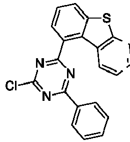 | 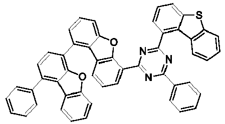 | 43% |
| 1-979 | 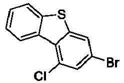 | 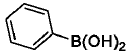 | 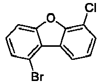 | 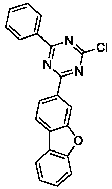 | 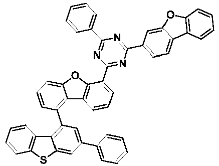 | 43% |
| 1-985 | 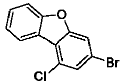 | 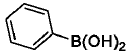 | 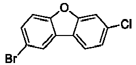 | 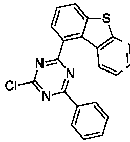 | 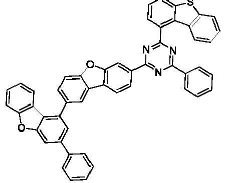 | 42% |
| 1-999 | 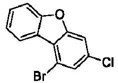 | 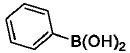 | 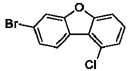 | 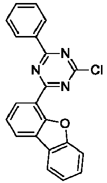 | 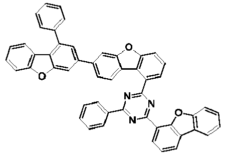 | 44% |
| 1-100 9 | 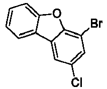 | 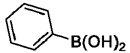 | 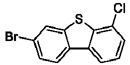 | 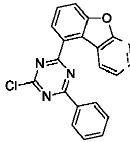 | 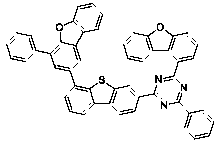 | 42% |
| 1-103 3 | 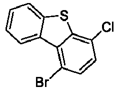 | 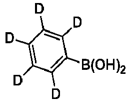 | 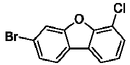 | 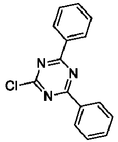 | 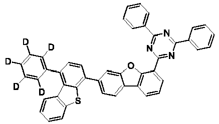 | 41% |
| 1-103 9 | 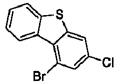 | 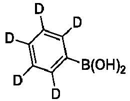 | 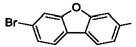 | 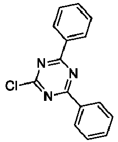 | 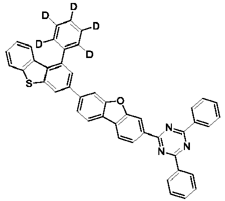 | 43% |
| 1-104 2 | 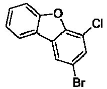 | 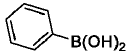 | 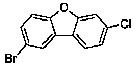 | 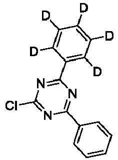 | 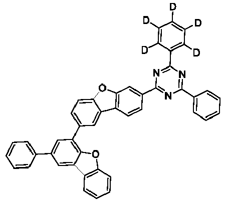 | 41% |
| 1-107 2 | 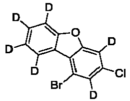 | 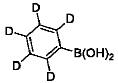 | 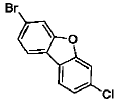 | 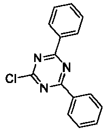 | 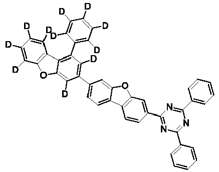 | 40% |
| 1-107 9 | 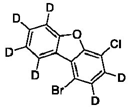 | 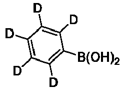 | 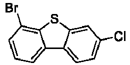 | 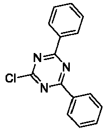 | 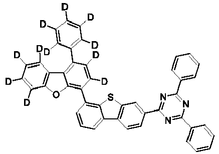 | 42% |
| 1-108 8 | 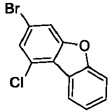 | 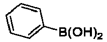 | 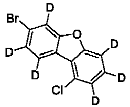 | 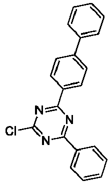 | 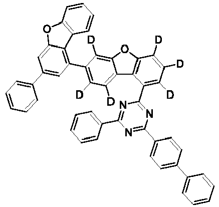 | 41% |
| 1-109 2 | 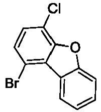 | 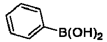 | 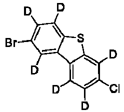 | 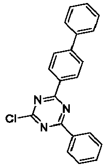 | 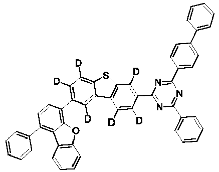 | 41% |
| 1-109 3 | 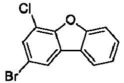 | 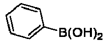 | 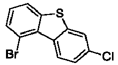 | 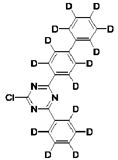 | 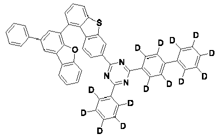 | 40% |
| 1-110 1 | 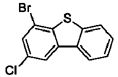 | 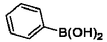 | 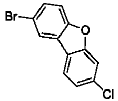 | 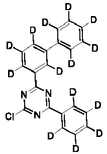 | 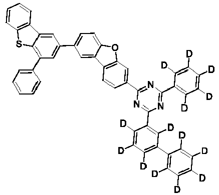 | 37% |
| 1-111 0 | 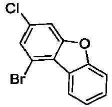 | 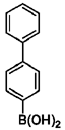 | 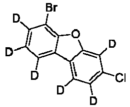 | 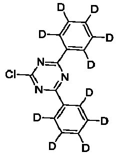 | 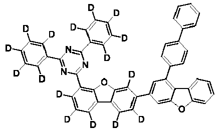 | 38% |
| 1-111 5 | 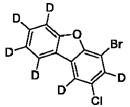 | 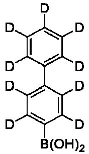 | 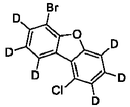 | 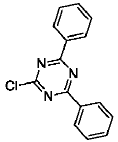 | 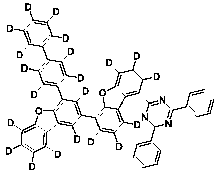 | 42% |

### <Preparation Example 4> Synthesis of compound 1-1124

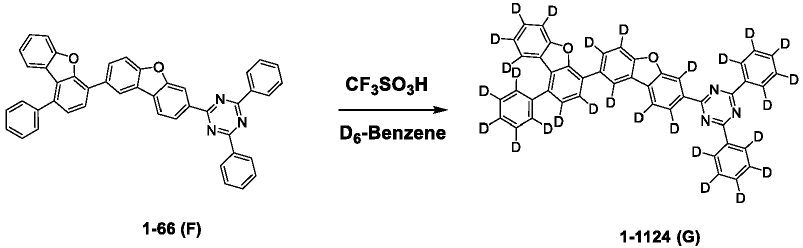

To one neck round bottom flask, a mixture of 10 g (0.016 mol) of compound 1-66, 33.6 g (0.27 mol) of triflic acid, and 100 ml of D6-benzene was added and refluxed at 50 °C. the resultant was extracted with dichloromethane and water, the organic layer was dried over MgSO₄, concentrated, and purified by column chromatography (DCM:hexane=1:2). Thereafter, the separated solution was concentrated and then recrystallized from methanol to obtain 8.5 g (yield: 82%) of the desired compound 1-1124.

The following desired compound G was synthesized in the same manner as in Preparation Example 4 above, except that in Preparation Example 4, compound F of Table 4 below instead of compound 1-66 was used.

**Table 4:**

| Compound | F | G | Yield |
|---|---|---|---|
| 1-1124 | 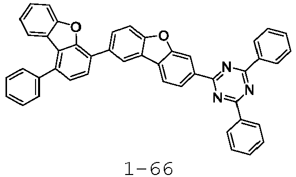 | 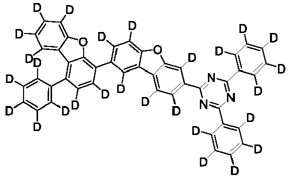 | 82% |
| 1-1133 | 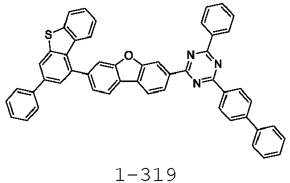 | 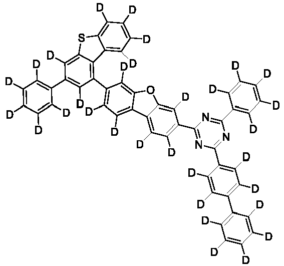 | 83% |
| 1-1148 | 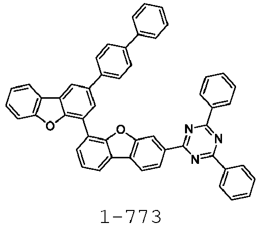 | 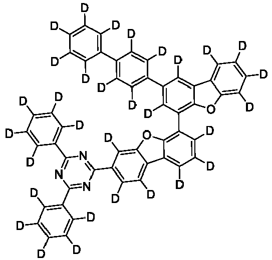 | 85% |
| 1-1155 | 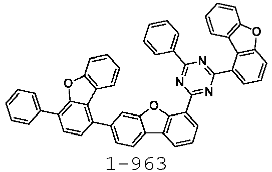 | 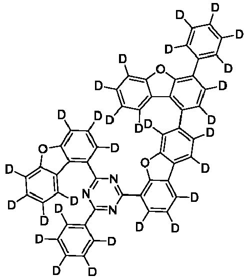 | 80% |

The remaining compounds other than the compounds described in Preparation Examples 1 to 4 and Tables 1 to 4 were prepared in the same manner as in the above-described Preparation Example, and the synthesis results are shown in Tables 5 and 6 below.

**Table 5:**

| Compound | FD-Mass | Compound | FD-Mass |
|---|---|---|---|
| 1-1 | m/z= 641.21 (C45H27N3O2=641.73) | 1-563 | m/z= 733.22 (C51H31N3OS=733.89) |
| 1-7 | m/z= 641.21 (C45H27N3O2=641.73) | 1-571 | m/z= 733.22 (C51H31N3OS=733.89) |
| 1-14 | m/z= 641.21 (C45H27N3O2=641.73) | 1-577 | m/z= 717.24 (C51H31N3O2=717.83) |
| 1-16 | m/z= 641.21 (C45H27N3O2=641.73) | 1-583 | m/z= 717.24 (C51H31N3O2=717.83) |
| 1-19 | m/z= 641.21 (C45H27N3O2=641.73) | 1-584 | m/z= 717.24 (C51H31N3O2=717.83) |
| 1-23 | m/z= 641.21 (C45H27N3O2=641.73) | 1-589 | m/z= 733.22 (C51H31N3OS=733.89) |
| 1-25 | m/z= 657.19 (C45H27N3OS=657.79) | 1-593 | m/z= 733.22 (C51H31N3OS=733.89) |
| 1-31 | m/z= 657.19 (C45H27N3OS=657.79) | 1-600 | m/z= 733.22 (C51H31N3OS=733.89) |
| 1-34 | m/z= 657.19 (C45H27N3OS=657.79) | 1-607 | m/z= 733.22 (C51H31N3OS=733.89) |
| 1-43 | m/z= 641.21 (C45H27N3O2=641.73) | 1-614 | m/z= 733.22 (C51H31N3OS=733.89) |
| 1-49 | m/z= 641.21 (C45H27N3O2=641.73) | 1-634 | m/z= 749.20 (C51H31N3S2=749.95) |
| 1-54 | m/z= 641.21 (C45H27N3O2=641.73) | 1-642 | m/z= 749.20 (C51H31N3S2=749.95) |
| 1-58 | m/z= 641.21 (C45H27N3O2=641.73) | 1-643 | m/z= 749.20 (C51H31N3S2=749.95) |
| 1-66 | m/z= 641.21 (C45H27N3O2=641.73) | 1-644 | m/z= 749.20 (C51H31N3S2=749.95) |
| 1-70 | m/z= 641.21 (C45H27N3O2=641.73) | 1-649 | m/z= 733.22 (C51H31N3OS=733.89) |
| 1-73 | m/z= 657.19 (C45H27N3OS=657.79) | 1-657 | m/z= 733.22 (C51H31N3OS=733.89) |
| 1-79 | m/z= 657.19 (C45H27N3OS=657.79) | 1-667 | m/z= 749.20 (C51H31N3S2=749.95) |
| 1-87 | m/z= 657.19 (C45H27N3OS=657.79) | 1-668 | m/z= 733.22 (C51H31N3OS=733.89) |
| 1-97 | m/z= 641.21 (C45H27N3O2=641.73) | 1-673 | m/z= 749.20 (C51H31N3S2=749.95) |
| 1-104 | m/z= 641.21 (C45H27N3O2=641.73) | 1-695 | m/z= 749.20 (C51H31N3S2=749.95) |
| 1-109 | m/z= 657.19 (C45H27N3OS=657.79) | 1-697 | m/z= 733.22 (C51H31N3OS=733.89) |
| 1-119 | m/z= 657.19 (C45H27N3OS=657.79) | 1-701 | m/z= 733.22 (C51H31N3OS=733.89) |
| 1-121 | m/z= 657.19 (C45H27N3OS=657.79) | 1-703 | m/z= 733.22 (C51H31N3OS=733.89) |
| 1-129 | m/z= 657.19 (C45H27N3OS=657.79) | 1-704 | m/z= 733.22 (C51H31N3OS=733.89) |
| 1-139 | m/z= 657.19 (C45H27N3OS=657.79) | 1-705 | m/z= 733.22 (C51H31N3OS=733.89) |
| 1-140 | m/z= 657.19 (C45H27N3OS=657.79) | 1-715 | m/z= 749.20 (C51H31N3S2=749.95) |
| 1-156 | m/z= 673.16 (C45H27N3S2=673.85) | 1-727 | m/z= 717.24 (C51H31N3O2=717.83) |
| 1-163 | m/z= 673.16 (C45H27N3S2=673.85) | 1-728 | m/z= 717.24 (C51H31N3O2=717.83) |
| 1-169 | m/z= 657.19 (C45H27N3OS=657.79) | 1-732 | m/z= 717.24 (C51H31N3O2=717.83) |
| 1-178 | m/z= 657.19 (C45H27N3OS=657.79) | 1-737 | m/z= 717.24 (C51H31N3O2=717.83) |
| 1-187 | m/z= 657.19 (C45H27N3OS=657.79) | 1-739 | m/z= 717.24 (C51H31N3O2=717.83) |
| 1-193 | m/z= 673.16 (C45H27N3S2=673.85) | 1-741 | m/z= 717.24 (C51H31N3O2=717.83) |
| 1-205 | m/z= 673.16 (C45H27N3S2=673.85) | 1-752 | m/z= 733.22 (C51H31N3OS=733.89) |
| 1-211 | m/z= 673.16 (C45H27N3S2=673.85) | 1-764 | m/z= 733.22 (C51H31N3OS=733.89) |
| 1-227 | m/z= 657.19 (C45H27N3OS=657.79) | 1-769 | m/z= 717.24 (C51H31N3O2=717.83) |
| 1-235 | m/z= 673.16 (C45H27N3S2=673.85) | 1-772 | m/z= 717.24 (C51H31N3O2=717.83) |
| 1-247 | m/z= 641.21 (C45H27N3O2=641.73) | 1-778 | m/z= 717.24 (C51H31N3O2=717.83) |
| 1-261 | m/z= 641.21 (C45H27N3O2=641.73) | 1-788 | m/z= 717.24 (C51H31N3O2=717.83) |
| 1-277 | m/z= 657.19 (C45H27N3OS=657.79) | 1-797 | m/z= 733.22 (C51H31N3OS=733.89) |
| 1-283 | m/z= 657.19 (C45H27N3OS=657.79) | 1-813 | m/z= 733.22 (C51H31N3OS=733.89) |
| 1-289 | m/z= 641.21 (C45H27N3O2=641.73) | 1-819 | m/z= 717.24 (C51H31N3O2=717.83) |
| 1-310 | m/z= 641.21 (C45H27N3O2=641.73) | 1-822 | m/z= 717.24 (C51H31N3O2=717.83) |
| 1-325 | m/z= 657.19 (C45H27N3OS=657.79) | 1-824 | m/z= 717.24 (C51H31N3O2=717.83) |
| 1-335 | m/z= 657.19 (C45H27N3OS=657.79) | 1-827 | m/z= 717.24 (C51H31N3O2=717.83) |
| 1-337 | m/z= 717.24 (C51H31N3O2=717.83) | 1-836 | m/z= 733.22 (C51H31N3OS=733.89) |
| 1-343 | m/z= 717.24 (C51H31N3O2=717.83) | 1-838 | m/z= 733.22 (C51H31N3OS=733.89) |
| 1-355 | m/z= 733.22 (C51H31N3OS=733.89) | 1-848 | m/z= 733.22 (C51H31N3OS=733.89) |
| 1-358 | m/z= 733.22 (C51H31N3OS=733.89) | 1-851 | m/z= 733.22 (C51H31N3OS=733.89) |
| 1-370 | m/z= 733.22 (C51H31N3OS=733.89) | 1-859 | m/z= 733.22 (C51H31N3OS=733.89) |
| 1-379 | m/z= 733.22 (C51H31N3OS=733.89) | 1-873 | m/z= 749.20 (C51H31N3S2=749.95) |
| 1-397 | m/z= 749.20 (C51H31N3S2=749.95) | 1-885 | m/z= 749.20 (C51H31N3S2=749.95) |
| 1-403 | m/z= 749.20 (C51H31N3S2=749.95) | 1-891 | m/z= 733.22 (C51H31N3OS=733.89) |
| 1-409 | m/z= 733.22 (C51H31N3OS=733.89) | 1-897 | m/z= 733.22 (C51H31N3OS=733.89) |
| 1-421 | m/z= 733.22 (C51H31N3OS=733.89) | 1-907 | m/z= 733.22 (C51H31N3OS=733.89) |
| 1-451 | m/z= 749.20 (C51H31N3S2=749.95) | 1-916 | m/z= 749.20 (C51H31N3S2=749.95) |
| 1-457 | m/z= 733.22 (C51H31N3OS=733.89) | 1-942 | m/z= 733.22 (C51H31N3OS=733.89) |
| 1-464 | m/z= 733.22 (C51H31N3OS=733.89) | 1-945 | m/z= 733.22 (C51H31N3OS=733.89) |
| 1-466 | m/z= 733.22 (C51H31N3OS=733.89) | 1-959 | m/z= 749.20 (C51H31N3S2=749.95) |
| 1-471 | m/z= 749.20 (C51H31N3S2=749.95) | 1-971 | m/z= 747.20 (C51H29N3O2S=747.87) |
| 1-475 | m/z= 749.20 (C51H31N3S2=749.95) | 1-979 | m/z= 733.22 (C51H31N3OS=733.89) |
| 1-490 | m/z= 717.24 (C51H31N3O2=717.83) | 1-985 | m/z= 747.20 (C51H29N3O2S=747.87) |
| 1-498 | m/z= 717.24 (C51H31N3O2=717.83) | 1-999 | m/z= 733.22 (C51H31N3OS=733.89) |
| 1-512 | m/z= 733.22 (C51H31N3OS=733.89) | 1-1009 | m/z= 747.20 (C51H29N3O2S=747.87) |
| 1-524 | m/z= 733.22 (C51H31N3OS=733.89) | 1-1033 | m/z= 662.22 (C45H22D5N3OS=662.82) |
| 1-553 | m/z= 733.22 (C51H31N3OS=733.89) | 1-1039 | m/z= 662.22 (C45H22D5N3OS=662.82) |
| 1-559 | m/z= 733.22 (C51H31N3OS=733.89) | 1-1042 | m/z= 646.24 (C45H22D5N3O2=646.76) |
| 1-1072 | m/z= 652.28 (C₄₅H₁₆D₁₁N₃O₂=652.80) | 1-1079 | m/z= 668.26 (C₄₅H₁₆D₁₁N₃OS=668.86) |
| 1-1088 | m/z= 723.28 (C₅₁H₂₅D₆N₃O₂=723.86) | 1-1092 | m/z= 739.26 (C₅₁H₂₅D₆N₃OS=739.93) |
| 1-1093 | m/z= 747.31 (C₅₁H₁₇D₁₄N₃OS=747.97) | 1-1101 | m/z= 747.31 (C₅₁H₁₇D₁₄N₃OS=747.97) |
| 1-1110 | m/z= 733.34 (C₅₁H₁₅D₁₆N₃O₂=733.93) | 1-1115 | m/z= 738.37 (C₅₁H₁₀D₂₁N₃O₂=738.96) |
| 1-1124 | m/z= 668.38 (C₄₅D₂₇N₃O₂=668.89) | 1-1133 | m/z= 764.41 (C₅₁D₃₁N₃OS=765.08) |
| 1-1148 | m/z= 748.44 (C₅₁D₃₁N₃O₂=749.02) | 1-1155 | m/z= 760.40 (C₅₁D₂₉N₃O₃=760.99) |

**Table 6:**

| Compound | ¹H NMR(CDCl₃, 200Mz) |
|---|---|
| 1-1 | δ = 8.36(4H, d), 8.08(2H, d), 8.02-7.98(2H, m), 7.88(2H, d), 7. 81(1H, s), 7.75(2H, d), 7.51-7.31(14H, m) |
| 1-7 | δ = 8.36(4H, d), 8.08-7.98(3H, m), 7.88-7.75(7H, m), 7.54-7.31(13H, m) |
| 1-14 | δ = 8.36(4H, d), 8.08(2H, d), 8.01-7.98(2H, m), 7.88-7.79(4H, m), 7.51-7.31(15H, m) |
| 1-16 | δ = 8.36(4H, d), 8.08(1H, d), 7.98(1H, d), 7.88-7.79(8H, m), 7.54-7.31(13H, m) |
| 1-19 | δ = 8.36(4H, d), 8.08(1H, d), 7.98(1H, d), 7.88-7.69(7H, m), 7.57-7.31(14H, m) |
| 1-23 | δ = 8.36(4H, d), 8.08(1H, d), 7.98(1H, d), 7.93(1H, s), 7.88-7.69(6H, m), 7.57-7.31(14H, m) |
| 1-25 | δ = 8.45 (1H, d), 8.36-8.30(5H, m), 8.08(2H, d), 8.02(2H, d), 7.94(1H, d), 7.88(1H, d), 7.75(2H, d), 7.56-7.41(13H, m) |
| 1-31 | δ = 8.45(1H, d), 8.36-8.30(5H, m), 8.08(2H, d), 8.01(1H, s), 7.93-7.82(4H, m), 7.75(2H, d), 7.56-7.41(12H, m) |
| 1-34 | δ = 8.45(1H, d), 8.36-8.30(5H, m), 8.08(2H, d), 8.01(1H, s), 7.93-7.79(6H, m), 7.56-7.31(12H, m) |
| 1-43 | δ = 8.45(1H, d), 8.36-8.30(5H, m), 8.08(1H, d), 8.01(1H, s), 7.93-7.82(5H, m), 7.69(1H, d), 7.57-7.41(13H, m) |
| 1-49 | δ = 8.36(4H, d), 8.08(1H, d), 8.03-7.98 (3H, m), 7.86-7.75(6H, m), 7.54-7.31(13H, m) |
| 1-54 | δ = 8.36(4H, d), 8.08-7.98(6H, m), 7.82-7.76(4H, m), 7.54-7.31(13H, m) |
| 1-58 | δ = 8.36(4H, d), 8.03(2H, d), 7.98(1H, d), 7.86-7.76(8H, m), 7.54-7.31(12H, m) |
| 1-66 | δ = 8.36(4H, d), 8.07-7.98(4H, m), 7.99-7.76(7H, m), 7.54-7.31(12H, m) |
| 1-70 | δ = 8.36(4H, d), 8.02(1H, d), 7.98(1H, d), 7. 82-7.76 (7H, m), 7.69(1H, d), 7.57-7.31(13H, m) |
| 1-73 | δ = 8.45(1H, d), 8.36(4H, d), 8.30(1H, s), 8.08(1H, d), 8.03-8.01(3H, m), 7.93(1H, d), 7.82(1H, d), 7.75(3H, d), 7.57-7.41(12H, m) |
| 1-79 | δ = 8.45(1H, d), 8.36(4H, d), 8.30(1H, s), 8.03(3H, d), 7.93(1H, d), 7.82-7.75(6H, m), 7.56-7.41(11H, m) |
| 1-87 | δ = 8.45(1H, d), 8.36(4H, d), 8.22(1H, s), 8.03(1h, d), 7.99(1H, d), 7.93-7.76(6H, m), 7.56-7.41(13H, m) |
| 1-97 | δ = 8.36(4H, d), 8.08(1H, d), 8.01(2H, d), 7.86-7.75(6H, m), 7.57-7.41(14H, m) |
| 1-104 | δ = 8.36(4H, d), 8.07-7.98(4H, m), 7.82(2H, d), 7.76 (1H, s), 7.69(1H, d), 7.57-7.31(15H, m) |
| 1-109 | δ = 8.45(1H, d), 8.36(4H, d), 8.30(1H, s), 8.06(2H, d), 8.01(1H, s), 7.93(1H, d), 7.82(2H, d), 7.75(2H, d), 7.69(1H, d), 7.57-7.41(13H, m) |
| 1-119 | δ = 8.45(1H, d), 8.36(4H, d), 8.22(1H, s), 8.03(1H, d), 7.98(1H, s), 7.93(1H, d), 7.82(2H, d), 7.79-7.75(4H, m), 7.57-7.41(12H, m) |
| 1-121 | δ = 8.55 (2H, d), 8.36-8.32 (5H, m), 7.98(1H, d), 7.92(1H, d), 7.86(1H, s), 7.81(1H, s), 7.75-7.69(4H, m), 7.57-7.41(12H, m) |
| 1-129 | δ = 8.55(1H, d), 8.36(4H, d), 8.24-8.19(3H, m), 7.98(1H, d), 7.93-7.87(3H, m), 7.70(1H, t), 7.51-7.31(14H, m) |
| 1-139 | δ = 8.55(1H, d), 8.36(4H, d), 8.03-7.70(10H, m), 7.54-7.31(12H, m) |
| 1-140 | δ = 8.55(1H, d), 8.36(4H, d), 8.07-7.92(6H, m), 7.70 (1H, d), 7.68(1H, d), 7.54-7.31 (14H, m) |
| 1-156 | δ = 8.55(1H, d), 8.45(1H, d), 8.36(4H, d), 8.24-8.11(5H, m), 7.92(2H, d), 7.79(2H, d), 7.70(1H, t), 7.56-7.41(11H, m) |
| 1-163 | δ = 8.55(1H, d), 8.45(1H, d), 8.36(4H, d), 8.30(1H, s), 8.03(2H, d), 7.94-7.92(3H, m), 7.75-7.68(4H, m), 7.56-7.41(11H, m) |
| 1-169 | δ = 8.45 (1H, d), 8.36-8.32 (5H, m), 8.24(2H, d), 7.94(2H, d), 7.86(1H, s), 7.81(1H, s), 7.75-7.70(3H, m), 7.54-7.39(12H, m) |
| 1-178 | δ = 8.36(4H, d), 8.24-8.17(5H, m), 7.98-7.94(2H, m), 7.86(1H, s), 7.81-7.79(3H, m), 7.54-7.31(12H, m) |
| 1-187 | δ = 8.36(4H, d), 8.24(1H, d), 8.20(1h, s), 8.03-7.94(4H, m), 7.86(1H, s), 7.77-7.68(4H, m), 7.54-7.31(12H, m) |
| 1-193 | δ = 8.55(1H, d), 8.45(1H, d), 8.36-8.24(8H, m), 8.01(1H, s), 7.94(2H, d), 7.75-7.70(3H, m), 7.56-7.31(11H, m) |
| 1-205 | δ = 8.45(1H, d), 8.36(4H, d), 8.30(1H, d), 8.24(1h, d), 8.20(1H, d), 8.12(2H, d), 8.01-7.94(4H, m), 7.75(2H, d), 7.56-7.41(11H, m) |
| 1-211 | δ = 8.45(1H, d), 8.36(4H, d), 8.30(1H, d), 8.24(2H, d), 8.03(2H, d), 7.94-7.91(3H, m), 7.75-7.68(3H, m), 7.56-7.41(11H, m) |
| 1-227 | δ = 8.36(4H, d), 8.24-8.17(3H, m), 8.03-7.93(4H, m), 7.87(1H, s), 7.75-7.68(3H, m), 7.56-7.31(12H, m) |
| 1-235 | δ = 8.45(1H, d), 8.36(4H, d), 8.30(1H, s), 8.24-8.17(3H, m), 8.03(2H, d), 7.94(2H, d), 7.75-7.68(3H, m), 7.51-7.41(11H, m) |
| 1-247 | δ = 8.36(2H, d), 8.08(1H, d), 8.03(1H, d), 7.98-7.96(3H, m), 7.88-7.75(9H, m) 7.54-7.41(15H, m) |
| 1-261 | δ = 8.36(2H, d), 8.08(1H, d), 8.03(1H, d), 7.98-7.94(3H, m), 7.86-7.75(9H, m), 7.54-7.25(15H, m) |
| 1-277 | δ = 8.36(2H, d), 8.08(1H, d), 7.98-7.69(10H, m), 7.54-7.31(18H, m) |
| 1-283 | δ = 8.45(1H, d), 8.36(2H, d), 8.30(1H, s), 8.08(1H, d), 8.01-7.75(12H, m), 7.51-7.41(12H, m), 7.25(2H, d) |
| 1-289 | δ = 8.45(1H, d), 8.46(2H, d), 8.30(1H, s), 8.08(1H, d), 8.01(1H, s), 7.93-7.75(10H, m), 7.57-7-41(13H, m), 7.25(2H, d) |
| 1-310 | δ = 8.36(2H, d), 7.98-7.74(4H, m), 7.82-7.69(10H, m), 7.57-7.25(15H, m) |
| 1-325 | δ = 8.45(1H, d), 8.36(2H, d), 8.30(1H, s), 8.01-7.75(14H, m), 7.56-7.41(11H, m), 7.25(2H, d) |
| 1-335 | δ = 8.45(1H, d), 8.36(2H, d), 8.22(1H, s), 8.03-7.93(5H, m), 7.82(2H, d), 7.76-7.69(6H, m), 7.57-7.41(12H, m), 7.25(2H, d) |
| 1-337 | δ = 8.36(2H, d), 8.08(1H, d),8.02-7.96(4H, m), 7.82-7.75(8H, m), 7.57-7.25(16H, m) |
| 1-343 | δ = 8.36(2H, d),8.03(1H, d), 7.98-7.96(3H, m), 7.86-7.69(10H, m), 7.57-7.25(15H, m) |
| 1-355 | δ = 8.45(1H, d), 8.36(2H, d), 8.30(1H, s), 8.03-7.93(5H, m), 7.82-7.69(8H, m), 7.57-7.31(12H, m), 7.25(2H, d) |
| 1-358 | δ = 8.45(1H, d), 8.36(2H, d), 8.30(1H, s), 8.03-7.96(5H, m), 7.82-7.69(8H, m), 7.57-7.31(12H, m), 7.25(2H, d) |
| 1-370 | δ = 8.55(1H, d), 8.36(2H, d), 8.24-8.20 (3h, m), 7.96-7.89(5H, m), 7.86(1H, s), 7.81-7.70(5H, m), 7.56-7.25(14H, m) |
| 1-379 | δ = 8.55(1H, d), 8.36(2H, d), 8.03-7.75(14H, m), 7.54-7.25(14H, m) |
| 1-397 | δ = 8.55(1H, d), 8.45(1H, d), 8.36(2H, d), 8.30(1H, s), 8.12(2H, d), 8.01-7.96(6H, m), 7.75-7.70(5H, m), 7.56-7.41(11H, m), 7.25(2H, d) |
| 1-403 | δ = 8.55(1H, d), 8.45(1H, d), 8.36(2H, d), 8.30(1H, s), 8.03(2H, d), 7.96-7.92 (5H, m), 7.75-7.69(6H, m), 7.56-7.41(11H, m), 7.25(2H, d) |
| 1-409 | δ = 8.55(1H, d) 8.36-8.32(3H, m), 8.24(1H, d), 8.20(1H, s), 7.98-7.94(4H, m), 7.86(1H, s), 7.81(1H, s), 7.75-7.71(5H, m), 7.54-7.25(14H, m) |
| 1-421 | δ = 8.36(2H, d), 8.24(1H, d), 8.20(1H, s), 8.12(2H, d), 7.99-7.94(5H, m), 7.86-7.75(6H, m), 7.54-7.25(14H, m) |
| 1-451 | δ = 8.45(1H, d), 8.36(2H, d), 8.30(1H, s), 8.20(2H, d), 8.03-7.96(7H, m), 7.75-7.68(5H, m), 7.56-7.41(11H, m)7.25(2H, d) |
| 1-457 | δ = 8.55(1H, d), 8.36(2H, d), 8.30(1H, s), 8.03-7.94(5H, m), 7.86(1H, s), 7.81(1H, s), 7.75-7.68(6H, m), 7.54-7.25(14H, m) |
| 1-464 | δ = 8.36(2H, d), 8.24-8.17(3H, m), 8.07-7.94(7H, m), 7.75(2H, d), 7.68(1H, d), 7.54-7.25(16H, m), |
| 1-466 | δ = 8.36(2H, d), 8.24-8.17(3H, m), 8.05-7.94(5H, m), 7.86(1H, s), 7.81-7.68(6H, m), 7.57-7.25(14H, m) |
| 1-471 | δ = 8.55(1H, d), 8.45(1H, d), 8.36-8.32(3H, m), 8.22(1H, s), 8.03-7.93(6H, m), 7.75-7.68(4H, m), 7.56-7.41(13H, m), 7.25(2H, d) |
| 1-475 | δ = 8.45(1H, d), 8.36(2H, d), 8.30(1H, s), 8.24-8.17(3H, m), 8.03-7.94(6H, m), 7.75-7.69(5H, m), 7.56-7.41(11H, m), 7.25(2H, d) |
| 1-490 | δ = 8.38-8.36(3H, m), 8.08-7.73(14H, m), 7.61-7.31(14H, m) |
| 1-498 | δ = 8.38-8.36(3H, m), 8.08(2H, d), 8.01-7.73(12H, m) 7.61-7.31(14H, m) |
| 1-512 | δ = 8.45(1H, d), 8.38-8.36(3H, m), 8.13-8.03(4H, m), 7.93-7.73(8H, m), 7.61-7.41(15H, m) |
| 1-524 | δ = 8.45(1H, d), 8.38-8.36(3H, m), 8.13-8.08(3H, m), 7.94-7.73(8H, m), 7.61-7.41(16H, m) |
| 1-553 | δ = 8.45(1H, d), 8.38-8.36(3H, m), 8.08(1H, d), 8.03-8.01(3H, m), 7.94(2H, d), 7.82(1H, d), 7.76-7.73(6H, m), 7.61-7.41(13H, m) |
| 1-559 | δ = 8.45(1H, d), 8.38(2H, d), 8.30(1H, s), 8.03-8.01(3H, m), 7.94(2H, d), 7.82(2H, d), 7.76-7.73(7H, m), 7.56-7.41(12H, m) |
| 1-563 | δ = 8.45(1H, d), 8.38-8.36(3H, m), 8.22(1H, s), 8.03(2H, d), 7.99(1H, s), 7.94(2H, d), 7.82(2H, d), 7.76-7.73(7H, m), 7.56-7.41(12H, m) |
| 1-571 | δ = 8.45(1H, d), 8.38-8.30(4H, m)8.03(2H, d), 7.94(2H, d), 7.82-7.73(9H, m), 7.61-7.41(13H, m) |
| 1-577 | δ = 8.38-8.36(3H, m), 8.08(1H, d), 8.02(1H, d), 7.98(2H, d), 7.82-7.73(9H, m), 7.57-7.41(15H, m) |
| 1-583 | δ = 8.38-8.36(3H, m), 8.03(1H, d), 7.94(2H, d), 7.82-7.69(11H, m), 7.61-7.41(14H, m) |
| 1-584 | δ = 8.38-8.36(3H, m), 8.07-7.94(5H, m), 7.82(2H, d), 7.76-7.69(5H, m), 7.61-7.31(16H, m) |
| 1-589 | δ = 8.45 (1H, d), 8.38-8.30 (4H, m), 8.08(1H, d), 8.02(2H, d), 7.93(2H, d), 7.82(1H, d), 7.76-7.73(6H, m),7.61-7.41(13H, m) |
| 1-593 | δ = 8.45(1H, d), 8.38-8.36(3H, m), 8.22(1H, s), 8.08(1H, d), 8.02-7.94(4H, m), 7.82(1H, d), 7.75-7.69(6H, m), 7.61-7.41(14H, m) |
| 1-600 | δ = 8.45(1H, d), 8.38-8.36(3H, m), 8.13-8.11(2H, m), 8.03(1H, d), 7.93(2H, d) 7.82-7.69(9H, m), 7.61-7.41(13H, m) |
| 1-607 | δ = 8.55(1H, d), 8.38-8.36(3H, m), 8.24-8.17(3H, m), 7.94-7.70(11H, m), 7.61-7.41(13H, m) |
| 1-614 | δ = 8.55(1H, d), 8.38-8.36(3H, m),8.12-7.92(8H, m), 7.75-7.70(4H, m), 7.61-7.31(15H, m) |
| 1-634 | δ = 8.55(1H, d), 8.45(1H, d), 8.38-8.36(3H, m), 8.30(1H, s), 8.24-8.17(3H, m), 8.01(1H, s), 7.93(3H, d), 7.79-7.70(6H, m), 7.61-7.41(12H, m) |
| 1-642 | δ = 8.55(1H, d), 8.45(1H, d), 8.38-8.36(3H, m), 8.13-8.11(4H, m), 7.99-7.92(4H, m), 7.79-7.70(6H, m), 7.61-7.41(12H m) |
| 1-643 | δ = 8.55(1H, d), 8.45(1H, d), 8.38-8.36(3H, m), 8.30(1H, s), 8.03(2H, d), 7.96-7.92(4H, m), 7.75-7.41(19H, m) |
| 1-644 | δ = 8.55(1H, d), 8.45 (1H, d), 8.38-8.36(3H, m), 8.11(2H, d), 8.03(1H, d), 7.94-7.92(4H, m), 7.75(19H, m) |
| 1-649 | δ = 8.55(1H, d), 8.38-8.32(4H, m), 8.21(2H, d), 7.98-7.94(3H, m), 7.86(1H, s), 7.81(1H, s), 7.75-7.70(6H, m), 7.61-7.39(13H, m) |
| 1-657 | δ = 8.38-8.36(3H, m), 8.24-8.17(5H, m), 7.98(1H, d), 7.94(2H, s), 7.87(1H, s), 7.75-7.73(3H, m), 7.61-7.31(15H, m) |
| 1-667 | δ = 8.45 (1H, d), 8.38-8.30 (4H, m), 8.20(2H, d), 8.03(2H, d), 7.94-7.92(4H, m), 7.75-7.41(18H, m) |
| 1-668 | δ = 8.38-8.36(3H, m), 8.24(1H, d), 8.20(1H, s), 8.07-7.94(7H, m), 775-7.31(19H, m) |
| 1-673 | δ = 8.55(1H, d), 8.45(1H, d), 8.38-8.30 (5H, m), 8.24-8.20(3H, |
| | m), 8.01(1H, s), 7.94-7.92(3H, m), 7.75-7.70(6H, m), 7.61-7.41(12H, m) |
| 1-695 | δ = 8.45(1H, d), 8.38-8.36(3H, m), 8.24-8.20(3H, m), 8.03-7.94(6H, m), 7.75-7.41(18H, m) |
| 1-697 | δ = 8.55(1H, d), 8.38-8.32(4H, m), 8.03(1H, d), 7.94-7.93(3H, m), 7.86(1H, s), 7.81(1H, s), 7.75-7.41(20H, m) |
| 1-701 | δ = 8.55(1H, d), 8.38-8.32(4H, m), 8.03-7.93(5H, m), 7.87(1H, s), 7.75-7.41(19H, m) |
| 1-703 | δ = 8.38-8.36(3H, m), 8.24-8.20(3H, m), 8.03(1H, d), 7.98-7.94(3H, m), 7.86(1H, s), 7.81(1H, s), 7.75-7.31(19H, m) |
| 1-704 | δ = 8.38-8.36(3H, m), 8.24-8.20(3H, m), 8.07-7.94(6H, m), 7.75-7.31(19H, m) |
| 1-705 | δ = 8.38-8.36(3H, m), 8.24-8.20(3H, m), 8.03(1H, d), 7.98-7.93(4H, m), 7.87(1H, s), 7.78-7.31(19H, m) |
| 1-715 | δ = 8.45(1H, d), 8.38-8.36(3H, m), 8.30(1H, s), 8.22-8.19(3H, m), 8.03(2H, d), 7.94(2H, d), 7.73-7.41(18H, m) |
| 1-727 | δ = 8.36(4H, d), 8.08(1H, d), 8.03(1H, d), 7.98-7.73(10H, m), 7.61(2H, d), 7.54-7.31(13H, m) |
| 1-728 | δ = 8.36(4H, d), 8.08-7.98(5H, m), 7.94(1H, d), 7.88-7.73(6H, m), 7.61(2H, m), 7.54-7.31(13H, m) |
| 1-732 | δ = 8.36(4H, d), 8.08-7.98(5H, m), 7.94(1H, m), 7.88-7.73(6H, m), 7.64-7.31(15H, m) |
| 1-737 | δ = 8.36(4H, d), 8.08(1H, d), 7.98-7.73(11H, m), 7.61(2H, m), 7.54-7.31(13H, m) |
| 1-739 | δ = 8.36(4H, d), 8.08(1H, d), 7.98-7.73(10H, m), 7.61-7.31(16H, m) |
| 1-741 | δ = 8.36(4H, d), 8.08(1H, d), 7.98(2H, d), 7.93(1H, s), 7.88-7.69(6H, m), 7.57-7.31(18H, m) |
| 1-752 | δ = 8.45(1H, d), 8.36(4H, d), 8.13-8.03(4H, m), 7.94-7.73(8H, m), 7.61-7.41(14H, m) |
| 1-764 | δ = 8.45(1H, d), 8.36(4H, d), 8.11-8.08(3H, m), 7.94-7.73(8H, m), 7.57-7.41(15H, m) |
| 1-769 | δ = 8.36(4H, d), 8.08-7.94(5H, m), 7.86(1H, s), 7.82(2H, d), 7.76-7.73(4H, m), 7.61(2H, d), 7.54-7.41(13H, m) |
| 1-772 | δ = 8.36(4H, d), 8.08-7.94(5H, m), 7.86(1H, s), 7.82-7.73(6H, m), 7.61(2H, d), 7.56-7.31(13H, m) |
| 1-778 | δ = 8.36(4H, d), 8.03(2H, d), 7.98(1H, d), 7.94(1H, d), 7.86-7.73(9H, m), 7.61(2H, d), 7.54-7.31(12H, m) |
| 1-788 | δ = 8.36(4H, d), 8.07(1H, d), 8.02(2H, d), 7.94(2H, d), 7.82(2H, d), 7.76-7.73(5H, m), 7.61-7.31(15H, m) |
| 1-797 | δ = 8.45(2H, d), 8.36(4H, d), 8.22(1H, s), 8.08(1H, d), 8.03-7.94(5H, m), 7.82(2H, d), 7.76-7.73(4H, m), 7.61-7.41(14H, m) |
| 1-813 | δ = 8.45(1H, d), 8.36(4H, d),8.22(1H, s), 7.98(2H, d), 7.94(2H, d), 7.82(2H, d), 7.76-7.69(6H, m), 7.61-7.41(14H, m) |
| 1-819 | δ = 8.36(4H, d),8.08(1H, d), 8.02(1H, d), 7.98(1H, d), 7.93(1H, s), 7.87(1H, s), 7.82-7.75(4H, m), 7.57-7.31(18H, m) |
| 1-822 | δ = 8.36(4H, d), 8.08-7.94(6H, m), 7.82(1H, d), 7.75-7.69(4H, m), 7.57-5.31(16H, m) |
| 1-824 | δ = 8.36(4H, d), 8.07-7.94(5H, m), 7.82(2H, d), 7.76-7.73(5H, m), 7.61-7.31(15H, m) |
| 1-827 | δ = 8.36(4H, d), 8.03(1H, d), 7.98-7.93(3H, m), 7. 87(1H, s), 7.82(2H, s), 7.75-7.69(5H, m),7.57-7.31(15H, m) |
| 1-836 | δ = 8.36(4H, d), 8.11(2H, d). 8.03(1H, d), 7.94(2H, d), 7.82(2H, d), 7.76-7.70(5H, m), 7.61-7.41(14H, m) |
| 1-838 | δ = 8.45 (1H, d), 8.38-8.30 (5H, m), 8.02(2H, d), 7.93(2H, d), 7.82(2H, d), 7.76-7.69(5H, m), 7.61-7.41(14H, m) |
| 1-848 | δ = 8.36(4H, d), 8.24-8.20(3H, m), 8.07(1H, d), 8.01-7.94(4H, m), 7.75-7.70(4H, m), 7.61(2H, d), 7.54-7.31(12H, m) |
| 1-851 | δ = 8.55(1H, d), 8.36(4H, d), 8.22-8.20(3H, m), 7.98(1H, d), 7.93(2H, d), 7.87(1H, s), 7.75-7.73(3H, m), 7.51-7.25(16H, m) |
| 1-859 | δ = 8.55(1H, d), 8.36(4H, d), 8.03(1H, d), 7.98-7.92(4H, m), 7.86(1H, s), 7.82(1H, s), 7.75-7.70(3H, m), 7.54-7.25(16H, m) |
| 1-873 | δ = 8.55(1H, d), 8.45(1H, d), 8.36(4H, d), 8.24-8.17(4H, m), 7.99(1H, s), 7.94-7.92(3H, m), 7.75-7.70(4H, m), 7.61-7.41(13H, m) |
| 1-885 | δ = 8.55(1H, d), 8.45(1H, d), 8.36(4H, d), 8.22(1H, s), 8.03(1H, d), 7.99(1H, s), 7.94-7.92(3H, m), 7.75-7.68(4H, m), 7.56-7.41(11H, m) |
| 1-891 | δ = 8.55(1H, d), 8.36-8.32(5H, m), 8.24(1H, d), 8.20(1H, s), 7.98-7.94(3H, m), 7.87(1H, s), 7.75-7.70(3H, m), 7.54-7.25(16H, m) |
| 1-897 | δ = 8.36(4H, d), 8.24-8.17(5H, m), 7.98-7.93(4H, m), 7.87(1H, s), 7.75-7.73(3H, m), 7.61(2H, d), 7.54-7.31(12H, m) |
| 1-907 | δ = 8.36(4H, d),8.24(1H, d), 8.20(1H, s), 8.03(1H, d), 7.98-7.94(3H, m), 7.86(1H, s), 7.81(1H, s), 7.75(2H, d), 7.69(1H, d), 7.57-7.31(16H, m) |
| 1-916 | δ = 8.55(1H, d), 8.45(1H, d), 8.36(4H, d),8.30(2H, d), 8.24(2H, d), 8.20(1H, s), 8.01(1H, s), 7.94(2H, d), 7.75-7.73(3H, m), 7.56-7.41(11H, m) |
| 1-942 | δ = 8.55(1H, d), 8.36(4H, d), 8.31(1H, d), 8.03-7.94(6H, m), 7.73-7.41(19H, m) |
| 1-945 | δ = 8.36(4H, d), 8.24-8.20(3H, m), 8.03(1h, d), 7.98-7.93(3H, m), 7.87(1H, s), 7.75(2H, d), 7.68(1H, d), 7.54-7.31(16H, m) |
| 1-959 | δ = 8.45(1H, d), 8.36(4H, d),8.24-8.17(4H, m), 8.03(1H, d), 7.99-7.93(4H, m), 7.73-7.41(17H, m) |
| 1-971 | δ = 8.45(1H, d), 8.36(2H, d), 8.08-7.79(11H, m), 7.68(2H, d), 7.57-7.31(13H, m) |
| 1-979 | δ = 8.45(1H, d), 8.36(2H, d), 8.30(1H, s), 8.03-7.69(12H, m), 7.57-7.31(13H, m), |
| 1-985 | δ = 8.45(1H, d), 8.36(2H, d), 8.30(1H, s), 8.03-7.69(12H, m), 7.54-7.41(13H, m) |
| 1-999 | δ = 8.36(4H, d), 8.08(lH, d), 8.03(1H, d) 7.98(2H, d), 7.88-7.79(9H, m), 7.57-7.31(14H, m) |
| 1-1009 | δ = 8.55(1H, d), 8.36-8.32(3H, m), 8.24(1H, d), 8.20(1H, s), 7.98-7.82(6H, m), 7.70(2H, d), 7.57-7.31(15H, m) |
| 1-1033 | δ = 8.45(1H, d), 8.36(4H, d), 8.13-8.03(4H, m), 7.93(1H, d), 7.88(1H, d), 7.82(1H, d), 7.76(1H, s), 7.56-7.47(9H, m) |
| 1-1039 | δ = 8.45(1H, d), 8.36(4H, d), 8.30(1H, s), 8.03(2H, d), 8.01(1H, s), 7.93(1H, d), 7.82(2H, d), 7.76(2H, s), 7.56-7.49(8H, m) |
| 1-1042 | δ = 8.36(2H, d), 8.03(1H, d), 7.98(1H, d), 8.03-7.75(9H, m), 7.54-7.41(8H, m) |
| 1-1072 | δ = 8.36(4H, d), 8.03 (2H, d), 7.82-7.76(4H, m), 7.50(6H, m) |
| 1-1079 | δ = 8.55(1H, d), 8.36-7.82(5H, m), 8.24-8.20(2H, m), 7.94(1H, d), 7.70(1H, t), 7.50(6H, m) |
| 1-1088 | δ =8.36(2H, d), 7.98-7.96(3H, m), 7.86-7.75(6H, m), 7.54-7.25(14H, m) |
| 1-1092 | δ =8.36(2H, d), 8.07-7.96(5H, m), 7.79-7.75(4H, m), 7.54-7.25(14H, m) |
| 1-1093 | δ = 8.24-8.20(2H, m), 8.03-7.87(6H, m), 7.75-7.68 (3H, m), 7.54-7.31(6H, m) |
| 1-1101 | δ = 8.45(1H, d), 8.22(1H, s), 8.03-7.76(8H, m), 7.56-7.41(7H, m) |
| 1-1110 | δ = 7.98(1H, d), 7.86(1H, s), 7.81(1H, s), 7.75(2H, d), 7.54-7.25(10H, m) |
| 1-1115 | δ = 8.36(4H, d), 7.50(6H, m) |
| 1-1124 | δ = deuterium content 100%, no ¹H NMR peak |
| 1-1133 | δ = deuterium content 100%, no ¹H NMR peak |
| 1-1148 | δ = deuterium content 100%, no ¹H NMR peak |
| 1-1155 | δ = deuterium content 100%, no ¹H NMR peak |

### <Preparation Example 5> Synthesis of compound 2-3

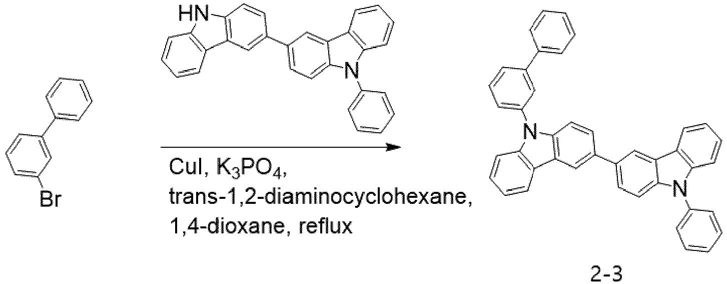

3.7g (15.8mM) of 3-bromo-1,1'-biphenyl, 6.5g (15.8mM) of 9-phenyl-9H,9'H-3,3'-bicarbazole, 3.0g (15.8mM) of CuI, 1.9mL (15.8mM) of trans-1,2-diaminocyclohexane, 3.3g (31.6mM) of K₃PO₄ were dissolved in 100 mL of 1,4-dioxane, and refluxed for 24 hours. After the reaction was completed, the resultant was extracted with distilled water and DCM at room temperature, and the organic layer was dried over MgSO₄ and the solvent was removed using a rotary evaporator. The reaction product was purified by column chromatography (DCM:hexane=1:3), and recrystallized from methanol to obtain 7.5 g (85%) of the desired compound 2-3.

The following desired compounds were prepared and synthesized in the same manner as in Preparation Example 5, except that in Preparation Example 5, intermediate A of Table 7 below instead of 3-bromo-1,1'-biphenyl was used, and intermediate B of Table 7 below instead of 9-phenyl-9H,9'H-3,3'-bicarbazole was used.

**Table 7:**

| Compound | Intermediate A | Intermediate B | Desired compound A | Yield |
|---|---|---|---|---|
| 2-3 | 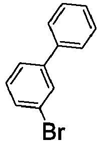 | 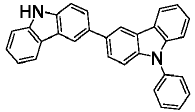 | 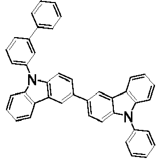 | 85% |
| 2-4 | 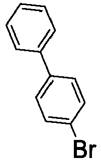 | | 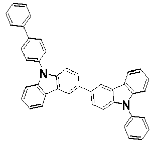 | 83% |
| 2-5 | 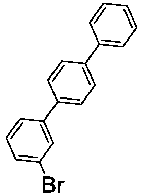 | | 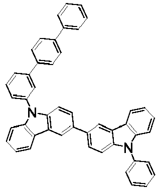 | 85% |
| 2-7 | 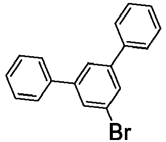 | | 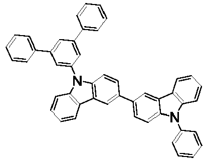 | 84% |
| 2-9 | 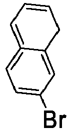 | | 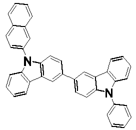 | 80% |
| 2-31 | 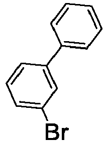 | 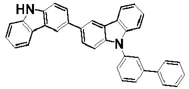 | 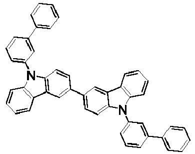 | 81% |
| 2-32 | 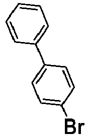 | | 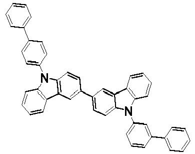 | 80% |
| 2-42 | 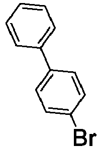 | 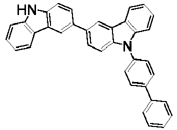 | 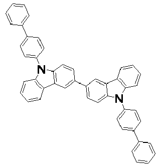 | 82% |

### <Preparation Example 6> Synthesis of compound 2-98

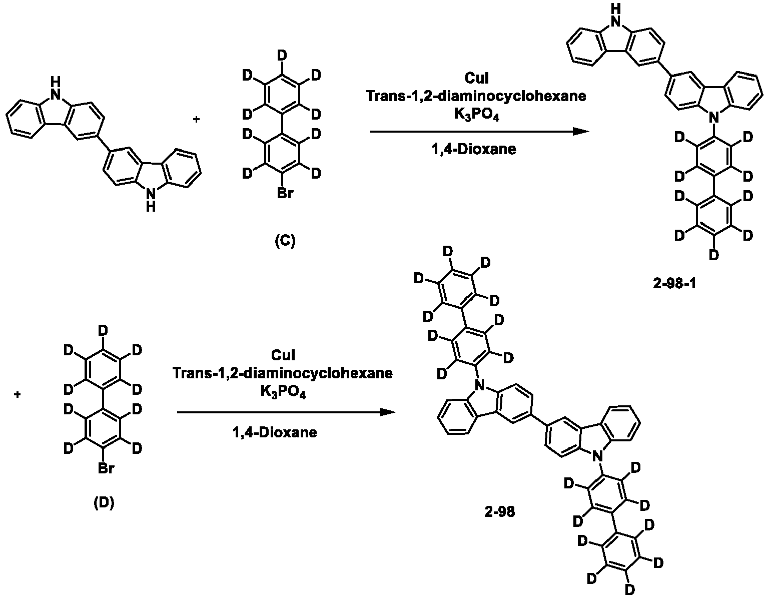

### 1) Preparation of compound 2-98-1

In a one neck round bottom flask, 10g (0.030 mol) of 9H,9'H-3,3'-bicarbazole, 7.26g (0.030 mol) of 4-bromo-1,1'-biphenyl-2,2',3,3',4',5,5',6,6'-D9 (C), 0.57g (0.003 mol) of CuI, 0.34g (0.003 mol) of trans-1,2-diaminocyclohexane, and 12.7g (0.06 mol) of K₃PO₄ were dissolved in 100 ml of 1,4-dioxane, and then refluxed at 125 °C for 8 hours. After the reaction was completed, the mixture was cooled, and the resultant extracted with dichloromethane, and then dried over MgSO₄. The separated organic layer was concentrated under reduced pressure and separated by column chromatography to obtain 13.92 g of compound 2-98-1 (yield: 94%).

### 2) Preparation of compound 2-98

In a one neck round bottom flask, 13.92g (0.028 mol) of intermediate 2-98-1, 6.83g (0.028 mol) of 4-bromo-1,1'-biphenyl-2,2',3,3',4',5,5',6,6'-D9 (D), 0.53g (0.0028 mol) of CuI, 0.32g (0.0028 mol) of trans-1,2-diaminocyclohexane, and 11.89g (0.056 mol) of K₃PO₄ were dissolved in 140 ml of 1,4-dioxane, and then refluxed at 125 °C for 8 hours. After the reaction was completed, the mixture was cooled, and the resultant was extracted with dichloromethane, and dried over MgSO₄. The separated organic layer was concentrated under reduced pressure and separated by column chromatography to obtain 16.14 g of compound 2-98 (yield: 88%) .

When the intermediate C and the intermediate D are identical, the desired compound can be directly synthesized by adding 2 equivalents of the intermediate C in the synthesis method of compound 2-98-1 of Preparation Example 6 above.

The following desired compound B was synthesized in the same manner as in Preparation Example 6 above, except that in Preparation Example 6, intermediate compounds C and D of Table 8 below instead of 4-bromo-1,1'-biphenyl-2,2',3,3',4',5,5',6,6'-D9 (intermediate C) and 4-bromo-1,1'-biphenyl-2,2',3,3',4',5,5',6,6'-D9 (intermediate D) were used, respectively.

**Table 8:**

| Compoun d No. | Intermediate C | Intermediate D | Desired compound B | Yield |
|---|---|---|---|---|
| 2-98 | 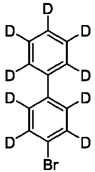 | 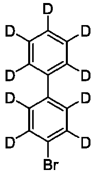 | 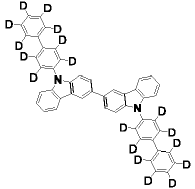 | 88% |
| 2-99 | 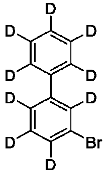 | 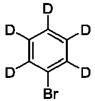 | 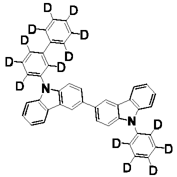 | 95% |
| 2-101 | 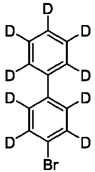 | 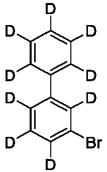 | 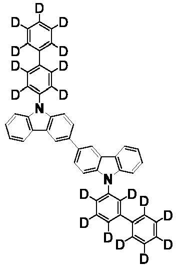 | 74% |
| 2-104 | 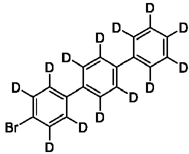 | 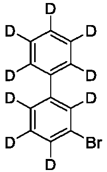 | 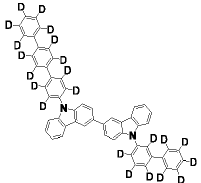 | 69% |

### <Preparation Example 7> Synthesis of compound 2-106

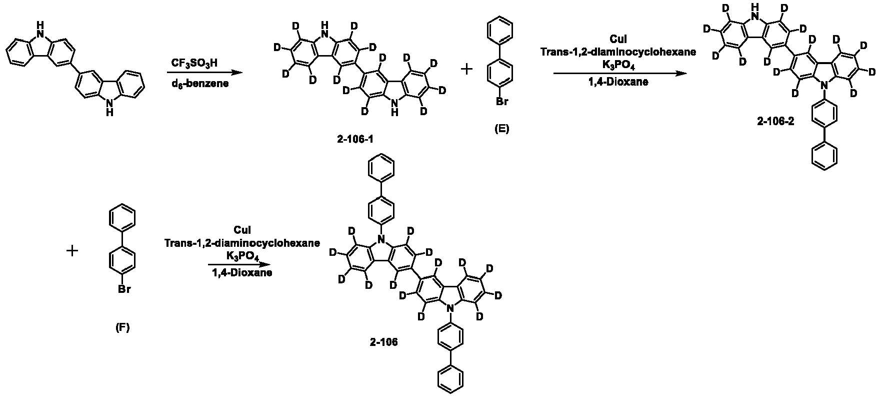

### 1) Preparation of compound 2-106-1

In one neck round bottom flask, a mixture of 10g (0.030 mol) of 9H,9'H-3,3'-biscarbazole, 34g (0.023 mol) of triflic acid, and 100ml of D6-benzene was refluxed at 50 °C. The mixture was cooled, and the resultant was extracted with dichloromethane and dried over MgSO₄. The resultant was separated by column chromatography to obtain 7.07 g of intermediate 2-106-1 (yield: 68%).

### 2) Preparation of compound 2-106-2

In a one neck round bottom flask, 7.07g(0.02 mol) of intermediate 2-106-1, 0.38g (0.002 mol) of CuI, 4.66g (0.02 mol) of 4-bromo-1,1'-biphenyl (E), 0.23g (0.002 mol) of trans-1,4-diaminocyclohexane, and 8.49g(0.04 mol) of K₃PO₄ were dissolved in 70 ml of 1,4-dioxane, and refluxed at 125 °C for 8 hours. After the reaction was completed, the resultant was extracted with distilled water and dichloromethane at room temperature, and the organic layer was dried over MgSO₄ and the solvent was removed using a rotary evaporator. The reactant was purified by column chromatography (DCM:hexane=1:3), and recrystallized from methanol to obtain 8.28 g of intermediate 2-106-2 (yield: 83%).

### 3) Preparation of compound 2-106

In a one neck round bottom flask, 8.28g (0.017 mol) of intermediate 2-106-2, 0.32g (0.0017 mol) of CuI, 3.96g (0.017 mol) of 4-bromo-1,1'-biphenyl(F), 0.19g(0.0017 mol) of trans-1,4-diaminocyclohexane, and 7.22g (0.034 mol) of K₃PO₄ were dissolved in 80 ml of 1,4-dioxane, and refluxed at 125 °C for 8 hours. After the reaction was completed, the resultant was extracted with distilled water and dichloromethane at room temperature, and the organic layer was dried over MgSO₄ and the solvent was removed using a rotary evaporator. The reactant was purified by column chromatography (DCM:hexane=1:3), and recrystallized from methanol to obtain 8.63 g of desired compound 2-106 (yield: 78%).

If the intermediate E and intermediate F are identical, the desired compound can be directly synthesized by adding 2 equivalents of intermediate E in the synthesis method of compound 2-106-1 of Preparation Example 7 above.

The following desired compounds C were synthesized in the same manner as in Preparation Example 7, except that in Preparation Example 7, intermediates E and F of Table 9 instead of 4-bromo-1,1'-biphenyl (E) and 4-bromo-1,1'-biphenyl (F) were used.

**Table 9:**

| Compound No. | Intermediate E | Intermediate F | Desired compound C | Yield |
|---|---|---|---|---|
| 2-106 | 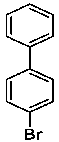 | 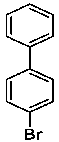 | 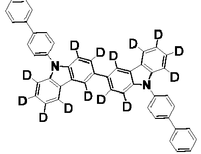 | 78% |
| 2-107 | 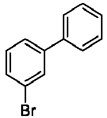 | 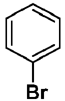 | 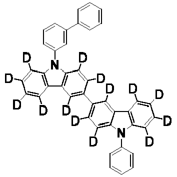 | 90% |
| 2-108 | 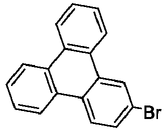 | 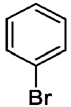 | 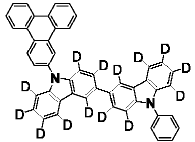 | 72% |

### <Preparation Example 8> Synthesis of compound 2-110

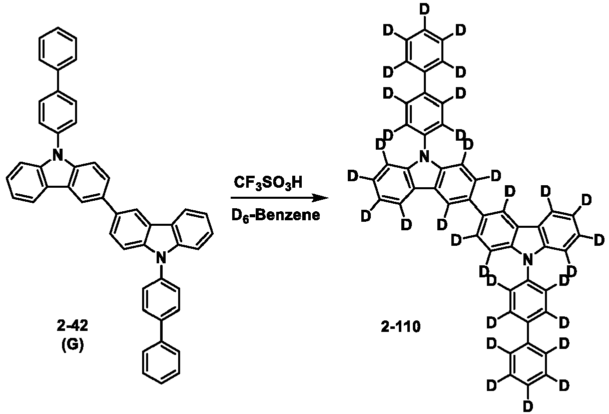

### Preparation of compound 2-110

In a one neck round bottom flask, a mixture of 12.17g(0.017 mol) of intermediate compound 2-42, 40.8g(0.27 mol) of triflic acid, and 120ml of D6-benzene was refluxed at 50 °C. the resultant was extracted with dichloromethane and water, the organic layer was dried over MgSO₄, concentrated, and then filtered through silica gel. The reactant was concentrated and recrystallized from methanol to obtain 8.87 g of desired compound 2-110 (yield: 78%).

The following desired compound D was synthesized in the same manner as in Preparation Example 8, except that in Preparation Example 8, intermediate compound G of Table 10 below instead of intermediate compound 2-42 (G) was used.

**Table 10:**

| Compound No. | Intermediate G | Desired compound D | Yield |
|---|---|---|---|
| 2-110 | 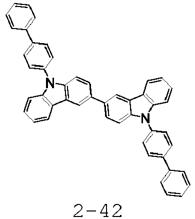 | 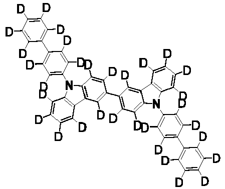 | 78% |
| 2-111 | 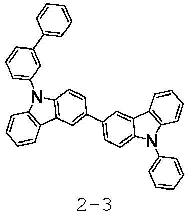 | 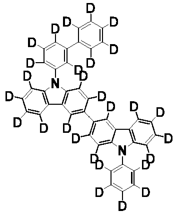 | 91% |
| 2-113 | 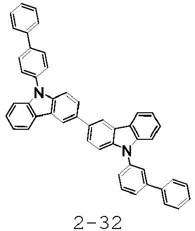 | 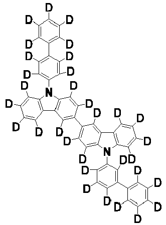 | 74% |
| 2-114 | 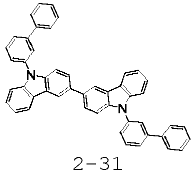 | 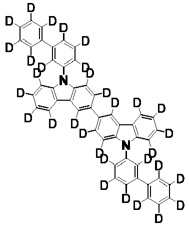 | 84% |
| 2-116 | 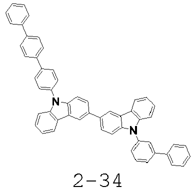 | 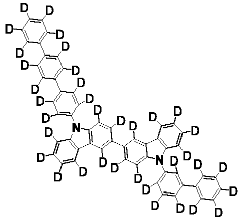 | 72% |

Compounds represented by Formula 2 other than the compounds shown in Preparation Examples 5 to 8 and Tables 7 to 10 were also prepared in the same manner as in the above-described Preparation Example, and the synthesis results are shown in Tables 11 and 12 below.

**Table 11:**

| Compound | FD-Mass | Compo und | FD-Mass |
|---|---|---|---|
| 2-3 | m/z= 560.23 (C₄₂H₂₈N₂=560.70) | 2-4 | m/z= 560.23 (C₄₂H₂₈N₂=560.70) |
| 2-5 | m/z= 636.26 (C₄₈H₃₂N₂=636.80) | 2-7 | m/z= 636.26 (C₄₈H₃₃N₂=636.80) |
| 2-9 | m/z=534.21 (C₄₀H₂₆N₂=534.66) | 2-31 | m/z= 636.26 (C₄₈H₃₂N₂=636.80) |
| 2-32 | m/z= 636.26 (C₄₈H₃₂N₂=636.80) | 2-42 | m/z= 636.26 (C₄₈H₃₂N₂=636.80) |
| 2-98 | m/z= 654.37 (C₄₈H₁₄D₁₈N₂=654.91) | 2-99 | m/z= 574.31 (C₄₂H₁₄D₁₄N₂=574.79) |
| 2-101 | m/z= 654.37 (C₄₈H₁₄D₁₈N₂=654.91) | 2-102 | m/z= 734.43 (C₅₄H₁₄D₂₂N₂=735.03) |
| 2-104 | m/z= 734.03 (C₅₄H₁₄D₂₂N₂=735.43) | 2-106 | m/z= 650.34 (C₄₈H₁₈D₁₄N₂=650.88) |
| 2-107 | m/z= 574.31 (C₄₂H₁₄D₁₄N₂=574.79) | 2-108 | m/z= 648.87 (C₄₈H₁₆D₁₄N₂=648.33) |
| 2-110 | m/z= 668.46 (C₄₈D₃₂N₂=668.99) | 2-111 | m/z= 588.40 (C₄₂D₂₈N₂=588.87) |
| 2-113 | m/z= 668.46 (C₄₈D₃₂N₂=668.99) | 2-114 | m/z= 668.46 (C₄₈D₃₂N₂=668.99) |
| 2-116 | m/z= 748.51 (C₅₄D₃₆N₂=749.12) | | |

**Table 12**

| Compound | ¹H NMR (CDCl₃, 200Mz) |
|---|---|
| 2-3 | δ= 8.55 (1H, d), 8.30 (1H, d), 8.21-8.13 (3H, m), 7.99-7.89 (4H, m), 7.77-7.35 (17H, m), 7.20-7.16 (2H, m) |
| 2-4 | δ= 8.55 (1H, d), 8.30(1H, d), 8.19-8.13(2H, m), 7.99-7.89(8H, m), 7.77-7.75 (3H, m), 7.62-7.35 (11H, m), 7.20-7.16 (2H, m) |
| 2-5 | δ= 8.55 (1H, d), 8.30(1H, d), 8.21-8.13(3H, m), 7.99-7.94(2H, m), 7.89(2H, s), 7.77-7.35 (17H, m), 7.25-7.16 (6H, m) |
| 2-7 | δ= 8.55 (1H, d), 8.31-8.30 (3H, d), 8.19-8.13 (2H, m), 7.99-7.89 (5H, m), 7.77-7.75 (5H, m), 7.62-7.35 (14H, m), 7.20-7.16 (2H, m) |
| 2-9 | δ= 8.55 (1H, d), 8.30 (1H, d), 8.19-8.13 (2H, m), 8.03-7.77 (9H, m), 7.62-7.50 (9H, m), 7.36-7.35 (2H, m), 7.20-7.16 (2H, m) |
| 2-31 | δ= 8.55 (1H, d), 8.30 (1H, d), 8.21-8.13 (4H, m), 7.99-7.89 (4H, m), 7.77-7.35 (20H, m), 7.20-7.16 (2H, m) |
| 2-32 | δ= 8.55 (1H, d), 8.30 (1H, d), 8.21-8.13 (3H, m), 7.99-7.89 (8H, m), 7.77-7.35 (17H, m), 7.20-7.16 (2H, m) |
| 2-37 | δ= 8.55 (1H, d), 8.30 (1H, d), 8.21-8.13 (3H, m), 8.03-7.35 (23H, m), 7.20-7.16 (2H, m) |
| 2-42 | δ= 8.55 (1H, d), 8.30 (1H, d), 8.19(1H, d) 8.13 (1H, d), 7.99-7.89 (12H, m), 7.77-7.75 (5H, m), 7.50-7.35 (8H, m), 7.20-7.16 (2H, m) |
| 2-82 | δ= 8.55 (1H, d), 8.30 (1H, d), 8.19-8.13 (2H, m), 8.03-7.77 (13H, m), 7.59-7.50 (6H, m) 7.36-7.35 (3H, m), 7.20-7.16 (2H, m) |
| 2-98 | δ = 8.55 (1H, d), 8.30 (1H, d), 8.13-8.19 (2H, m), 7.89∼7.99 (4H, m), 7.77 (1H, d), 7.50-7.58 (2H, m), 7.35 (1H, t), 7.16-7.20 (2H, t) |
| 2-99 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-101 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-104 | δ = 8.55 (1H, d), 8.30 (1H, d), 8.13∼8.19 (2H, m), 7.89∼7.99 (4H, m), 7.77 (1H, d), 7.50∼7.58 (2H, m), 7.35 (1H, t), 7.16∼7.20 (2H, t) |
| 2-106 | δ = 7.91∼7.92 (8H, m), 7.75 (4H, d), 7.41∼7.49 (6H, m) |
| 2-107 | δ = 8.21 (1H, s), 7.41∼7.68 (13H, m) |
| 2-108 | δ = 9.05 (1H, s), 8.33∼8.25 (4H, m), 7.94 (1H, d), 7.70∼7.50 (10H, m) |
| 2-110 | δ = deuterium content 100%, no ¹H NMR peak |
| 2-111 | δ = deuterium content 100%, no ¹H NMR peak |
| 2-113 | δ = deuterium content 100%, no ¹H NMR peak |
| 2-114 | δ = deuterium content 100%, no ¹H NMR peak |
| 2-116 | δ = deuterium content 100%, no ¹H NMR peak |

### <Preparation Example 9> Synthesis of compound 3-2

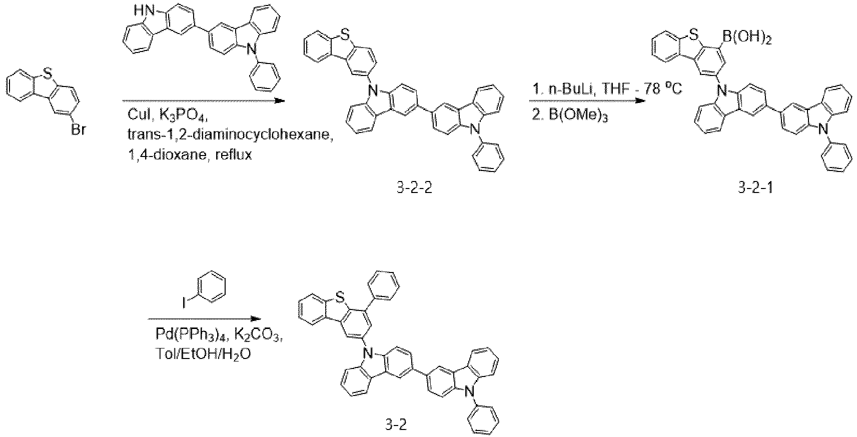

### 1) Preparation of compound 3-2-2

4.2g (15.8mM) of 2-bromodibenzo[b,d]thiophene, 6.5g (15.8mM) of 9-phenyl-9H,9'H-3,3'-bicarbazole, 3.0g (15.8mM) of CuI, 1.9mL (15.8mM) of trans-1,2-diaminocyclohexane, and 3.3g (31.6mM) of K₃PO₄ were dissolved in 100 mL of 1,4-dioxane and refluxed for 24 hours. After the reaction was completed, the resultant was extracted with distilled water and DCM at room temperature, and the organic layer was dried over MgSO₄ and the solvent was removed using a rotary evaporator. The reactant was purified by column chromatography (DCM:hexane=1:3) and recrystallized from methanol to obtain 7.9 g (85%) of desired compound 3-2-2.

### 2) Preparation of compound 3-2-1

To the mixed solution containing 8.4g (14.3mmol) of compound 3-2-2 and 100mL of tetrahydrofuran (THF), 7.4mL (18.6mmol) of 2.5M n-butyllithium (n-BuLi) was added dropwise at -78 °C, and the mixture was stirred at room temperature for 1 hour. 4.8 mL (42.9 mmol) of trimethyl borate was added dropwise to the reaction mixture, and the mixture was stirred at room temperature for 2 hours. After the reaction was completed, the resultant was extracted with distilled water and DCM at room temperature, and the organic layer was dried over MgSO₄ and the solvent was removed using a rotary evaporator. The reactant was purified by column chromatography (DCM:MeOH=100:3) and recrystallized from DCM to obtain 3.9 g (70%) of desired compound 3-2-1.

### 3) Preparation of compound 3-2

6.7g (10.5mM) of compound 3-2-1, 2.1g (10.5mM) of iodobenzene, 606mg (0.52mM) of Pd(PPh₃)₄, and 2.9g(21.0mM) of K₂CO₃ were dissolved in toluene/ethanol (EtOH)/H₂O 100/20/20 mL and refluxed for 12 hours. After the reaction was completed, the resultant was extracted with distilled water and DCM at room temperature, and the organic layer was dried over MgSO₄ and the solvent was removed using a rotary evaporator. The reactant was purified by column chromatography (DCM:hexane=1:3) and recrystallized from methanol to obtain 4.9 g (70%) of desired compound 3-2.

The following desired compounds B were obtained in the same manner as in 3-2, except that In Preparation Example 9, the following compound A instead of iodobenzene was used.

**Table 13:**

| Compound | Intermediate 3-2-1 | Intermediate A | Desired compound B | Yie ld |
|---|---|---|---|---|
| 3-2 | 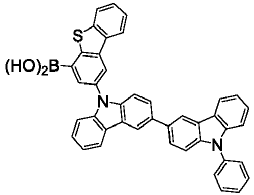 | 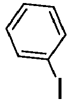 | 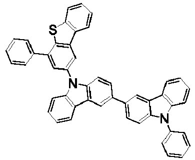 | 83% |
| 3-3 | | 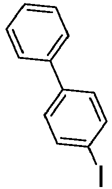 | 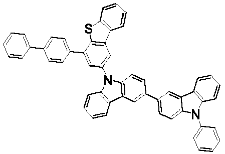 | 84% |

### <Preparation Example 10> Synthesis of compound 3-106

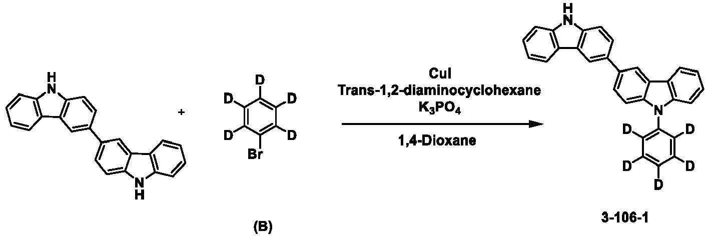

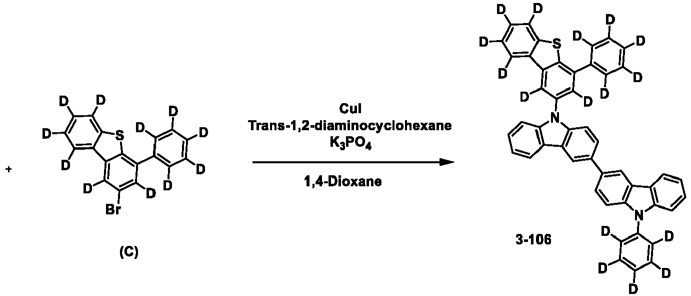

### 1) Preparation of compound 3-106-1

In a one neck round bottom flask, 10g (0.030 mol) of 9H,9'H-3,3'-bicarbazole, 4.86g(0.030 mol) of 1-bromobenzene-2,3,4,5,6-D5 (B), 0.57g(0.003 mol) of CuI, 0.34g(0.003 mol) of trans-1,2-diaminocyclohexane, and 12.7g(0.06 mol) of K₃PO₄ were dissolved in 100 ml of 1,4-dioxane, and then refluxed at 125 °C for 8 hours. After the reaction was completed, the mixture was cooled, and the resultant was extracted with dichloromethane, and dried over MgSO₄. The resulting organic layer was concentrated under reduced pressure and separated by column chromatography to obtain 10 g of compound 3-106-1 (yield: 81%).

### 2) Preparation of compound 3-106

In a one neck round bottom flask, 10g (0.024 mol) of intermediate compound 3-106-1, 8.4g(0.024 mol) of 2-bromo-4-(phenyl-D5)dibenzo[b,d]thiophene1,3,6,7,8,9-D6 (C), 0.45g(0.0024 mol) of CuI, 0.27g(0.0024 mol) of trans-1,2-diaminocyclohexane, and 10.2g(0.048 mol) of K₃PO₄ were dissolved in 100 ml of 1,4-dioxane, and then refluxed at 125 °C for 8 hours. After the reaction was completed, the mixture was cooled, and the resultant was extracted with dichloromethane, and dried over MgSO₄. The filtered organic layer was concentrated under reduced pressure and separated by column chromatography to obtain 14.4 g of compound 3-106 (yield: 88%).

The following desired compounds F were synthesized in the same manner as in Preparation Example 10, except that in Preparation Example 10, intermediates B and C of Table 14 below instead of 1-bromobenzene-2,3,4,5,6-D5 (intermediate B) and 2-bromo-4-(phenyl-D5)dibenzo[b,d]thiophene1,3,6,7,8,9-D6 (intermediate C) were used, respectively.

**Table 14:**

| Compoun d No. | Intermediate B | Intermediate C | Desired compound F | Yield |
|---|---|---|---|---|
| 3-107 | 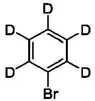 | 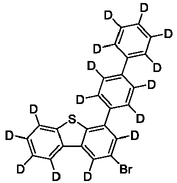 | 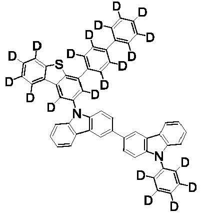 | 86% |
| 3-108 | 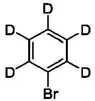 | 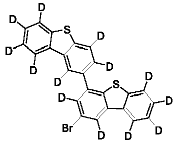 | 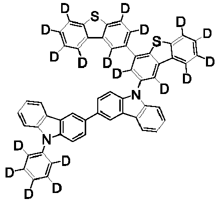 | 82% |
| 3-109 | 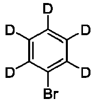 | 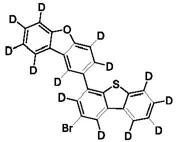 | 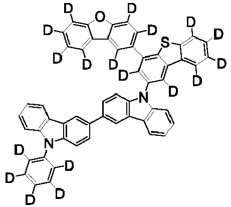 | 77% |

### <Preparation Example 11> Synthesis of compound 3-111

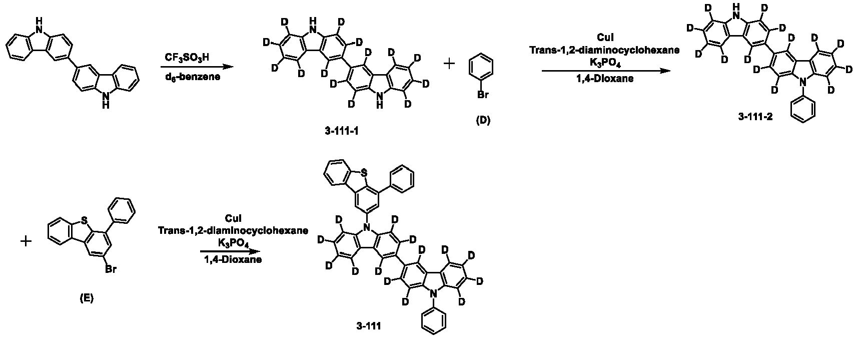

### 1) Preparation of compound 3-111-1

In a one neck round bottom flask, a mixture of 10g (0.030 mol) of 9H,9'H-3,3'-biscarbazole, 34g (0.023 mol) of triflic acid, and 100ml of D6-benzene was refluxed at 50 °C. The mixture was cooled, and the resultant was extracted with dichloromethane and dried over MgSO₄. The resultant was separated by column chromatography to obtain 7.07 g of intermediate 3-111-1 (yield: 68%) .

### 2) Preparation of compound 3-111-2

In a one neck round bottom flask, 7.07g (0.02 mol) of intermediate 3-111-1, 0.38g (0.002 mol) of CuI, 3.14g(0.02 mol) of bromobenzene (D), 0.23g (0.002 mol) of trans-1,4-diaminocyclohexane, 8.49g (0.04 mol) of K₃PO₄ were dissolved in 70 ml of 1,4-dioxane, and then refluxed at 125 °C for 8 hours. After the reaction was completed, the resultant was extracted with distilled water and dichloromethane at room temperature, the organic layer was dried over MgSO₄, and the solvent was removed using a rotary evaporator. The resultant was purified by column chromatography (DCM:hexane=1:3) and recrystallized from methanol to obtain 6.85 g of intermediate 3-111-2 (yield: 81%).

### 3) Preparation of compound 3-111

In a one neck round bottom flask, 6.85g (0.016 mol) of intermediate 3-111-2, 0.30g (0.0016 mol) of CuI, 3.73g (0.016 mol) of 2-bromo-4-phenyldibenzo[b,d]thiophene (E), 0.18g (0.0016 mol) of trans-1,4-diaminocyclohexane, and 6.79g (0.032 mol) of K₃PO₄ were dissolved in 70 ml of 1,4-dioxane, and refluxed at 125 °C for 8 hours. After the reaction was completed, the resultant was extracted with distilled water and dichloromethane at room temperature, the organic layer was dried over MgSO₄, and the solvent was removed using a rotary evaporator. The resultant was purified by column chromatography (DCM:hexane=1:3) and recrystallized from methanol to obtain 8.72 g of desired compound 3-111 (yield: 72%).

The following desired compound G was synthesized in the same manner as in Preparation Example 11, except that in Preparation Example 11, Intermediates D and E of Table 15 instead of bromobenzene (D) and 2-bromo-4-phenyldibenzo[b,d]thiophene (E) were used, respectively.

**Table 15:**

| Compou nd No. | Intermediate D | Intermediate E | Desired compound G | Yield |
|---|---|---|---|---|
| 3-112 | 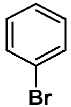 | 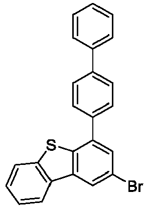 | 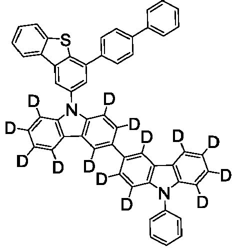 | 76% |
| 3-114 | 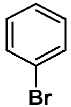 | 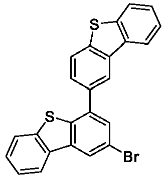 | 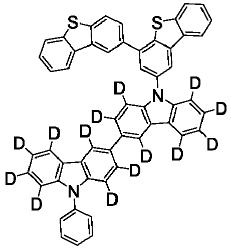 | 81% |
| 3-115 | 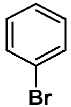 | 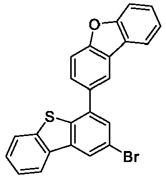 | 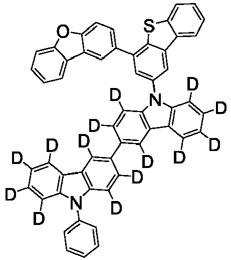 | 74% |

### <Preparation Example 12> Synthesis of compound 3-117

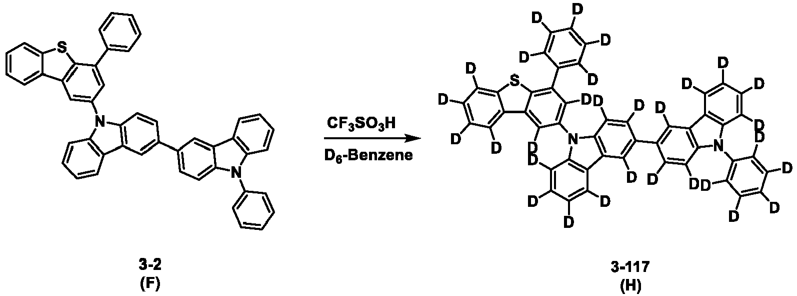

In a one neck round bottom flask, a mixture of 10g (0.015 mol) of intermediate compound 3-117, 34.8g (0.23 mol) of triflic acid, and 100ml of D6-benzene was refluxed at 50 °C. The resultant was extracted with dichloromethane and water, and the organic layer was dried over MgSO₄ and concentrated. The resultant was purified by column chromatography (DCM:hexane=1:2) and recrystallized from methanol to obtain 9.62 g of desired compound 3-117 (yield: 92%).

The following desired compound H was synthesized in the same manner as in Preparation Example 12, except that in Preparation Example 12, intermediate F of Table 16 below instead of intermediate compound 3-2 (F) was used.

**Table 16:**

| Compound No. | Intermediate F | Desired compound H | Yield |
|---|---|---|---|
| 3-118 | 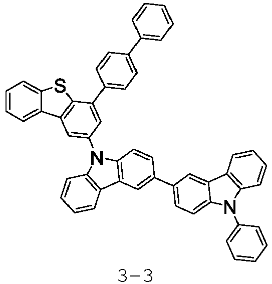 | 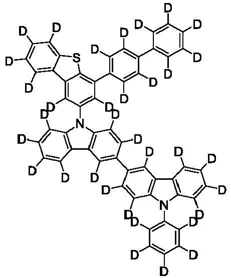 | 85% |
| 3-119 | 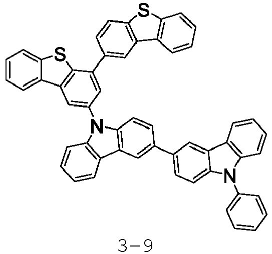 | 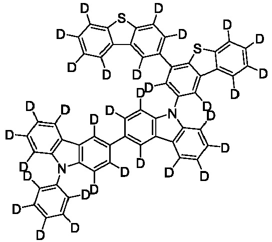 | 91% |
| 3-120 | 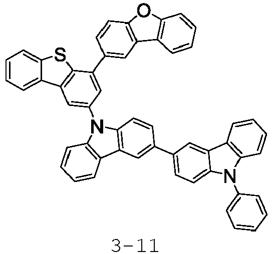 | 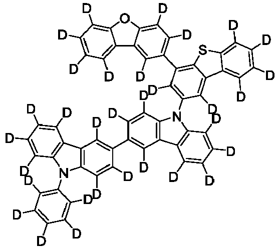 | 88% |
| 3-123 | 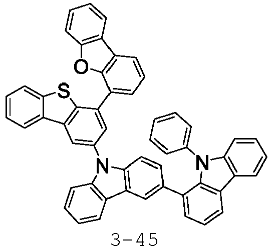 | 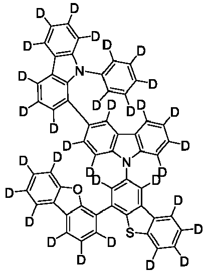 | 89% |
| 3-125 | 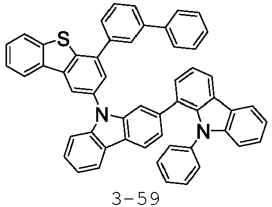 | 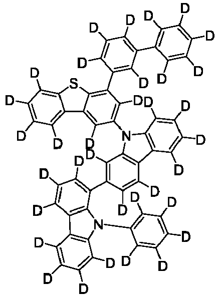 | 92% |

The compounds represented by Formula 2 other than the compounds shown in Preparation Examples 9 to 12 and Tables 13 to 16 were also prepared in the same manner as in the above-described Preparation Example, and the synthesis results are shown in Tables 17 and 18 below.

**Table 17:**

| Compound | FD-Mass | Compound | FD-Mass |
|---|---|---|---|
| 3-2 | m/z= 666.84 (C₄₈H₃₀N₂=666.21) | 3-3 | m/z= 742.24 (C₅₄H₃₄N₂S=742.94) |
| 3-106 | m/z= 682.31 (C₄₈H₁₄D₁₆N₂S=682.94) | 3-107 | m/z= 762.37 (C₅₄H₁₄D₂₀N₂S=763.06) |
| 3-108 | m/z= 790.31 (C₅₄H₁₄D₁₈N₂S₂=791.09) | 3-109 | m/z= 774.34 (C₅₄H₁₄D₁₈N₂OS=775.03) |
| 3-111 | m/z= 680.30 (C₄₈H₁₆D₁₄N₂S=680.93) | 3-112 | m/z= 756.33 (C₅₄H₂₀D₁₄N₂S=757.03) |
| 3-114 | m/z= 786.29 (C₅₄H₁₈D₁₄N₂S₂=787.07) | 3-115 | m/z= 770.31 (C₅₄H₁₈D₁₄N₂OS=771.01) |
| 3-117 | m/z= 696.40 (C₄₈D₃₀N₂S=697.03) | 3-118 | m/z= 776.46 (C₅₄D₃₄N₂S=777.15) |
| 3-119 | m/z= 804.40 (C₅₄D₃₂N₂S₂=805.18) | 3-120 | m/z= 788.42 (C₅₄D₃₂N₂OS=789.12) |
| 3-123 | m/z= 788.42 (C₅₄D₃₂N₂OS=789.12) | 3-125 | m/z= 776.46 (C₅₄D₃₄N₂S=777.15) |

**Table 18:**

| Compound | ¹H NMR (CDCl₃, 200Mz) |
|---|---|
| 3-2 | δ = 8.55(1H, d), 8.45(1H, d), 8.30(1H, d), 8.19(1H, d), 8.13(1H, d), 8.00~7.89(6H, m), 7.77(2H, m), 7.62∼7.35(15H, |
| | m), 7.20∼7.16(2H, m) |
| 3-3 | δ = 8.55(1H, d), 8.45(1H, d), 8.30(1H, d), 8.19(1H, d), 8.13(1H, d), 8.00∼7.89(6H, m), 7.77∼7.75(4H, m), 7.62∼7.35(13H, m), 7.25∼7.16(6H, m) |
| 3-106 | δ = 8.55 (2H, d), 7.99-7.89 (6H, m), 7.77 (2H, d), 7.35 (2H, t), 7.16 (2H, t) |
| 3-107 | δ = 8.55 (2H, d), 7.99-7.89 (6H, m), 7.77 (2H, d), 7.35 (2H, t), 7.16 (2H, t) |
| 3-108 | δ = 8.55 (2H, d), 7.99-7.89 (6H, m), 7.77 (2H, d), 7.35 (2H, t), 7.16 (2H, t) |
| 3-109 | δ = 8.55 (2H, d), 7.99-7.89 (6H, m), 7.77 (2H, d), 7.35 (2H, t), 7.16 (2H, t) |
| 3-111 | δ = 8.45 (1H, d), 8.00 (1H, s), 7.93 (1H, d), 7.77 (1H, s), 7.62-7.41 (12H, m) |
| 3-112 | δ = 8.45 (1H, d), 8.00 (1H, s), 7.93-7.91 (5H, m), 7.77-7.75 (3H, m), 7.56-7.41 (10H, m) |
| 3-114 | δ = 8.45 (2H, d), 8.12 (2H, m), 8.00-7.93 (4H, m), 7.77 (1H, s), 7.62-7.49 (9H, m) |
| 3-115 | δ = 8.45 (1H, d), 8.00-7.77 (7H, m), 7.62-7.31 (10H, m) |
| 3-117 | δ = deuterium content 100%, no ¹H NMR peak |
| 3-118 | δ = deuterium content 100%, no ¹H NMR peak |
| 3-119 | δ = deuterium content 100%, no ¹H NMR peak |
| 3-120 | δ = deuterium content 100%, no ¹H NMR peak |
| 3-123 | δ = deuterium content 100%, no ¹H NMR peak |
| 3-125 | δ = deuterium content 100%, no ¹H NMR peak |

### <Experimental Example 1>

### (1) Manufacturing of organic light emitting device

A glass substrate coated with a thin film of indium tin oxide (ITO) to a thickness of 1,500 Å was ultrasonically washed with distilled water. After washing with distilled water, the substrate was ultrasonically washed with a solvent such as acetone, methanol, isopropyl alcohol, etc., dried, and then treated with ultraviolet ozone (UVO) for 5 minutes using ultraviolet (UV) in a UV cleaner. Thereafter, the substrate was transferred to a plasma cleaner (PT), and then plasma-treated in a vacuum to increase the work function of ITO and remove the remaining film, and transferred to a thermal deposition equipment for organic deposition.

A hole injection layer 2-TNATA (4,4',4''-tris[2-naphthyl(phenyl)amino]triphenylamine) and a hole transport layer NPB (N,N'-Di(1-naphthyl)-N,N'-diphenyl-(1,1'-biphenyl)-4,4'-diamine) as a common layer were formed on the ITO transparent electrode (positive electrode).

On top of that, a light emitting layer was thermally vacuum deposited as follows. The light emitting layer was formed by depositing the compound for the light emitting layer described in Tables 19 and 21 as a host to a thickness of 400 Å, and doping and depositing a green phosphorescent dopant [Ir(ppy)3] to a thickness of 7% of the deposition thickness of the light emitting layer. In the case of manufacturing a blue phosphorescent device, A blue phosphorescent dopant [Firpic] was deposited. Thereafter, bathocuproine (BCP) was deposited to a thickness of 60 Å as a hole blocking layer, and on it, Alq₃ was deposited to a thickness of 200 Å as an electron transport layer. Finally, on the electron transport layer, lithium fluoride (LiF) was deposited to a thickness of 10 Å to form an electron injection layer, and then a negative electrode of aluminum (Al) was deposited to a thickness of 1,200 Å on the electron injection layer to form a negative electrode, thereby manufacturing an organic light emitting device.

On the other hand, all organic compounds required for the manufacture of OLED devices were purified by vacuum sublimation under 10⁻⁸ to 10⁻⁶ torr for each material, and used for the manufacture of OLED.

### (2) Operating voltage and luminous efficiency of organic light emitting device

For the organic light emitting device manufactured as described above, electroluminescence (EL) characteristics were measured with M7000 from McScience Co. With the measurement results, T₉₀ of the green phosphorescent device was measured at a reference luminance of 6,000 cd/m², and T₉₀ of the blue phosphorescent device was measured at a reference luminance of 1,000 cd/m² through the lifetime measuring device (M6000) manufactured by McScience Co. In the above, T₉₀ means a lifetime (unit: hour (h)) that is a time at which the luminance becomes 90% compared to the initial luminance.

The results of measuring the operating voltage, luminous efficiency, color coordinate (CIE), and lifetime of the organic light emitting device manufactured according to the present invention are shown in Tables 19 to 20 below. In addition, the results of measuring the driving voltage, luminous efficiency, color coordinate (CIE), and lifetime of the organic light emitting device manufactured using the following comparative compound as a compound for the light emitting layer are shown in Table 21 below.

**Table 19:**

| | Light emitting layer Compound | Operating voltage (V) | Efficiency (cd/A) | Luminous color | Lifetime (T90) |
|---|---|---|---|---|---|
| Example 1 | 1-1 | 4.40 | 72.5 | green | 161 |
| Example 2 | 1-7 | 4.37 | 74.1 | green | 182 |
| Example 3 | 1-14 | 4.36 | 74.4 | green | 185 |
| Example 4 | 1-16 | 4.35 | 74.9 | green | 188 |
| Example 5 | 1-19 | 4.41 | 75.0 | green | 164 |
| Example 6 | 1-23 | 4.40 | 75.4 | green | 160 |
| Example 7 | 1-25 | 4.52 | 73.9 | green | 170 |
| Example 8 | 1-31 | 4.49 | 75.0 | green | 189 |
| Example 9 | 1-34 | 4.48 | 75.4 | green | 192 |
| Example 10 | 1-43 | 4.48 | 76.6 | green | 172 |
| Example 11 | 1-49 | 4.35 | 75.7 | green | 177 |
| Example 12 | 1-54 | 4.34 | 75.0 | green | 182 |
| Example 13 | 1-58 | 4.30 | 76.3 | green | 194 |
| Example 14 | 1-66 | 4.33 | 76.0 | green | 195 |
| Example 15 | 1-70 | 4.41 | 75.4 | green | 174 |
| Example 16 | 1-73 | 4.52 | 76.6 | green | 183 |
| Example 17 | 1-79 | 4.47 | 77.2 | green | 195 |
| Example 18 | 1-87 | 4.45 | 77.6 | green | 193 |
| Example 19 | 1-97 | 4.42 | 74.4 | green | 171 |
| Example 20 | 1-104 | 4.29 | 74.1 | green | 166 |
| Example 21 | 1-109 | 4.50 | 77.8 | green | 180 |
| Example 22 | 1-119 | 4.51 | 77.0 | green | 182 |
| Example 23 | 1-121 | 4.45 | 75.4 | green | 163 |
| Example 24 | 1-129 | 4.40 | 73.5 | green | 169 |
| Example 25 | 1-139 | 4.35 | 74.6 | green | 172 |
| Example 26 | 1-140 | 4.37 | 75.0 | green | 174 |
| Example 27 | 1-156 | 4.50 | 75.2 | green | 180 |
| Example 28 | 1-163 | 4.44 | 74.9 | green | 178 |
| Example 29 | 1-169 | 4.45 | 76.0 | green | 184 |
| Example 30 | 1-178 | 4.39 | 76.7 | green | 192 |
| Example 31 | 1-187 | 4.33 | 75.1 | green | 166 |
| Example 32 | 1-193 | 4.44 | 75.0 | green | 175 |
| Example 33 | 1-205 | 4.43 | 78.2 | green | 195 |
| Example 34 | 1-211 | 4.35 | 74.7 | green | 179 |
| Example 35 | 1-227 | 4.29 | 74.2 | green | 170 |
| Example 36 | 1-235 | 4.34 | 75.6 | green | 178 |
| Example 37 | 1-247 | 4.29 | 75.9 | green | 181 |
| Example 38 | 1-261 | 4.32 | 76.0 | green | 177 |
| Example 39 | 1-277 | 4.30 | 79.2 | green | 194 |
| Example 40 | 1-283 | 4.31 | 78.8 | green | 182 |
| Example 41 | 1-289 | 4.33 | 78.9 | green | 182 |
| Example 42 | 1-310 | 4.34 | 79.5 | green | 195 |
| Example 43 | 1-325 | 4.29 | 79.0 | green | 199 |
| Example 44 | 1-335 | 4.21 | 74.9 | green | 176 |
| Example 45 | 1-337 | 4.18 | 75.0 | green | 173 |
| Example 46 | 1-343 | 4.24 | 75.3 | green | 179 |
| Example 47 | 1-355 | 4.26 | 75.5 | green | 181 |
| Example 48 | 1-358 | 4.25 | 75.4 | green | 182 |
| Example 49 | 1-370 | 4.35 | 76.9 | green | 185 |
| Example 50 | 1-379 | 4.29 | 77.0 | green | 180 |
| Example 51 | 1-397 | 4.30 | 79.2 | green | 195 |
| Example 52 | 1-403 | 4.35 | 77.5 | green | 188 |
| Example 53 | 1-409 | 4.35 | 76.5 | green | 186 |
| Example 54 | 1-421 | 4.26 | 78.8 | green | 193 |
| Example 55 | 1-451 | 4.30 | 79.2 | green | 180 |
| Example 56 | 1-457 | 4.29 | 78.7 | green | 185 |
| Example 57 | 1-464 | 4.29 | 79.3 | green | 181 |
| Example 58 | 1-466 | 4.26 | 79.6 | green | 178 |
| Example 59 | 1-471 | 4.36 | 76.0 | green | 183 |
| Example 60 | 1-475 | 4.36 | 79.9 | green | 190 |
| Example 61 | 1-490 | 4.26 | 78.2 | green | 175 |
| Example 62 | 1-498 | 4.30 | 79.5 | green | 168 |
| Example 63 | 1-512 | 4.32 | 79.3 | green | 170 |
| Example 64 | 1-524 | 4.35 | 80.6 | green | 175 |
| Example 65 | 1-553 | 4.43 | 82.4 | green | 182 |
| Example 66 | 1-559 | 4.35 | 84.5 | green | 189 |
| Example 67 | 1-563 | 4.34 | 83.7 | green | 186 |
| Example 68 | 1-571 | 4.31 | 80.5 | green | 169 |
| Example 69 | 1-577 | 4.24 | 81.0 | green | 163 |
| Example 70 | 1-583 | 4.26 | 82.1 | green | 160 |
| Example 71 | 1-584 | 4.22 | 80.6 | green | 159 |
| Example 72 | 1-589 | 4.15 | 78.6 | green | 158 |
| Example 73 | 1-593 | 4.17 | 79.0 | green | 163 |
| Example 74 | 1-600 | 4.29 | 78.9 | green | 170 |
| Example 75 | 1-607 | 4.30 | 78.7 | green | 174 |
| Example 76 | 1-614 | 4.31 | 80.1 | green | 177 |
| Example 77 | 1-634 | 4.26 | 77.9 | green | 168 |
| Example 78 | 1-642 | 4.27 | 78.2 | green | 180 |
| Example 79 | 1-643 | 4.22 | 79.0 | green | 172 |
| Example 80 | 1-644 | 4.20 | 79.1 | green | 173 |
| Example 81 | 1-649 | 4.32 | 79.2 | green | 171 |
| Example 82 | 1-657 | 4.29 | 77.6 | green | 180 |
| Example 83 | 1-667 | 4.27 | 78.7 | green | 172 |
| Example 84 | 1-668 | 4.24 | 78.1 | green | 158 |
| Example 85 | 1-673 | 4.38 | 80.1 | green | 174 |
| Example 86 | 1-695 | 4.33 | 79.0 | green | 170 |
| Example 87 | 1-697 | 4.21 | 78.8 | green | 160 |
| Example 88 | 1-701 | 4.19 | 79.0 | green | 160 |
| Example 89 | 1-703 | 4.29 | 77.3 | green | 176 |
| Example 90 | 1-704 | 4.26 | 77.5 | green | 172 |
| Example 91 | 1-705 | 4.25 | 78.1 | green | 167 |
| Example 92 | 1-715 | 4.31 | 78.0 | green | 173 |
| Example 93 | 1-727 | 4.36 | 75.3 | green | 175 |
| Example 94 | 1-728 | 4.35 | 76.2 | green | 182 |
| Example 95 | 1-732 | 4.35 | 76.6 | green | 182 |
| Example 96 | 1-737 | 4.31 | 78.1 | green | 188 |
| Example 97 | 1-739 | 4.26 | 78.4 | green | 170 |
| Example 98 | 1-741 | 4.24 | 77.1 | green | 166 |
| Example 99 | 1-752 | 4.29 | 77.3 | green | 173 |
| Example 100 | 1-764 | 4.29 | 78.5 | green | 168 |
| Example 101 | 1-769 | 4.25 | 79.0 | green | 167 |
| Example 102 | 1-772 | 4.26 | 78.3 | green | 162 |
| Example 103 | 1-778 | 4.31 | 76.5 | green | 183 |
| Example 104 | 1-788 | 4.22 | 77.9 | green | 172 |
| Example 105 | 1-797 | 4.30 | 76.1 | green | 170 |
| Example 106 | 1-813 | 4.21 | 75.9 | green | 175 |
| Example 107 | 1-819 | 4.20 | 80.3 | green | 169 |
| Example 108 | 1-822 | 4.16 | 79.9 | green | 168 |
| Example 109 | 1-824 | 4.25 | 76.8 | green | 183 |
| Example 110 | 1-827 | 4.26 | 76.5 | green | 180 |
| Example 111 | 1-836 | 4.29 | 77.5 | green | 183 |
| Example 112 | 1-838 | 4.27 | 77.9 | green | 180 |
| Example 113 | 1-848 | 4.21 | 78.6 | green | 175 |
| Example 114 | 1-851 | 4.24 | 78.2 | green | 170 |
| Example 115 | 1-859 | 4.19 | 77.0 | green | 168 |
| Example 116 | 1-873 | 4.26 | 78.6 | green | 174 |
| Example 117 | 1-885 | 4.18 | 77.3 | green | 169 |
| Example 118 | 1-891 | 4.25 | 78.0 | green | 170 |
| Example 119 | 1-897 | 4.30 | 80.3 | green | 192 |
| Example 120 | 1-907 | 4.19 | 80.0 | green | 169 |
| Example 121 | 1-916 | 4.23 | 77.6 | green | 174 |
| Example 122 | 1-942 | 4.17 | 79.3 | green | 170 |
| Example 123 | 1-945 | 4.25 | 78.1 | green | 180 |
| Example 124 | 1-959 | 4.28 | 78.4 | green | 184 |
| Example 125 | 1-971 | 4.28 | 81.2 | green | 202 |
| Example 126 | 1-979 | 4.25 | 82.0 | green | 221 |
| Example 127 | 1-985 | 4.29 | 83.5 | green | 208 |
| Example 128 | 1-999 | 4.17 | 83.6 | green | 197 |
| Example 129 | 1-1009 | 4.19 | 84.1 | green | 190 |
| Example 130 | 1-1033 | 4.44 | 76.0 | green | 183 |
| Example 131 | 1-1039 | 4.46 | 77.3 | green | 193 |
| Example 132 | 1-1042 | 4.43 | 77.4 | green | 191 |
| Example 219 | 1-1072 | 4.18 | 86 | green | 284 |
| Example 220 | 1-1088 | 4.17 | 83 | green | 281 |
| Example 221 | 1-1093 | 4.16 | 88 | green | 287 |
| Example 222 | 1-1110 | 4.17 | 89 | green | 289 |
| Example 223 | 1-1124 | 4.14 | 93 | green | 293 |
| Example 224 | 1-1148 | 4.15 | 97 | green | 296 |

**Table 20:**

| | Light emitting layer Compound | | 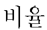 | Operating voltage (V) | Efficiency (cd/A) | Luminous color | Lifetime (T90) |
|---|---|---|---|---|---|---|---|
| Example 133 | 1-1 | 3-2 | 1:5 | 5.57 | 51.6 | green | 166 |
| Example 134 | | | 1:3 | 4.69 | 68.4 | green | 226 |
| Example 135 | | | 1:2 | 4.40 | 82.9 | green | 310 |
| Example 136 | | | 1:1 | 4.21 | 78.5 | green | 255 |
| Example 137 | | | 2:1 | 4.60 | 70.5 | green | 233 |
| Example 138 | | | 3:1 | 4.95 | 65.6 | green | 201 |
| Example 139 | | | 5:1 | 5.58 | 58.5 | green | 170 |
| Example 140 | | 2-3 | 1:2 | 4.45 | 84.1 | green | 331 |
| Example 141 | | | 1:1 | 4.25 | 76.9 | green | 263 |
| Example 143 | 1-7 | 2-7 | 1:2 | 4.39 | 85.1 | green | 343 |
| Example 144 | | | 1:1 | 4.13 | 78.2 | green | 269 |
| Example 145 | 1-14 | 2-4 | 1:2 | 4.33 | 83.1 | green | 361 |
| Example 146 | | | 1:1 | 4.20 | 77.8 | green | 271 |
| Example 147 | 1-19 | 2-3 | 1:2 | 4.39 | 85.2 | green | 355 |
| Example 148 | | | 1:1 | 4.19 | 78.7 | green | 257 |
| Example 149 | 1-31 | 2-4 | 1:2 | 4.45 | 84.6 | green | 384 |
| Example 150 | | | 1:1 | 4.22 | 79.2 | green | 275 |
| Example 151 | 1-43 | 2-3 | 1:2 | 4.44 | 86.6 | green | 368 |
| Example 152 | | | 1:1 | 4.20 | 79.9 | green | 260 |
| Example 153 | 1-54 | 2-5 | 1:2 | 4.31 | 86.2 | green | 371 |
| Example 154 | | | 1:1 | 4.12 | 79.2 | green | 270 |
| Example 155 | 1-66 | 2-32 | 1:2 | 4.33 | 84.9 | green | 377 |
| Example 156 | | | 1:1 | 4.17 | 78.6 | green | 259 |
| Example 157 | 1-87 | 2-42 | 1:2 | 4.43 | 88.2 | green | 385 |
| Example 158 | | | 1:1 | 4.20 | 80.1 | green | 291 |
| Example 159 | 1-104 | 2-32 | 1:2 | 4.27 | 85.2 | green | 363 |
| Example 160 | | | 1:1 | 4.08 | 79.0 | green | 252 |
| Example 161 | 1-139 | 2-31 | 1:2 | 4.31 | 84.8 | green | 362 |
| Example 162 | | | 1:1 | 4.29 | 77.9 | green | 266 |
| Example 163 | 1-187 | 2-5 | 1:2 | 4.29 | 86.2 | green | 349 |
| Example 164 | | | 1:1 | 4.15 | 79.2 | green | 241 |
| Example 165 | 1-227 | 3-2 | 1:2 | 4.28 | 83.7 | green | 359 |
| Example 166 | | | 1:1 | 4.01 | 79.1 | green | 239 |
| Example 167 | 1-235 | 2-42 | 1:2 | 4.31 | 88.4 | green | 392 |
| Example 168 | | | 1:1 | 4.11 | 77.8 | green | 261 |
| Example 169 | 1-310 | 2-4 | 1:2 | 4.34 | 87.2 | green | 382 |
| Example 170 | | | 1:1 | 4.10 | 82.2 | green | 261 |
| Example 171 | 1-355 | 2-5 | 1:2 | 4.27 | 84.9 | green | 377 |
| Example 172 | | | 1:1 | 4.12 | 79.3 | green | 271 |
| Example 173 | 1-370 | 2-32 | 1:2 | 4.31 | 88.4 | green | 389 |
| Example 174 | | | 1:1 | 4.10 | 77.8 | green | 271 |
| Example 175 | 1-451 | 2-42 | 1:2 | 4.27 | 91.0 | green | 401 |
| Example 176 | | | 1:1 | 4.04 | 82.6 | green | 278 |
| Example 177 | 1-498 | 2-42 | 1:2 | 4.25 | 90.1 | green | 389 |
| Example 178 | | | 1:1 | 4.03 | 81.8 | green | 262 |
| Example 179 | 1-571 | 2-32 | 1:2 | 4.28 | 94.3 | green | 376 |
| Example 180 | | | 1:1 | 4.13 | 83.7 | green | 268 |
| Example 181 | 1-577 | 2-7 | 1:2 | 4.29 | 91.3 | green | 372 |
| Example 182 | | | 1:1 | 4.10 | 82.2 | green | 255 |
| Example 183 | 1-584 | 3-2 | 1:2 | 4.20 | 92.1 | green | 361 |
| Example 184 | | | 1:1 | 3.99 | 83.7 | green | 250 |
| Example 185 | 1-600 | 2-4 | 1:2 | 4.25 | 88.0 | green | 354 |
| Example 186 | | | 1:1 | 4.06 | 81.4 | green | 251 |
| Example 187 | 1-643 | 2-32 | 1:2 | 4.19 | 87.2 | green | 361 |
| Example 188 | | | 1:1 | 4.00 | 81.5 | green | 244 |
| Example 189 | 1-697 | 2-5 | 1:2 | 4.17 | 86.9 | green | 342 |
| Example 190 | | | 1:1 | 3.97 | 82.1 | green | 249 |
| Example 191 | 1-704 | 3-2 | 1:2 | 4.23 | 86.2 | green | 350 |
| Example 192 | | | 1:1 | 3.95 | 80.6 | green | 244 |
| Example 193 | 1-737 | 2-31 | 1:2 | 4.27 | 89.0 | green | 383 |
| Example 194 | | | 1:1 | 4.13 | 82.2 | green | 270 |
| Example 195 | 1-764 | 2-42 | 1:2 | 4.24 | 89.7 | green | 380 |
| Example 196 | | | 1:1 | 4.07 | 80.6 | green | 267 |
| Example 197 | 1-813 | 3-2 | 1:2 | 4.19 | 84.9 | green | 349 |
| Example 198 | | | 1:1 | 3.90 | 79.4 | green | 242 |
| Example 199 | 1-848 | 2-32 | 1:2 | 4.17 | 86.5 | green | 365 |
| Example 200 | | | 1:1 | 3.97 | 80.9 | green | 251 |
| Example 201 | 1-897 | 3-3 | 1:2 | 4.25 | 91.7 | green | 403 |
| Example 202 | | | 1:1 | 4.01 | 83.3 | green | 271 |
| Example 203 | 1-907 | 3-3 | 1:2 | 4.16 | 92.3 | green | 387 |
| Example 204 | | | 1:1 | 3.94 | 83.6 | green | 274 |
| Example 205 | 1-916 | 2-42 | 1:2 | 4.18 | 88.6 | green | 378 |
| Example 206 | | | 1:1 | 4.00 | 80.5 | green | 266 |
| Example 207 | 1-945 | 2-32 | 1:2 | 4.22 | 89.4 | green | 365 |
| Example 208 | | | 1:1 | 4.01 | 81.7 | green | 249 |
| Example 209 | 1-971 | 2-42 | 1:2 | 4.22 | 93.6 | green | 419 |
| Example 210 | | | 1:1 | 3.99 | 84.4 | green | 301 |
| Example 211 | 1-999 | 2-42 | 1:2 | 4.14 | 96.2 | green | 401 |
| Example 212 | | | 1:1 | 3.92 | 85.7 | green | 299 |
| Example 213 | 1-1009 | 2-32 | 1:2 | 4.10 | 96.0 | green | 398 |
| Example 214 | | | 1:1 | 3.89 | 87.2 | green | 279 |
| Example 215 | 1-1033 | 2-5 | 1:2 | 4.39 | 87.2 | green | 372 |
| Example 216 | | | 1:1 | 4.19 | 79.3 | green | 258 |
| Example 217 | 1-1042 | 2-4 | 1:2 | 4.37 | 85.2 | green | 366 |
| Example 218 | | | 1:1 | 4.20 | 80.5 | green | 250 |
| Example 225 | 1-7 | 2-98 | 1:2 | 4.21 | 92.3 | green | 445 |
| Example 226 | | | 1:1 | 4.09 | 86.4 | green | 324 |
| Example 227 | 1-14 | 3-107 | 1:2 | 4.12 | 92.8 | green | 456 |
| Example 228 | | | 1:1 | 3.93 | 84.4 | green | 346 |
| Example 229 | 1-19 | 2-110 | 1:2 | 4.13 | 93.7 | green | 454 |
| Example 230 | | | 1:1 | 3.97 | 87.6 | green | 362 |
| Example 231 | 1-31 | 3-119 | 1:2 | 4.08 | 92.8 | green | 466 |
| Example 232 | | | 1:1 | 3.97 | 86.4 | green | 374 |
| Example 233 | 1-1072 | 2-3 | 1:2 | 4.18 | 89.6 | green | 443 |
| Example 234 | | | 1:1 | 3.97 | 84.2 | green | 322 |
| Example 235 | 1-1088 | 3-2 | 1:2 | 4.11 | 91.4 | green | 460 |
| Example 236 | | | 1:1 | 3.90 | 83.6 | green | 347 |
| Example 237 | 1-1093 | 2-101 | 1:2 | 4.08 | 93.3 | green | 753 |
| Example 238 | | | 1:1 | 3.95 | 87.4 | green | 357 |
| Example 239 | 1-1110 | 3-109 | 1:2 | 4.08 | 95.3 | green | 466 |
| Example 240 | | | 1:1 | 3.90 | 88.3 | green | 361 |
| Example 241 | 1-1072 | 2-113 | 1:2 | 4.00 | 99.6 | green | 473 |
| Example 242 | | | 1:1 | 3.91 | 91.2 | green | 380 |
| Example 243 | 1-1093 | 3-120 | 1:2 | 4.06 | 100.1 | green | 476 |
| Example 244 | | | 1:1 | 3.88 | 92.6 | green | 383 |
| Example 245 | 1-1124 | 2-7 | 1:2 | 4.08 | 92.4 | green | 466 |
| Example 246 | | | 1:1 | 3.95 | 86.7 | green | 377 |
| Example 247 | 1-1133 | 3-3 | 1:2 | 4.07 | 93.4 | green | 468 |
| Example 248 | | | 1:1 | 3.97 | 87.6 | green | 376 |
| Example 249 | 1-1148 | 2-106 | 1:2 | 4.03 | 99.4 | green | 474 |
| Example 250 | | | 1:1 | 3.92 | 91.8 | green | 386 |
| Example 251 | 1-1155 | 3-112 | 1:2 | 4.01 | 100.2 | green | 476 |
| Example 252 | | | 1:1 | 3.89 | 92.1 | green | 385 |
| Example 253 | 1-1124 | 2-116 | 1:2 | 3.92 | 111.4 | green | 503 |
| Example 254 | | | 1:1 | 3.86 | 107.5 | green | 482 |
| Example 255 | 1-1133 | 3-123 | 1:2 | 3.94 | 112.6 | green | 511 |
| Example 256 | | | 1:1 | 3.89 | 108.2 | green | 487 |

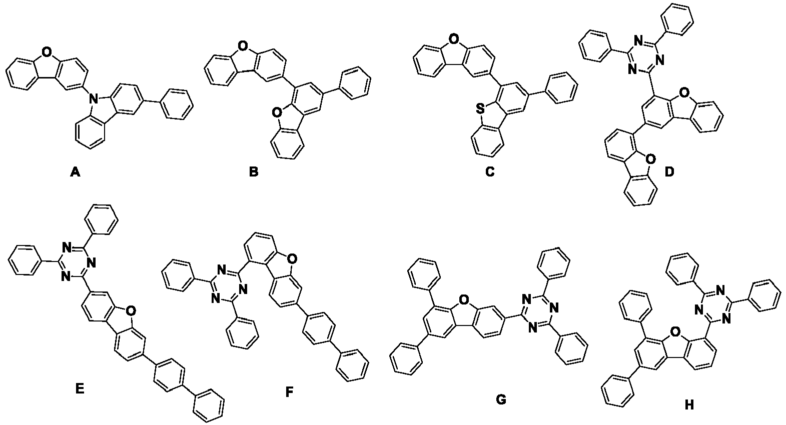

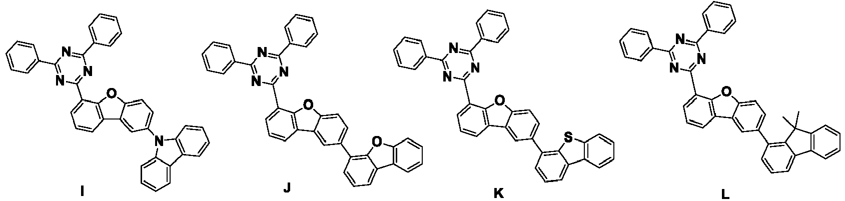

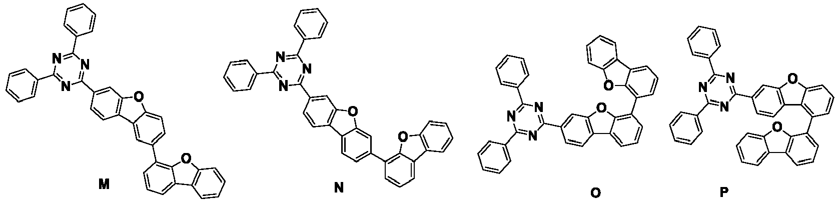

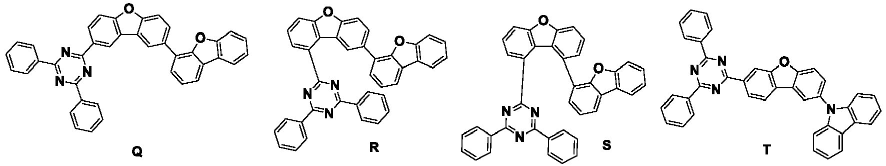

**Table 21**

| | Light emitting layer Compound | Operating voltage (V) | Efficiency (cd/A) | Luminous color | Lifetime (T90) |
|---|---|---|---|---|---|
| Comparative Example 1 | A | 6.51 | 23.5 | green | 29 |
| Comparative Example 2 | B | 6.44 | 23.4 | green | 24 |
| Comparative Example 3 | C | 6.45 | 23.9 | green | 24 |
| Comparative Example 4 | D | 4.58 | 68.3 | green | 79 |
| Comparative Example 5 | E | 5.00 | 44.2 | green | 96 |
| Comparative Example 6 | F | 4.94 | 43.9 | green | 90 |
| Comparative Example 7 | G | 5.04 | 43.5 | green | 106 |
| Comparative Example 8 | H | 4.95 | 42.9 | green | 101 |
| Comparative Example 9 | I | 4.60 | 69.1 | green | 139 |
| Comparative Example 10 | J | 4.51 | 68.2 | green | 123 |
| Comparative Example 11 | K | 4.64 | 69.7 | green | 131 |
| Comparative Example 12 | L | 4.46 | 67.4 | green | 94 |
| Comparative Example 13 | M | 4.49 | 68.4 | green | 128 |
| Comparative Example 14 | N | 4.52 | 69.7 | green | 135 |
| Comparative Example 15 | O | 4.46 | 70.5 | green | 116 |
| Comparative Example 16 | P | 4.45 | 66.8 | green | 117 |
| Comparative Example 17 | Q | 4.47 | 72.1 | green | 60 |
| Comparative Example 18 | R | 4.40 | 70.3 | green | 107 |
| Comparative Example 19 | S | 4.62 | 54.9 | green | 72 |
| Comparative Example 20 | T | 4.63 | 71.7 | green | 157 |
| Comparative Example 21 | 2-3 | 5.21 | 47.0 | green | 55 |
| Comparative Example 22 | 2-4 | 5.75 | 41.1 | green | 49 |
| Comparative Example 23 | 2-5 | 5.71 | 42.6 | green | 51 |
| Comparative Example 24 | 2-7 | 5.48 | 45.3 | green | 56 |
| Comparative Example 25 | 2-31 | 5.75 | 41.2 | green | 41 |
| Comparative Example 26 | 2-32 | 5.48 | 40.2 | green | 54 |
| Comparative Example 27 | 2-42 | 5.52 | 41.0 | green | 53 |
| Comparative Example 28 | 3-2 | 5.83 | 42.9 | green | 45 |
| Comparative Example 29 | 3-3 | 5.84 | 43.0 | green | 45 |
| Comparative Example 30 | 2-98 | 5.39 | 45.1 | green | 59 |
| Comparative Example 31 | 2-110 | 5.35 | 48.2 | green | 68 |
| Comparative Example 32 | 3-107 | 5.46 | 46.3 | green | 62 |
| Comparative Example 33 | 3-119 | 5.43 | 46.7 | green | 71 |

The comparative compounds A, B and C were substituted with carbazole, dibenzofuran, and dibenzothiophene substituted with phenyl at position 2 of dibenzofuran, respectively. It can be seen that the lifetime in the case of a carbazole group and the operating voltage in the case of dibenzofuran or dibenzothiophene are relatively good or almost similar.

In the case of comparative compound D, the 2 and 4 positions of dibenzofuran were substituted with dibenzofuran and diphenyl triazine. The operating voltage and efficiency were excellent, but the lifetime was insufficient, as compared to the comparative compounds E, F, G, and H, and the lifetime was insufficient as compared to comparative compounds I, J, and K with similar operating voltage and efficiency. The reason is that when only one side of the core of dibenzofuran is used, since the LUMO/HOMO levels are not properly divided and are mixed with each other, the structural stability is lower than that of comparative compounds E, F, G, and H.

Comparative compounds I, J, and K showed improved operating voltage, lifetime, and efficiency as compared to comparative compounds E, F, G, and H. Substituents constituting the HOMO level in the compounds are biphenyl, diphenyl, carbazole, dibenzofuran, and dibenzothiophene, respectively. The phenyl group is inferior in its property as a relative electron donor compared to carbazole, dibenzofuran, and dibenzothiophene. Therefore, in the case of the phenyl group, since the property as an electron donor toward triazine, which is a withdrawing unit, is weak, and the flow of electrons in the molecule is inferior, a stronger voltage is required. However, the luminous efficiency is lowered, and non-radiative recombination occurs due to charge accumulation in the light emitting layer by a strong voltage, thereby resulting in a short lifetime.

In the case of L in comparative compounds I, J, K, and L, dimethylfluorene, which is a ring group containing no hetero atom, is substituted. This structure showed a low voltage and a similar level of efficiency, but showed a low lifetime due to the weak bonding force of the methyl-bonded position in dimethylfluorene. Dibenzofuran, dibenzothiophene, or carbazole, which have strong resonance structures because N, O, and S are substituted at the same position, have high structural stability and thus longer lifetime.

Comparative compounds I, J, and K have similar tendencies, but it can be seen that the stable electron donor property of the carbazole group is advantageous for long lifetime characteristics. However, even when dibenzofuran and dibenzothiophene were combined, there was a lifetime deviation of about 10%, but it can be confirmed that the operating voltage was slightly improved while maintaining similar efficiency.

The properties of the electron donor and structural stability of the carbazole group are superior to those of dibenzofuran or dibenzothiophene, but the strong electronegativity of O and S atoms has an advantage in increasing the charge mobility in a material. Therefore, the movement of electrons is made faster and operating voltage is low. However, as the mobility of electrons increases, since the stability of the molecule itself is affected, there is a disadvantage that when electrons are concentrated in a positionally vulnerable part, radicalization may proceed and thus molecular degradation may proceed.

In the case of comparative compounds J, M, N, O, P, Q, R, and S, changes can be seen depending on the bonding position of triazine and dibenzofuran. In the case of comparative compounds J, M, and N with enhanced orthogonality, it can be seen that the relative absolute lifetime values are increased. In the case of comparative compounds O, P, and R, it can be seen that if a stronger steric hindrance is given to the molecular structure, the lifetime can be reduced, but the operating voltage or efficiency can be improved.

In the case of comparative compound Q, it is a substance in which a triazine group is substituted at position 2 of dibenzofuran. It can be seen that operating voltage and efficiency are good, but lifetime is only half. When a triazine group is introduced at the corresponding position, since it has a high T1 level, it may be advantageous in terms of efficiency, but due to the heteroatom substitution properties of dibenzofuran/dibenzothiophene, position 2 is equivalent to the substitution of the para position of O and S. This position is a good position for degradation by radicalization in dibenzofuran and dibenzothiophene, and if the acceptor that attracts electrons is substituted, as radicalization is proceeded more easily, the lifetime tends to decrease rapidly.

In the case of comparative compound S, it is a substance in which positions 1 and 9 are substituted, and has the form with the greatest steric hindrance. In this case, it can be seen that structural distortion is deepened due to steric hindrance, and thus structural stability is lowered. Both HOMO and LUMO levels do not spread widely, and remain narrow within the substituents. Therefore, even with a phenyl group substituted in dibenzofuran or triazine in the center, the orbital does not expand and thus it has an unstable form, showing low efficiency and lifetime.

Comparative Examples 21 to 29 are all P-type donor hosts centered on a carbazole group, which is a strong electron donor. These have a strong electron donor tendency, but since their electron acceptor is composed of an arylene group and a substituted dibenzothiophene group which have very poor properties compared to the triazine group, these exhibit high operating voltage, and low efficiency and lifetime.

According to the present invention, it is possible to obtain a host material having a long lifetime and high efficiency by enhancing the low-power characteristics of dibenzofuran and dibenzothiophene, and at the same time introducing an aryl substituent into the material, and thus extending the HOMO level and enhancing the structural stability of the material.

It was confirmed that as shown in Table 19 (Examples 1 to 132 and Examples 219 to 224), the heterocyclic compound represented by Formula 1 according to the present invention has improved operating voltage, efficiency, and lifetime by having Rc of Formula 1 above, as compared to Comparative Examples 10 to 16 (comparative compounds J, K, M, N, O, and P). In the case of dibenzofuran/dibenzothiophene to which triazine is bound, LUMO is established around triazine. At this time, the LUMO extends and affects even to the dibenzofuran/dibenzothiophene core (hereafter, core) located at the center of the compound, due to the influence of O and S, which are atoms with strong electronegativity.

The cyclic group such as dibenzofuran/dibenzothiophene or other aryl, arylene, and heteroarylene bonded to the opposite side of the core has relatively strong electron donor properties, so that HOMO is located. However, the region occupied by the HOMO in the entire compound does not extend to the core, and has a form limited to an arylene or heterocyclic group substituted in the core. Therefore, it has a molecular structure having a very strong acceptor tendency. In the case of such a molecular structure, fast electron movement is achieved during operation of the device, and it has advantageous properties in constructing a device having a lower operating voltage.

The more electrons are attracted, the greater the electrical stress applied to the compound. However, the core unit has two substituents, and in particular, due to the influence of the triazine unit, electrons in the entire core become insufficient, and thus the possibility of radicalization is lowered.

Triazine and its substituted cyclic groups (Ra, Rb) also lack electrons due to the strong electron-withdrawing tendency of triazine, and thus deterioration due to chemical properties is not large even during operation of the device. Unlike triazine, dibenzofuran/dibenzothiophene opposite to the core may have high molecular stability due to the resonance effect of the condensed ring group, but may cause sufficient radicalization or cleavage of the benzene ring, due to the heat generated during operation of the device and the energy of the supplied current. In particular, the P (para) position of the O and S atoms is a good position for radicalization, and the energy transferred by non-radiative recombination such as TTA and TPA generated during operation of the device transfers enough energy to break the bonds of molecular structure. The deterioration of the light emitting layer caused by it causes insufficient lifetime performance when a carbazole group having relatively high stability is substituted, as compared with Comparative Example T.

However, it is possible to increase the stability of the molecular structure by introducing an aryl group and thus extending the conjugation region of dibenzofuran/dibenzothiophene. In addition, as the conjugation region is extended, the region of the HOMO level existing at the corresponding position is also widened and has a more stable region.

The dibenzofuran/dibenzothiophene introduced into the host has a role as a weak donor, as compared to the carbazole group mainly used in the existing bipolar host. Of course, it has a low voltage characteristic due to the strong acceptor tendency, and higher efficiency could be pursued depending on the structure of the device. However, excessive current flow and disruption of the balance within the molecule deteriorated the stability of the entire light emitting layer, causing fatal weakness in the lifetime. The HOMO level distributed in a narrow area due to the influence of strong acceptor was expanded by introducing this additional aryl group, and accordingly, the electrical stability of the compound was strengthened. At this time, since the acceptor unit maintains the same substituents as the existing ones, it is possible to increase the stability of the unstable HOMO level, so that a long-life host with low voltage and high efficiency characteristics can be obtained, while maintaining fast electrical characteristics.

Referring to the results of Table 20 (Examples 133 to 218 and Examples 225 to 256), when the compound (n-type) represented by the formula 1 according to the present invention is used together with the compound represented by the Formula 2, better efficiency and lifetime effect are exhibited. This result is because when both compounds are included at the same time, an exciplex phenomenon occurs.

The exciplex phenomenon is a phenomenon in which energy having the size of the HOMO energy level of the donor (p-host) and the LUMO energy level of the acceptor (n-host) is emitted by the exchange of electrons between two molecules. When the intermolecular exciplex phenomenon occurs, Reverse Intersystem Crossing (RISC) occurs, which can increase the internal quantum efficiency of fluorescence to 100%. When a donor (p-host) with good hole transport ability and an acceptor (n-host) with good electron transport ability are used as hosts for the light emitting layer, since holes are injected into the p-host and electrons are injected into the n-host, the operating voltage can be lowered, thereby helping to improve the lifetime. In the present invention, it was confirmed that when the compound of Formula 2 as a donor and the compound of Formula 1 as an acceptor are used as a host of the light emitting layer, excellent device properties are exhibited.

As can be seen from the results of Tables 19 to 21, it was confirmed that the organic electroluminescent device using the material for the light emitting layer of the organic electroluminescent device of the present invention has a low operating voltage, and not only improved luminous efficiency, but also significantly improved lifetime, as compared to the comparative compounds J, K, M, N, O, P, Q, R, S, and T.

The condensed ring group introduced into the triazine group can make the strong electron withdrawal properties of triazine stronger. Irrespective of the presence or absence of heteroatoms, it is possible to expand the LUMO level by introducing a polycondensed ring having a stable structure and thus securing a wide conjugation region. Through this, the electron mobility is made stronger, and it plays a role in maintaining sufficient current efficiency even at low voltage, and adjustment to an appropriate level of T1 energy required for red light emission is also performed.

Since multiple colors can be implemented in the same structure based on the existing structure depending on whether or not the condensed ring of the substituent is introduced, the extensibility of the structure is limitless, and even if various device structures such as RGB method, 2-stack, and 3-stack are introduced, there is an advantage that devices having similar shapes can be constructed.

Since a material having an appropriate work function can be manufactured depending on the position of the substituent, even if a new device is constructed in the future, it can be used as an element to control the energy level as well as the electron mobility of the entire device.

When aryl or non-condensed arylene is introduced as a specific substituent of dibenzofuran/dibenzothiophene, the conjugation region of the substance itself is expanded, but it is connected by a single bond. Accordingly, although it affects the expansion and energy level of a certain level of the HOMO level, there is no significant change in the T1 energy level of the material. The reason is that the electron withdrawal or donating tendency of the substituents substituted at this position is not large. Rather, the aryl or arylene group that can be substituted as a relatively weak donor and acceptor compared to carbazole group/dibenzofuran/dibenzothiophene/amine group or triazine group/pyrimidine group/pyridine group/imidazole group has a close relationship with which substituent it is substituted. Thanks to this, it expands the conjugation region of the substituted unit and does not significantly affect the work function at any level. Therefore, since it does not have a significant effect on maintaining the intrinsic T1 energy level of dibenzofuran/dibenzothiophene, excellent efficiency and long lifetime characteristics without hindrance in use as a phosphorescent green host could be expressed.

However, when the condensed ring group is substituted, since a stronger effect of pi-pi stacking occurs, the energy level of the core unit itself is also affected by the wide conjugation region of the condensed ring itself and the low T1 energy level, and it has a relatively low T1 energy level.

The pre-mixing according to the present invention refers to first mixing the heterocyclic compound represented by Formula 1 and the heterocyclic compound represented by Formula 2 and putting them in one source to mix them, before deposition on the organic layer. In the case of pre-mixing, there is an advantage of making the process simpler because one deposition source is used instead of using two or three deposition sources.

When pre-mixing as described above, the unique thermal properties of each material should be checked and mixed. In this case, when the pre-mixed host material is deposited from a single deposition source, the deposition conditions including the deposition rate may be greatly affected according to the intrinsic thermal properties of the materials. If the thermal properties between two or more premixed materials are not similar and very different, repeatability and reproducibility in the deposition process cannot be maintained, which means that uniform OLED devices cannot be manufactured in one deposition process.

In order to overcome this, while tuning the electrical properties of materials by utilizing an appropriate combination of the basic structure and substituents of each material, it is also possible to control the thermal properties depending on the shape of the molecular structure. Therefore, it is possible to achieve an improvement in the performance of the device by utilizing not only C-N bonding of carbazole as shown in Formula 2 but also several substituents on the triazine group or dibenzofuran/dibenzothiophene in Formula 1 above, in addition to the basic skeleton, and it is also possible to secure a variety of different premixed deposition processes between host and host by controlling the thermal properties of each material. Through this, there is an advantage that it is possible to secure the diversity of the premix deposition process using 3 ~ 4 or more host materials as well as 2 compounds as a host.

Mixing of Formula 1 and Formula 2 may mix two or more types of materials, and the above experimental example is only a representative example, but is not limited thereto.

Through the mixing of Formula 1 and Formula 2, it is possible not only to obtain an effect of partially improving the current efficiency of the light emitting layer, but also to construct a device having a long lifetime. Referring to the results of Tables 19 to 21, when the compound of Formula 1 and the compound of Formula 2 are included at the same time, better efficiency and lifetime effect are exhibited. This is due to the exciplex phenomenon that occurs when two or more substances are mixed. The exciplex phenomenon is a phenomenon in which energy having the size of the HOMO energy level of the donor (p-host) and the LUMO energy level of the acceptor (n-host) is released through electron exchange between two molecules. When the exciplex phenomenon between two molecules occurs, Reverse Intersystem Crossing (RISC) occurs, which can increase the internal quantum efficiency of fluorescence to 100%. When a donor (p-host) with good hole transport ability and an acceptor (n-host) with good electron transport ability are used as hosts for the light emitting layer, since holes are injected into the p-host and electrons are injected into the n-host, and at this time, excitons are not quenched due to intermolecular electron exchange, and the lifetime of excitons that can have energy is increased. As a result, the overall current efficiency can be improved and also it can help to improve the lifetime of the device. In the present invention, it was confirmed that when the compound of Formula 2 serving as a donor and the compound of Formula 1 serving as an acceptor are used as a host of the light emitting layer, excellent device properties are exhibited.

When an exciplex phenomenon occurs, in some cases, the operating voltage rises. The reason is that an imbalance of holes and electrons occurred in the light emitting layer of the device. This is a problem that occurs due to the deviation of hole and electron mobility of each material between mixed hosts. Therefore, only when the balance of electron flow in the device is properly maintained, a device with optimal performance can be constructed.

## Claims

1. A heterocyclic compound represented by the following formula 1: 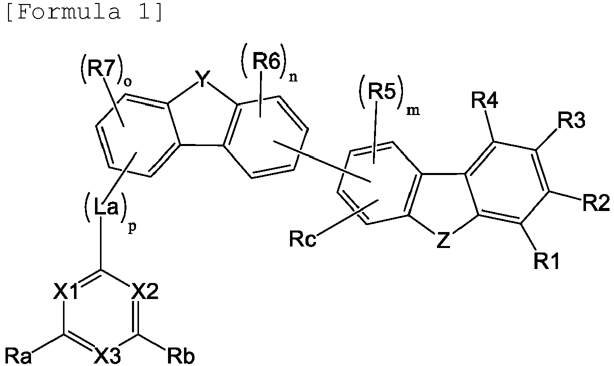 wherein,
X1 to X3 are each independently N or CR10, and at least two of X1 to X3 are N,
R10 is hydrogen, deuterium, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group,
Ra and Rb are the same as or different from each other, and are each independently a substituted or unsubstituted C6 to C60 aryl group, or a substituted or unsubstituted C2 to C60 heteroaryl group,
Rc is a substituted or unsubstituted C6 to C60 aryl group, or a substituted or unsubstituted C2 to C60 heteroaryl group,
La is a single bond, a substituted or unsubstituted C6 to C60 arylene group, or a substituted or unsubstituted C2 to C60 heteroarylene group,
Y and Z are the same as or different from each other and are each independently O, S or CR11R12,
R11 and R12 is hydrogen, deuterium, a cyano group, a substituted or unsubstituted C1 to C60 alkyl group, or a substituted or unsubstituted C6 to C60 aryl group,
R1 to R7 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, or form a substituted or unsubstituted C6 to C60 aromatic hydrocarbon ring or a substituted or unsubstituted C2 to C60 heterocyclic ring by combining two or more groups adjacent to each other, R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
m is an integer from 0 to 2, n is an integer from 0 to 3, o is an integer from 0 to 3, and p is an integer from 0 to 5.

2. The heterocyclic compound according to claim 1, wherein Formula 1 is represented by any one of Formulas 1-1 to 1-3 below: 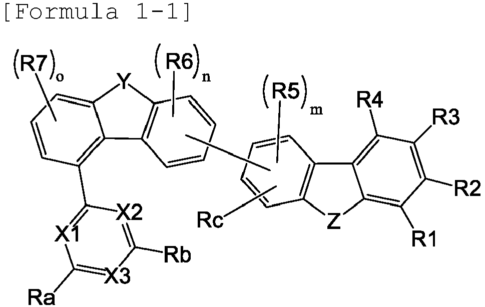 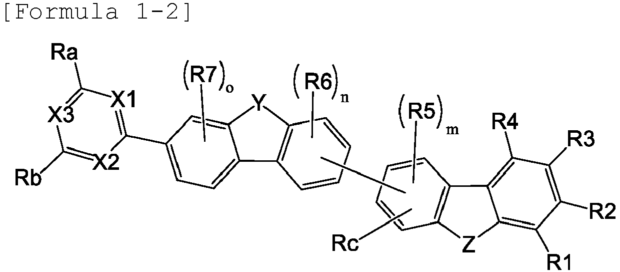 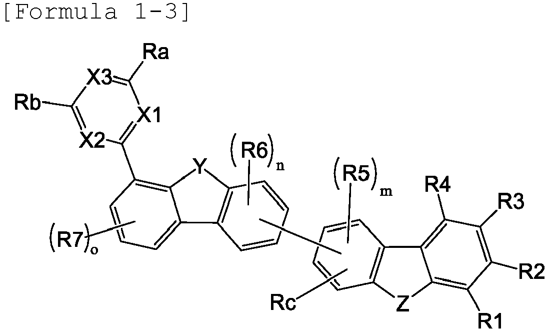 wherein the definitions of X1 to X3, Ra, Rb, Rc, Y, Z, R1 to R7, m, n and o are the same as those in Formula 1 above.

3. The heterocyclic compound according to claim 2, wherein Formula 1-1 is represented by Formula 1-4 or Formula 1-5: 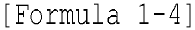 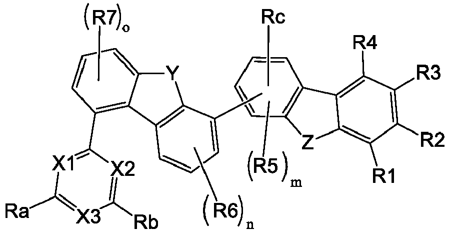 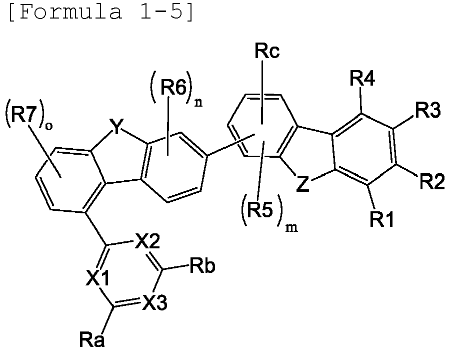 wherein X1 to X3, Ra, Rb, Rc, Y, Z, R1 to R7, m, n, and o have the same definitions as in Formula 1 above.

4. The heterocyclic compound according to claim 1, wherein Formula 1 is represented by any one of Formulas 1-6 to 1-11: 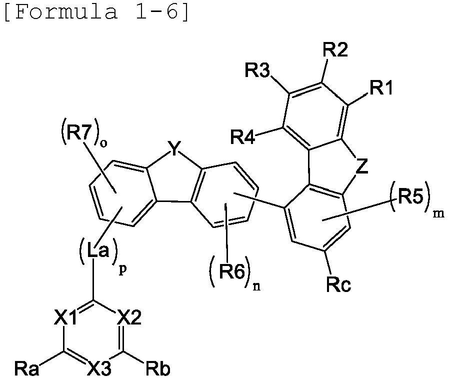 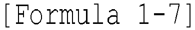 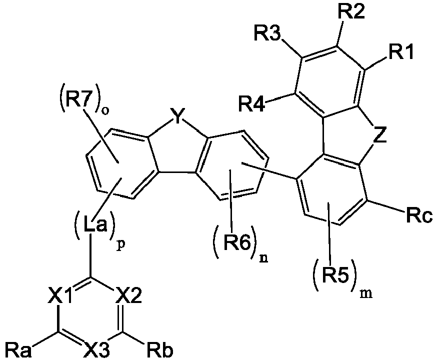 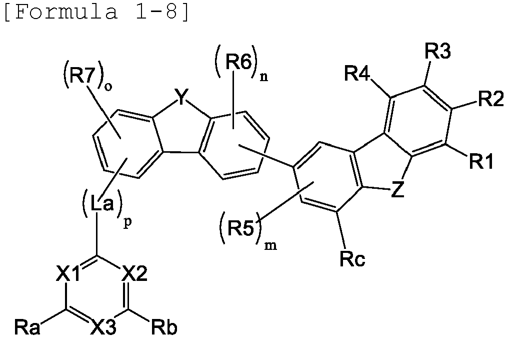 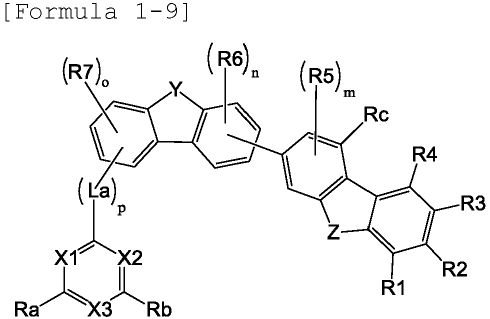 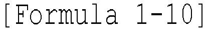 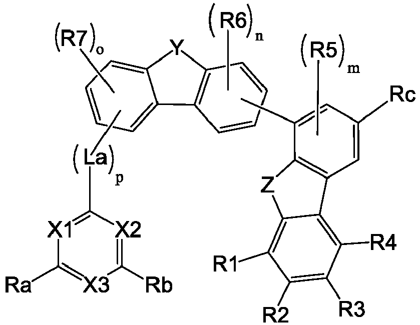 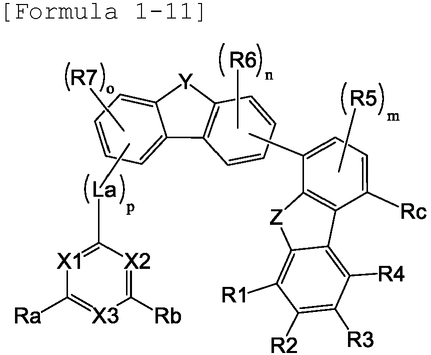 wherein the definitions of X1 to X3, Ra, Rb, Rc, La, Y, Z, R1 to R7, m, n, o and p are the same as those in Formula 1 above.

5. The heterocyclic compound according to claim 1, wherein All of X1 to X3 are N.

6. The heterocyclic compound according to claim 1, wherein La is a single bond.

7. The heterocyclic compound according to claim 1, wherein Rc is a substituted or unsubstituted C6 to C12 aryl group.

8. The heterocyclic compound according to claim 1, wherein Ra and Rb are the same as or different from each other and are each independently a substituted or unsubstituted C6 to C12 aryl group, or a substituted or unsubstituted C2 to C12 heteroaryl group.

9. The heterocyclic compound according to claim 1, wherein R1 to R7 are the same as or different from each other and are each independently hydrogen, deuterium or a substituted or unsubstituted C1 to C5 alkyl group.

10. The heterocyclic compound according to claim 1, wherein the heterocyclic compound represented by Formula 1 does not contain deuterium as a substituent, or the content of deuterium is 1% to 100% relative to the total number of hydrogen atoms and deuterium atoms.

11. The heterocyclic compound according to claim 1, wherein Formula 1 is represented by any one of the following compounds:

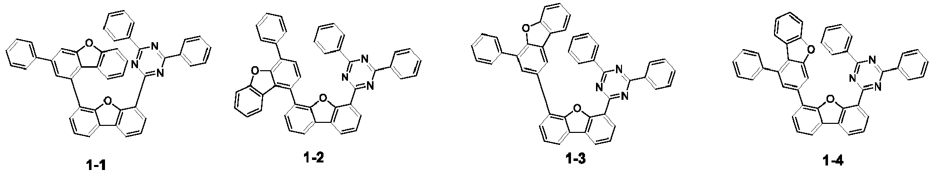

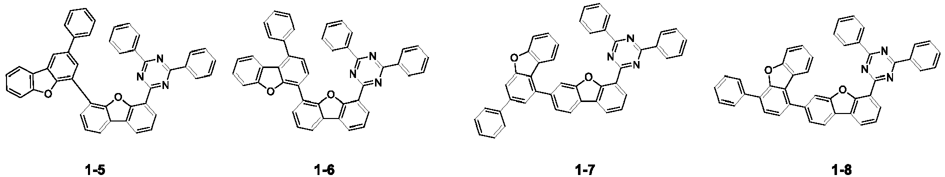

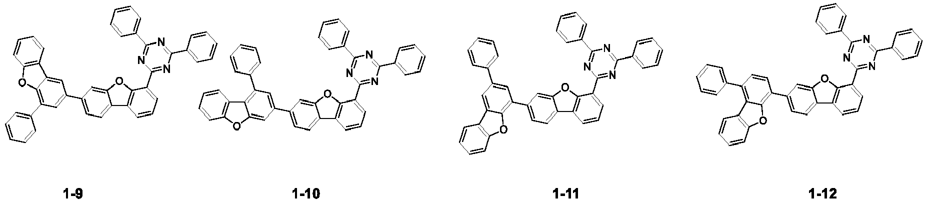

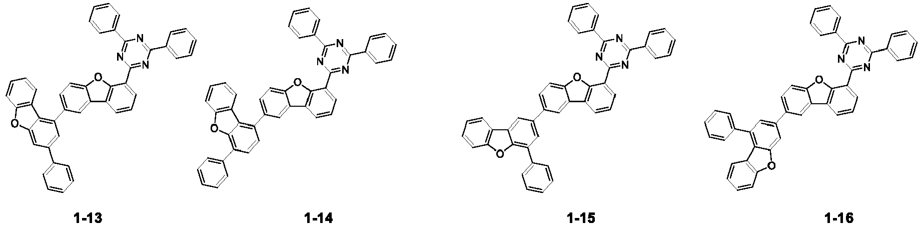

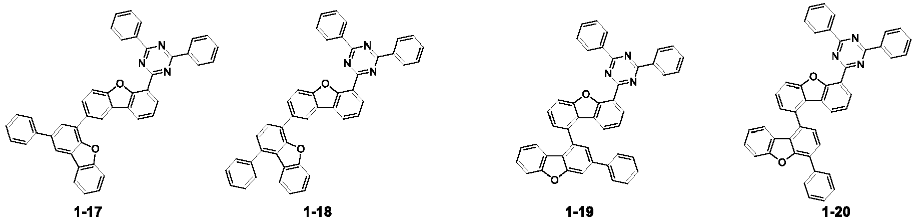

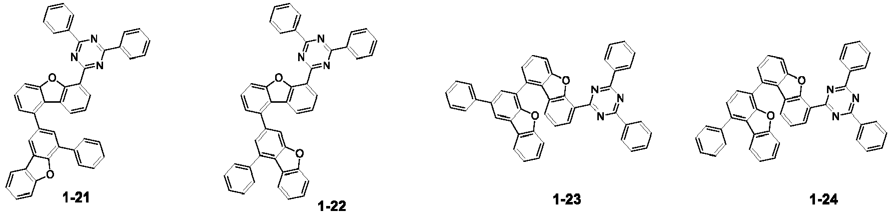

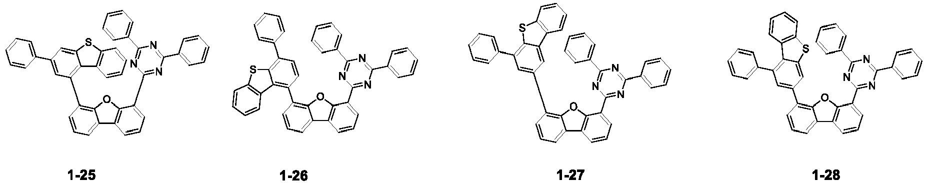

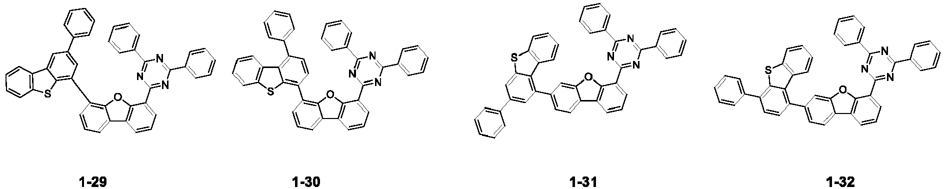

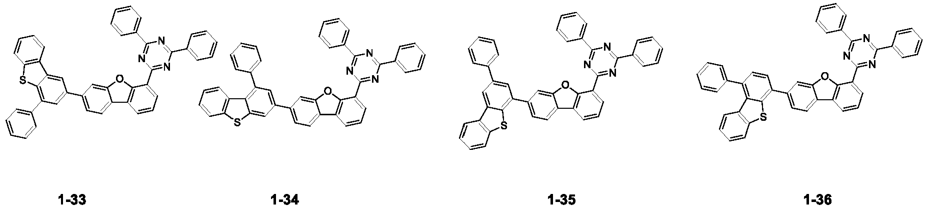

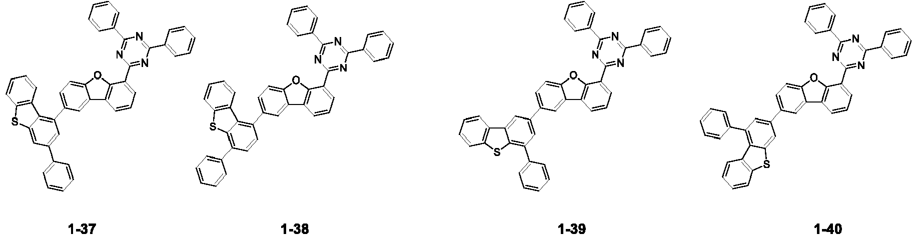

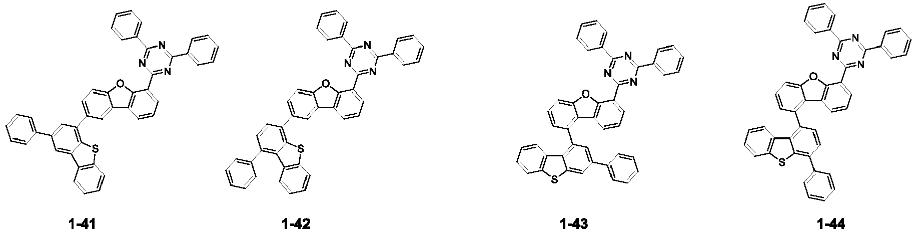

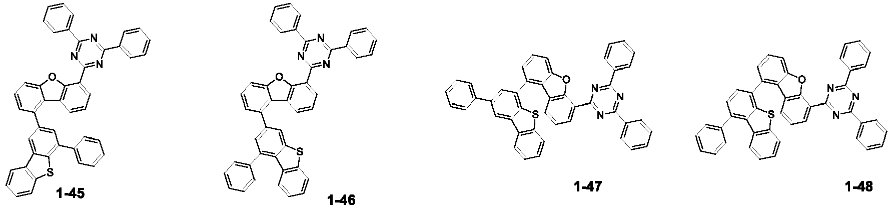

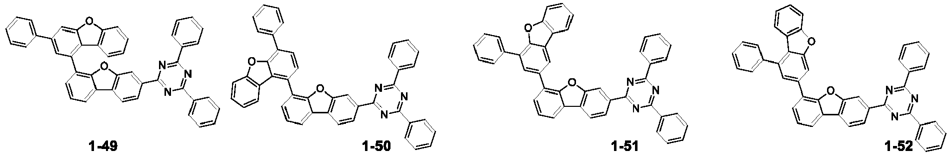

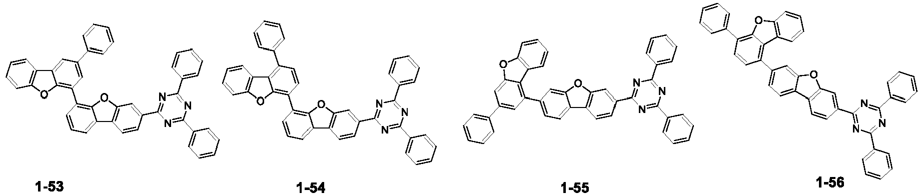

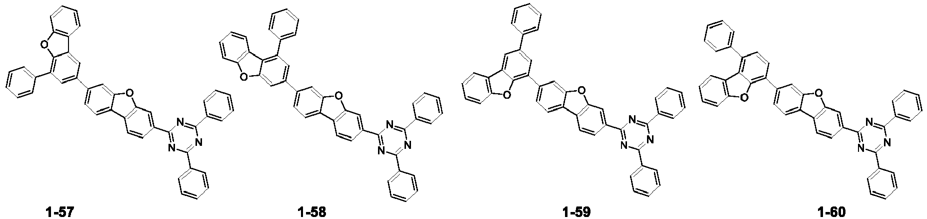

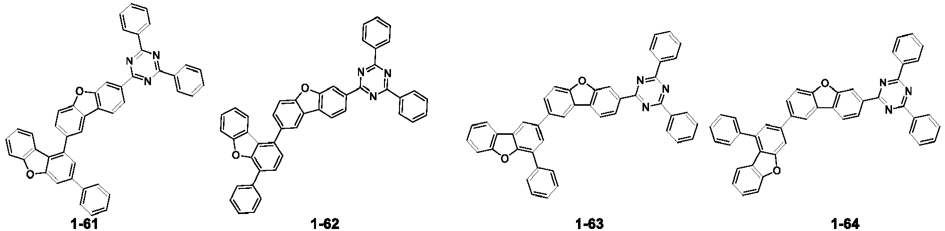

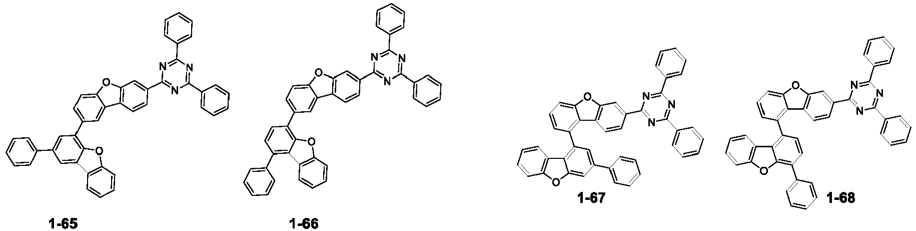

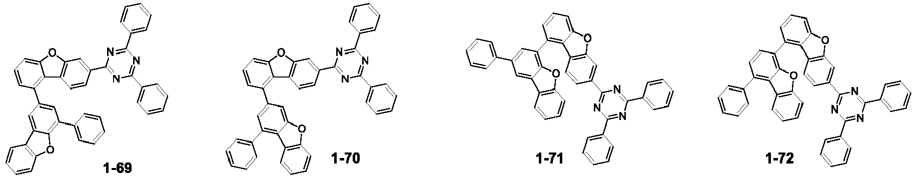

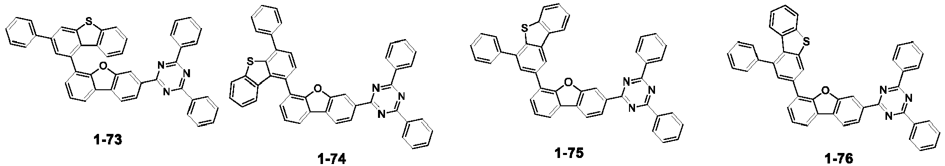

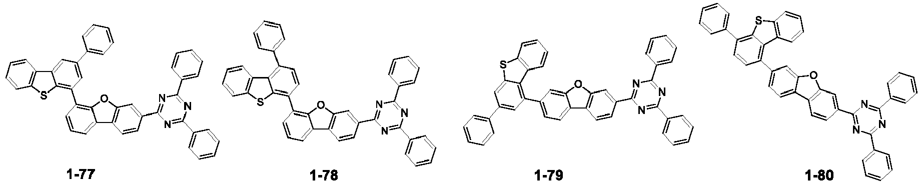

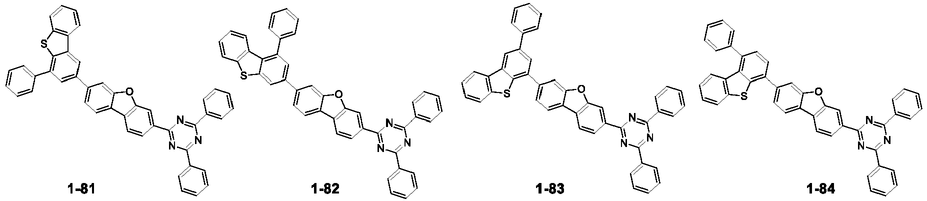

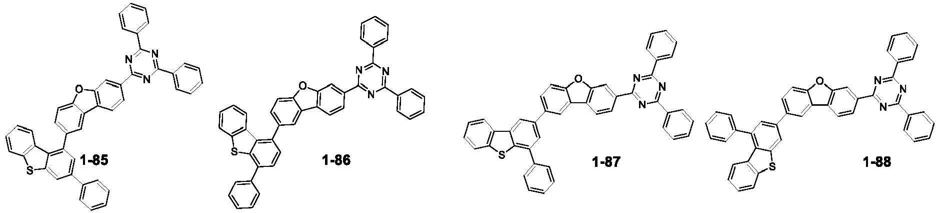

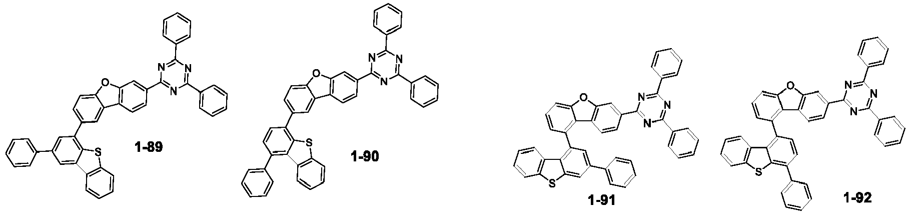

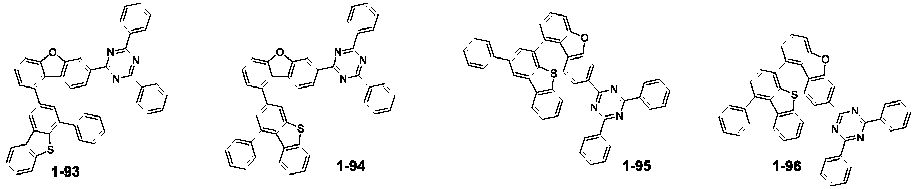

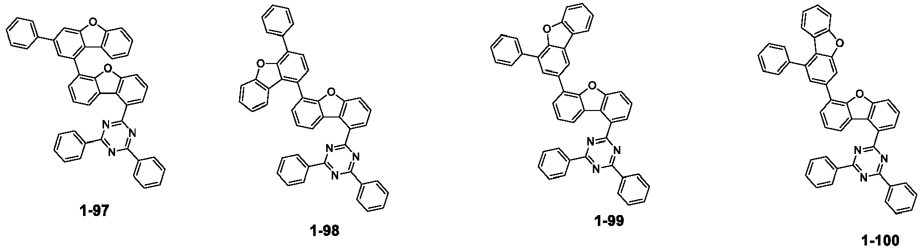

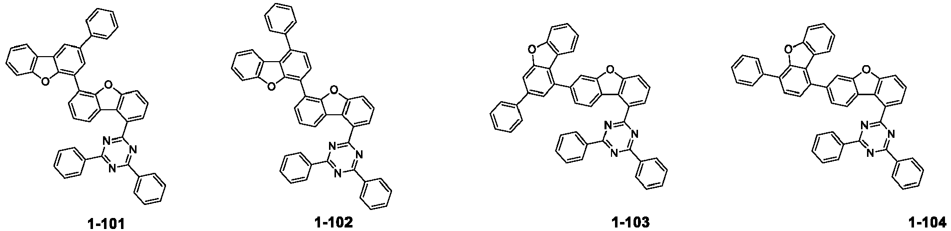

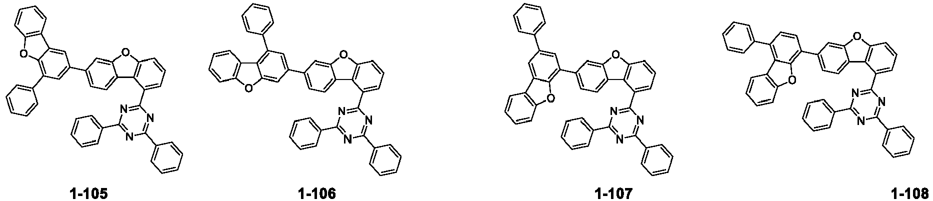

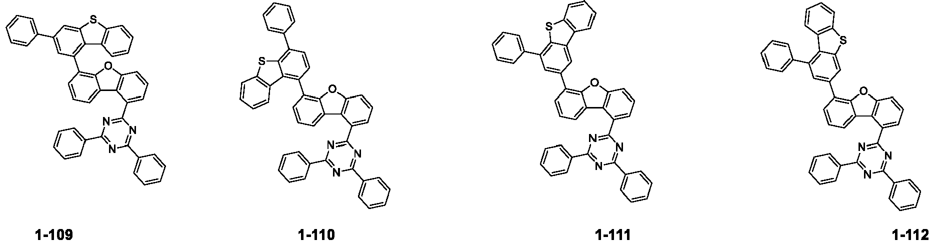

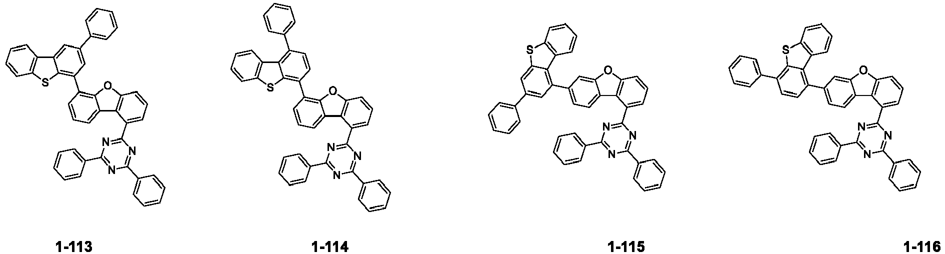

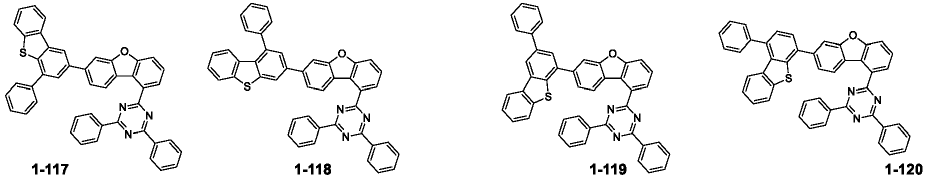

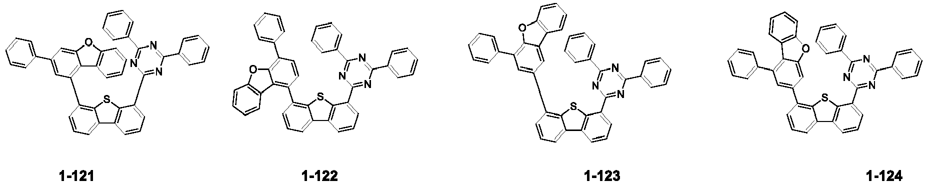

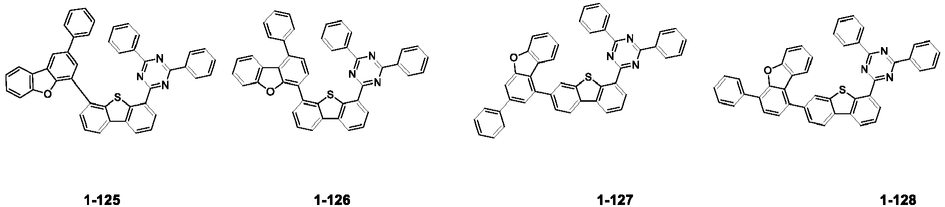

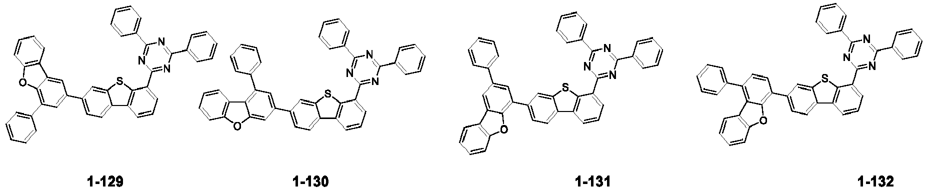

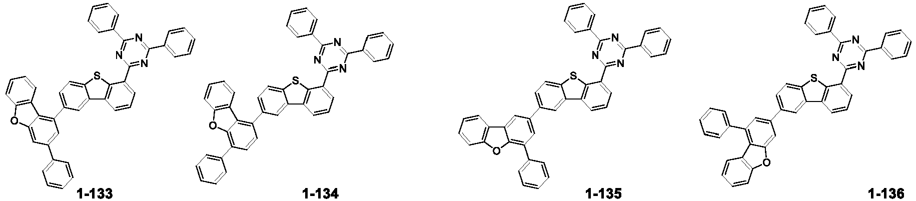

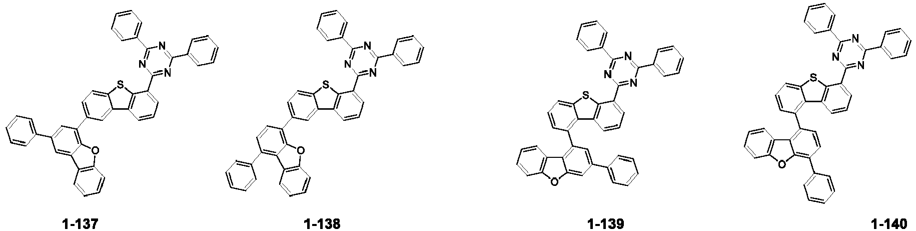

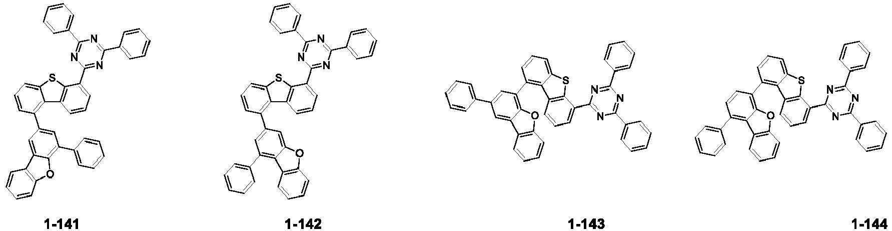

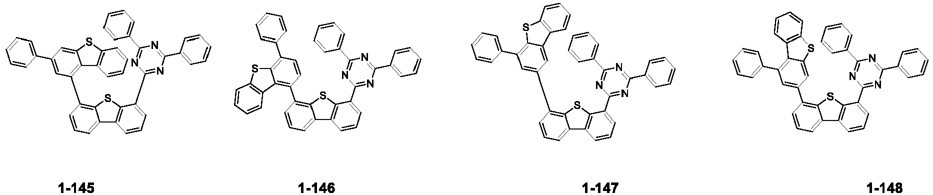

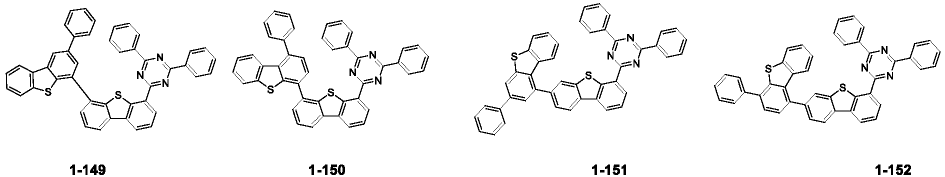

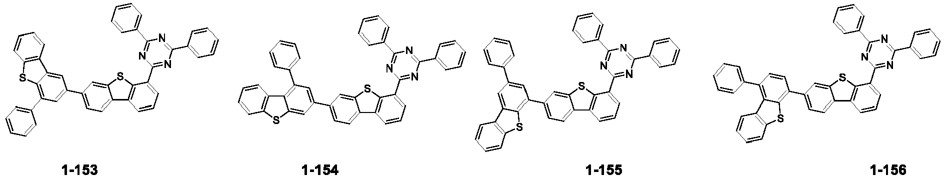

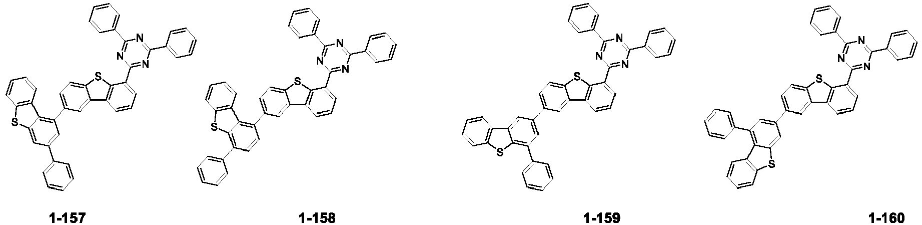

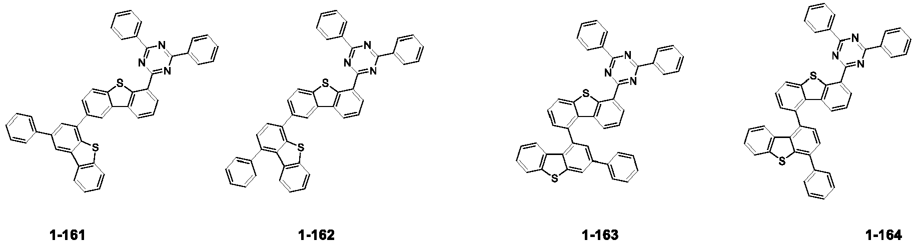

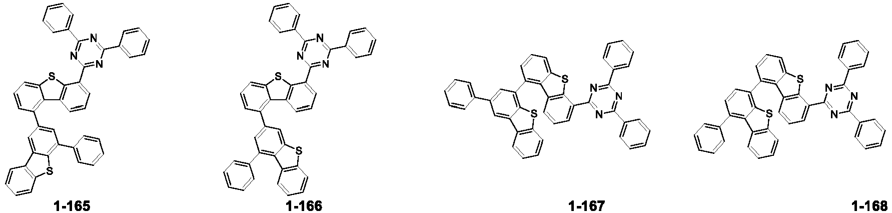

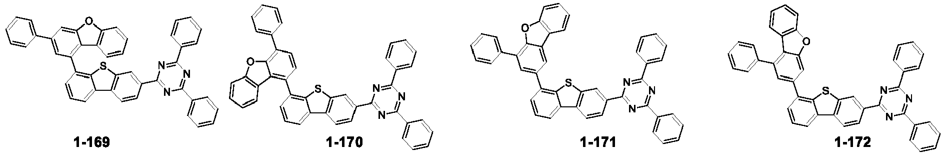

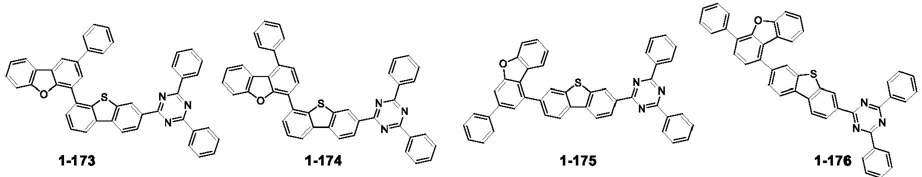

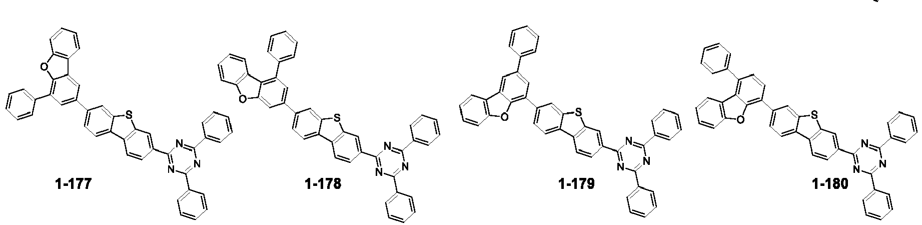

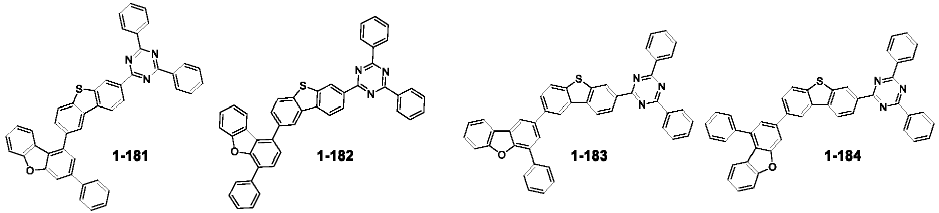

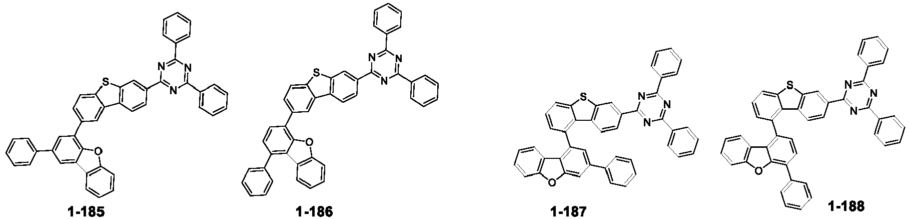

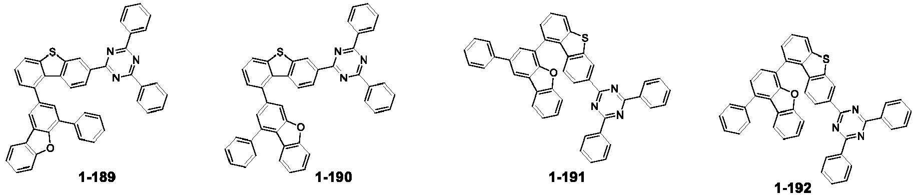

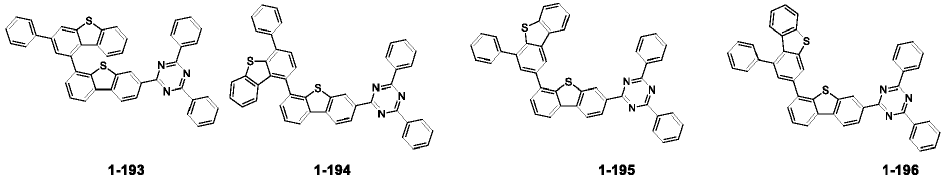

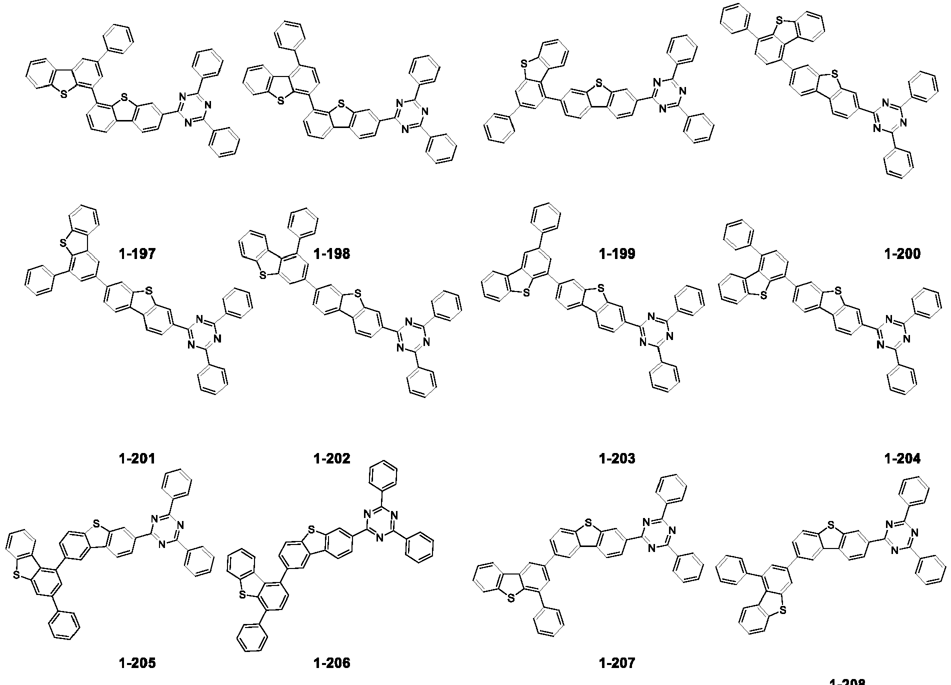

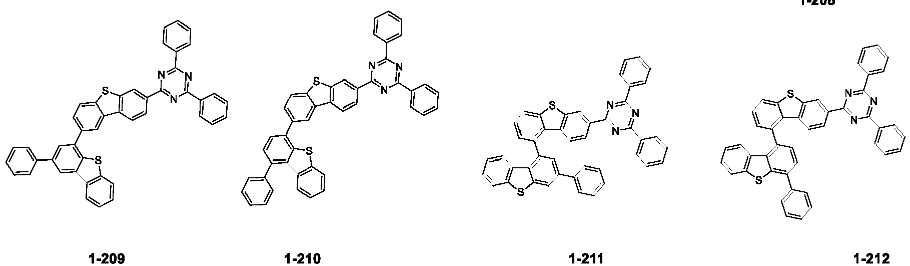

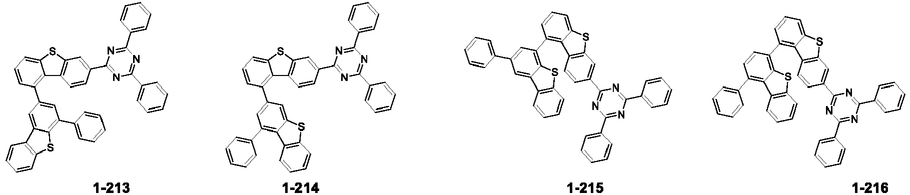

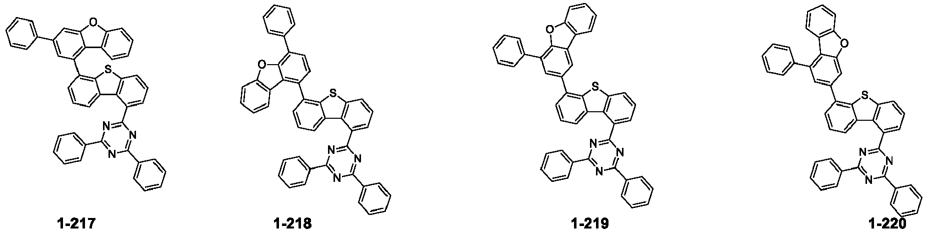

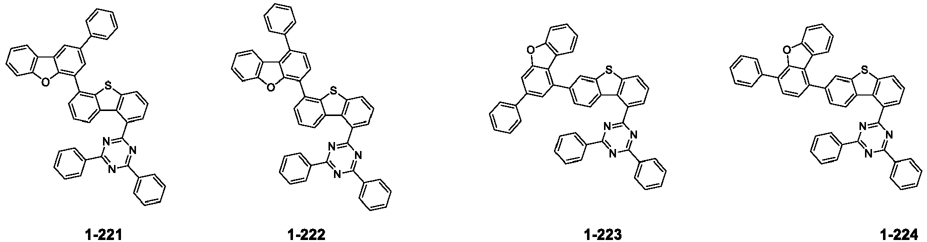

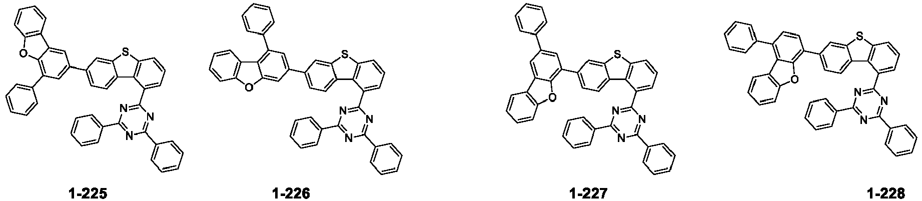

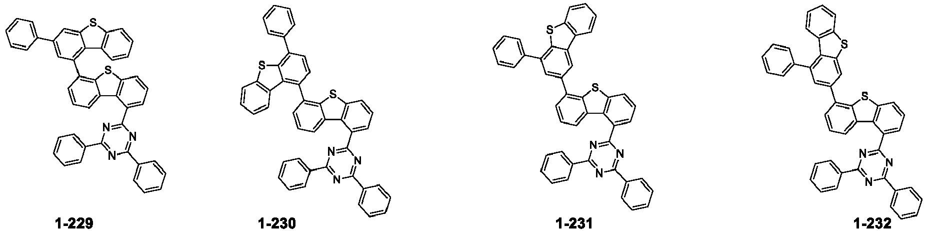

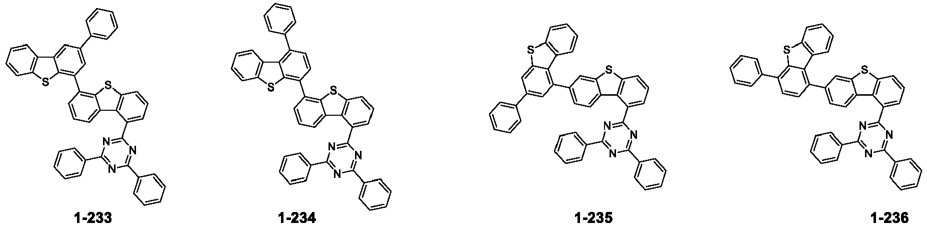

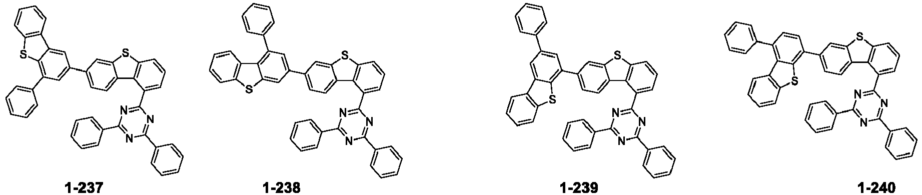

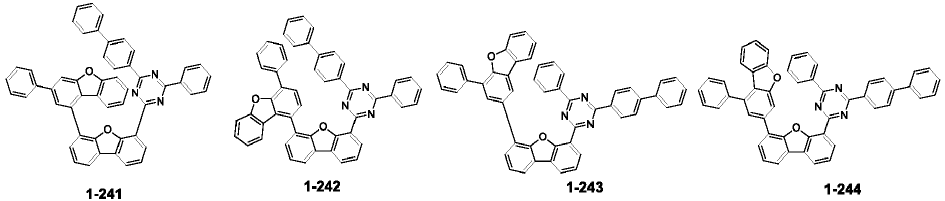

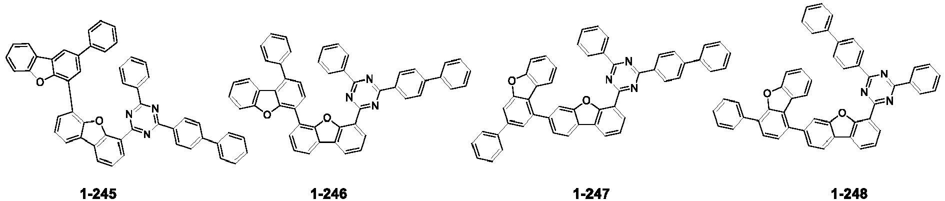

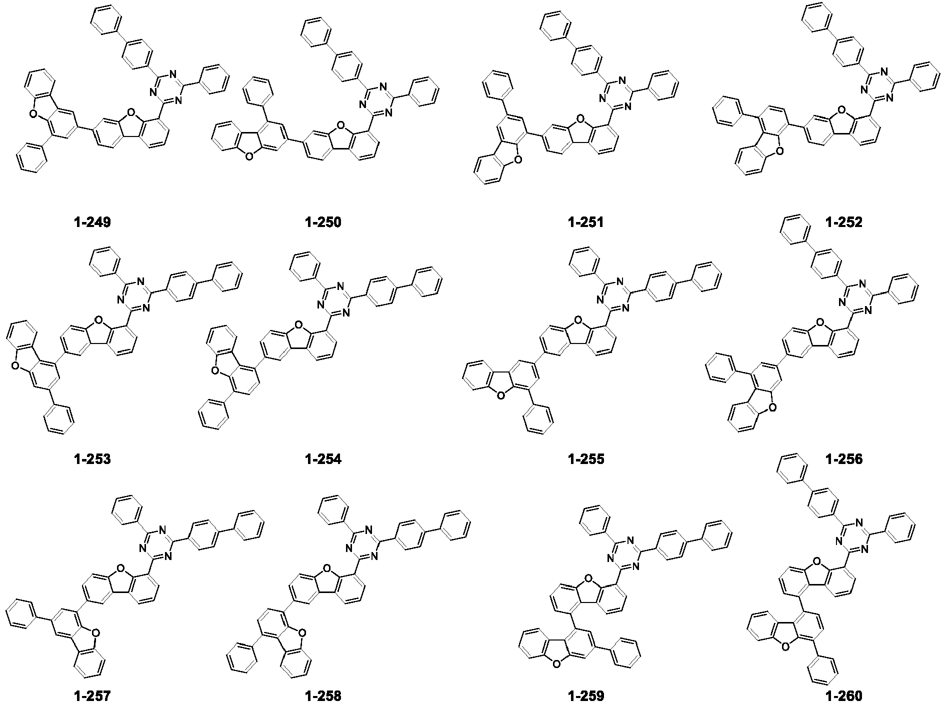

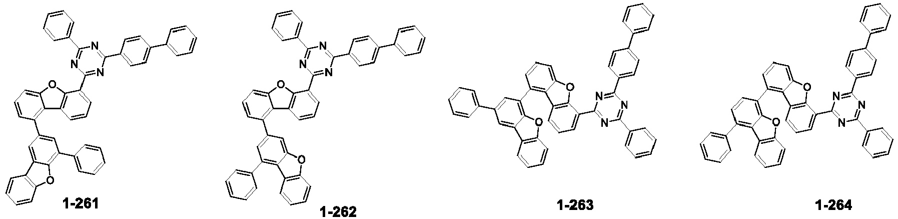

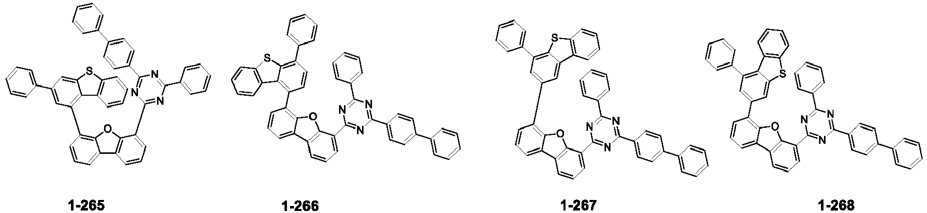

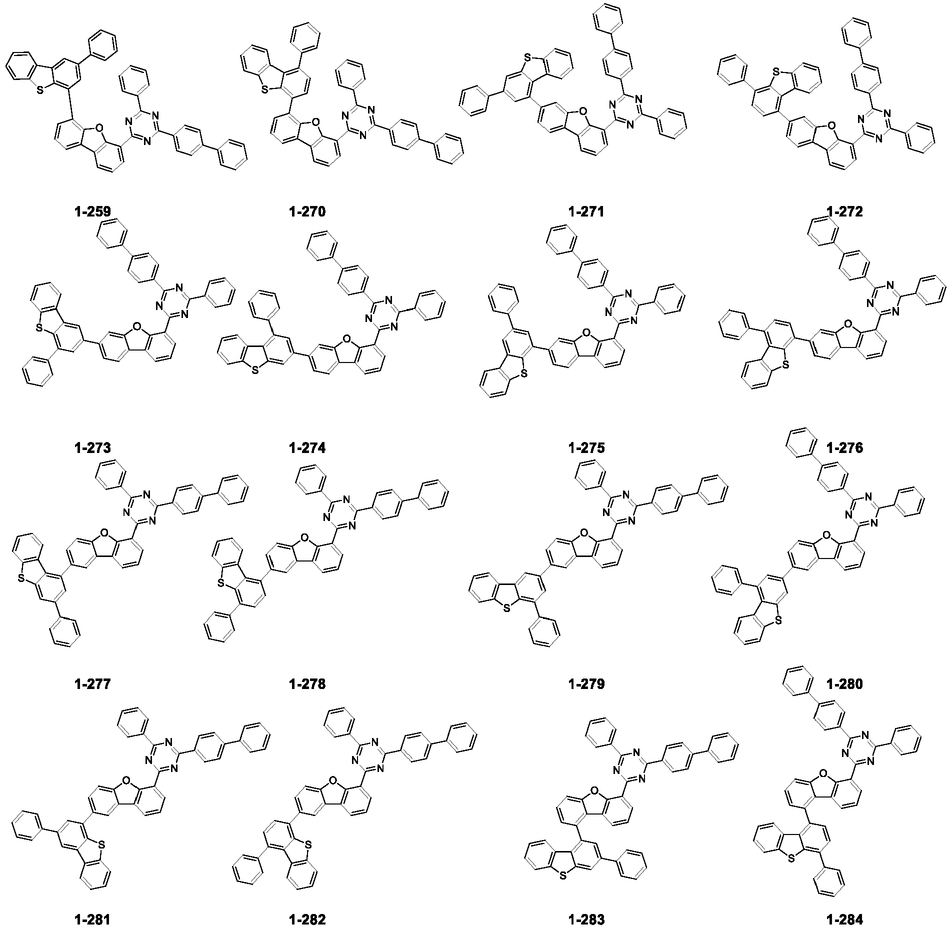

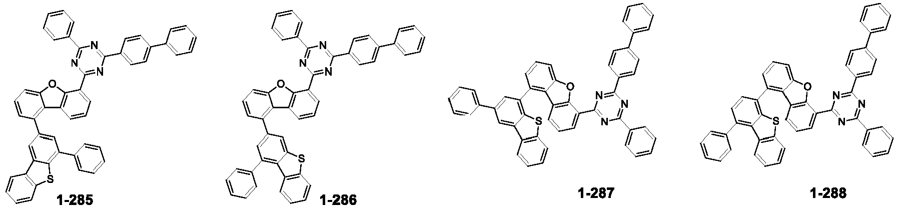

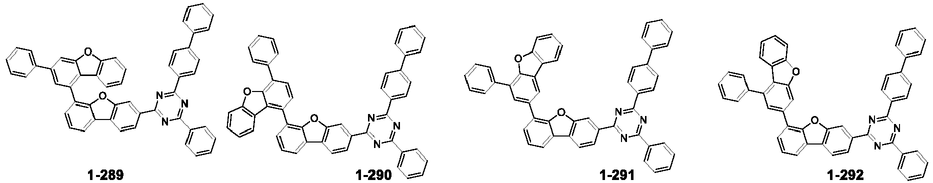

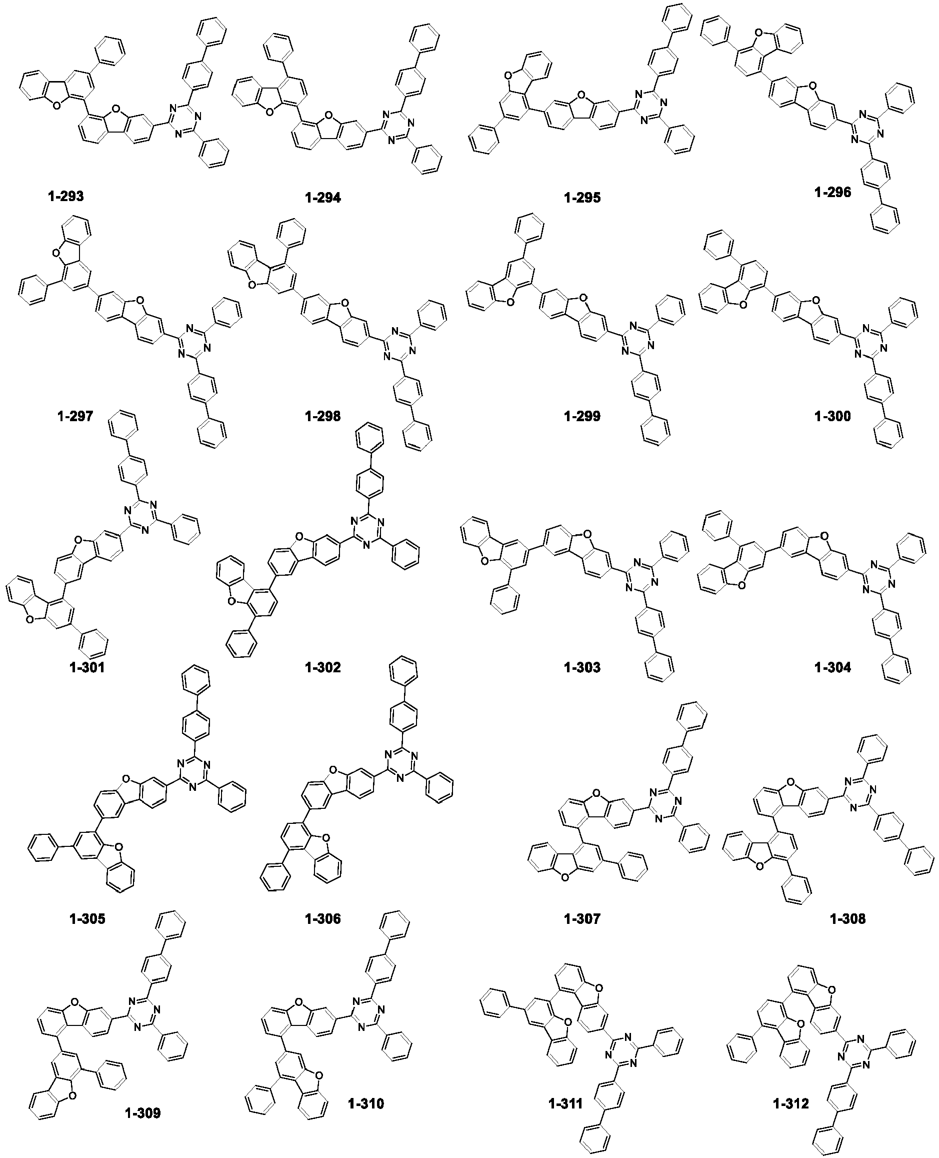

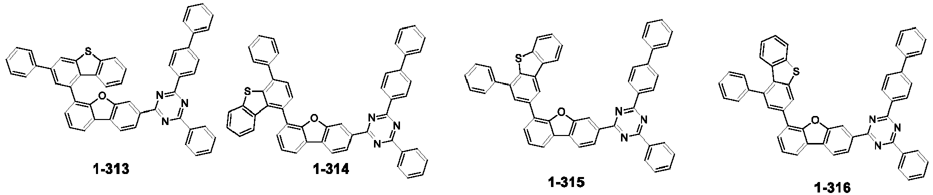

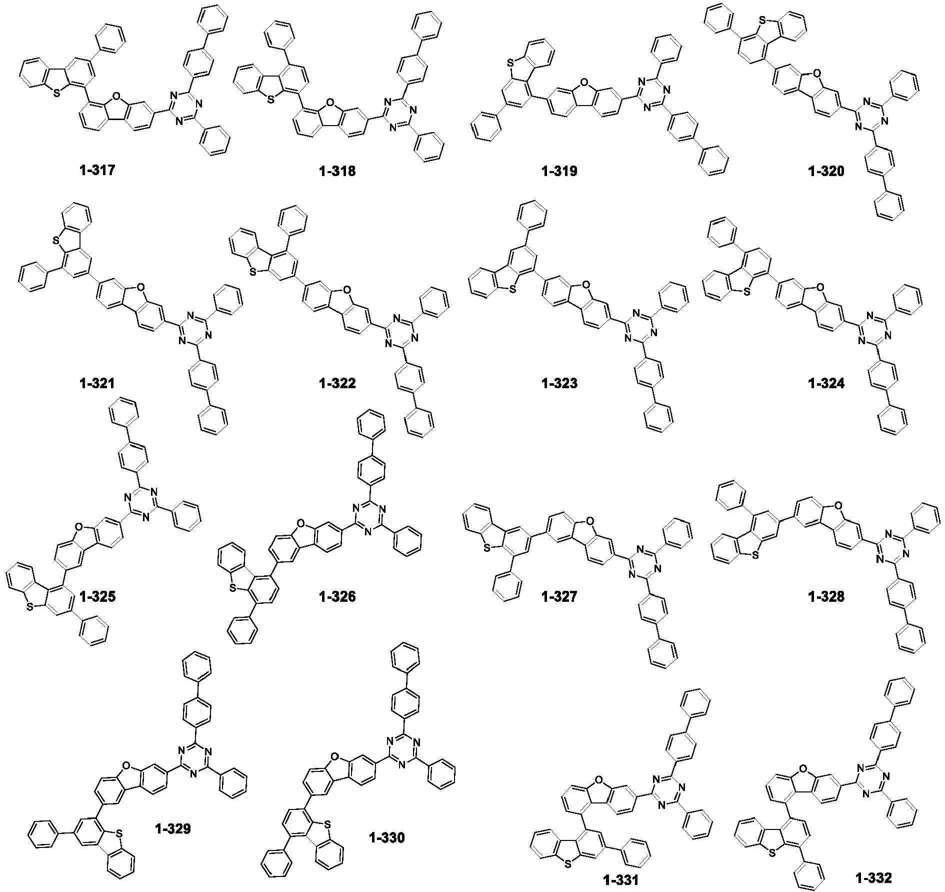

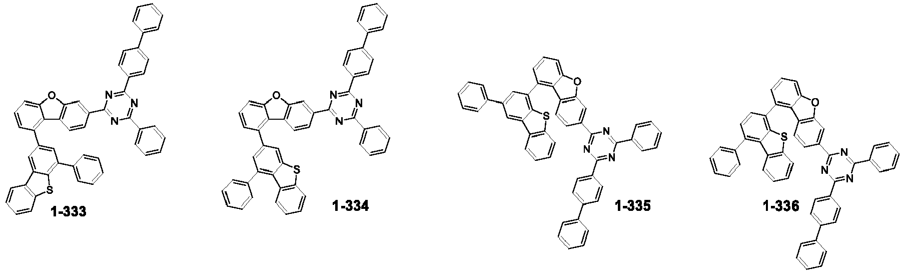

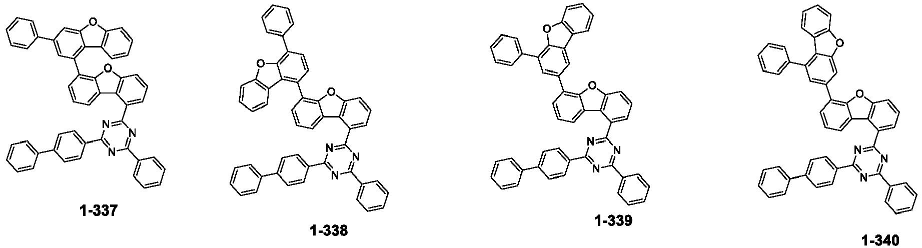

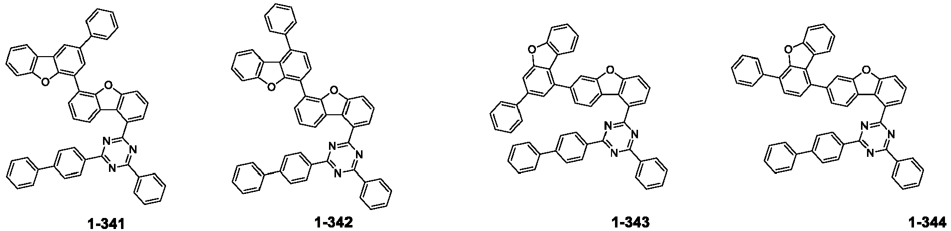

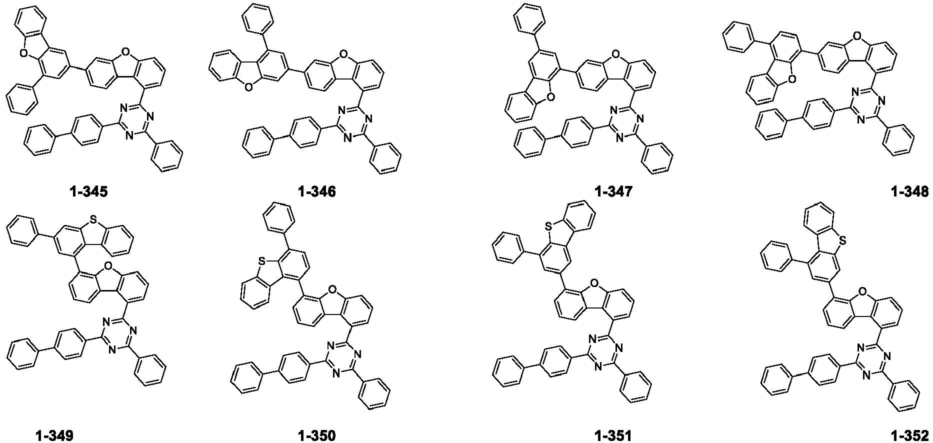

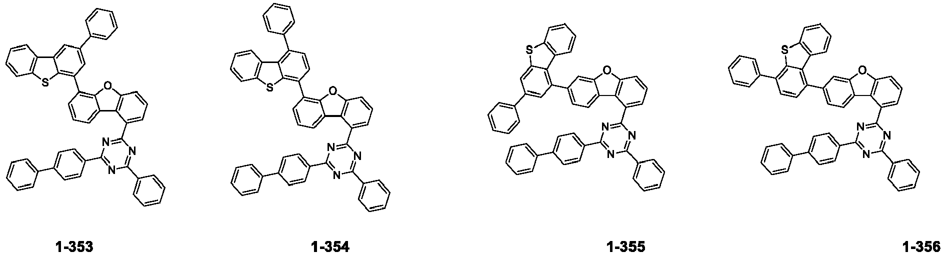

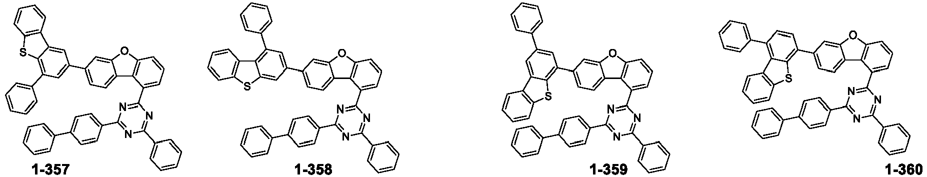

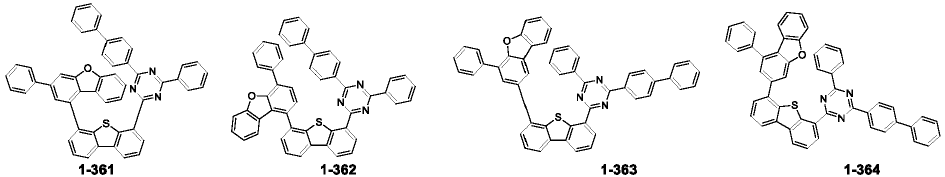

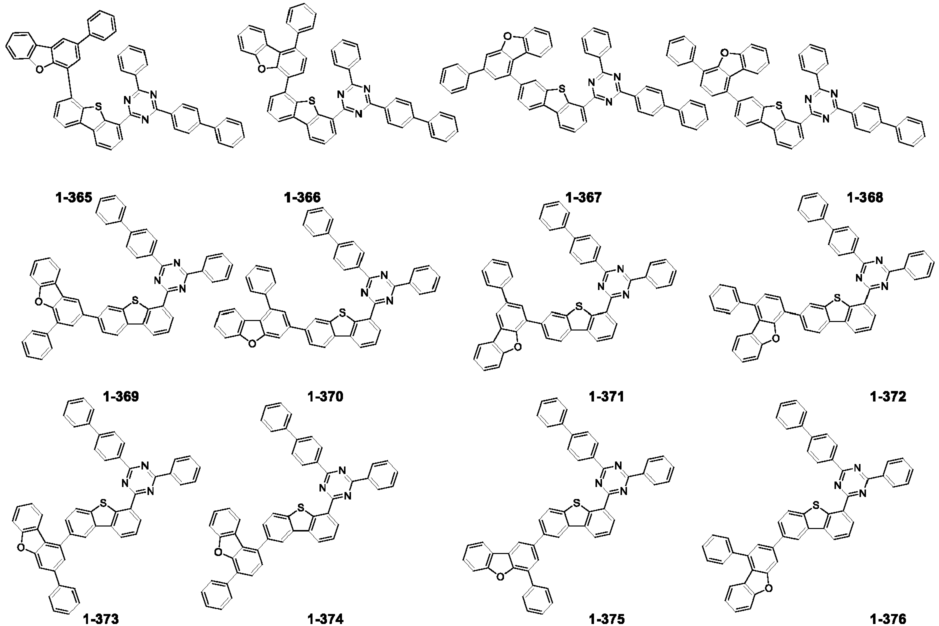

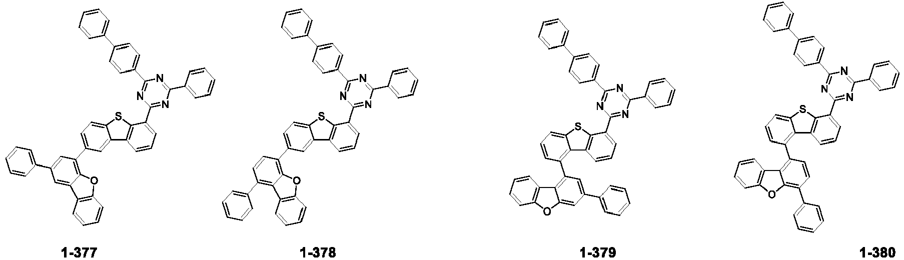

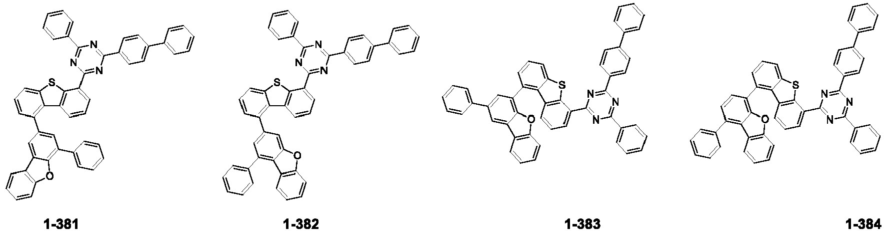

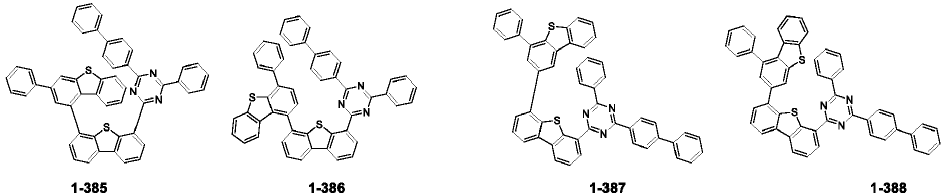

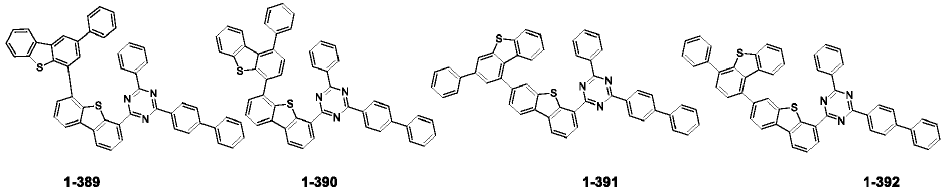

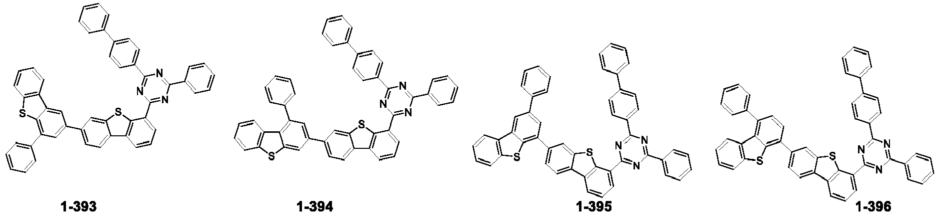

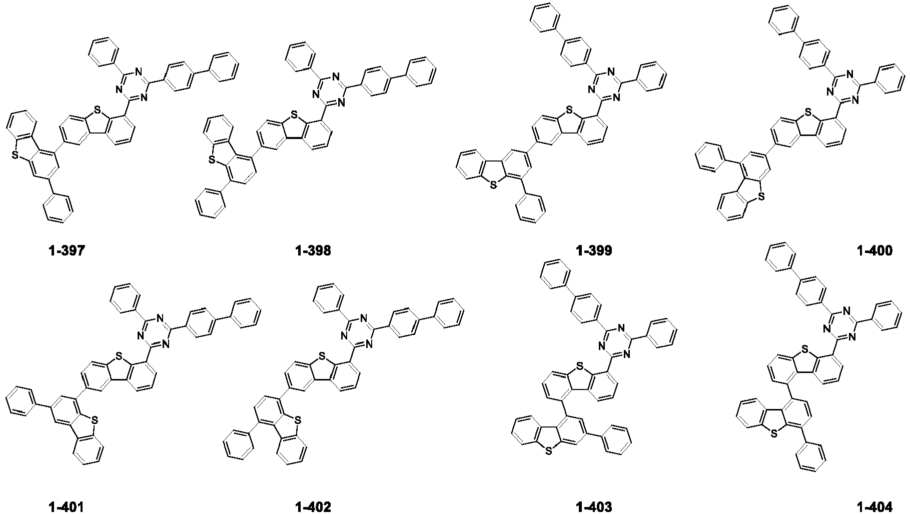

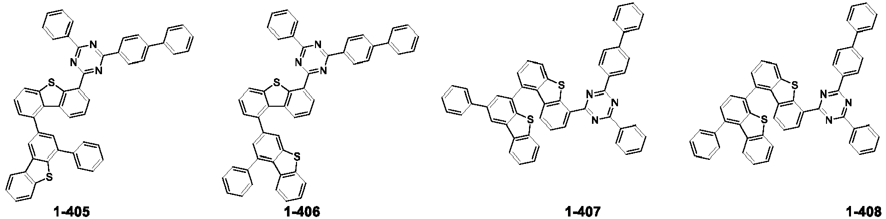

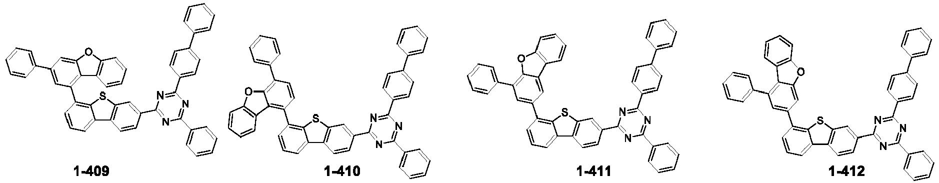

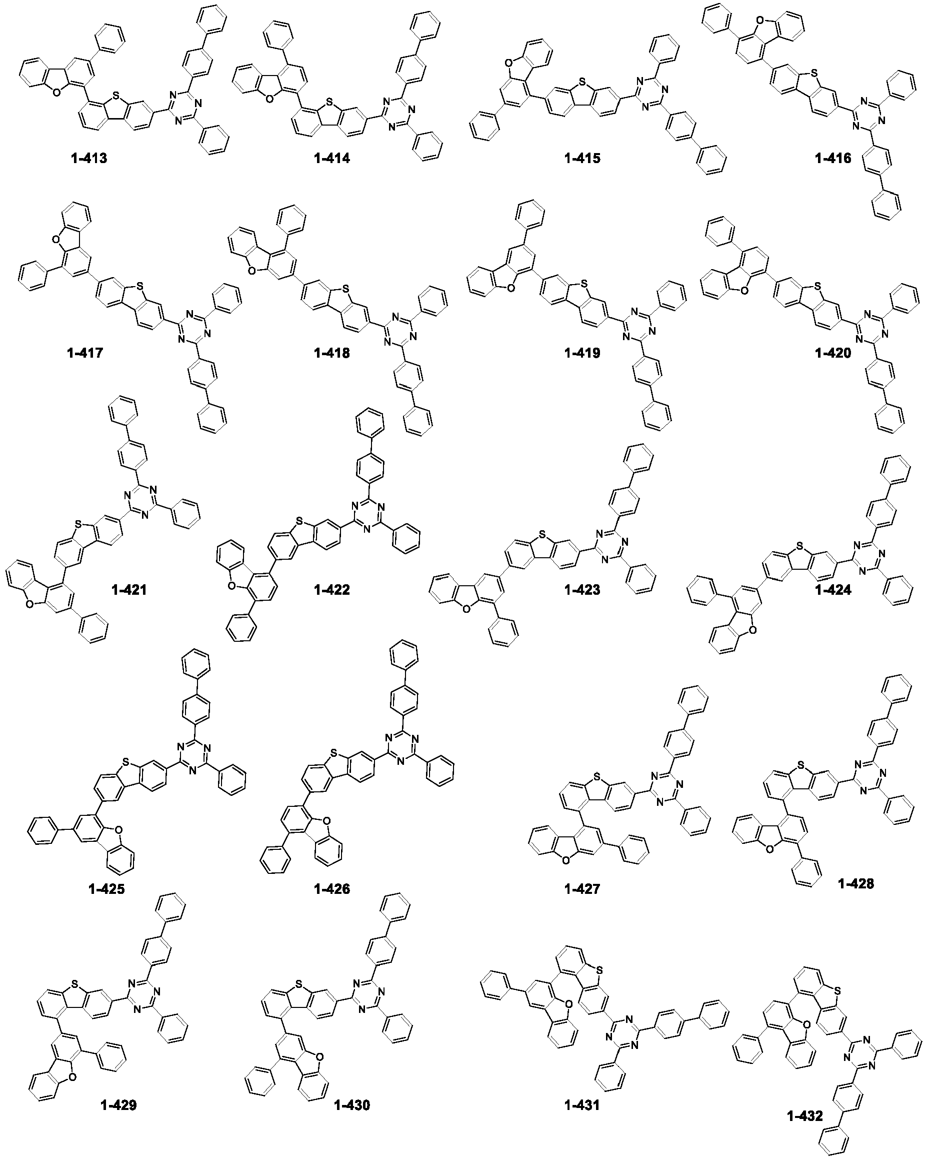

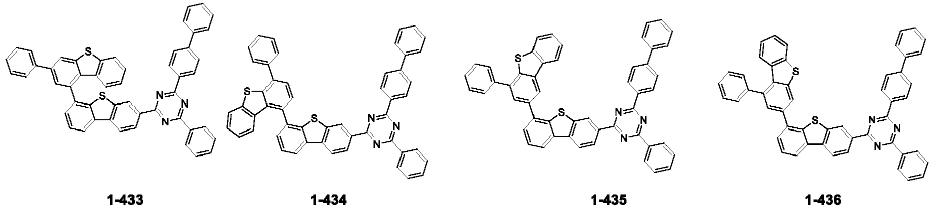

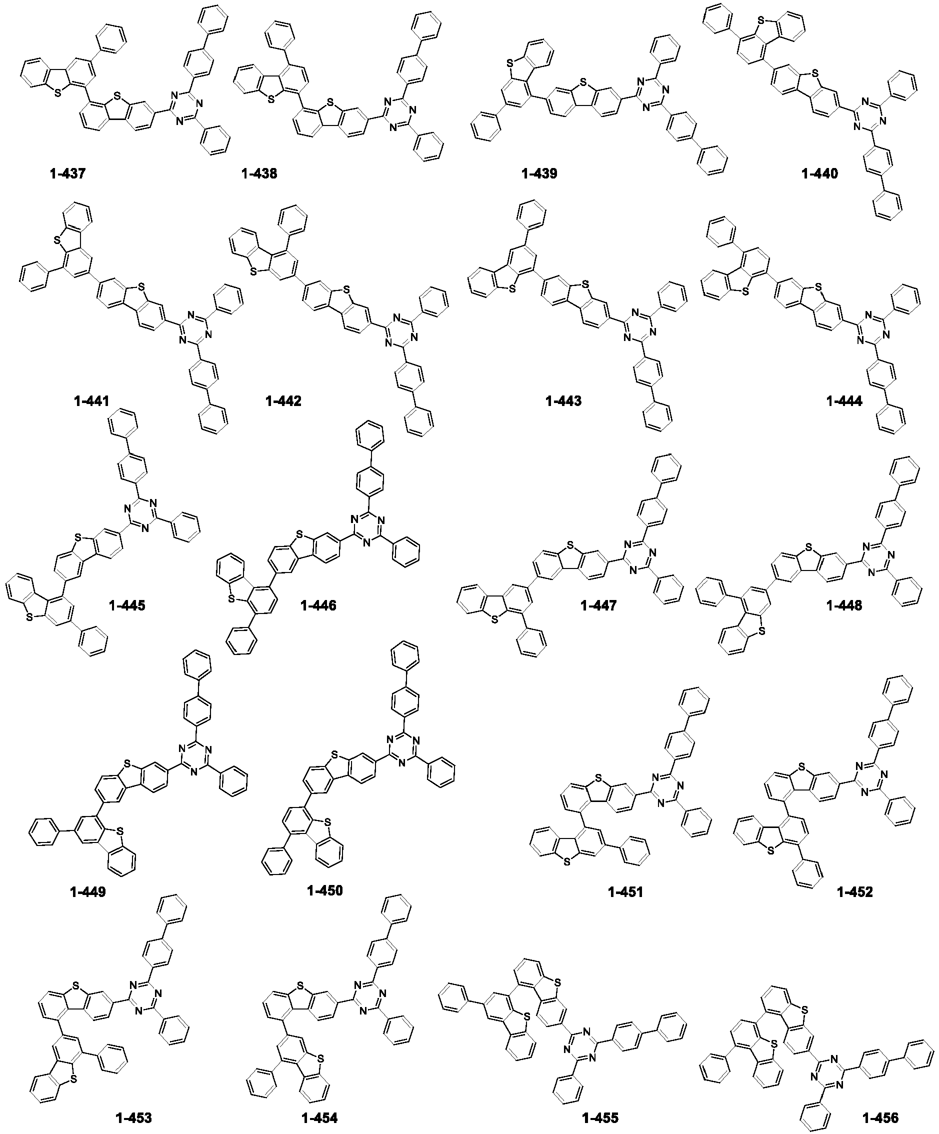

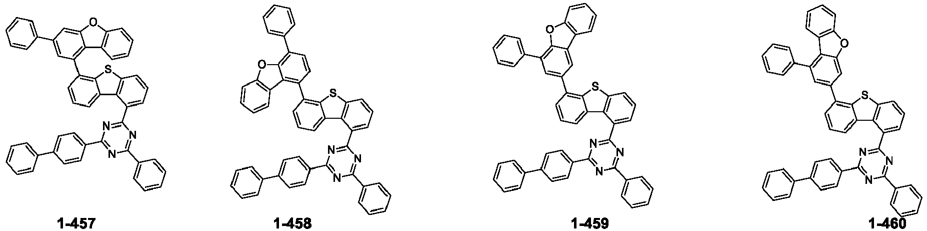

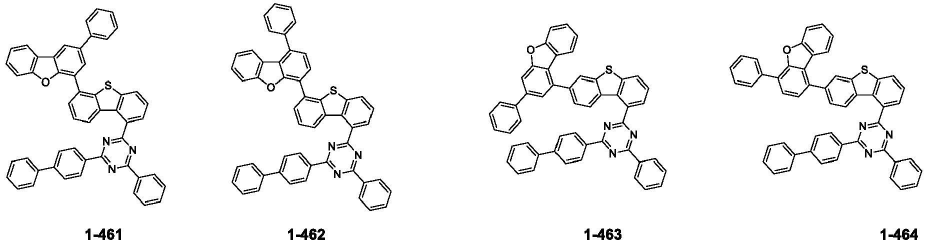

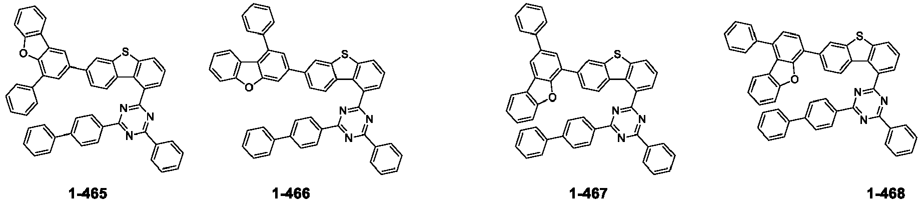

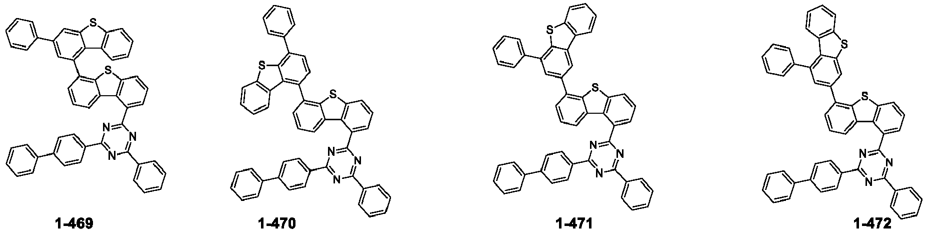

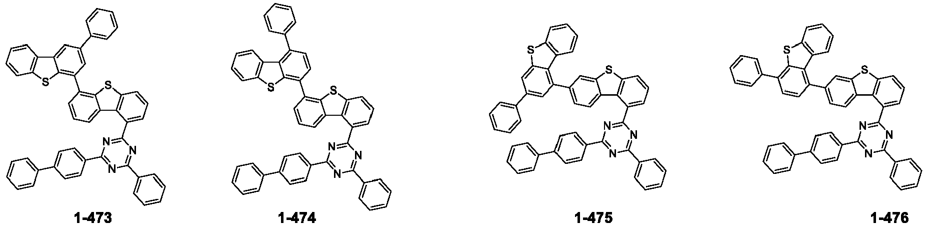

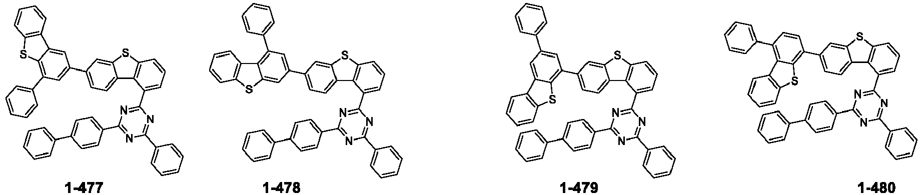

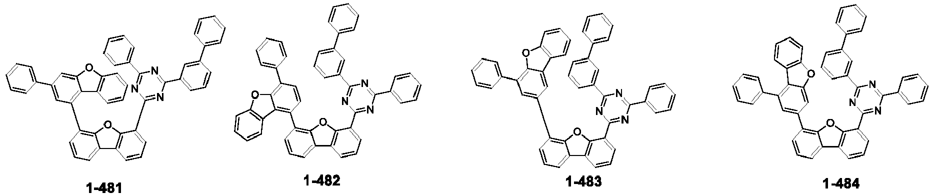

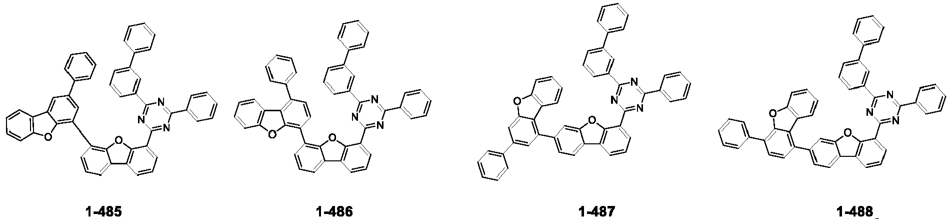

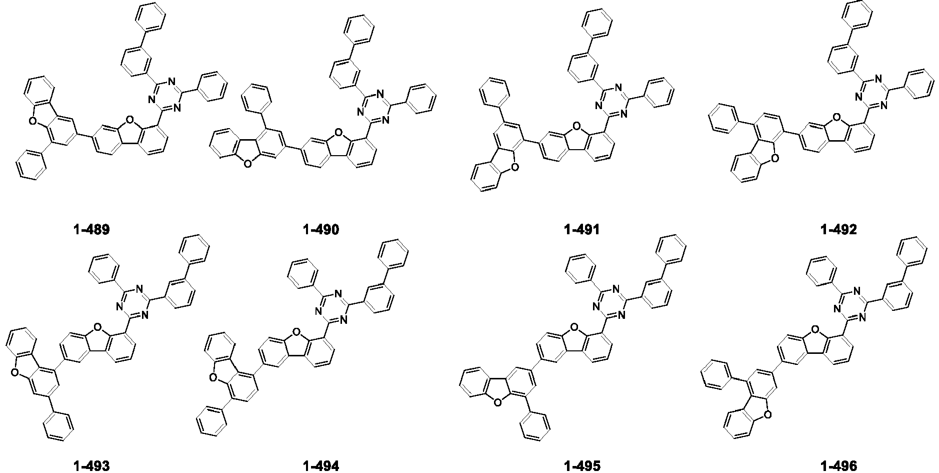

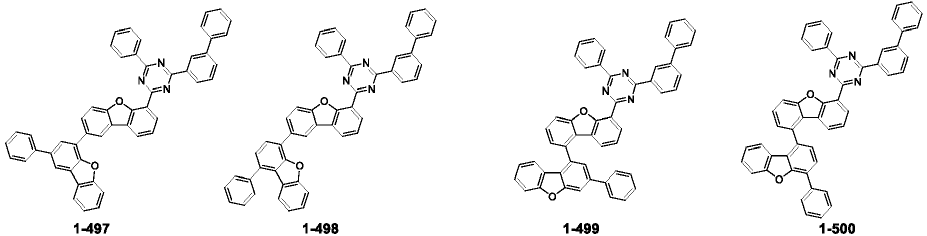

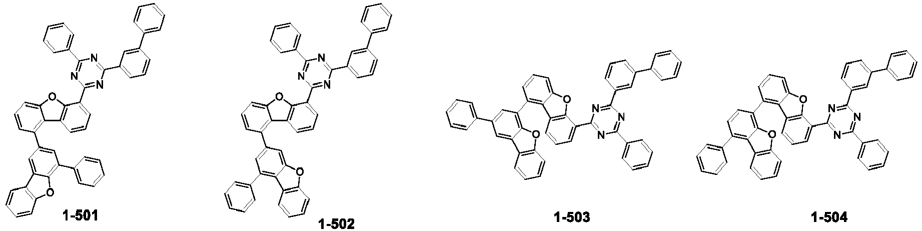

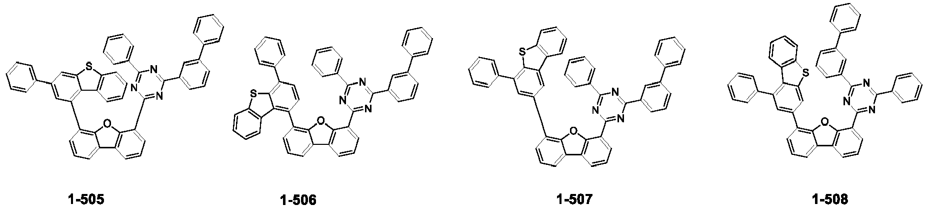

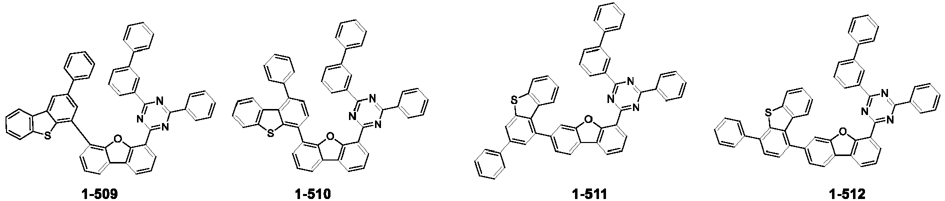

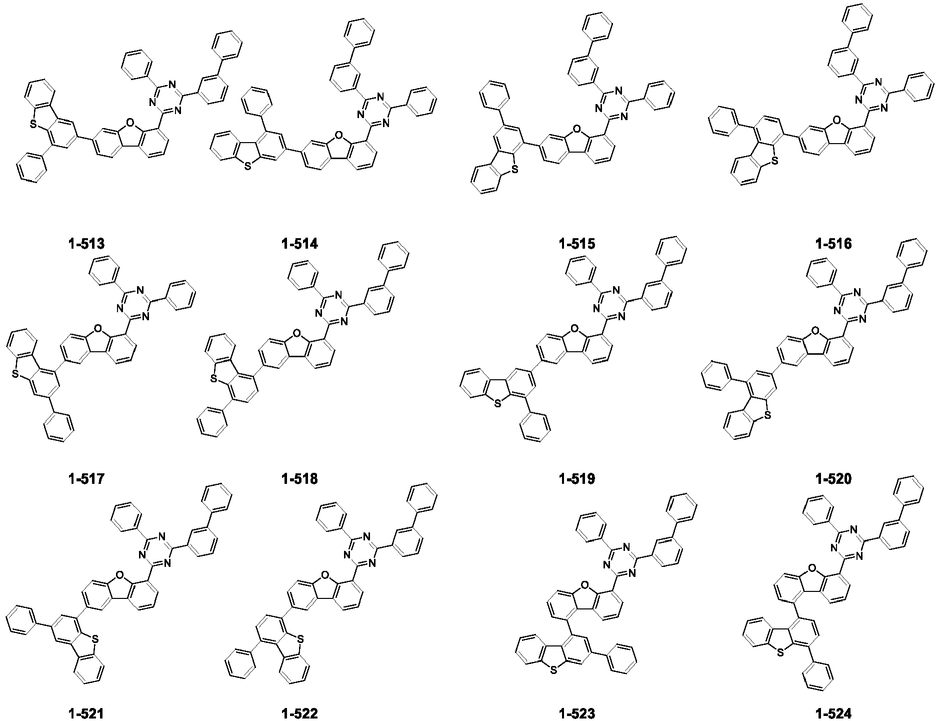

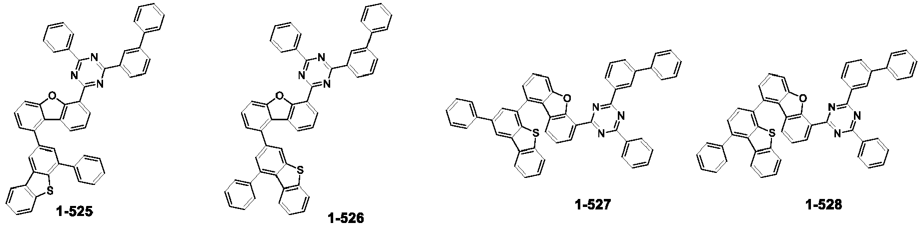

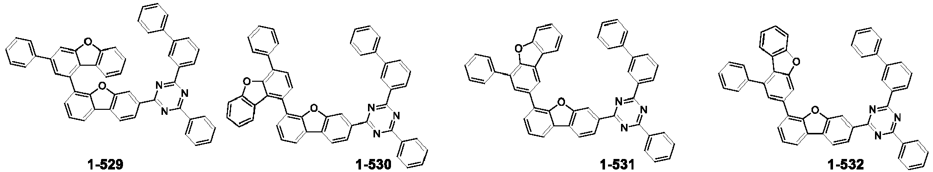

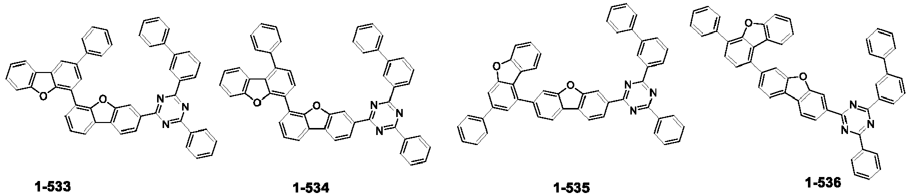

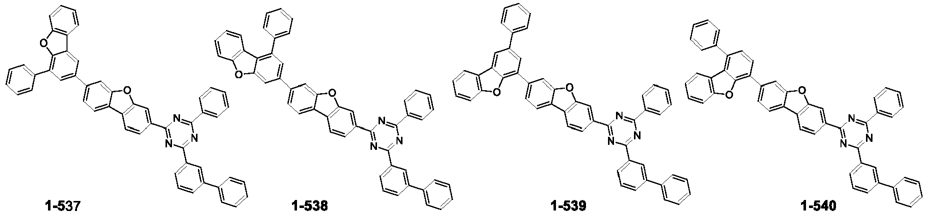

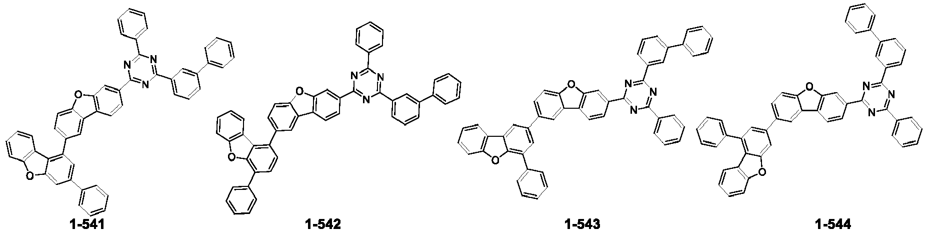

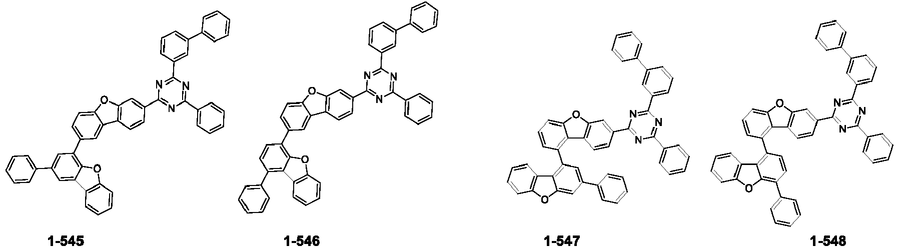

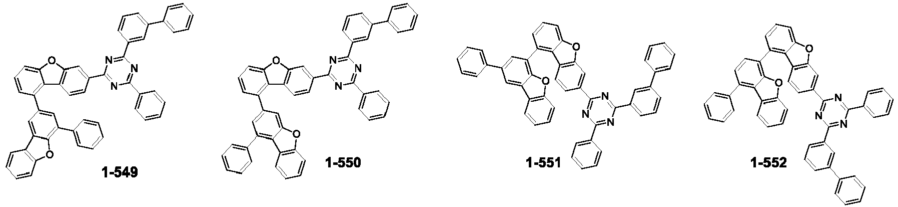

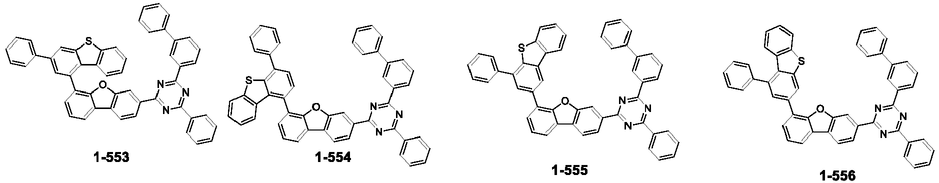

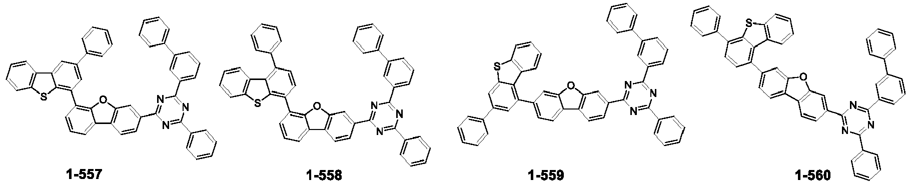

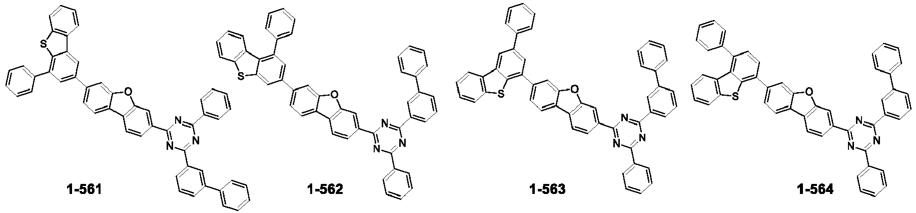

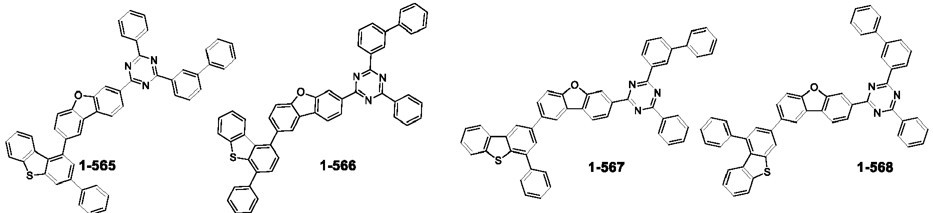

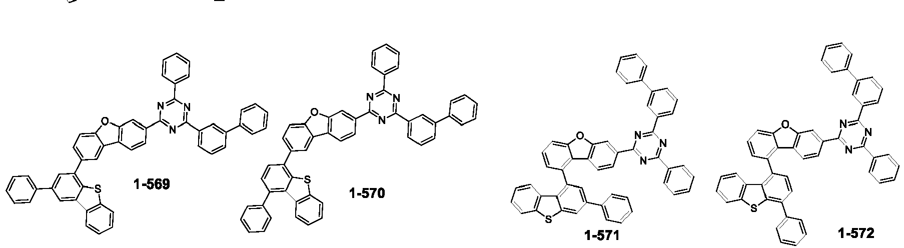

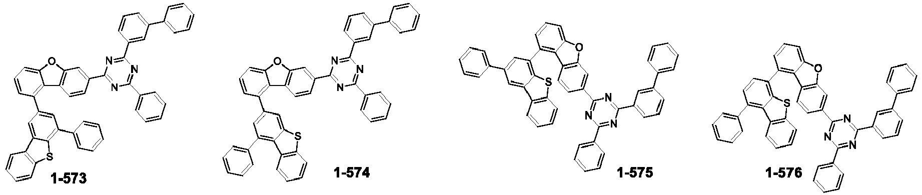

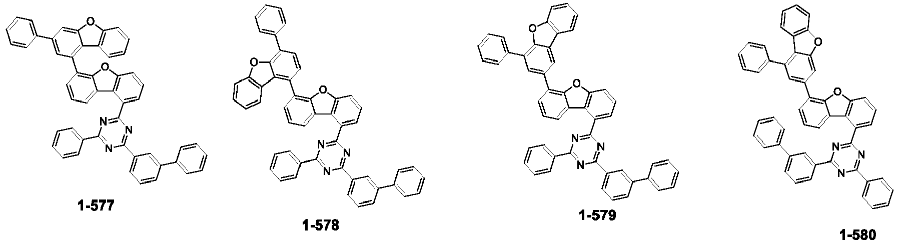

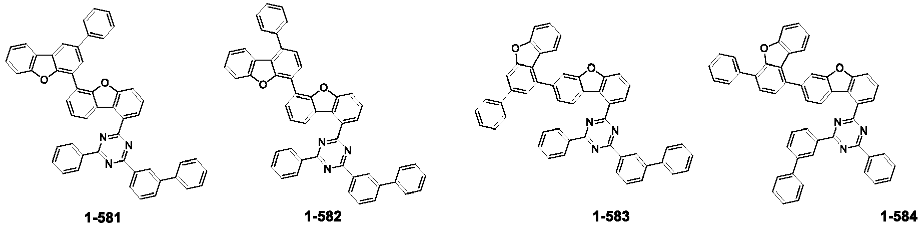

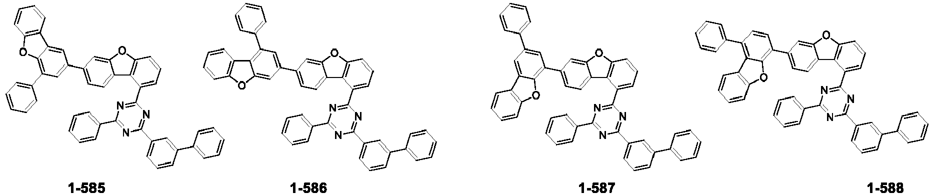

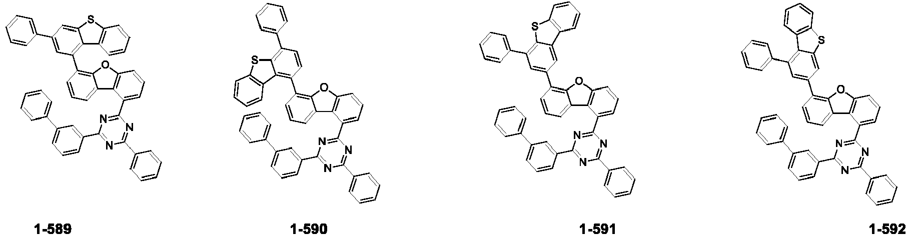

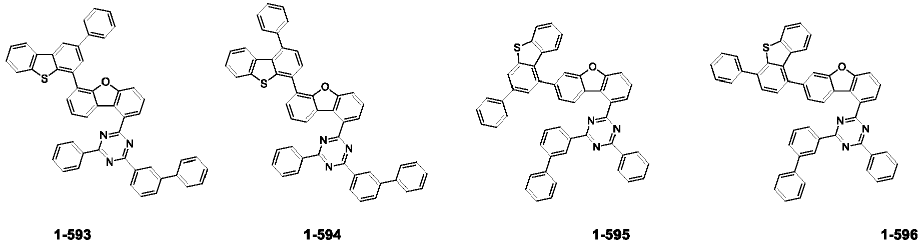

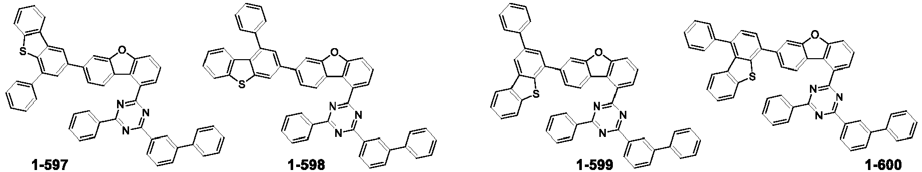

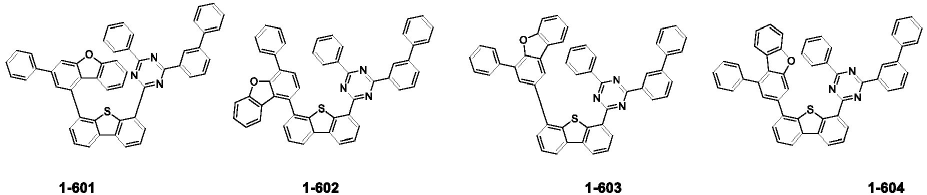

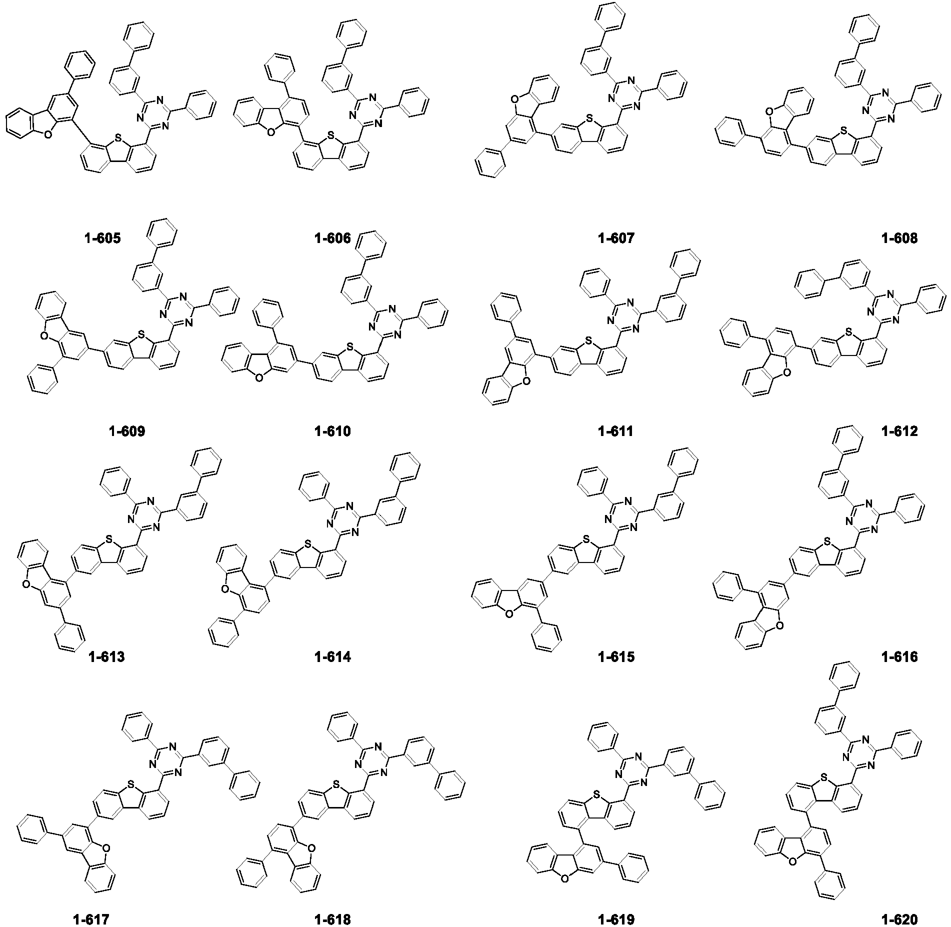

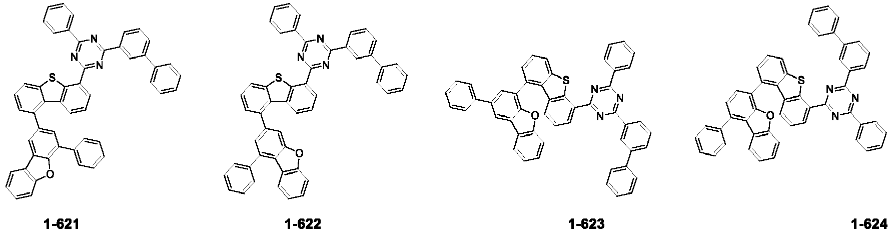

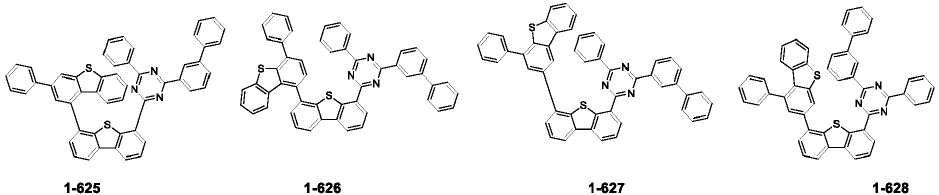

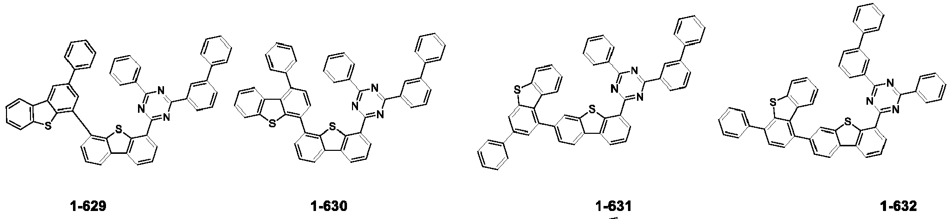

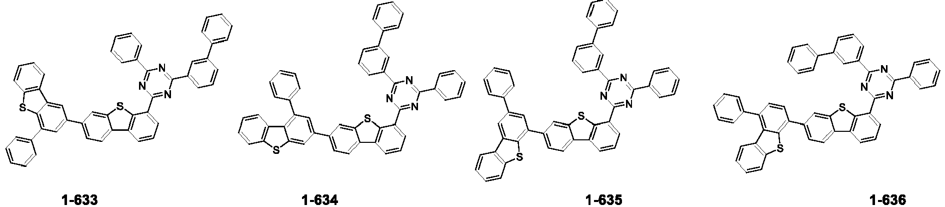

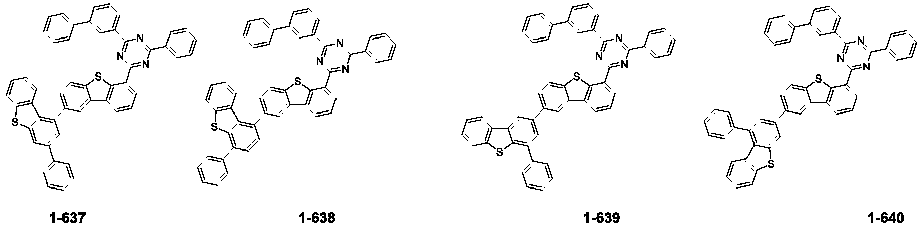

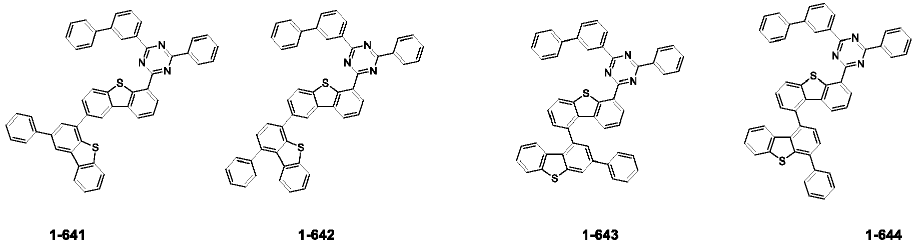

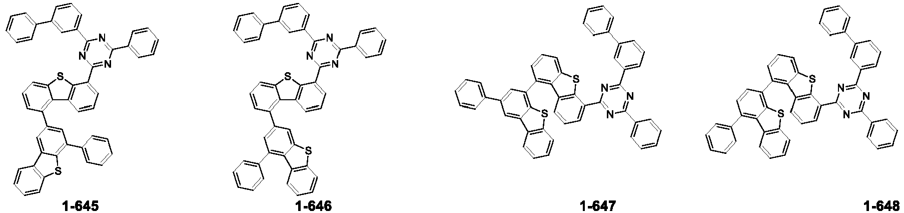

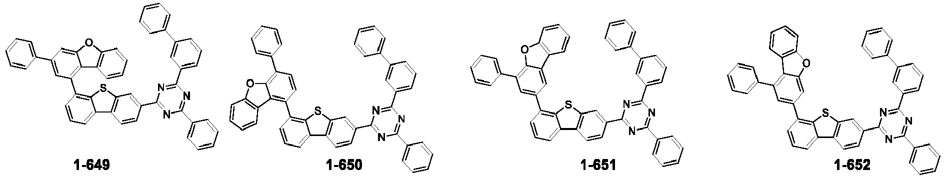

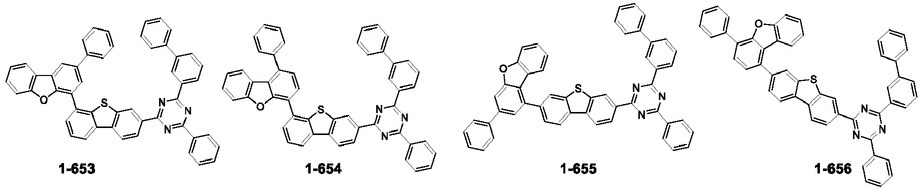

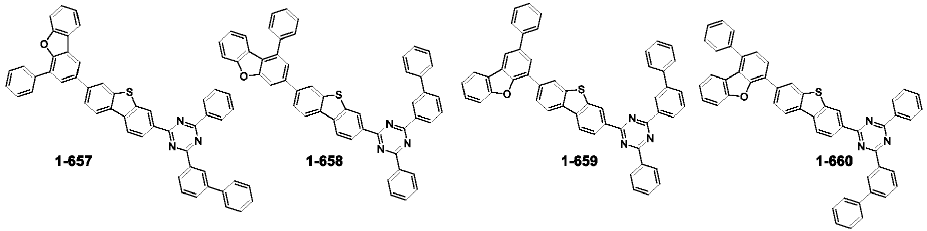

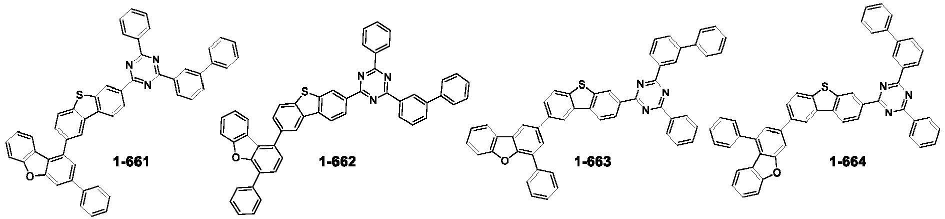

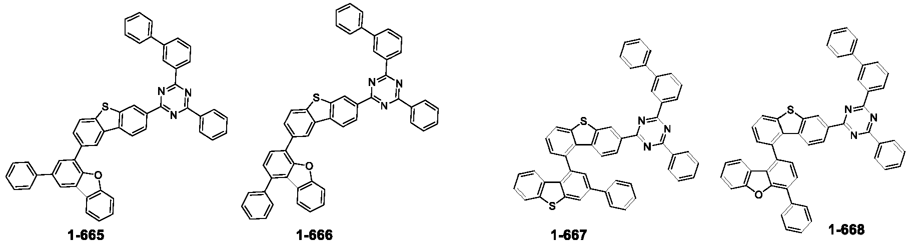

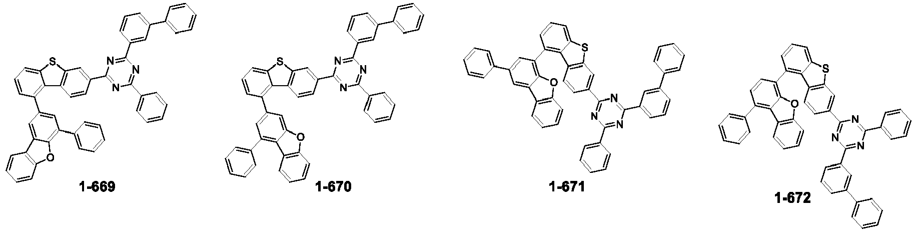

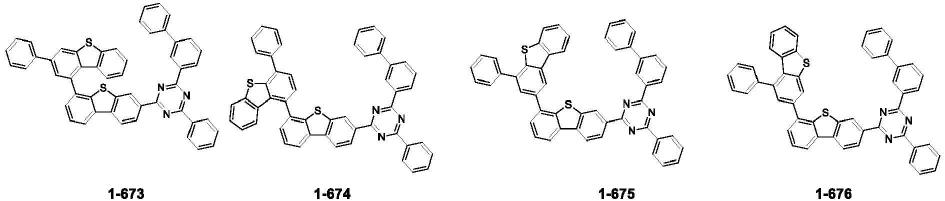

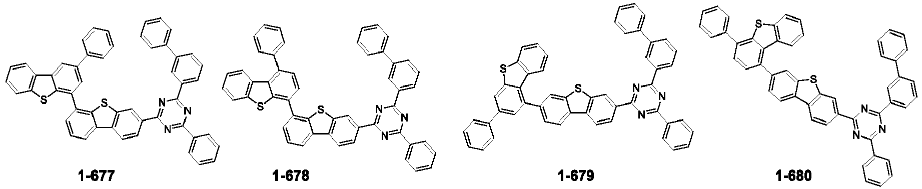

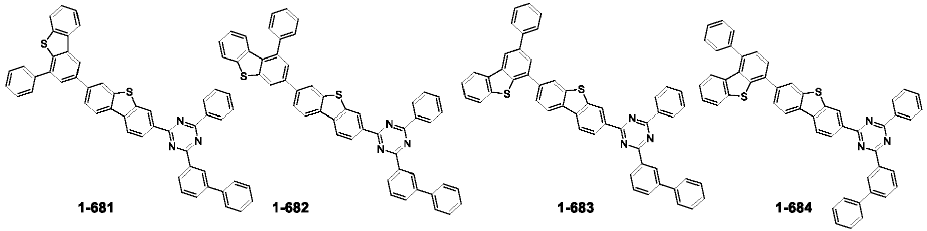

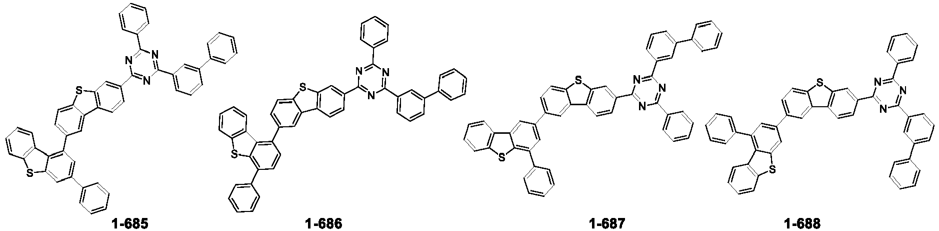

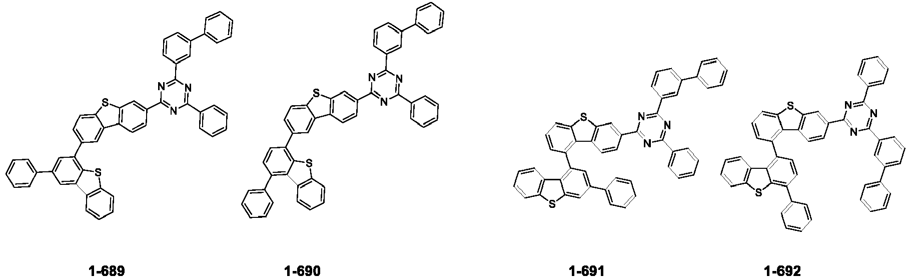

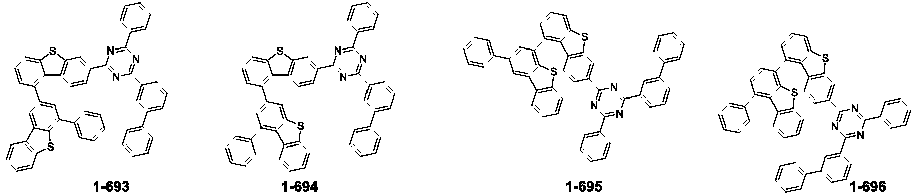

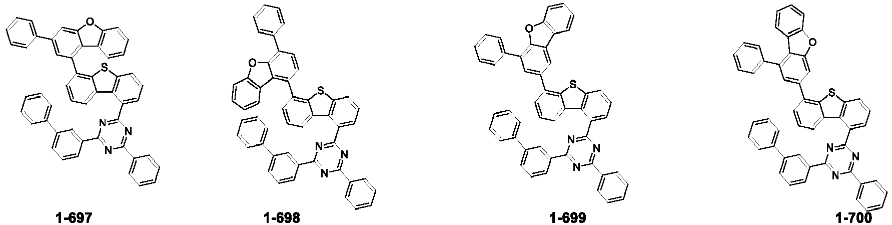

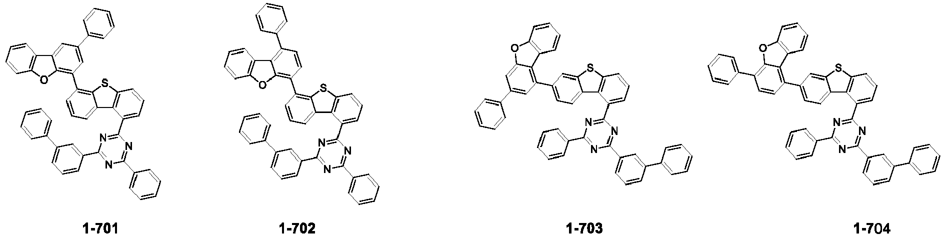

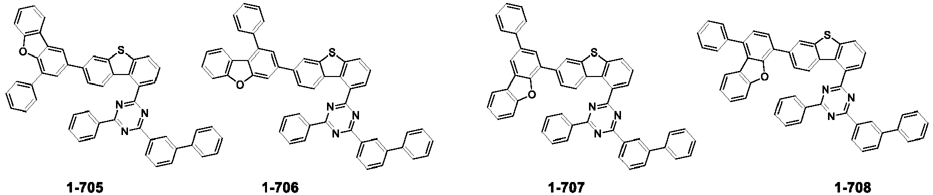

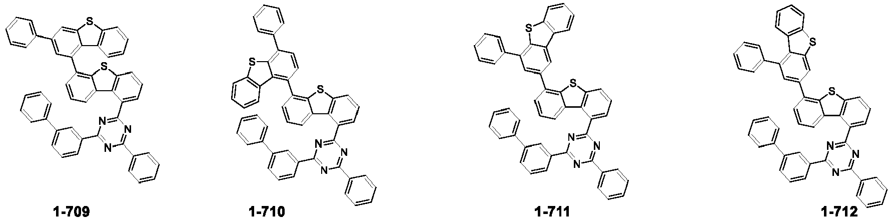

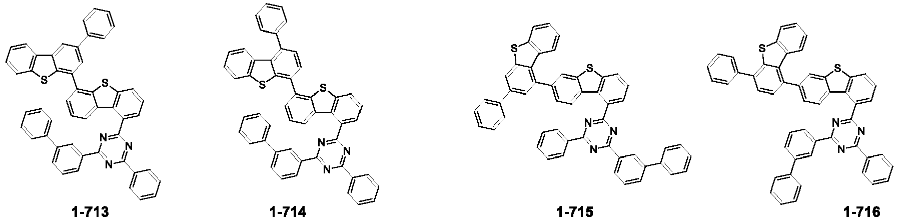

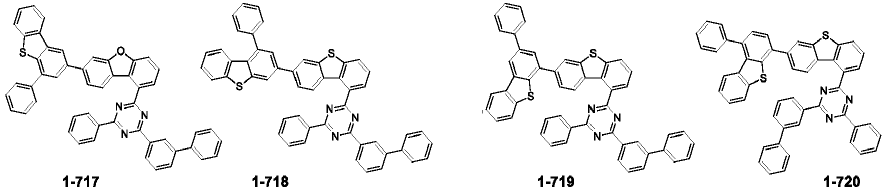

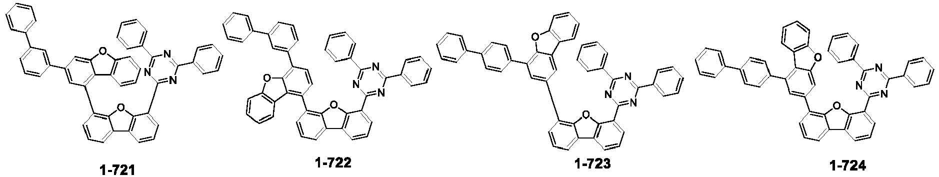

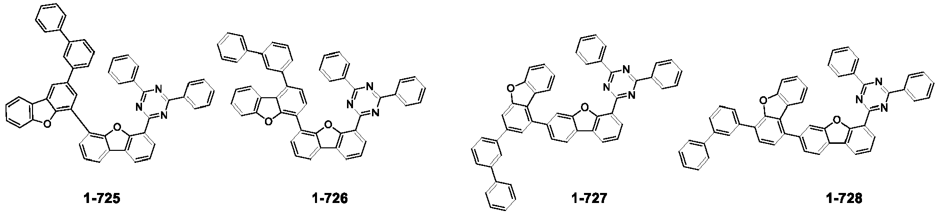

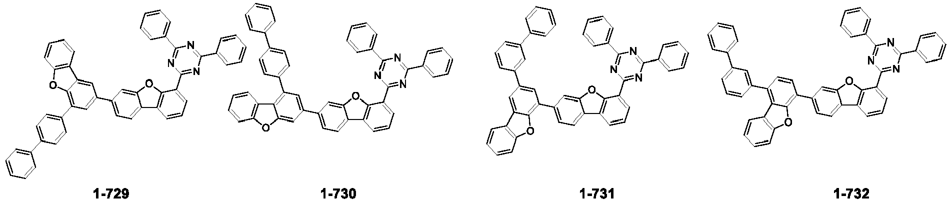

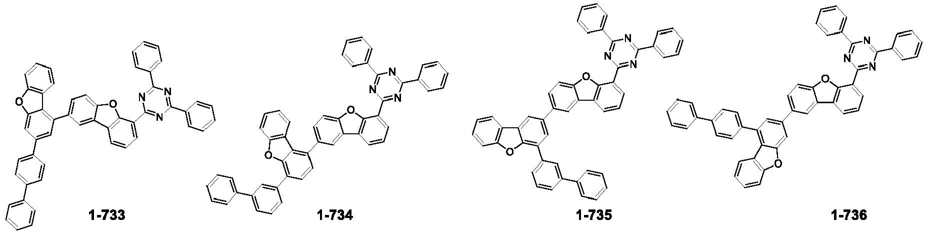

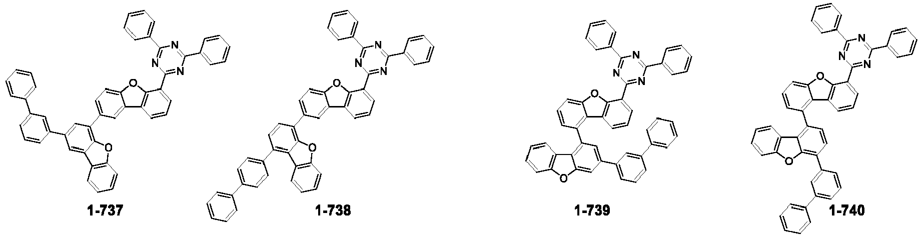

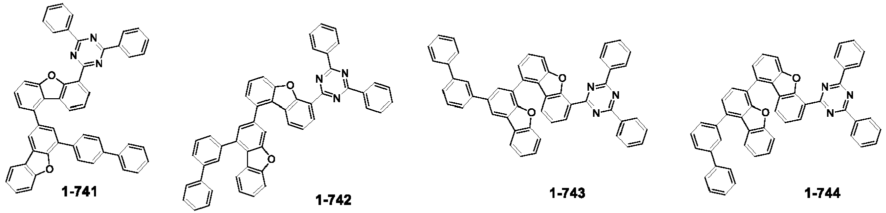

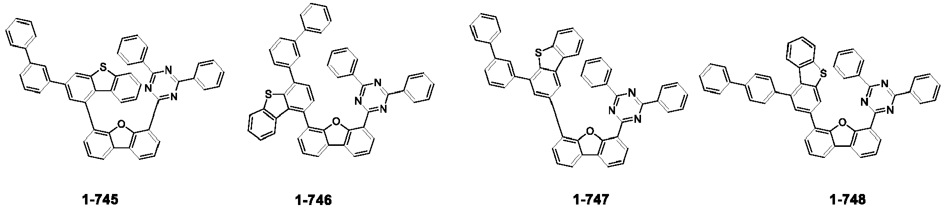

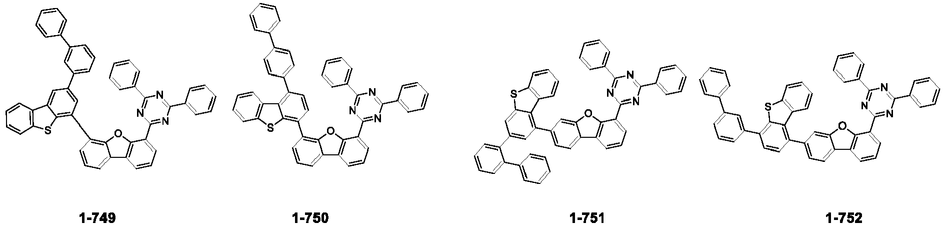

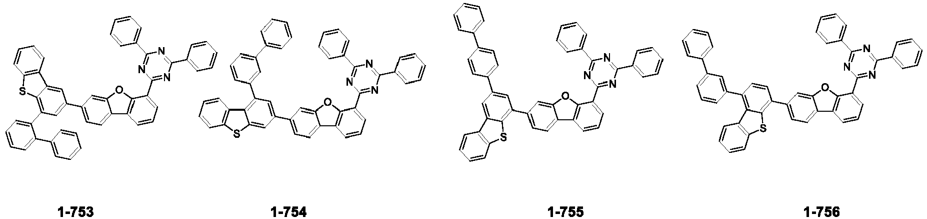

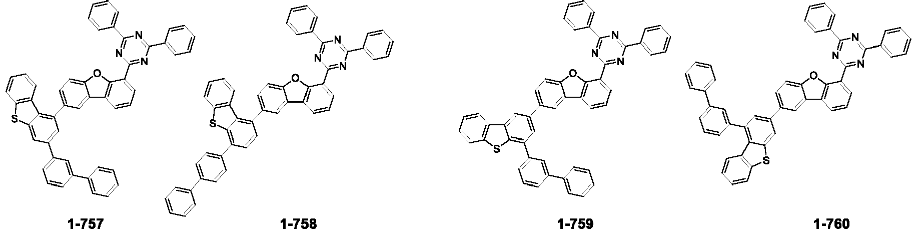

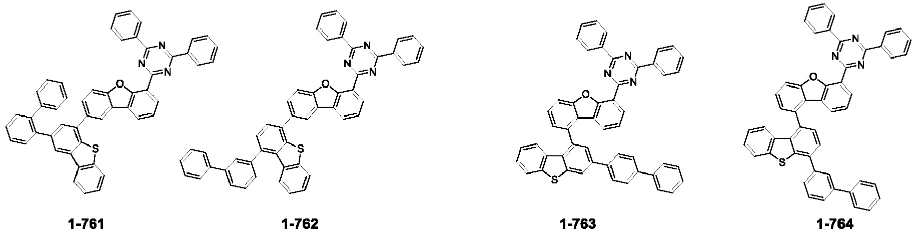

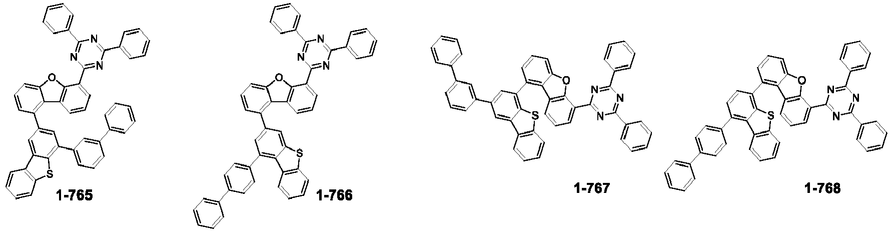

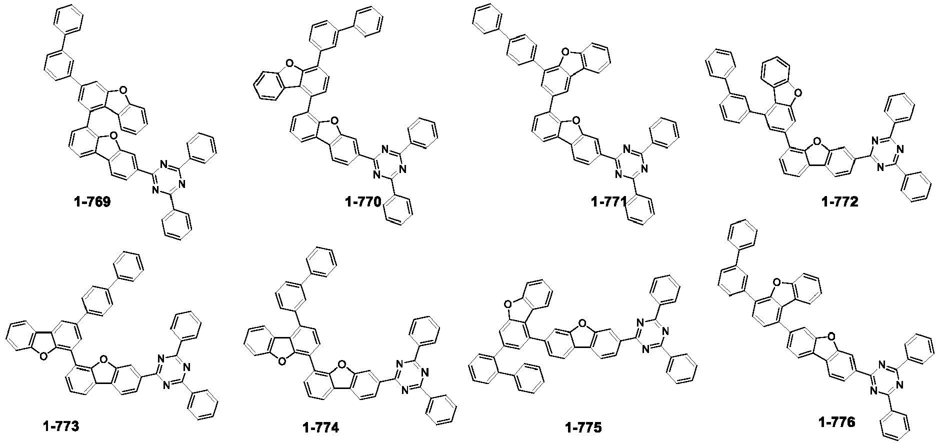

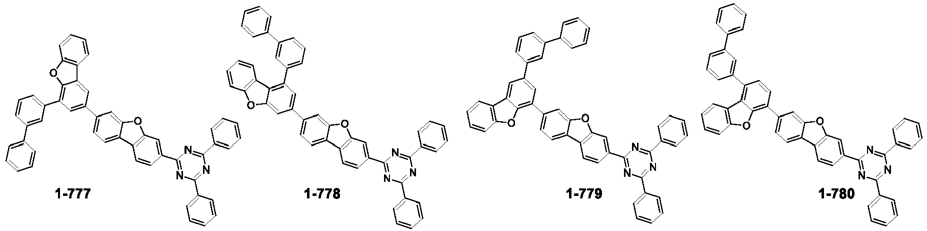

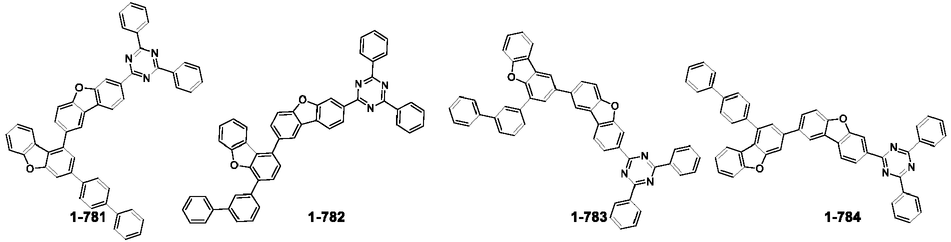

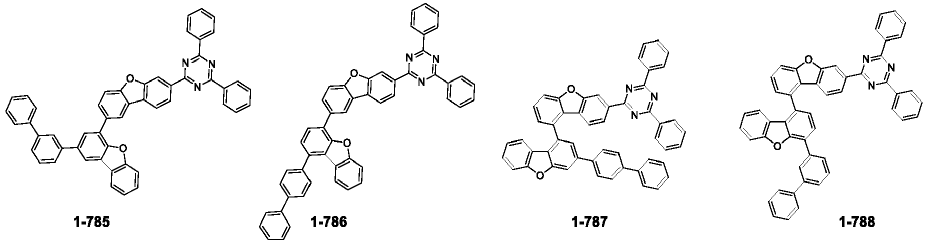

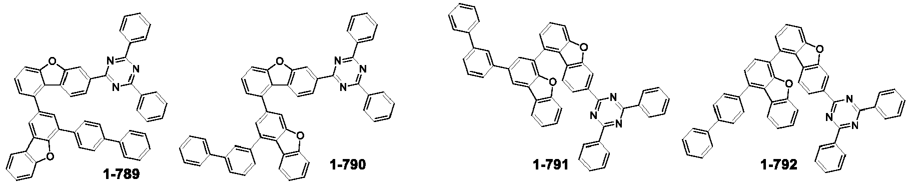

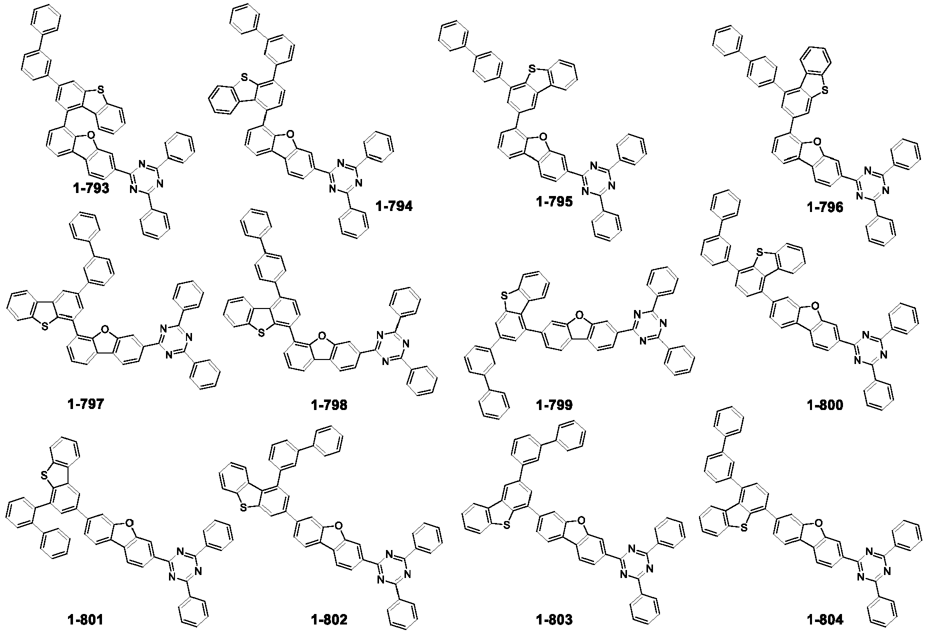

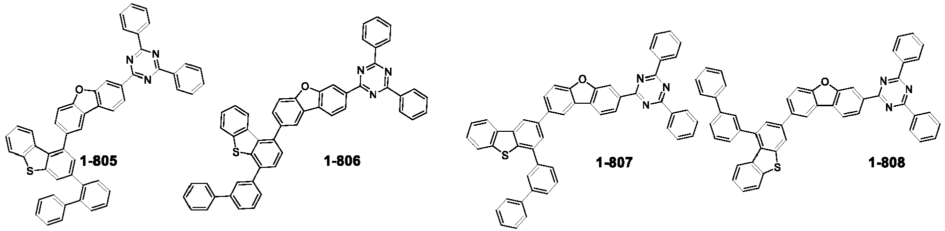

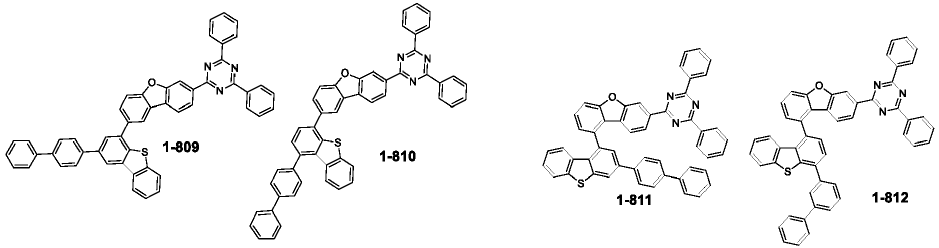

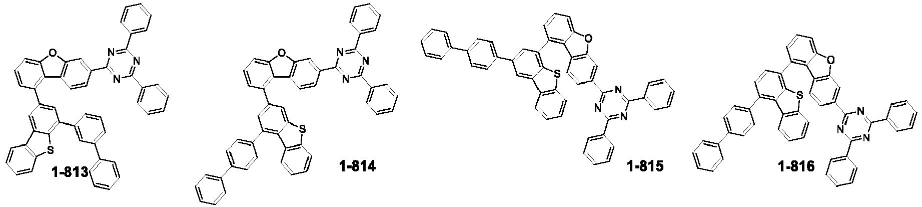

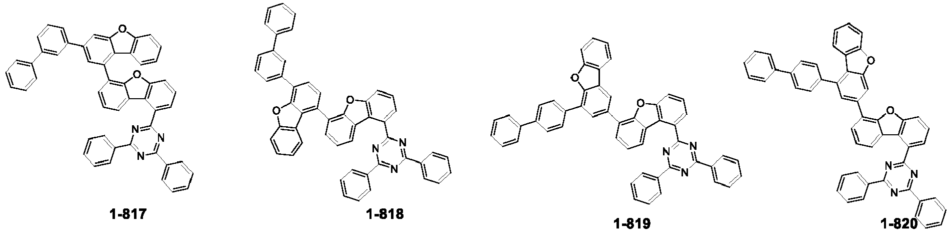

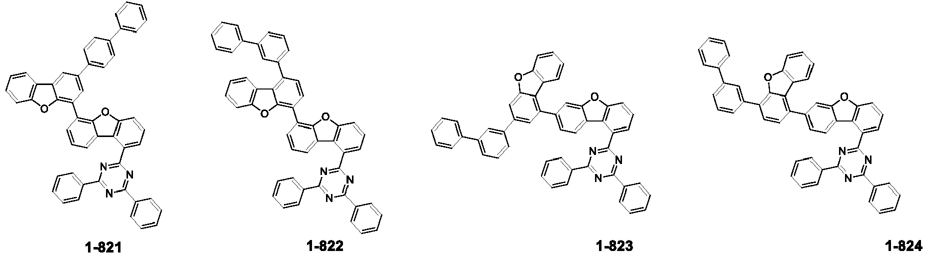

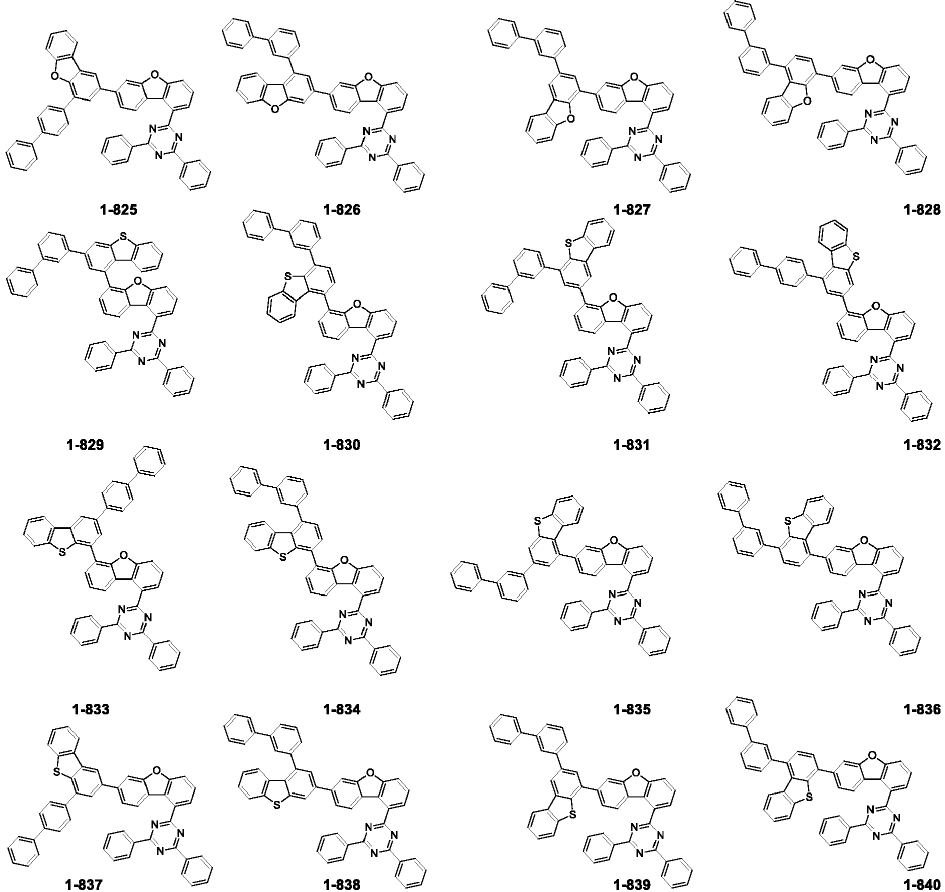

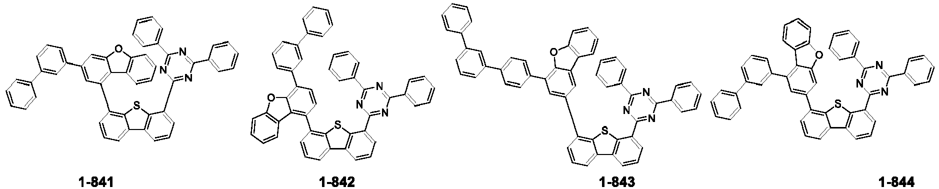

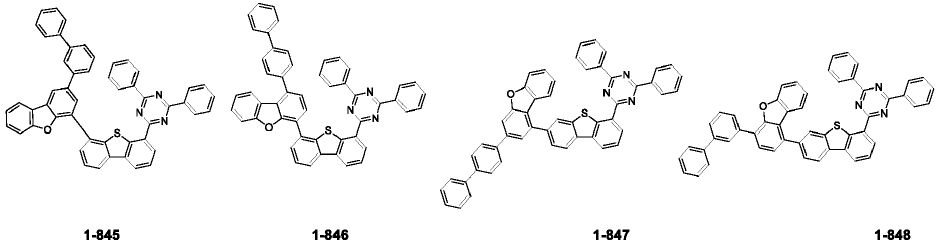

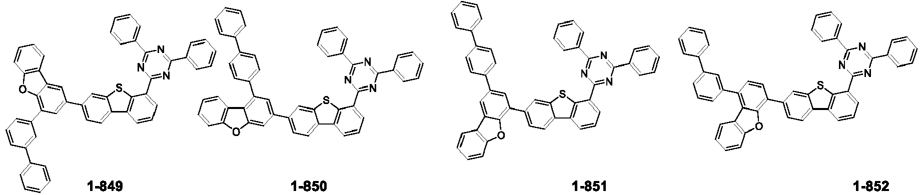

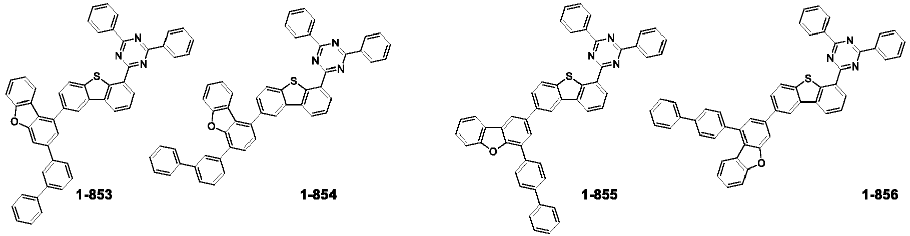

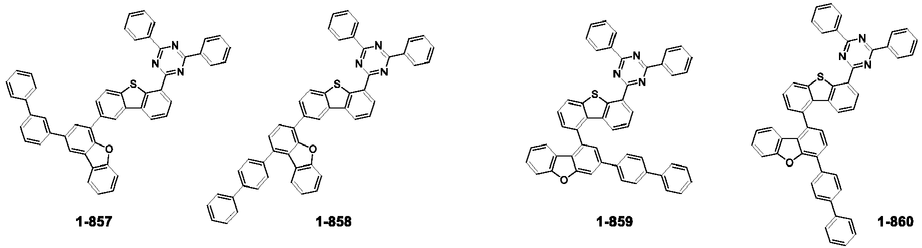

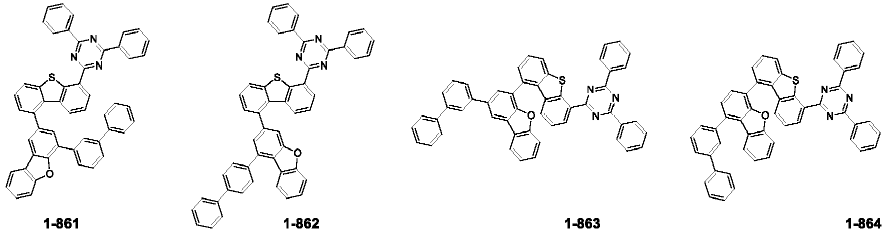

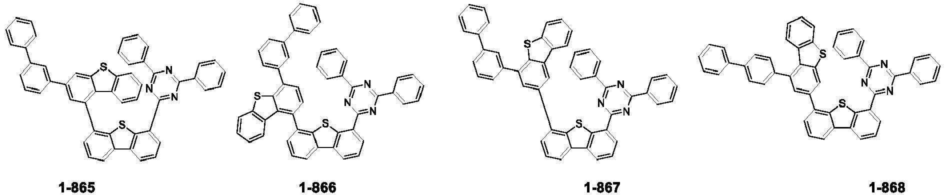

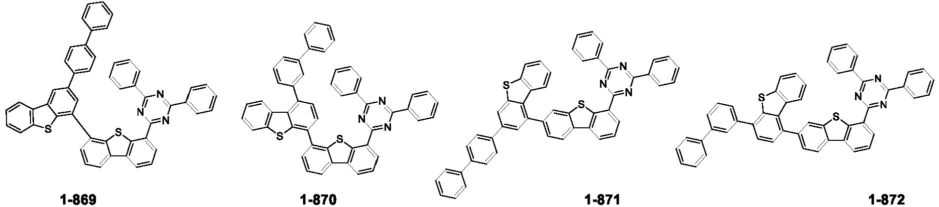

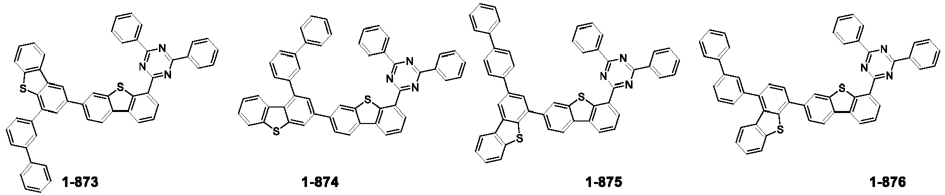

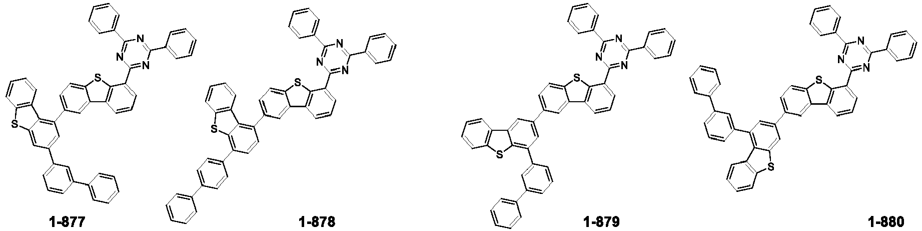

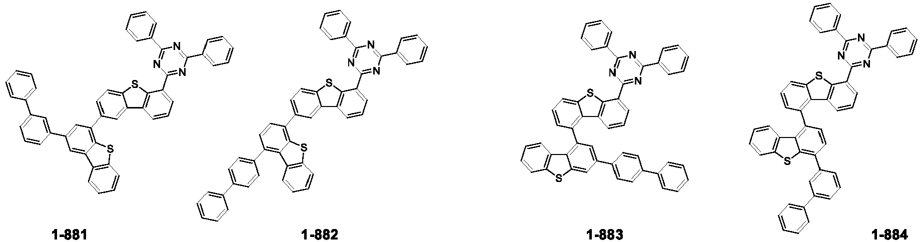

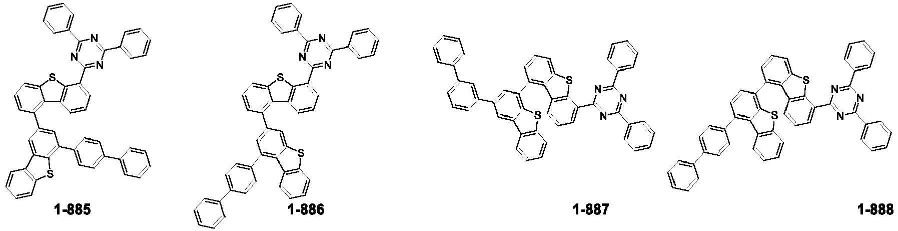

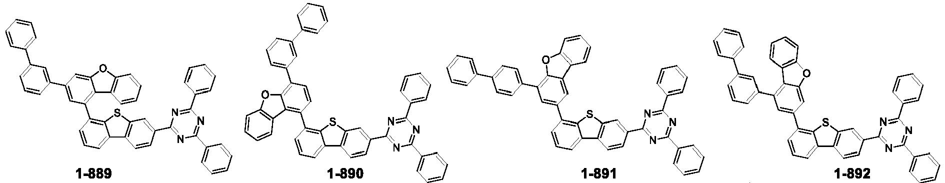

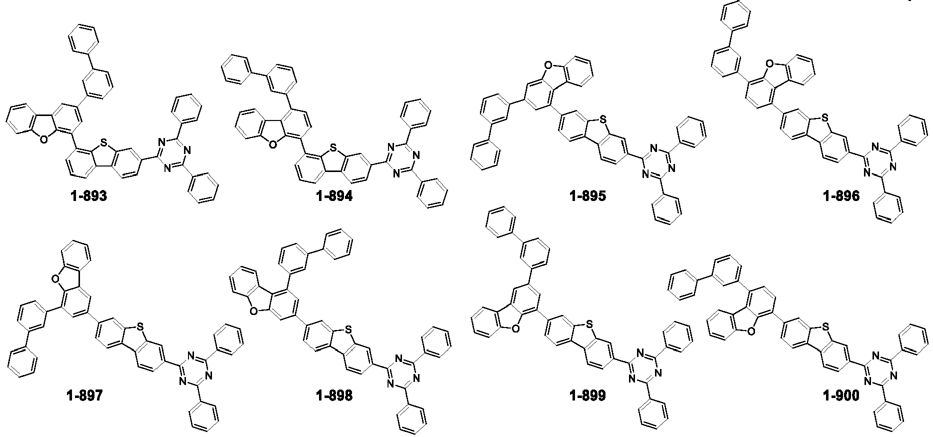

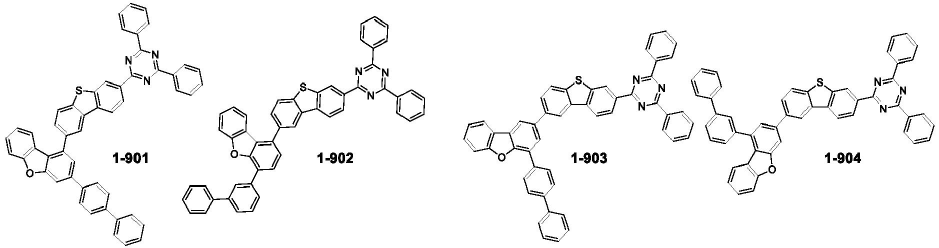

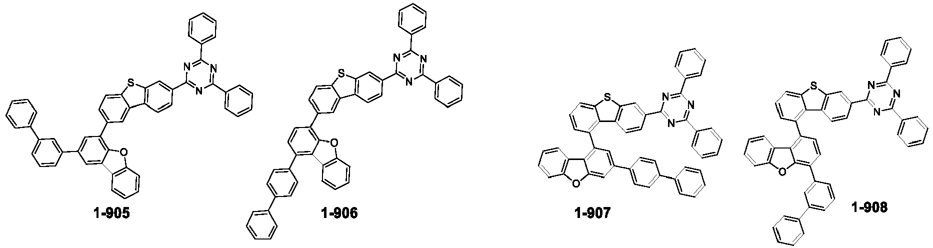

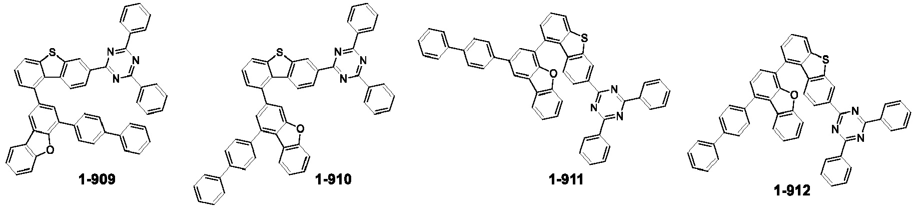

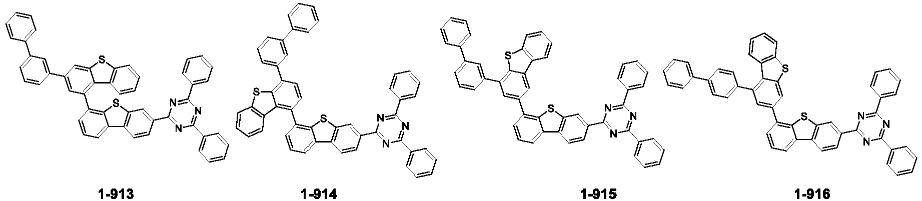

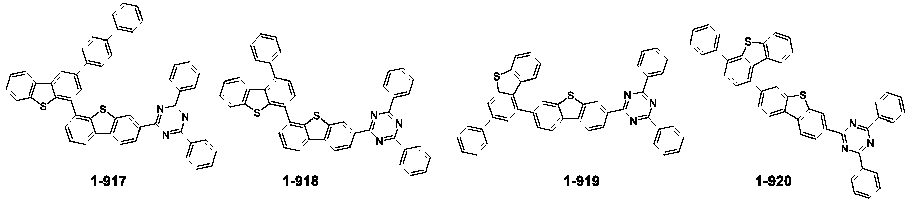

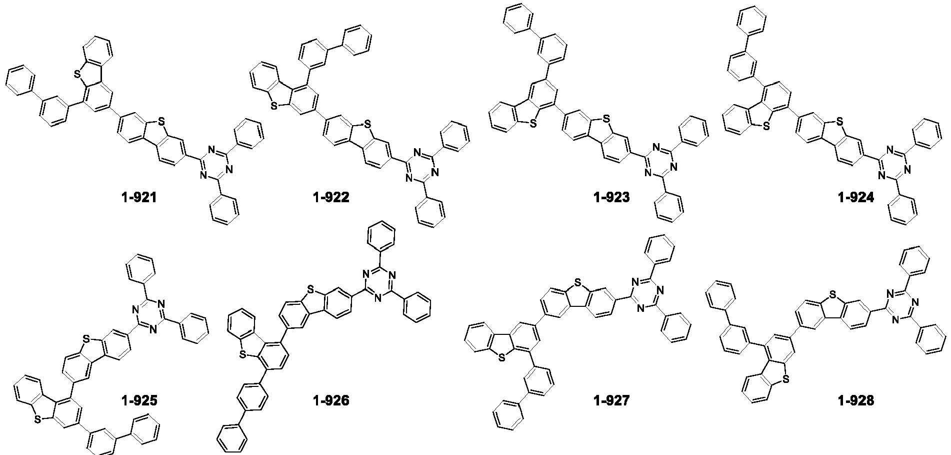

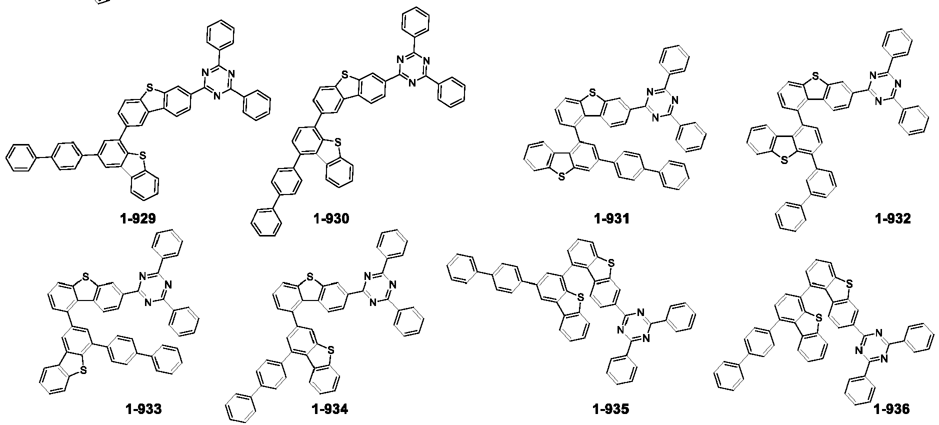

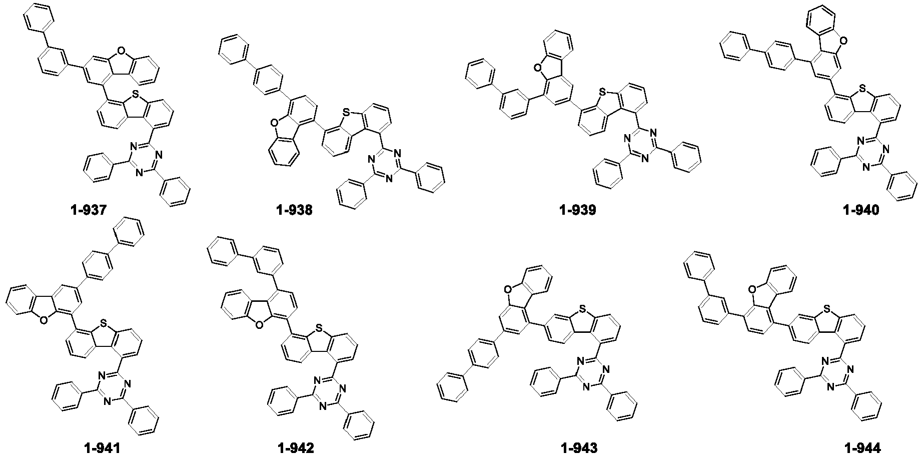

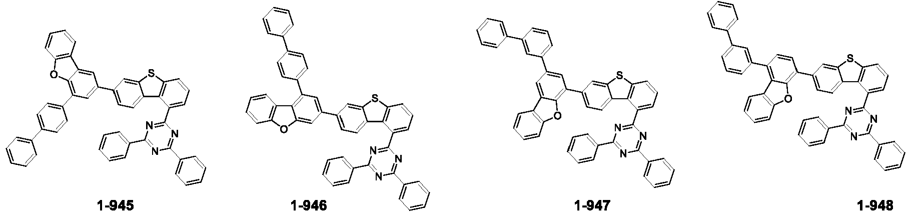

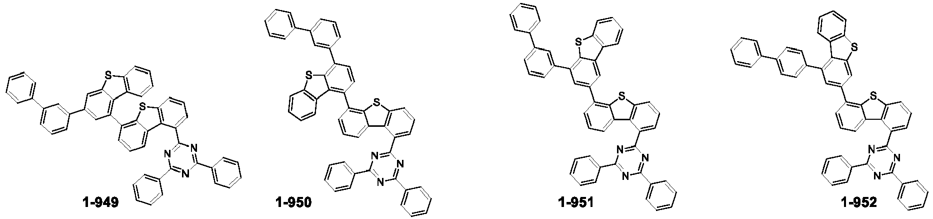

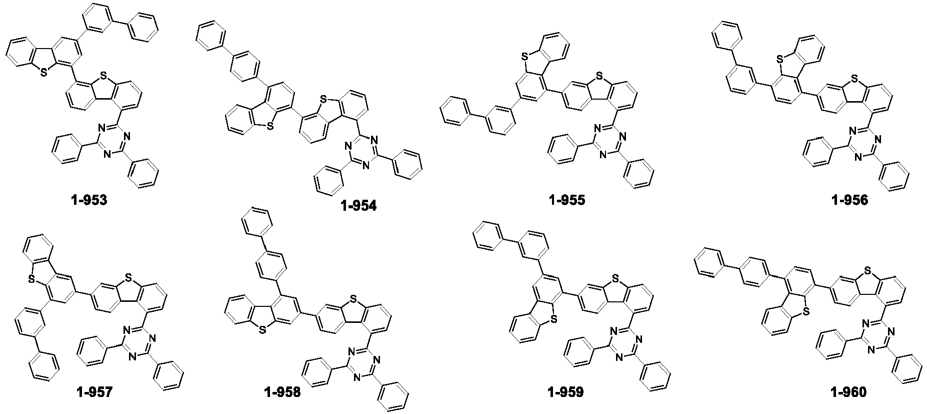

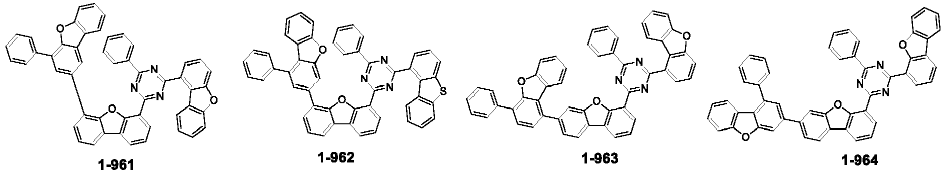

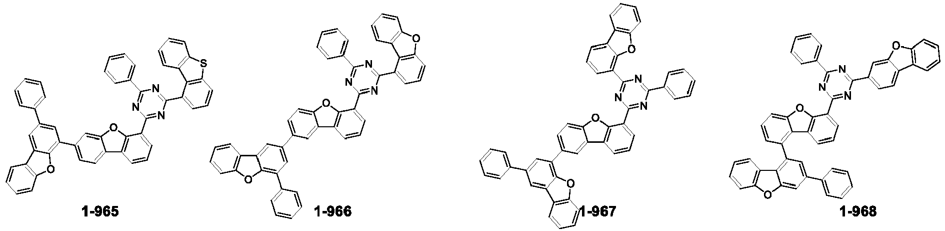

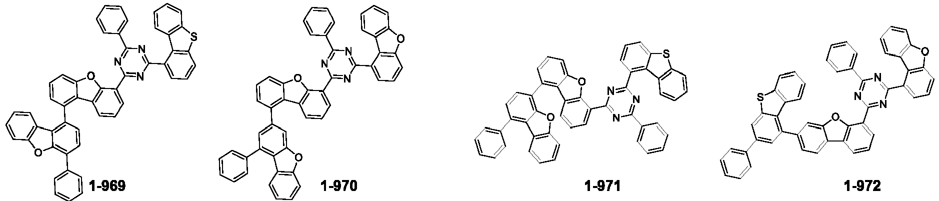

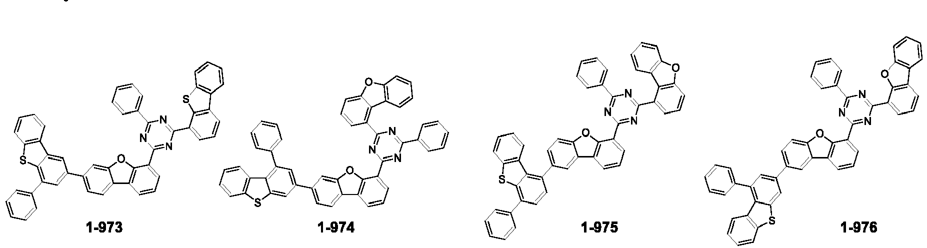

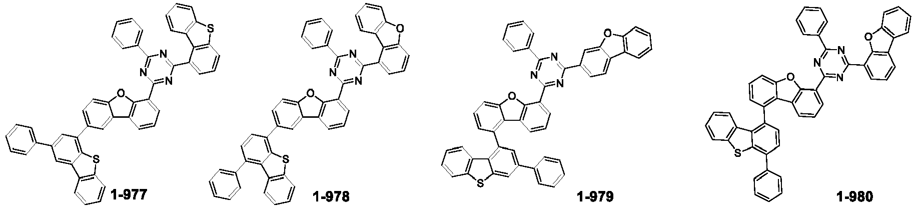

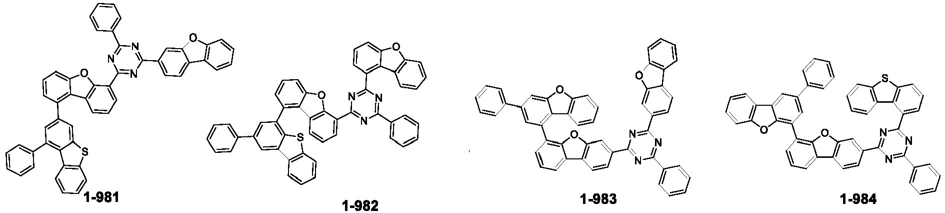

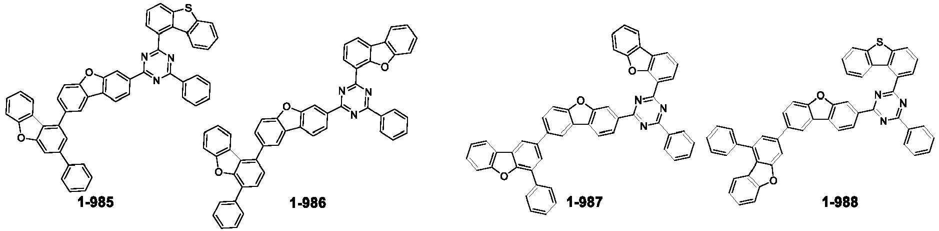

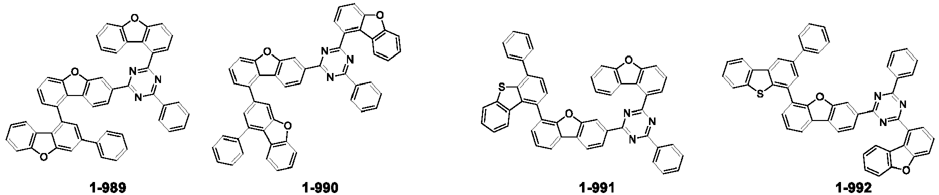

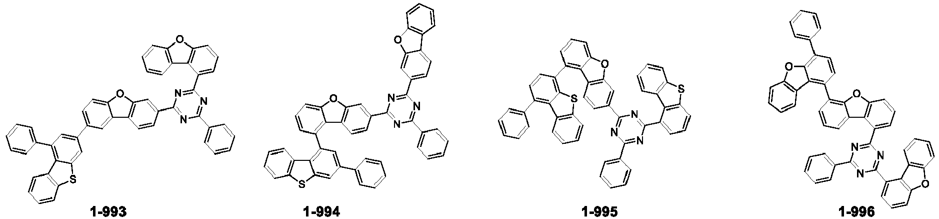

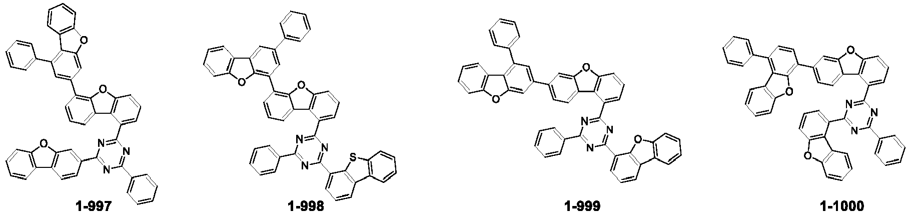

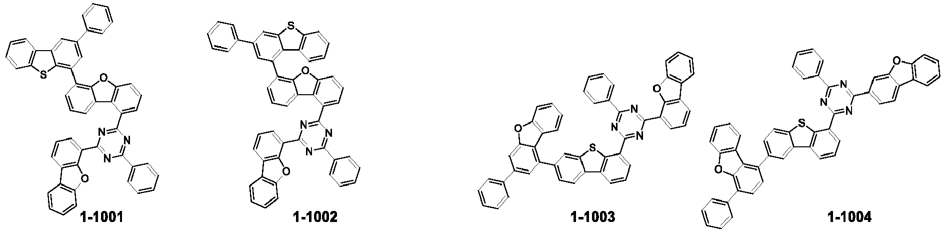

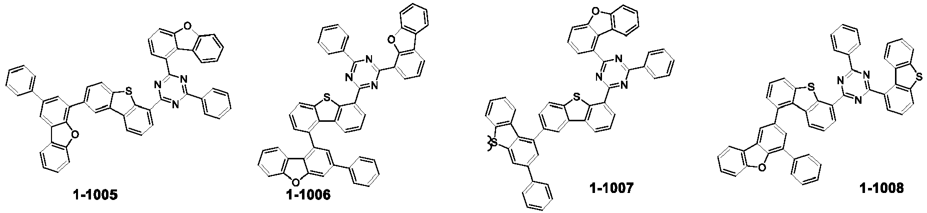

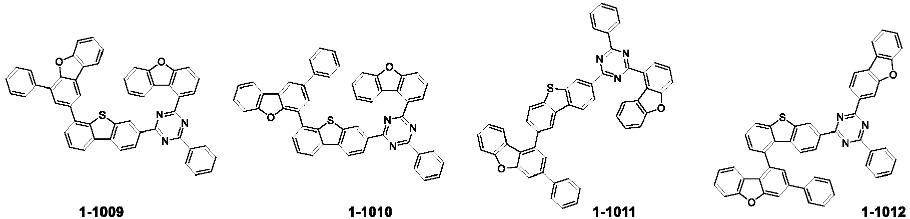

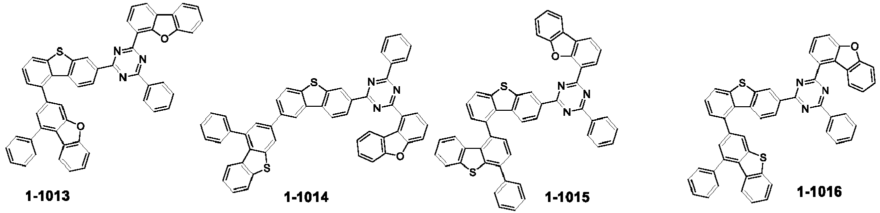

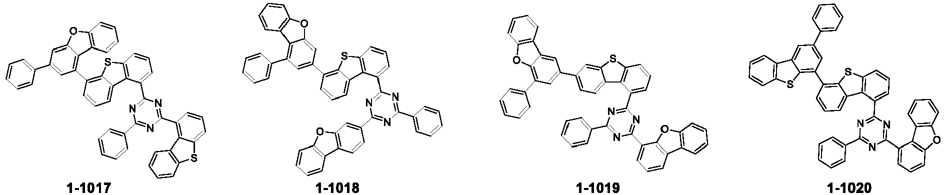

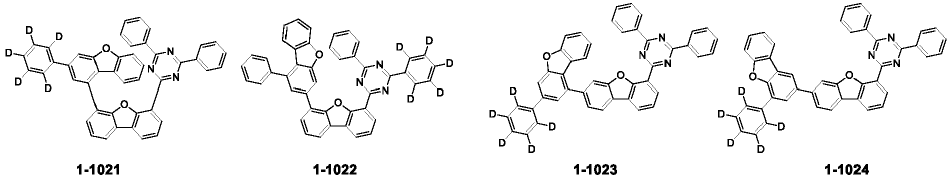

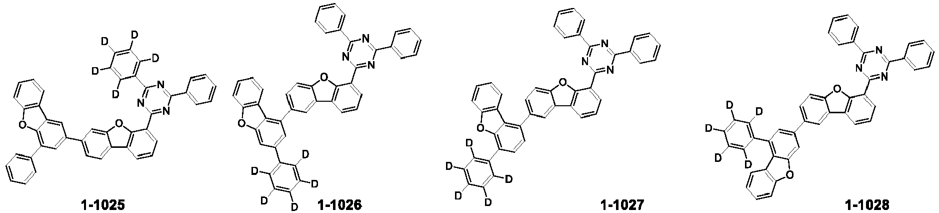

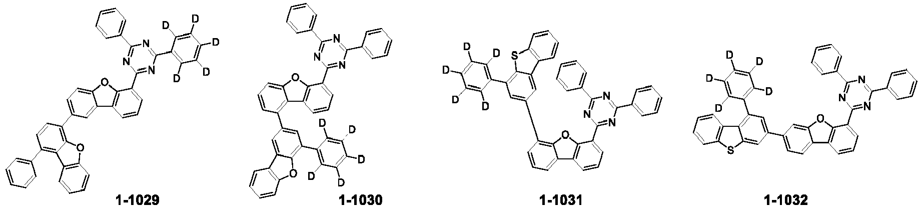

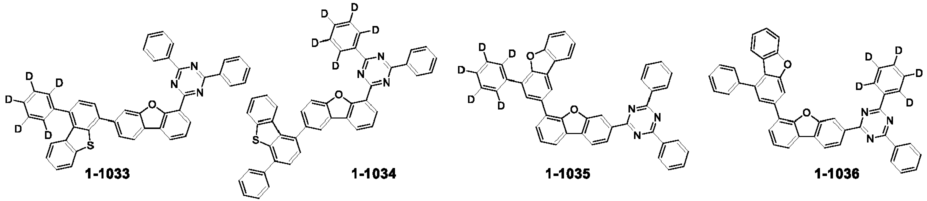

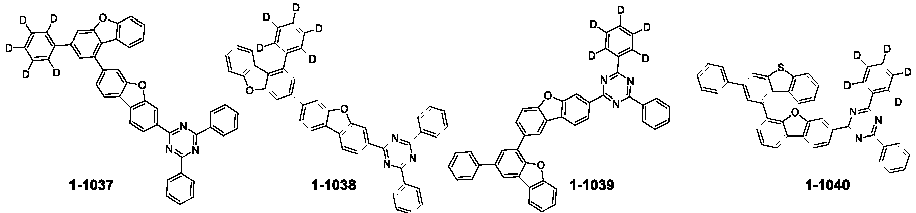

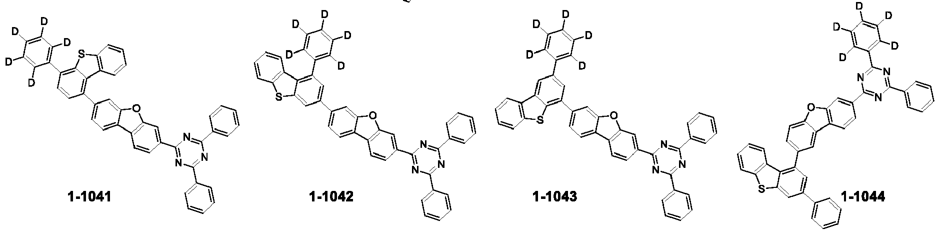

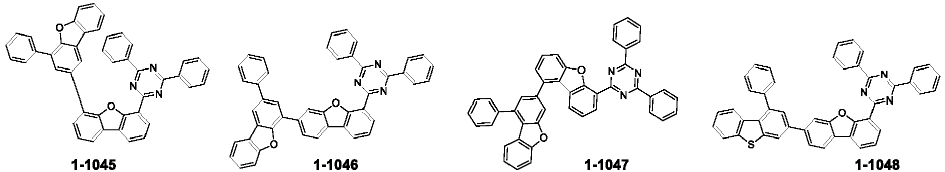

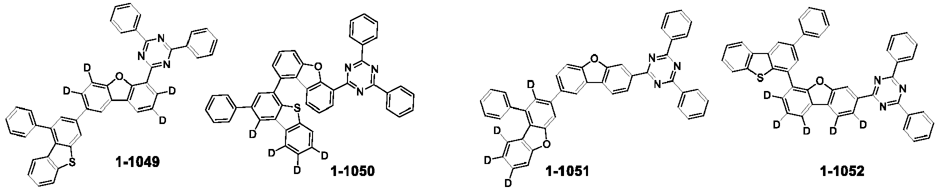

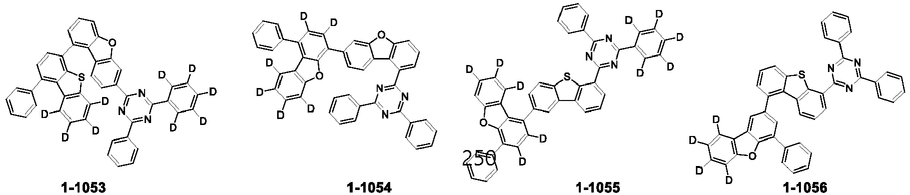

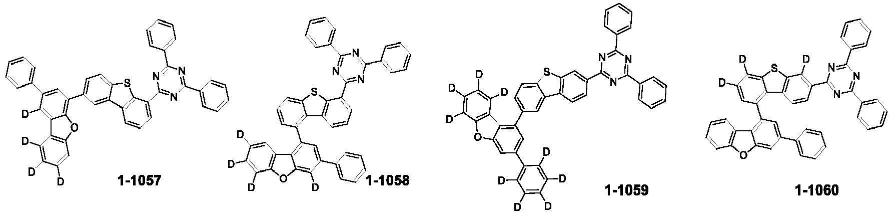

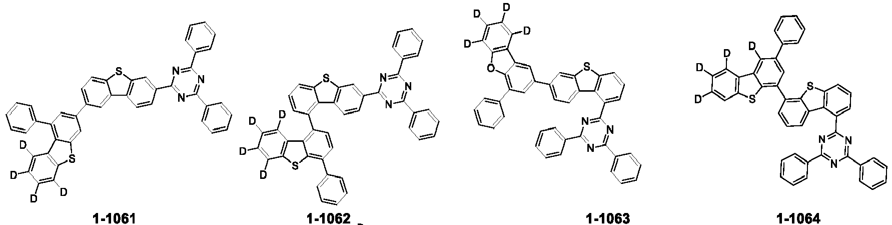

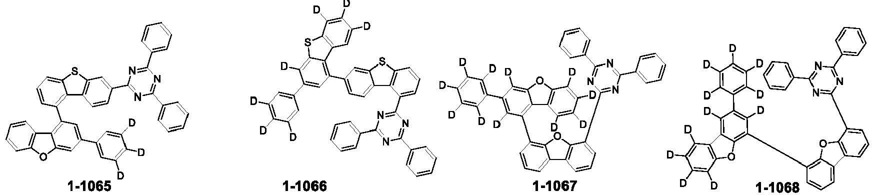

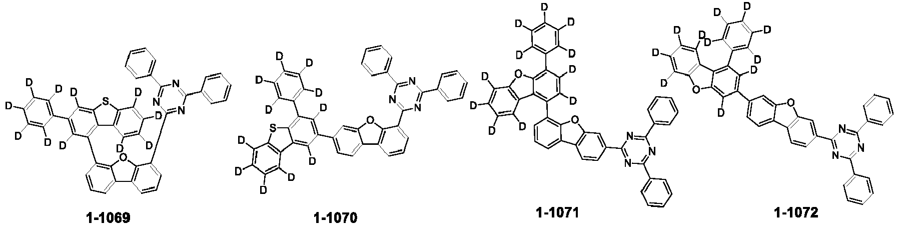

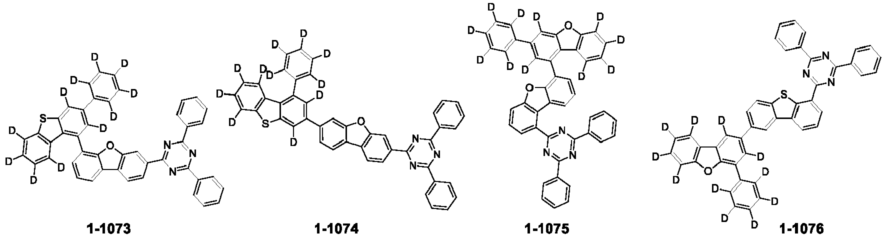

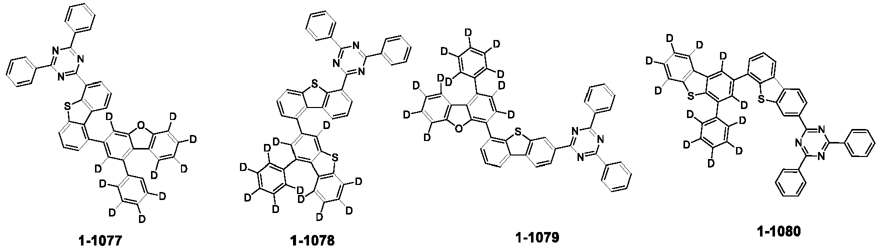

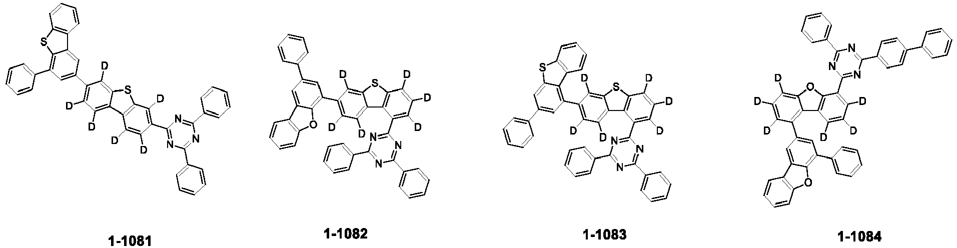

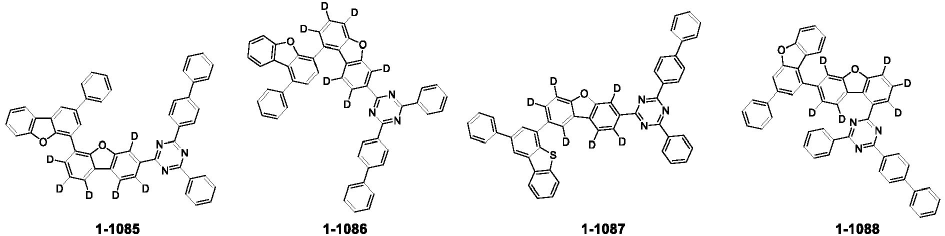

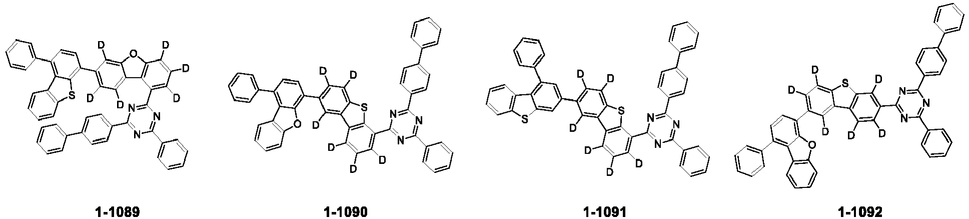

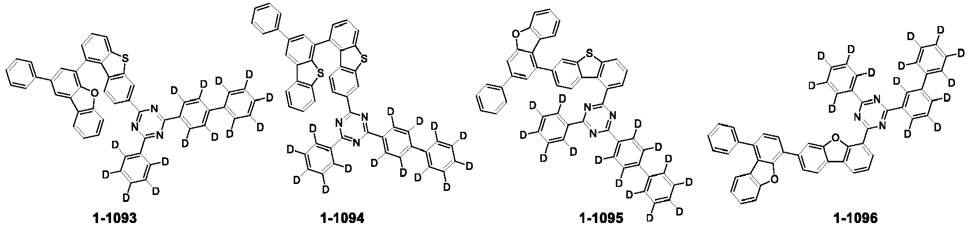

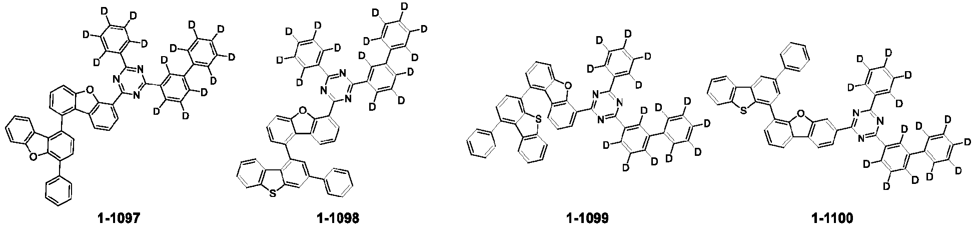

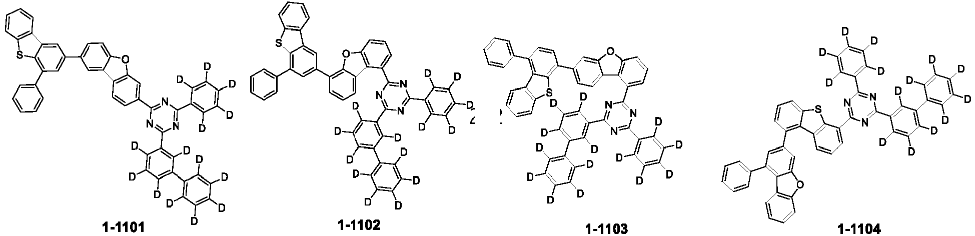

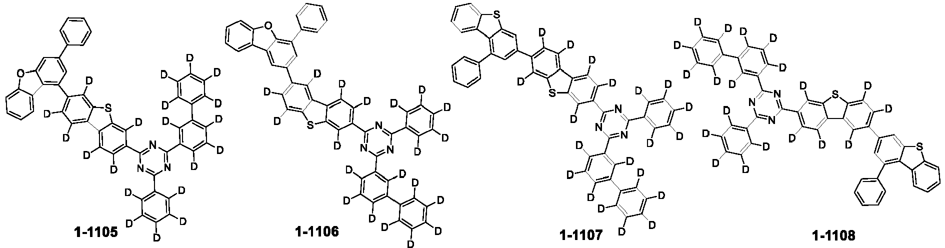

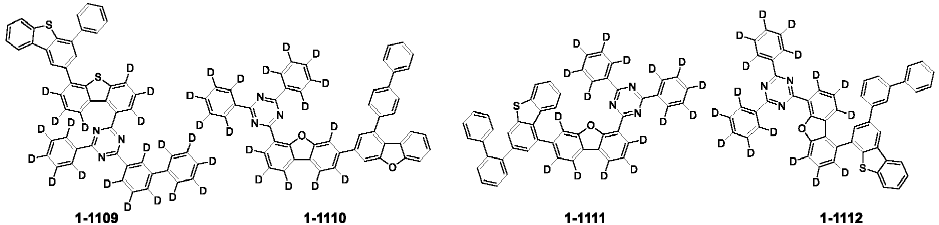

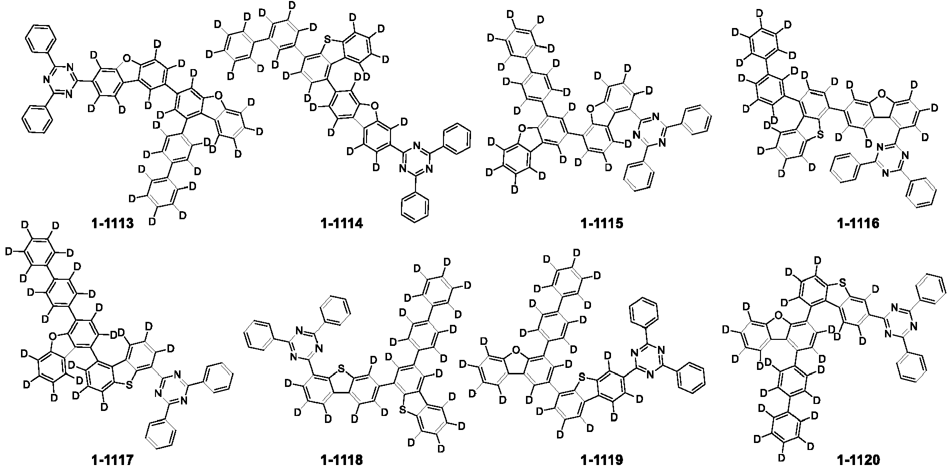

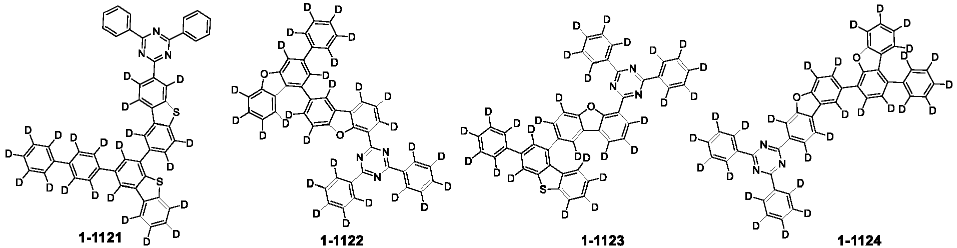

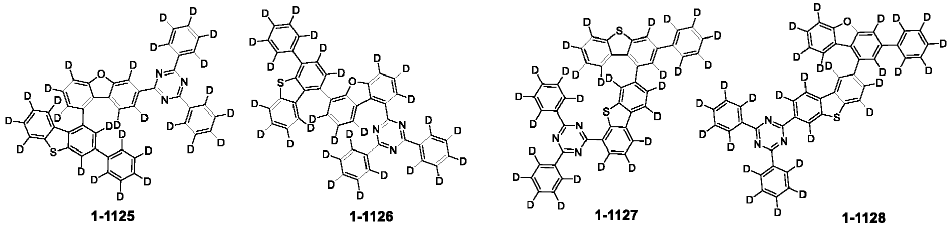

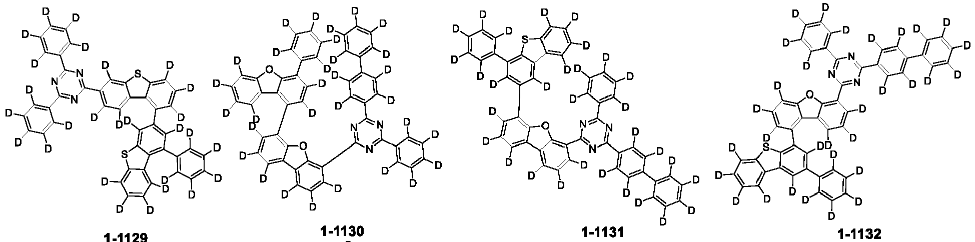

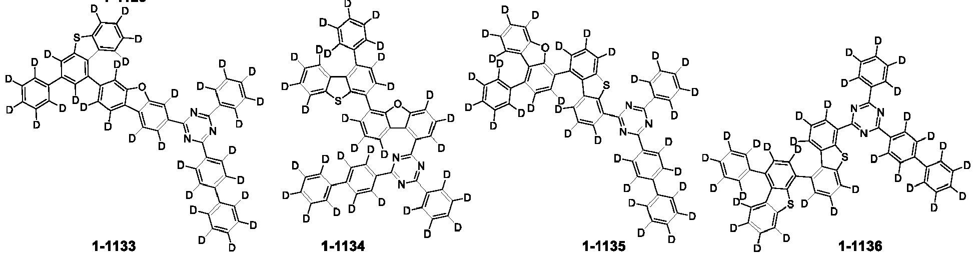

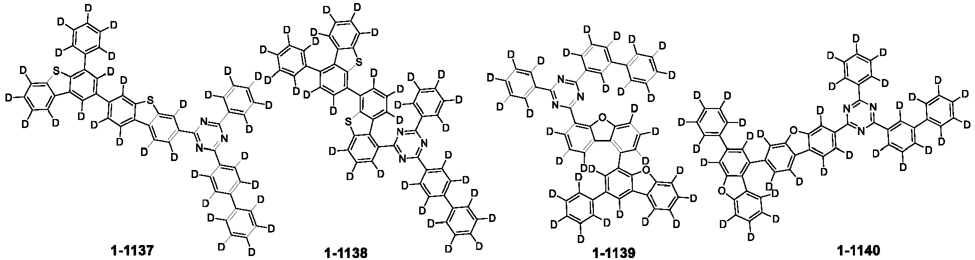

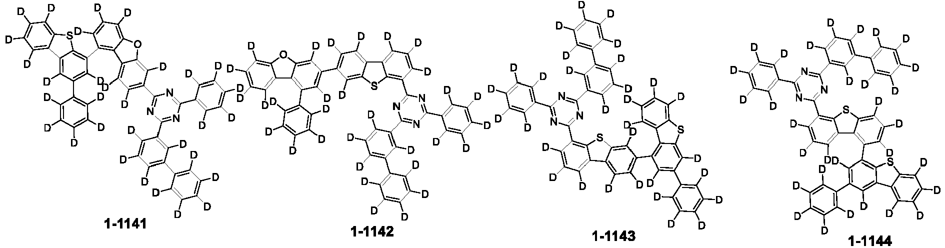

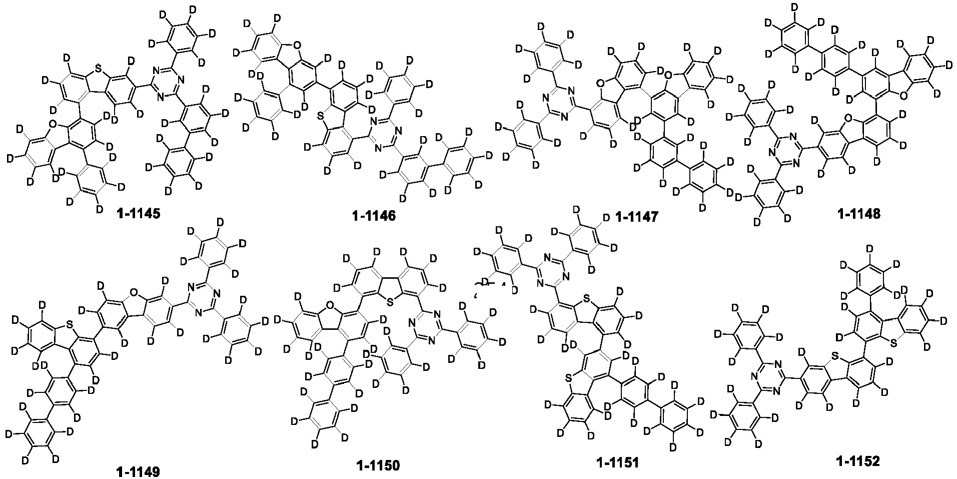

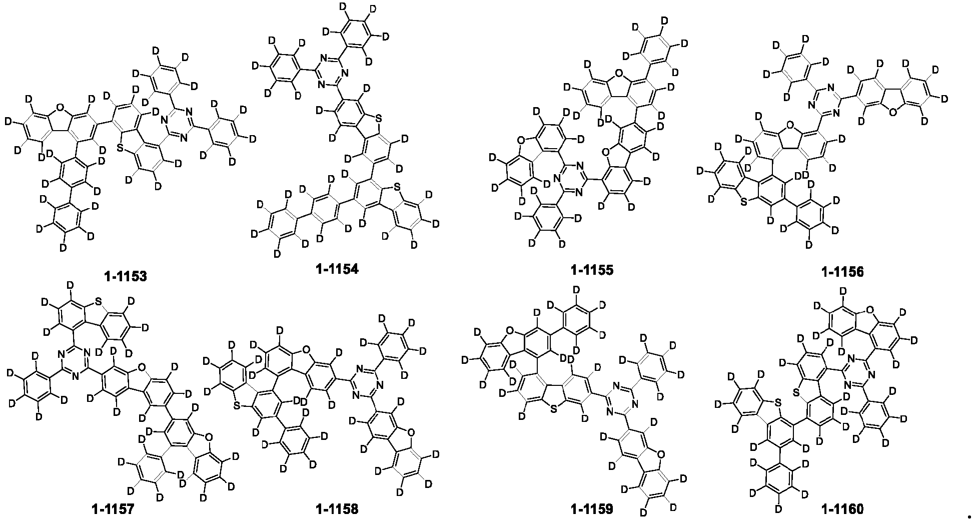

12. An organic light emitting device comprising,
a first electrode;
a second electrode provided to face the first electrode; and
one or more organic layers provided between the first electrode and the second electrode,
wherein at least one of the one or more organic layers comprises a heterocyclic compound represented by Formula 1 according to any one of claims 1 to 11.

13. The organic light emitting device according to claim 12, wherein the one or more organic layers further comprises a heterocyclic compound represented by Formula 2 below: 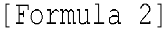 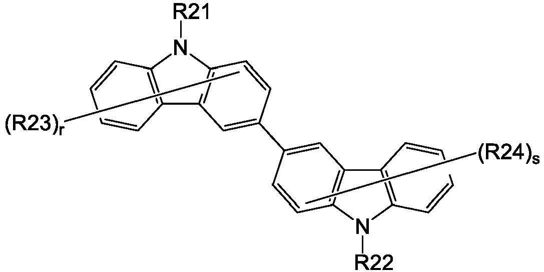 wherein,
R21 and R22 are the same as or different from each other and are each independently a substituted or unsubstituted C6 to C60 aryl group or a substituted or unsubstituted C2 to C60 heteroaryl group,
R23 and R24 are the same as or different from each other and are each independently selected from hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR201R202R203, - P(=O)R201R202; and an amine group substituted or unsubstituted with a substituted or unsubstituted C1 to C60 alkyl group, a substituted or unsubstituted C6 to C60 aryl group or a substituted or unsubstituted C2 to C60 heteroaryl group, or form a substituted or unsubstituted C6 to C60 aromatic hydrocarbon ring or a substituted or unsubstituted C2 to C60 heterocyclic ring by combining two or more groups adjacent to each other,
R201, R202, and R203 are the same as or different from each other and are each independently hydrogen, deuterium, a cyano group, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group, and
r and s are integers from 0 to 7.

14. The organic light emitting device according to claim 13, wherein Formula 2 is a heterocyclic compound represented by Formula 2-1 below: 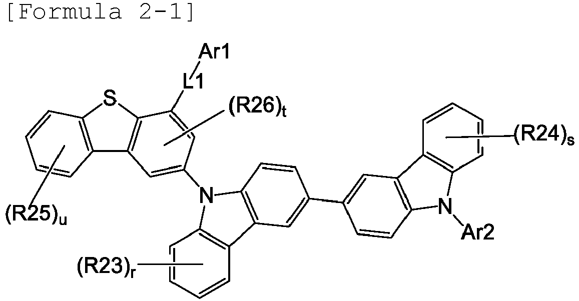 wherein,
R25 and R26 are the same as or different from each other and are each independently selected from the group consisting of hydrogen, deuterium, a substituted or unsubstituted C1 to C60 alkyl group and a substituted or unsubstituted C2 to C60 heteroaryl group, t is an integer from 0 to 2, u is an integer from 0 to 4,
L1 is a single bond, a substituted or unsubstituted C1 to C60 arylene group, or a substituted or unsubstituted C2 to C60 heteroarylene group,
Ar1 and Ar2 are the same as or different from each other and are each independently a substituted or unsubstituted C1 to C60 alkyl group or a substituted or unsubstituted C2 to C60 heteroaryl group, and
the definitions of R23, R24, r and s are the same as those in Formula 2 above.

15. The organic light emitting device according to claim 13, wherein the heterocyclic compound represented by Formula 2 does not contain deuterium as a substituent, or the content of deuterium is 1% to 100% relative to the total number of hydrogen atoms and deuterium atoms.

16. The organic light emitting device according to claim 13, wherein Formula 2 is represented by any one of the following compounds:

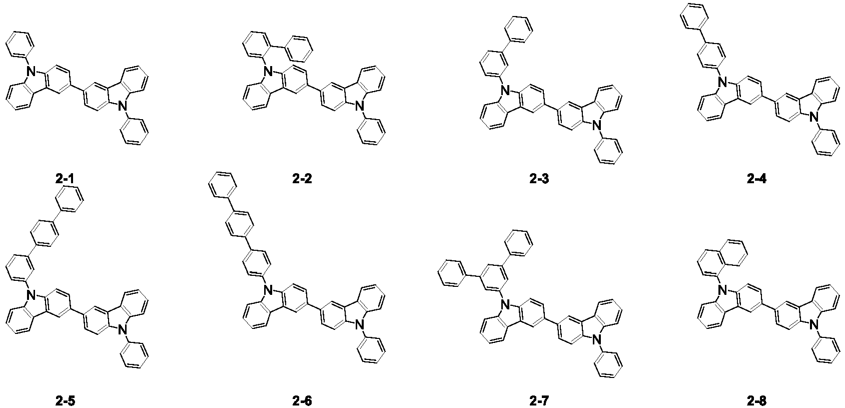

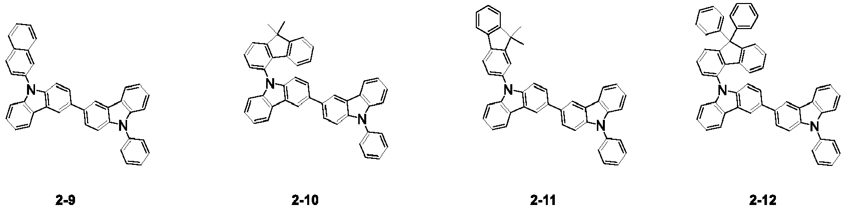

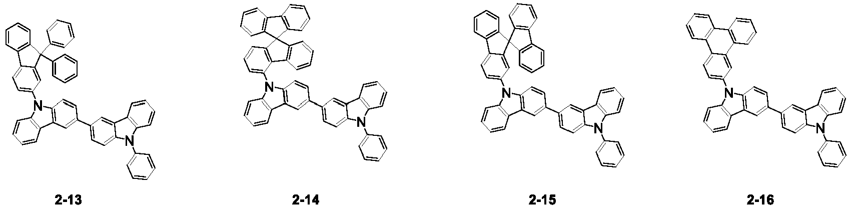

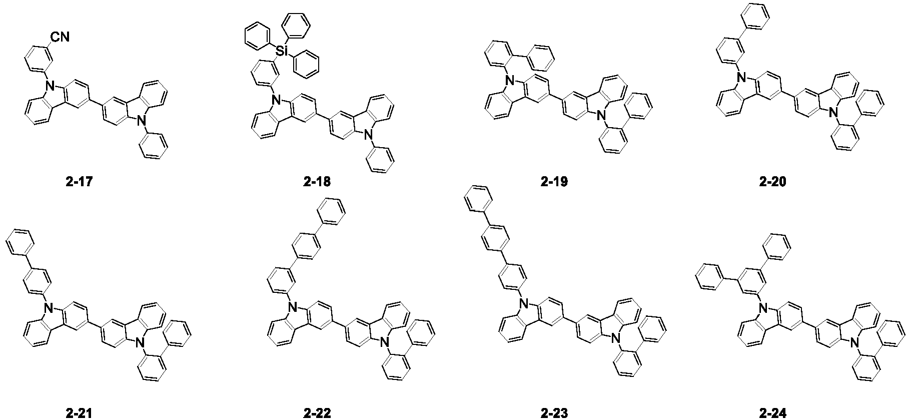

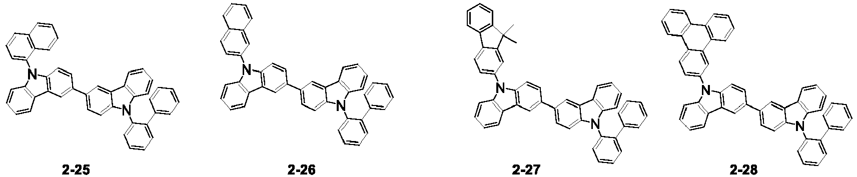

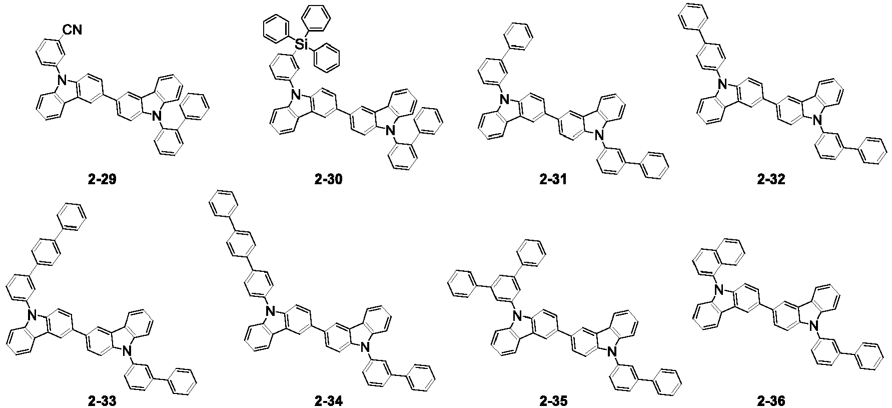

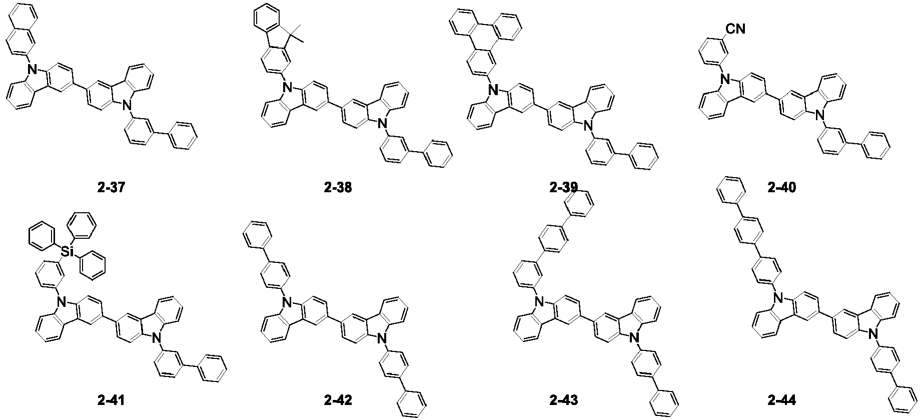

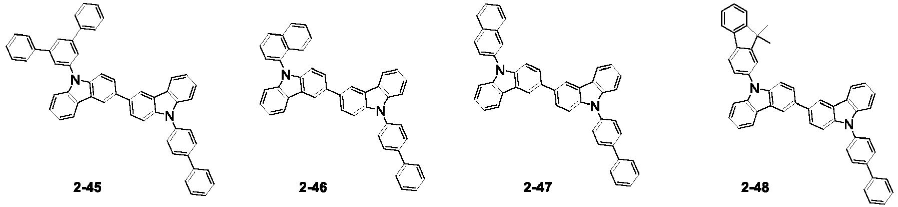

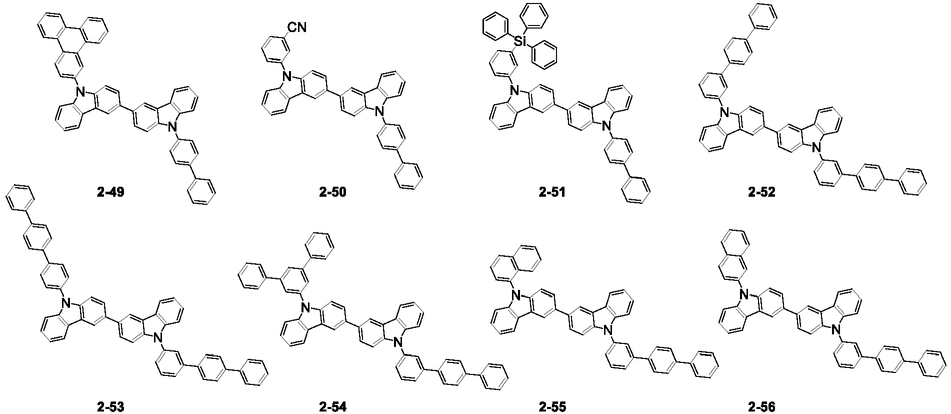

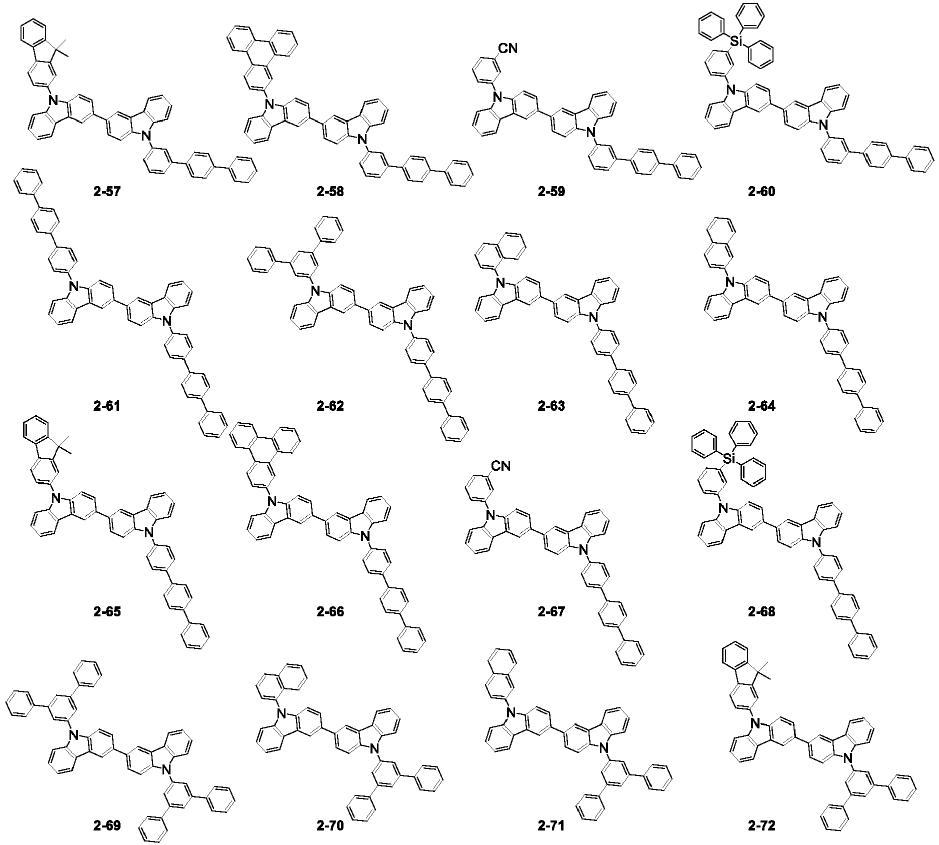

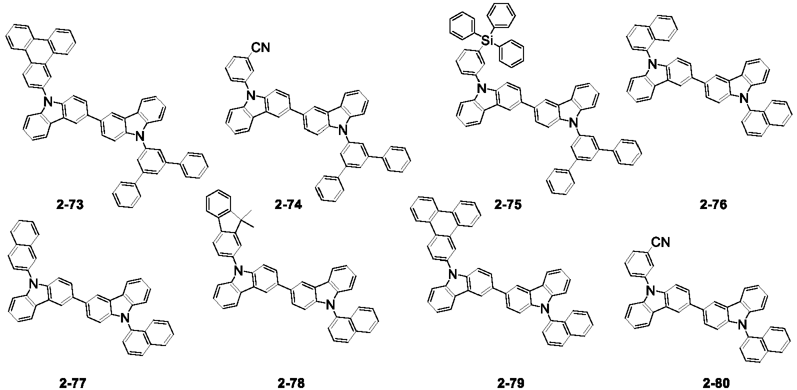

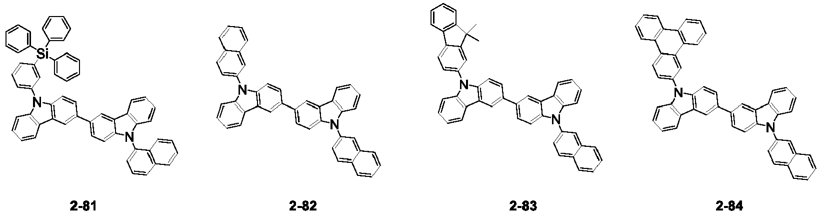

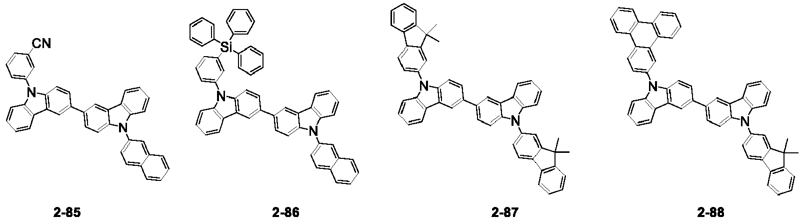

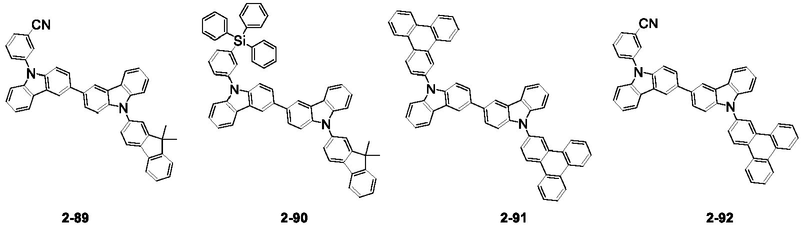

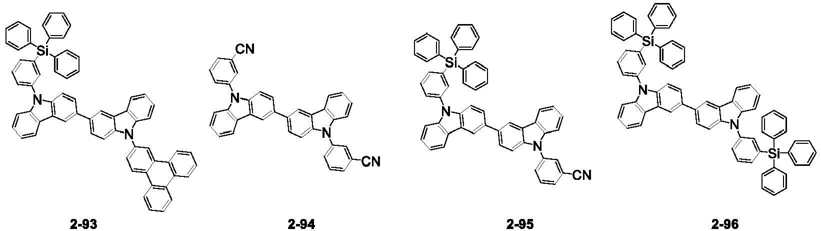

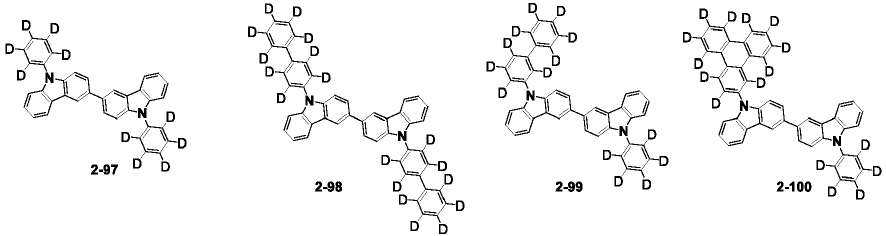

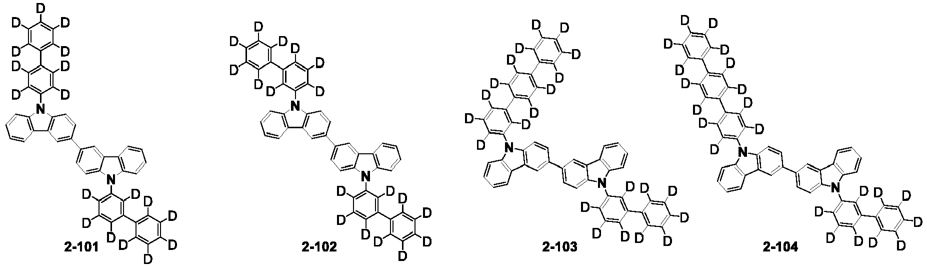

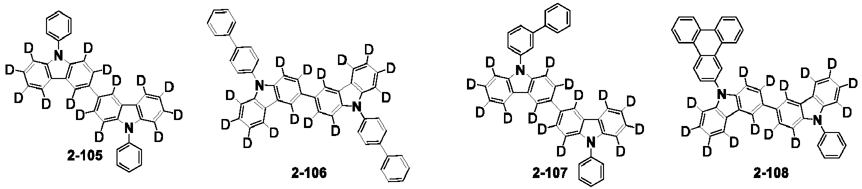

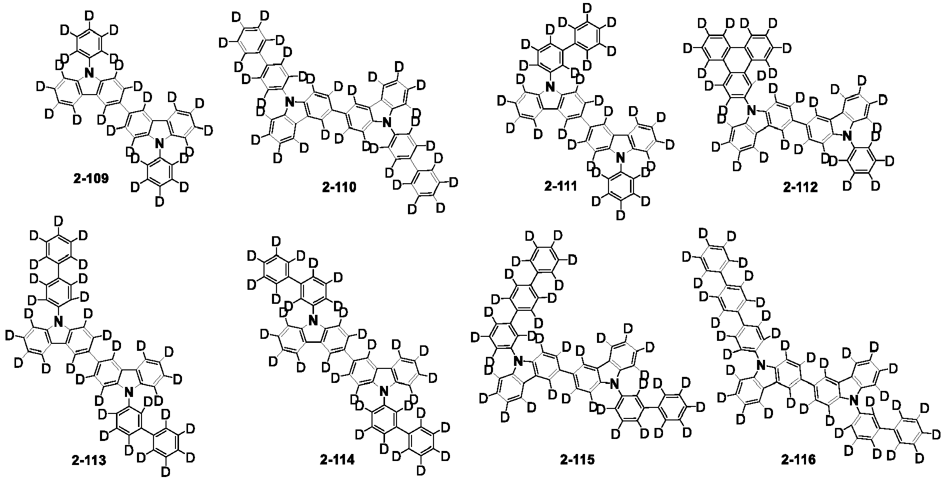

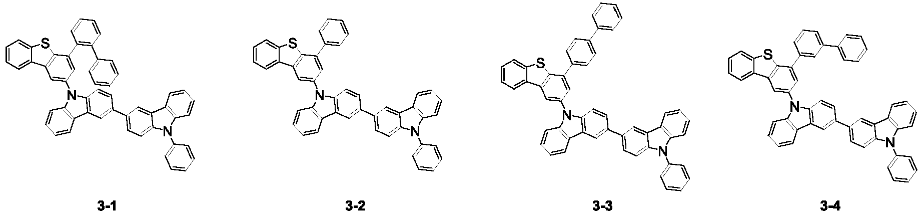

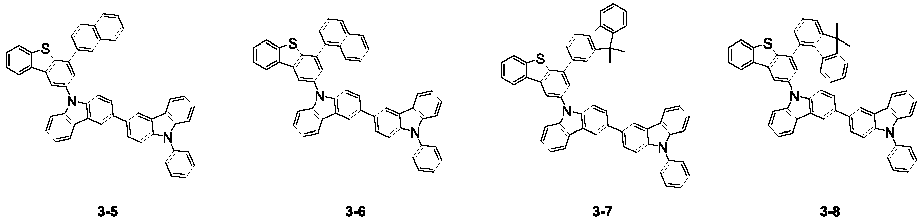

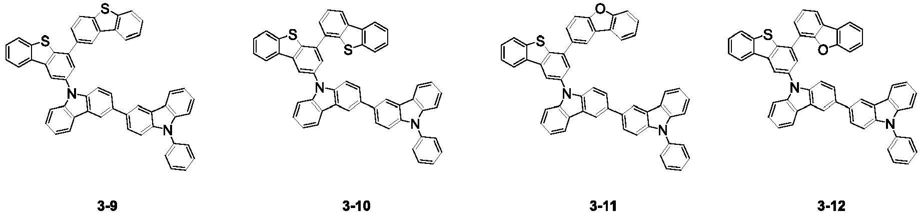

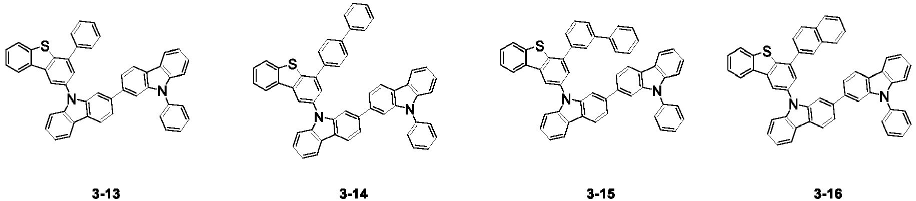

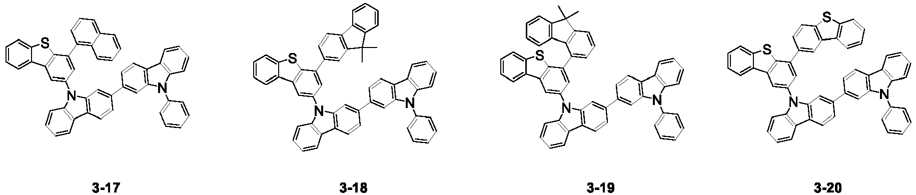

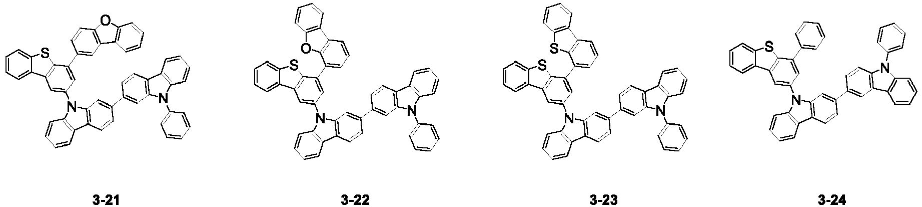

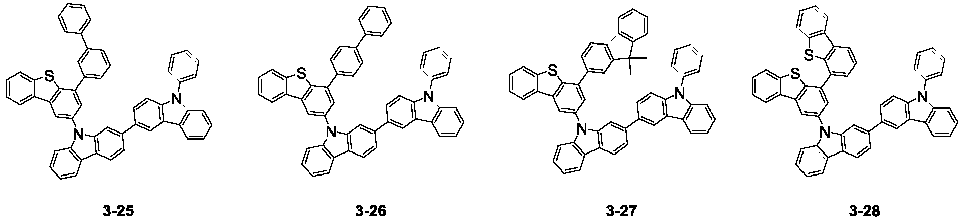

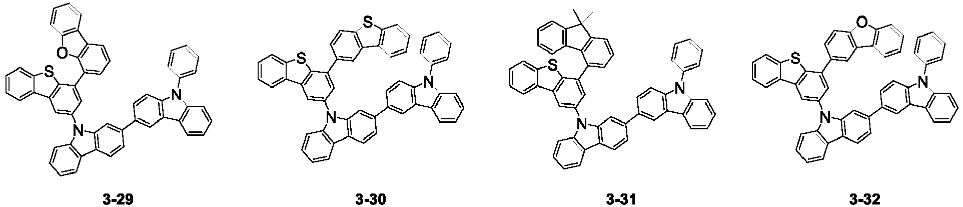

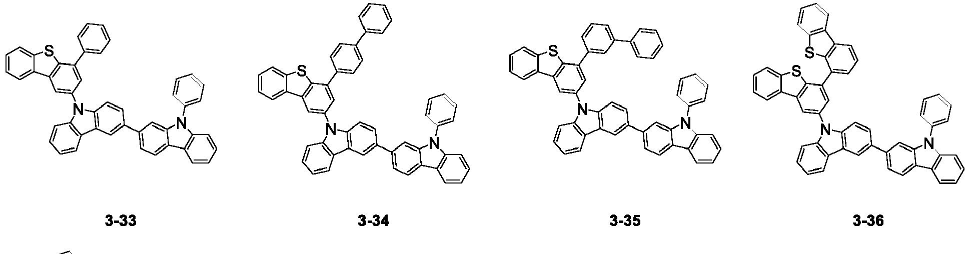

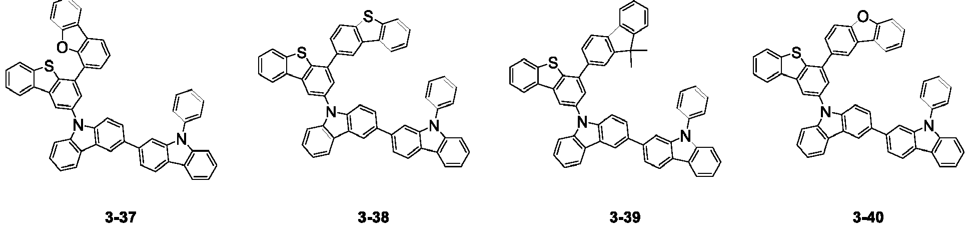

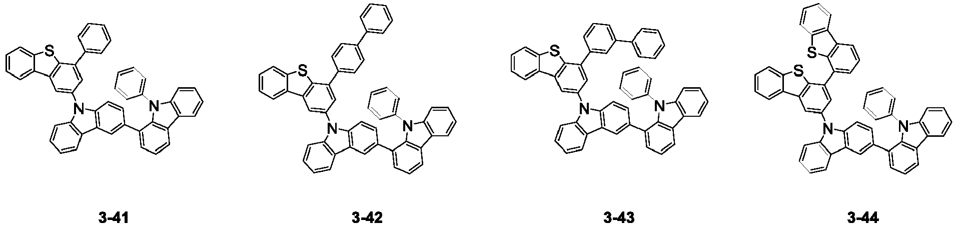

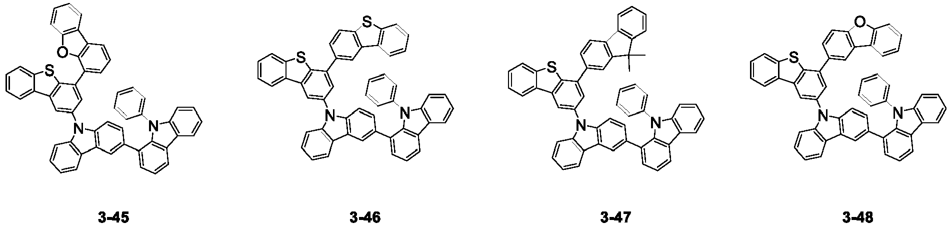

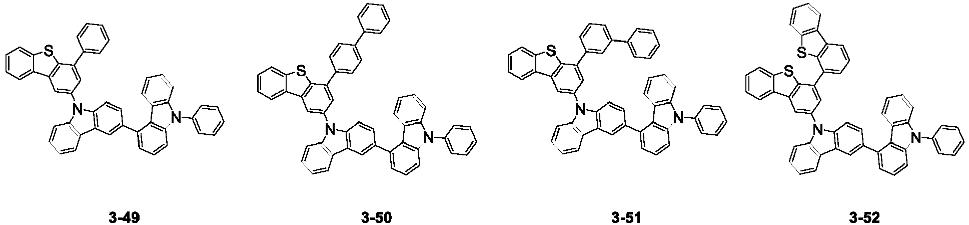

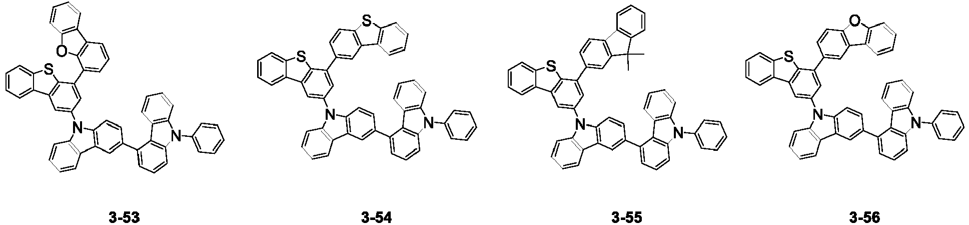

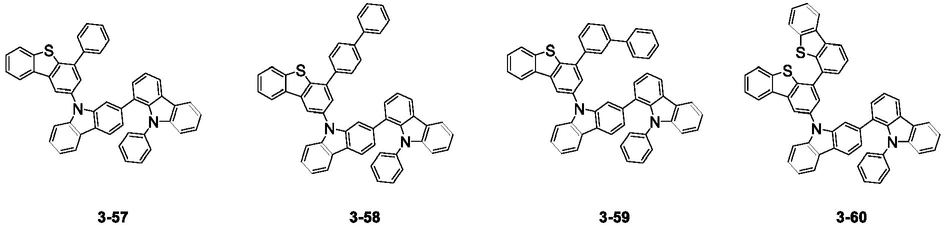

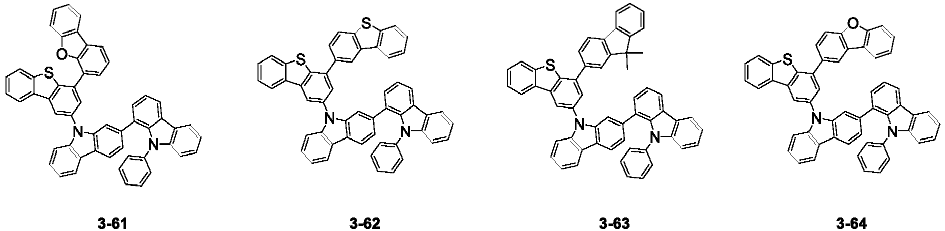

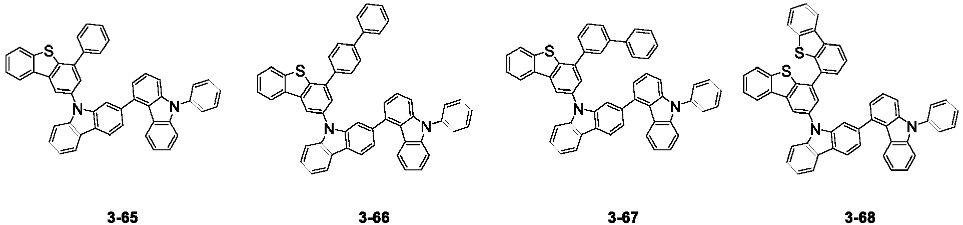

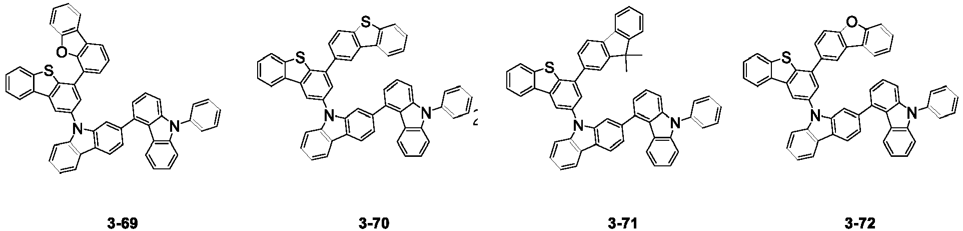

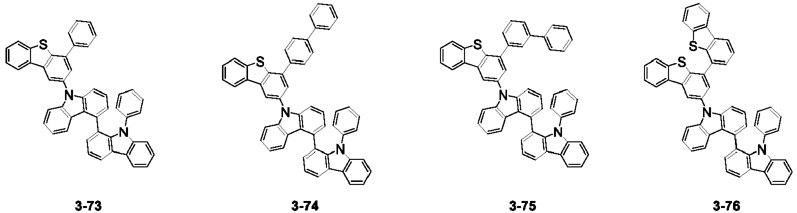

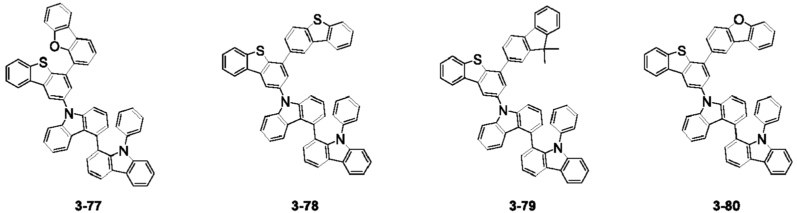

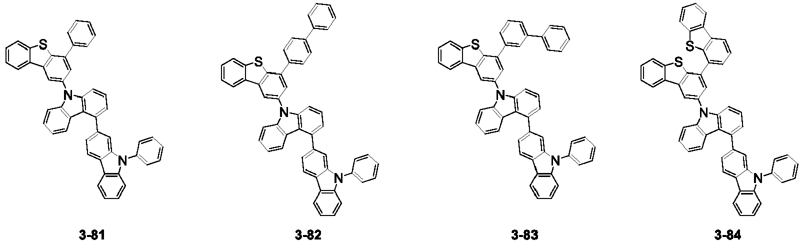

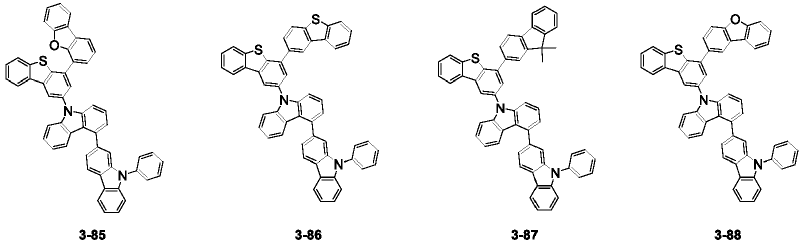

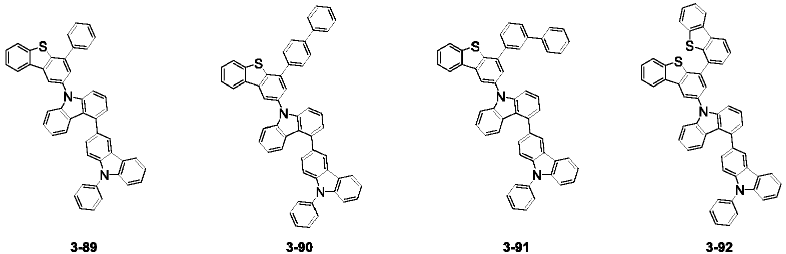

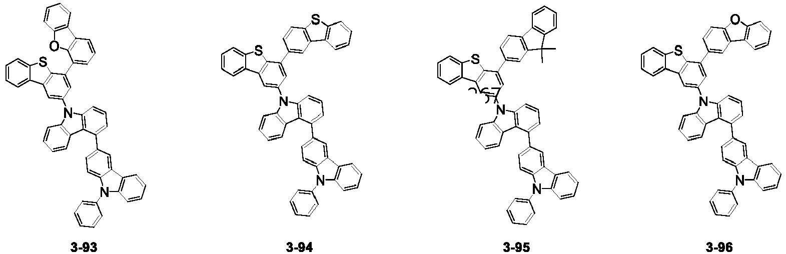

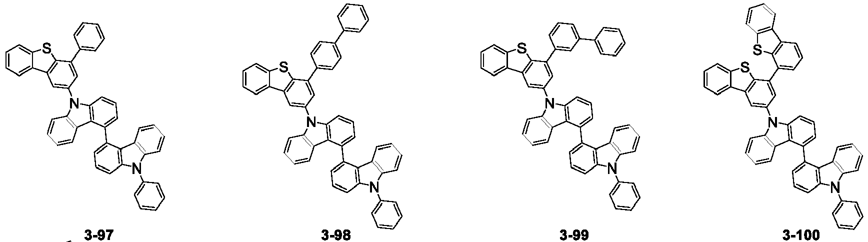

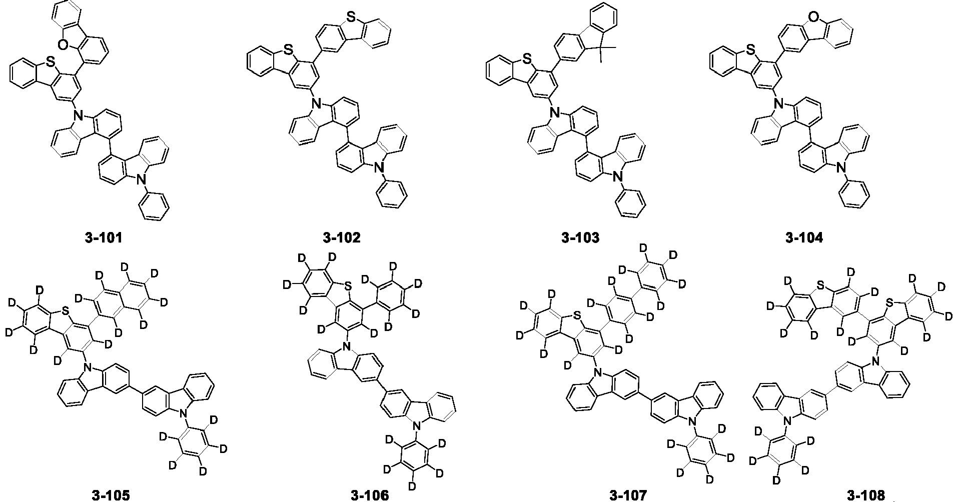

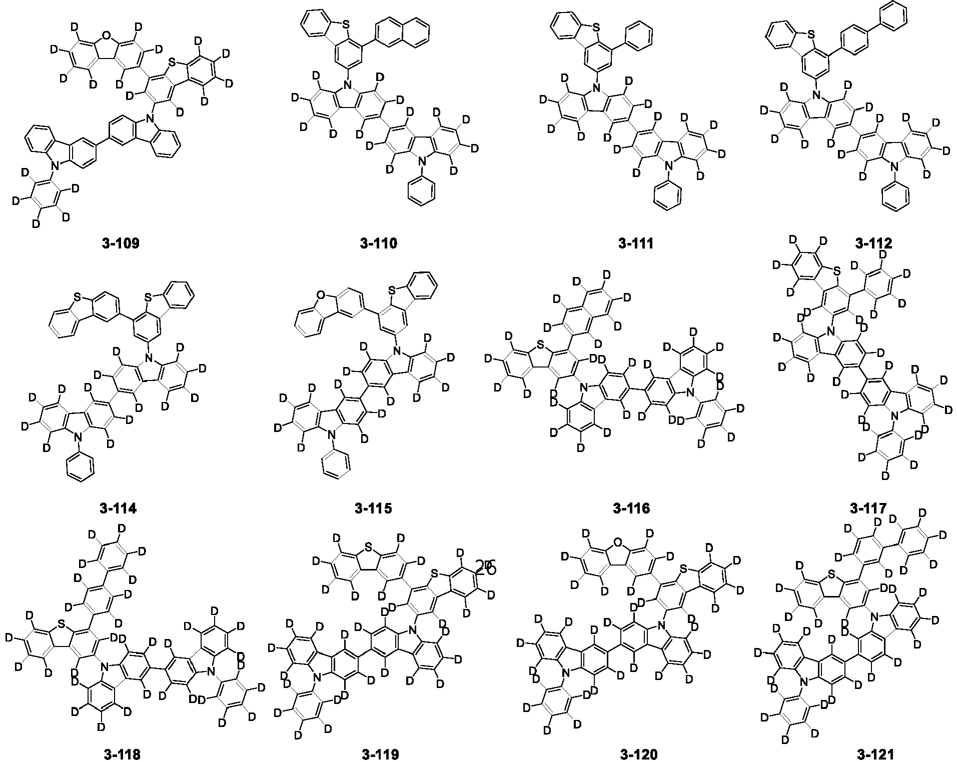

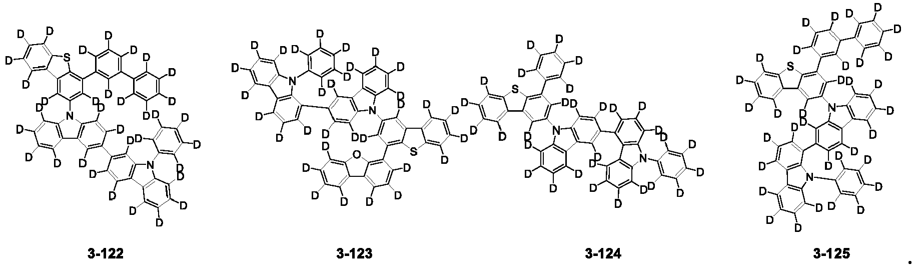

17. The organic light emitting device according to claim 12, wherein the one or more organic layers comprises a light emitting layer, the light emitting layer comprises a host material, and the host material comprises the heterocyclic compound represented by Formula 1 above and a heterocyclic compound represented by Formula 2 below: 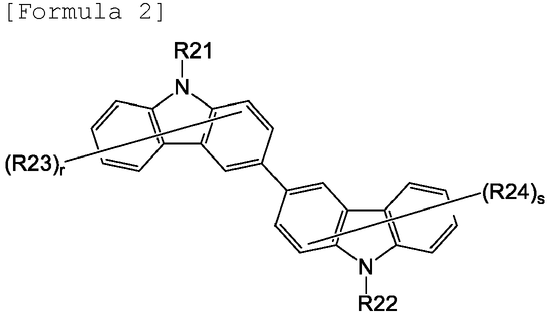 wherein,
R21 and R22 are the same as or different from each other and are each independently a substituted or unsubstituted C6 to C60 aryl group or a substituted or unsubstituted C2 to C60 heteroaryl group,
R23 and R24 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR201R202R203, - P(=O)R201R202; and an amine group substituted or unsubstituted with a substituted or unsubstituted C1 to C60 alkyl group, a substituted or unsubstituted C6 to C60 aryl group or a substituted or unsubstituted C2 to C60 heteroaryl group, or form a substituted or unsubstituted C6 to C60 aromatic hydrocarbon ring or a substituted or unsubstituted C2 to C60 heterocyclic ring by combining two or more groups adjacent to each other,
R201, R202, and R203 are the same as or different from each other and are each independently hydrogen, deuterium, a cyano group, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group, and
r and s are integers from 0 to 7.

18. The organic light emitting device according to claim 12, wherein the organic light emitting device further comprises one or more layers selected from the group consisting of a light emitting layer, a hole injection layer, a hole transport layer, an electron injection layer, an electron transport layer, an electron blocking layer and a hole blocking layer.

19. A composition for organic layer of a organic light emitting device comprising a heterocyclic compound represented by Formula 1 below and a heterocyclic compound represented by Formula 2 below: 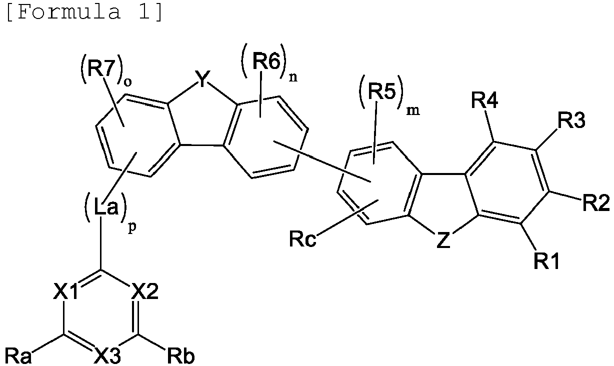 wherein,
X1 to X3 are each independently N or CR10, and at least two of X1 to X3 are N,
R10 is hydrogen, deuterium, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group,
Ra and Rb are the same as or different from each other, and are each independently a substituted or unsubstituted C6 to C60 aryl group, or a substituted or unsubstituted C2 to C60 heteroaryl group,
Rc is a substituted or unsubstituted C6 to C60 aryl group, or a substituted or unsubstituted C2 to C60 heteroaryl group,
La is a single bond, a substituted or unsubstituted C6 to C60 arylene group, or a substituted or unsubstituted C2 to C60 heteroarylene group,
Y and Z are the same as or different from each other and are each independently O, S or CR11R12,
R11 and R12 are hydrogen, deuterium, a cyano group, a substituted or unsubstituted C1 to C60 alkyl group, or a substituted or unsubstituted C6 to C60 aryl group,
R1 to R7 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)R101R102; - SiR101R102R103; and -NR101R102, or form a substituted or unsubstituted C6 to C60 aromatic hydrocarbon ring or a substituted or unsubstituted C2 to C60 heterocyclic ring by combining two or more groups adjacent to each other, and R101, R102, and R103 are the same as or different from each other and are each independently a substituted or unsubstituted C1 to C60 alkyl group; a substituted or unsubstituted C6 to C60 aryl group; or a substituted or unsubstituted C2 to C60 heteroaryl group, and
m is an integer from 0 to 2, n is an integer from 0 to 3, o is an integer from 0 to 3, p is an integer from 0 to 5, 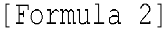 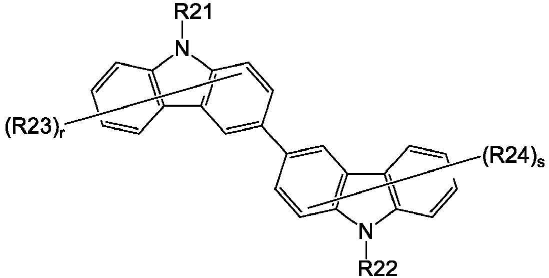
wherein,
R21 and R22 are the same as or different from each other and are each independently a substituted or unsubstituted C6 to C60 aryl group or a substituted or unsubstituted C2 to C60 heteroaryl group,
R23 and R24 are the same as or different from each other and are each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR201R202R203, - P(=O)R201R202; and an amine group substituted or unsubstituted with a substituted or unsubstituted C1 to C60 alkyl group, a substituted or unsubstituted C6 to C60 aryl group or a substituted or unsubstituted C2 to C60 heteroaryl group, or form a substituted or unsubstituted C6 to C60 aromatic hydrocarbon ring or a substituted or unsubstituted C2 to C60 heterocyclic ring by combining two or more groups adjacent to each other,
R201, R202, and R203 are the same as or different from each other and are each independently hydrogen, deuterium, a cyano group, a substituted or unsubstituted C1 to C60 alkyl group, a substituted or unsubstituted C3 to C60 cycloalkyl group, a substituted or unsubstituted C6 to C60 aryl group, or a substituted or unsubstituted C2 to C60 heteroaryl group, and
r and s are integers from 0 to 7.

20. The composition for organic layer of the organic light emitting device according to claim 19, wherein the weight ratio of the heterocyclic compound represented by Formula 1:the heterocyclic compound represented by Formula 2 is 1:10 to 10:1.

21. A method for manufacturing an organic light emitting device comprising the steps of,
preparing a substrate;
forming a first electrode on the substrate;
forming one or more organic layers on the first electrode; and
forming a second electrode on the one or more organic layers,
wherein the step of forming the one or more organic layers comprises a step of forming the one or more organic layers using the composition for organic layer according to claim 19.

22. The method for manufacturing the organic light emitting device according to claim 21, wherein the step of forming the one or more organic layers comprises pre-mixing the heterocyclic compound represented by Formula 1 and the heterocyclic compound represented by Formula 2, and forming the one or more organic layers using a thermal vacuum deposition method.
